# EUROPEAN PATENT APPLICATION

(11) **EP 2 145 936 A2**
(43) Date of publication of application: **20.01.2010**
(21) Application number: 09156757.8
(22) Date of filing: 30.03.2009
(51) Int. Cl.: C09K 11/06

(54) **Fluorene and pyrene derivatives and organic electroluminescent device using the same**

(30) Priority: 14.07.2008 KR 20080068194
(71) Applicant: Gracel Display Inc., Seoul 133-833 (KR)
(72) Inventor: Lee, Hyo Jung, 153-784, Seoul (KR); Cho, Young Jun, 136-060, Seoul (KR); Kwon, Hyuck Joo, 130-100, Seoul (KR); Kim, Bong Ok, 135-090, Seoul (KR); Kim, Sung Min, 157-886, Seoul (KR); Yoon, Seung Soo, 135-884, Seoul (KR)
(74) Representative: Kent, Venetia Katherine

(57) **Abstract**

Provided are novel organic electroluminescent compounds and organic electroluminescent devices comprising the same as electroluminescent material. Specifically, the organic electroluminescent compounds according to the invention are **characterized in that** they are represented by Chemical Formula (1) : 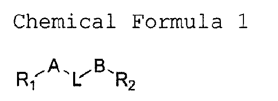 wherein, L is selected from the following structures.

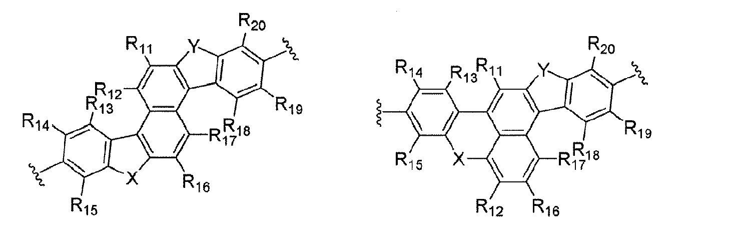

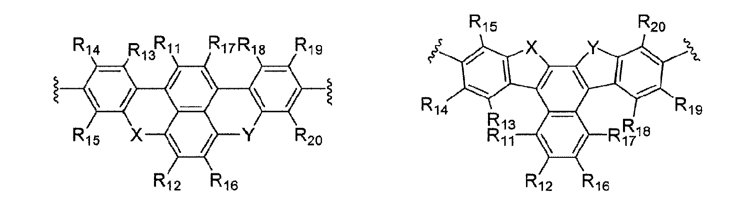

The organic electroluminescent compounds according to the invention exhibit high luminous efficiency in blue color and excellent life property as a material, so that an OLED having very good operation life can be prepared therefrom.

## Description

### FIELD OF THE INVENTION

The present invention relates to novel organic electroluminescent compounds and organic electroluminescent devices employing the same.

### BACKGROUND OF THE INVENTION

Among display devices, electroluminescence devices (EL devices) are self-luminescent display devices showing the advantage of wide angle of view, excellent contrast and rapid response rate. Eastman Kodak developed in 1987 an organic EL device which employs a low molecular weight aromatic diamine and an aluminum complex as material for forming an EL layer, for the first time [Appl. Phys. Lett. 51, 913, 1987].

An organic EL device is a device wherein, when charge is applied to an organic film formed between an electron injection electrode (cathode) and a hole injection electrode (anode), an electron and a hole form a pair and then become extinct with emitting light. A device can be formed on a transparent flexible substrate such as plastics. The device can be operated at a lower voltage (not more than 10 V) with relatively lower power consumption but excellent color purity, as compared to a plasma display panel or an inorganic EL display.

Since the organic electroluminescent (EL) devices can develop three colors (green, blue and red), they have been focused as full colored display devices for next generation.

The procedure for manufacturing an organic EL device comprises the following steps:
(1) First, anode material is coated on a transparent substrate. As the anode material, indium tin oxide (ITO) is usually employed.
(2) A hole injecting layer (HIL) is coated thereon. As the hole injecting layer, it is common to coat copper phthalocyanine (CuPc) with a thickness of 10 nm to 30 nm.
(3) Then, a hole transport layer (HTL) is introduced. As the hole transport layer, 4,4'-bis[N-(1-naphthyl)-N-phenylamino]biphenyl (NPB) is vapor-deposited with a thickness of about 30 nm to 60 nm.
(4) An organic emitting layer is formed thereon. If required, dopant is added. In case of green electroluminescence, tris(8-hydroxyquinolate)aluminium (Alq₃) is commonly vapor-deposited with a thickness of 30 to 60 nm as the organic emitting layer, and MQD(N-methylquinacridone) is usually employed as dopant.
(5) An electron transport layer (ETL) and an electron injecting layer (EIL) is then sequentially coated thereon, or an electron injecting transport layer is formed. In case of green electroluminescence, since Alq₃ of (4) has good capability of electron transport, an electron injecting/transport layer may not be necessarily employed.
(6) Then, a cathode is coated, and finally passivation is carried out.

Depending upon how the emitting layer is formed in such a structure, a blue, green or red electroluminescent device can be realized. In the meanwhile, conventional substances used as green electroluminescent compound for realizing a green electroluminescent device had problems of insufficient life and poor luminous efficiency.

The most important factor to determine luminous efficiency, lifetime or the like in an organic EL device is electroluminescent material. Several properties required for such electroluminescent materials include that the material should have high fluorescent quantum yield in solid state and high mobility of electrons and holes, is not easily decomposed during vapor-deposition in vacuo, and forms uniform and stable thin film.

Organic electroluminescent materials can be generally classified into high-molecular materials and low-molecular materials. The low-molecular materials include metal complexes and thoroughly organic electroluminescent materials which do not contain metal, from the aspect of molecular structure. Such electroluminescent materials include chelate complexes such as tris(8-quinolinolato)aluminum complexes, coumarin derivatives, tetraphenylbutadiene derivatives, bis(styrylarylene) derivatives and oxadiazole derivatives. From those materials, it is reported that light emission of visible region from blue to red can be obtained, so that realization of full-colored display devices is anticipated thereby.

### SUMMARY OF THE INVENTION

With intensive efforts to overcome the problems of conventional techniques as described above, the present inventors have invented novel electroluminescent compounds which can realize organic electroluminescent devices having excellent luminous efficiency and noticeably improved life property.

The object of the present invention is to provide organic electroluminescent compounds having the backbone to provide better luminous efficiency and device life with appropriate color coordinate as compared to conventional host or dopant material, while overcoming the problems described above.

Another object of the invention is to provide organic electroluminescent devices having high luminous efficiency and long life, which employs the organic electroluminescent compounds as electroluminescent material.

Still another object of the invention is to provide organic electroluminescent devices which employ the organic electroluminescent compounds in the hole transport layer or electroluminescent layer.

Yet still another object of the invention is to provide organic solar cells comprising the organic electroluminescent compounds.

The present invention relates to organic electroluminescent compounds represented by Chemical Formula (1), and organic electroluminescent devices comprising the same. Since the organic electroluminescent compounds according to the invention show good luminous efficiency and excellent color purity and life property of material, OLED's having very good operation life can be manufactured therefrom. 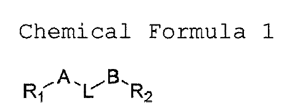
wherein, L is selected from the following structures: 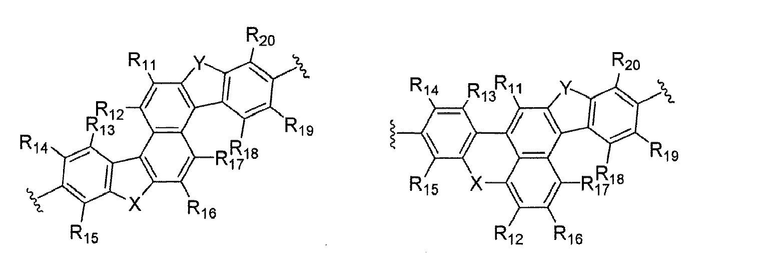 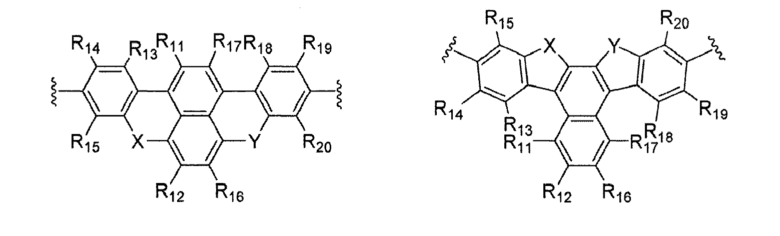 A and B independently represent a chemical bond, or (C6-C60)arylene, (C3-C60)heteroarylene, 5- or 6-membered heterocycloalkylene containing one or more heteroatom(s) selected from N, O and S, (C3-C60)cycloalkylene, (C2-C60)alkenylene, (C2-C60)alkynylene, (C1-C60)alkylenoxy, (C6-C60)arylenoxy or (C6-C60)arylenethio;
   R₁ and R₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, NR₃R₄, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or a substituent selected from the following structures: 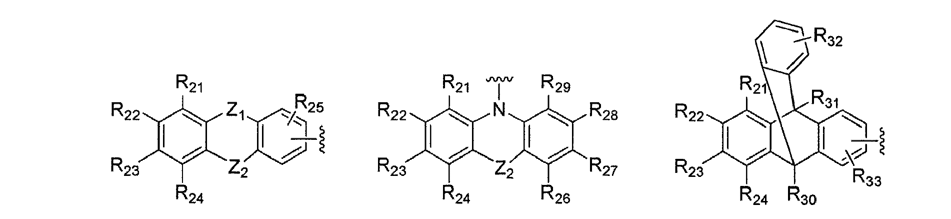
R₃ and R₄ independently represent (C1-C60)alkyl, (C6-C60)aryl, 5- or 6-membered heterocycloalkyl containing one or more heteroatom(s) selected from N, O and S, adamantyl, (C7-C60)bicycloalkyl, (C3-C60)cycloalkyl, (C3-C60)heteroaryl, tri(C1-C60)alkylsilyl, di(C1-C60)alkyl(C6-C60)arylsilyl or tri(C6-C60)arylsilyl, or R₃ and R₄ may be linked via (C3-C60)alkylene or (C3-C60)alkenylene with or without a fused ring to form an alicyclic ring, or a monocyclic or polycyclic aromatic ring;
X and Y independently represent a chemical bond, or - C(R₄₁)(R₄₂)-, -N(R₄₃)-, -S-, -O-, -Si(R₄₄)(R₄₅)-, -P(R₄₆)-, - C(=O)-, -B(R₄₇)-, -In(R₄₈)-, -Se-, -Ge(R₄₉)(R₅₀)-, -Sn(R₅₁)(R₅₂)-or -Ga(R₅₃)-;
R₄₁ through R₅₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₄₁ and R₄₂, R₄₄ and R₄₅, R₄₉ and R₅₀, or R₅₁ and R₅₂ may be linked via (C3-C60)alkylene or (C3-C60)alkenylene with or without a fused ring to form an alicyclic ring, or a monocyclic or polycyclic aromatic ring;
R₁₁ through R₂₀ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or a substituent selected from the following structures: 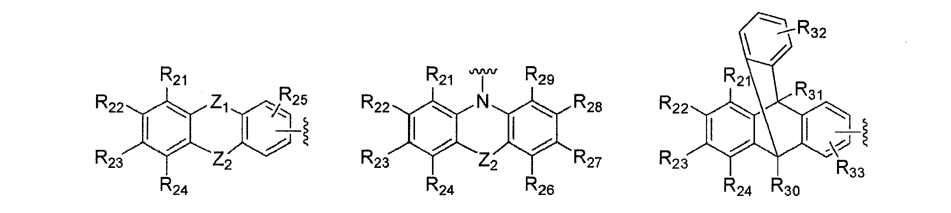
R₂₁ through R₃₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₂₁ through R₃₃ may be linked to an adjacent substituent via (C3-C60)alkylene or (C3-C60)alkenylene with or without a fused ring to form an alicyclic ring, or a monocyclic or polycyclic aromatic ring;
Z₁ and Z₂ independently represent a chemical bond, or - (CR₆₁R₆₂)ₘ-, -N(R₆₃)-, -S-, -O-, -Si(R₆₄)(R₆₅)-, -P(R₆₆)-, -C(=O)-, -B(R₆₇)-, -In(R₆₈)-, -Se-, -Ge(R₆₉)(R₇₀)-, -Sn(R₇₁)(R₇₂)-, - GaR₇₃)- or -(R₇₄)C=C(R₇₅)-;
wherein, R₆₁ through R₇₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₆₁ and R₆₂, R₆₄ and R₆₅, R₆₉ and R₇₀, R₇₁ and R₇₂, or R₇₄ and R₇₅ may be linked via (C3-C60)alkylene or (C3-C60)alkenylene with or without a fused ring to form an alicyclic ring, or a monocyclic or polycyclic aromatic ring;
the arylene, heteroarylene, heterocycloalkylene, cycloalkylene, alkenylene, alkynylene, alkylenoxy, arylenoxy or arylenethio of A and B; or the alkyl, aryl, heteroaryl, heterocycloalkyl, cycloalkyl, alkylsilyl, arylsilyl, adamantyl, bicycloalkyl, alkenyl, alkynyl, alkylamino or arylamino of R₁ through R₄, R₁₁ through R₃₃, R₄₁ through R₅₅ and R₆₁ through R₇₅ may be further substituted by one or more substituent(s) selected from halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl; and
m is an integer from 1 to 4.

### BRIEF DESCRIPTION OF THE DRAWINGS

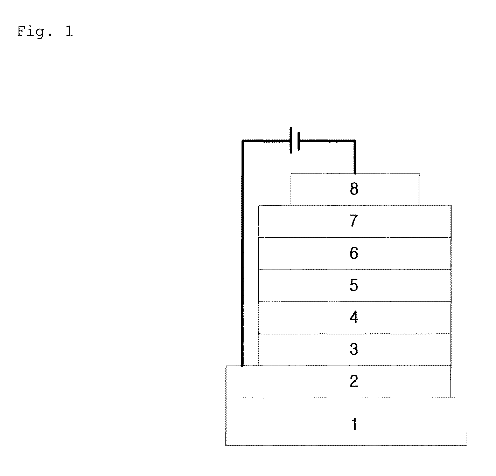

Fig. 1 is a cross-sectional view of an OLED.

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The term "alkyl" and any other substituents comprising "alkyl" moiety include linear and branched species.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Specific examples include phenyl, naphthyl, biphenyl, anthryl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S for the aromatic cyclic backbone atoms, and carbon atom(s) for remaining aromatic cyclic backbone atoms. The heteroaryl may be 5- or 6-membered monocyclic heteroaryl or a polycyclic heteroaryl which is fused with one or more benzene ring(s), and may be partially saturated. The heteroaryl groups include bivalent aryl group of which the heteroatom in the ring is oxidized or quarternized to form an N-oxide or a quaternary salt. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl and benzodioxolyl; and corresponding N-oxides (e.g., pyridyl N-oxides, quinolyl N-oxides) and quaternary salts thereof; but they are not restricted thereto.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms. The substituents comprising "(C3-C60)cycloalkyl" moiety may contain 3 to 60 carbon atoms, 3 to 20 carbon atoms, or 3 to 7 carbon atoms. The substituents comprising "(C2-C60)alkenyl or alkynyl" moiety may contain 2 to 60 carbon atoms, 2 to 20 carbon atoms, or 2 to 10 carbon atoms.

The organic electroluminescent compounds according to the present invention may be selected from the compounds represented by one of Chemical Formulas (2) to (5): 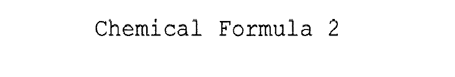 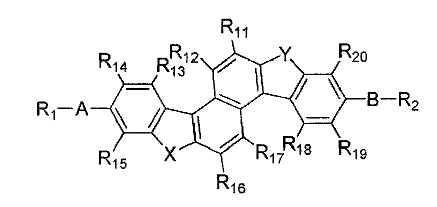 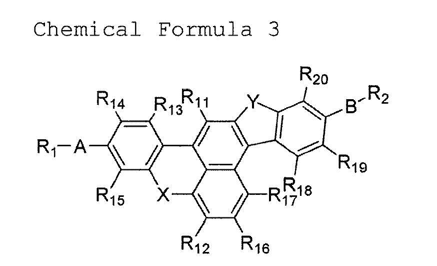 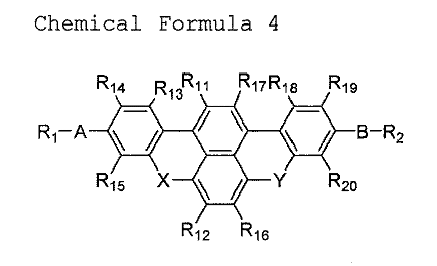 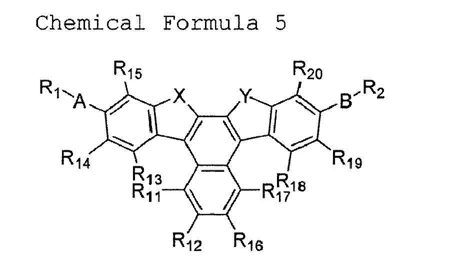 wherein, A, B, X, Y, R₁, R₂ and R₁₁ through R₂₀ are defined as in Chemical Formula (1).

In Chemical Formula (1), L is selected from the following structures, without restriction: 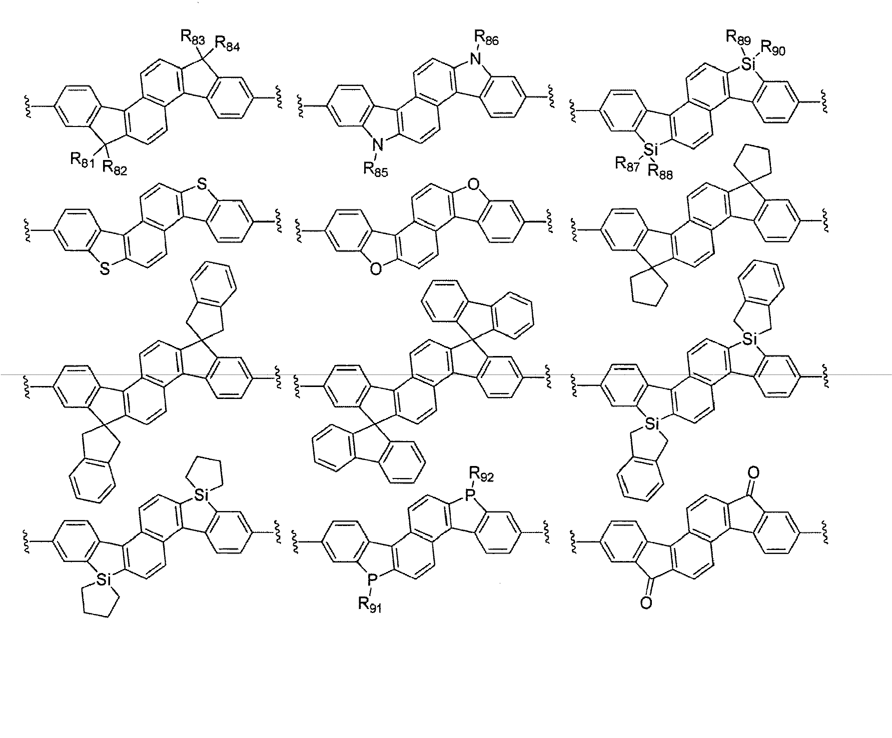 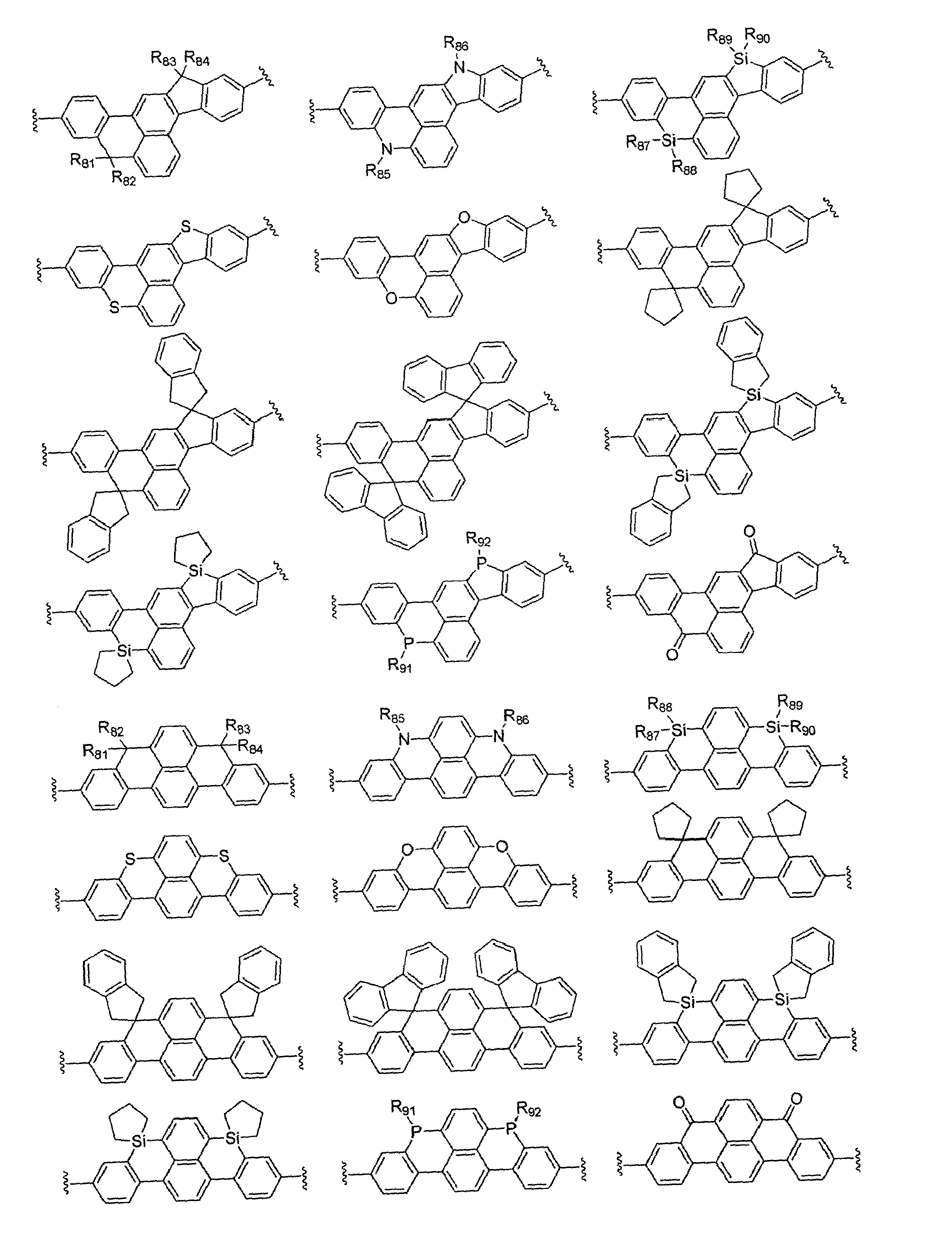 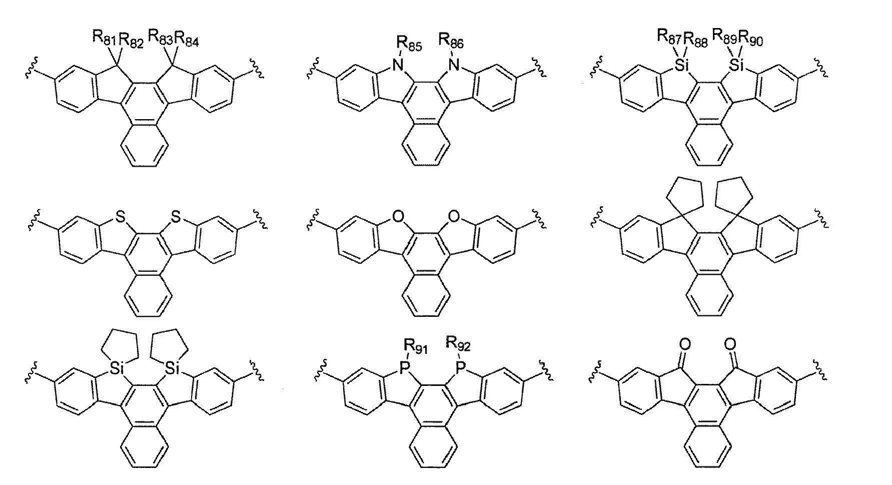 wherein, R₈₁ through R₉₂ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₈₁ and R₈₂, R₈₃ and R₈₄, R₈₇ and R₈₈, or R₈₉ and R₉₀ may be linked via (C3-C60)alkylene or (C3-C60)alkenylene with or without a fused ring to form an alicyclic ring, or a monocyclic or polycyclic aromatic ring; and
the alkyl, aryl, heteroaryl, heterocycloalkyl, cycloalkyl, alkylsilyl, arylsilyl, adamantyl, bicycloalkyl, alkenyl, alkynyl, alkylamino or arylamino of R₈₁, through R₉₂ may be further substituted by halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl.

In Chemical Formula (1), 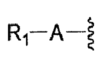 and 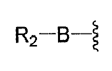 may be independently selected from the following structures, without restriction: 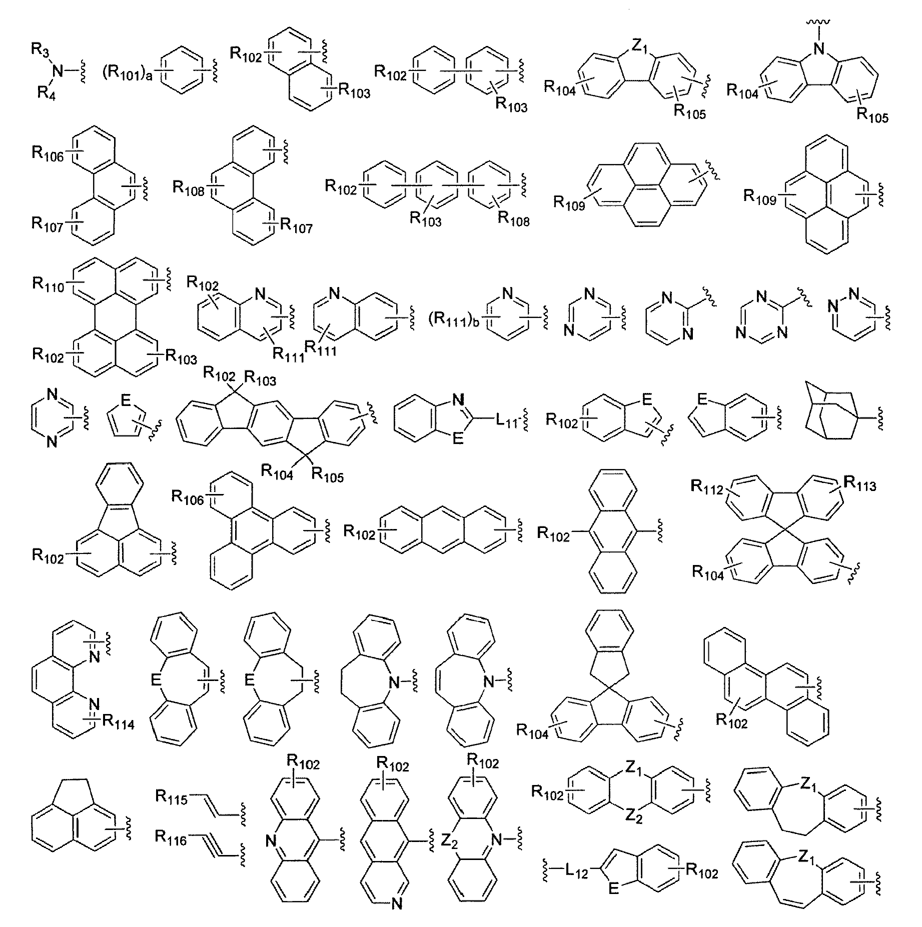
wherein, R₃ and R₄ are defined as in Chemical Formula (1);
R₁₀₁ through R₁₁₆ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl;
E, Z₁ and Z₂ independently represent -C(R₆₁)(R₆₂)-, -N(R₆₃)-, -S-, -O-, -Si(R₆₄)(R₆₅)-, -P(R₆₆)-, -C(=O)-, -B(R₆₇)-, -In(R₆₈)-, -Se-, -Ge(R₆₉)(R₇₀)-, -Sn(R₇₁)(R₇₂)-, -Ga(R₇₃)- or -(R₇₄)C=C(R₇₅)-;
R₆₁ through R₇₅ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₆₁ and R₆₂, R₆₄ and R₆₅, R₆₉ and R₇₀, R₇₁ and R₇₂, or R₇₄ and R₇₅ may be linked via (C3-C60)alkylene or (C3-C60)alkenylene with or without a fused ring to form an alicyclic ring, or a monocyclic or polycyclic aromatic ring;
L₁₁ and L₁₂ independently represent a chemical bond, (C6-C60)arylene, (C3-C60)heteroarylene, 5- or 6-membered heterocycloalkylene containing one or more heteroatom(s) selected from N, O and S, (C3-C60)cycloalkylene, (C2-C60)alkenylene, (C2-C60)alkynylene, (C1-C60)alkylenoxy, (C6-C60)arylenoxy or (C6-C60)arylenethio;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₃, R₄, R₆₁ through R₇₅, and R₁₀₁ through R₁₁₆ ; or arylene, heteroarylene, heterocycloalkylene, cycloalkylene, alkenylene, alkynylene, alkylenoxy, arylenoxy or arylenethio of L₁₁ and L₁₂ may be further substituted by halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl; and
a is an integer from 1 to 5, and b is an integer from 1 to 4.

More specifically, R₁-A- 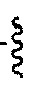 and R₂-B- 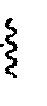 are independently selected from the following structures, without restriction:

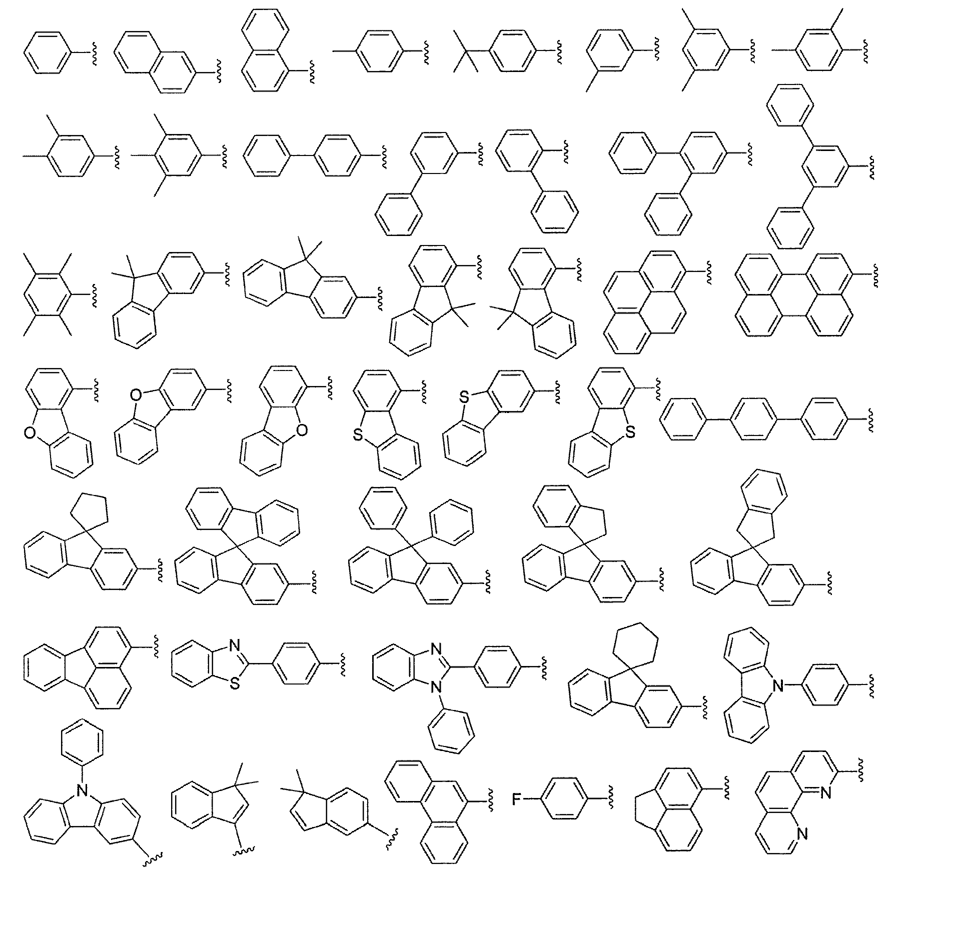

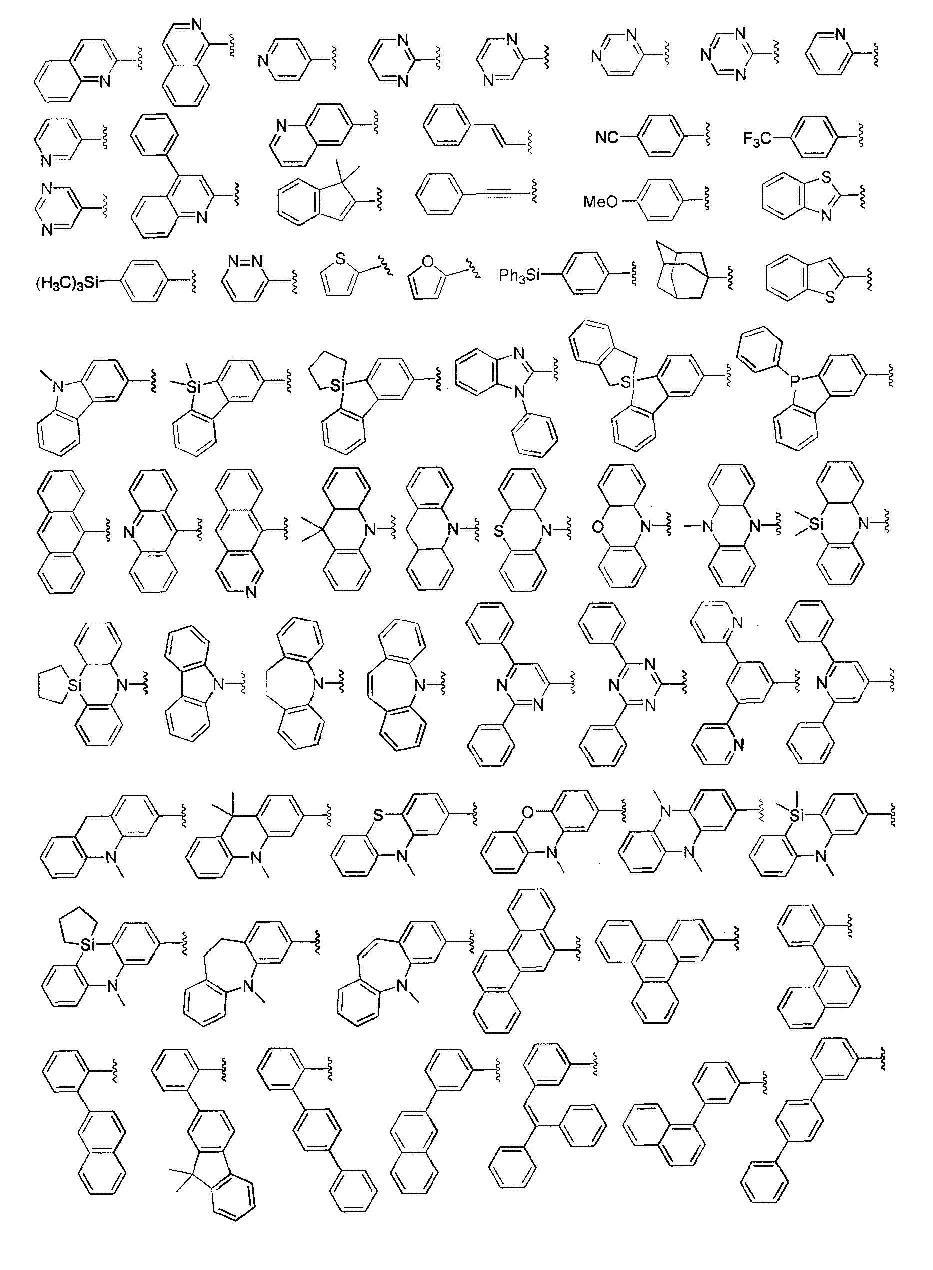

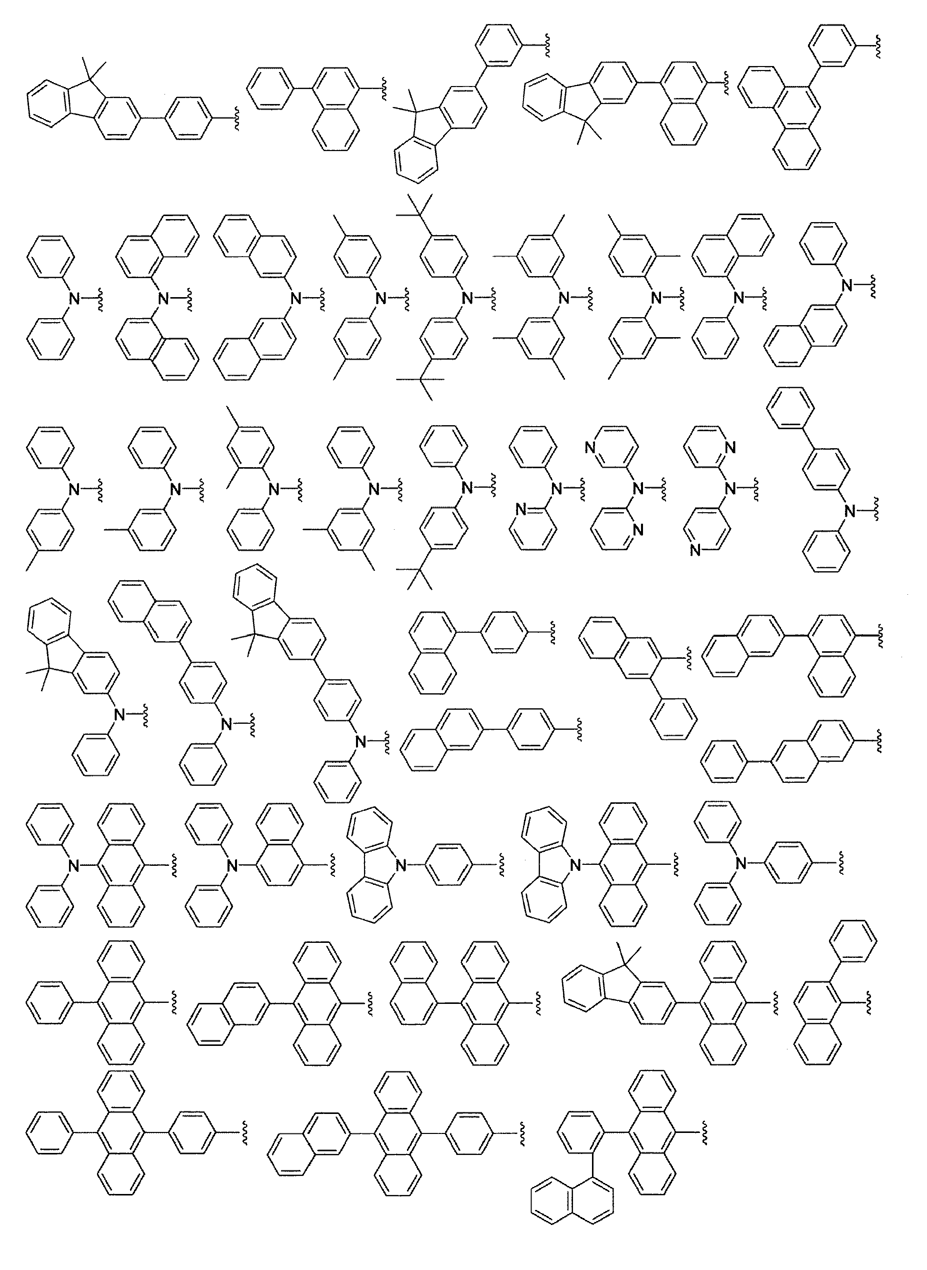

The organic electroluminescent compounds according to the present invention can be more specifically exemplified by the following compounds, without restriction:

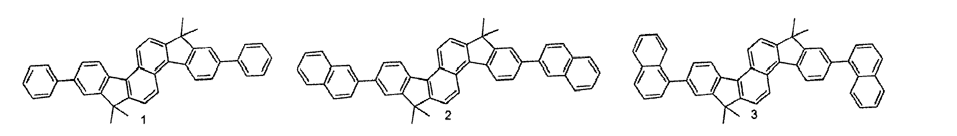

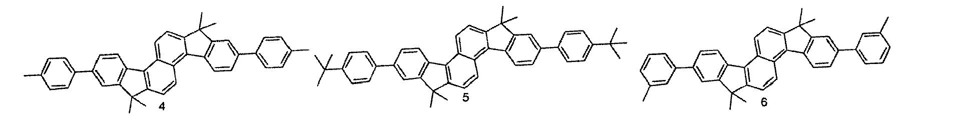

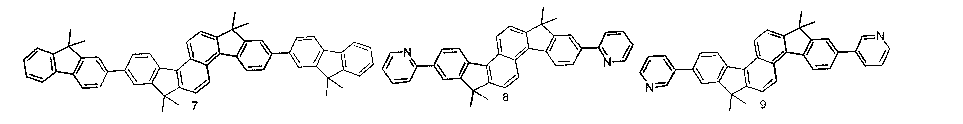

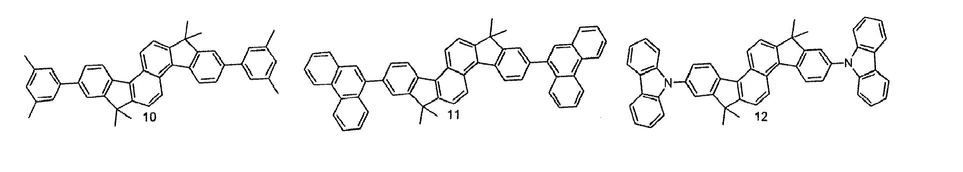

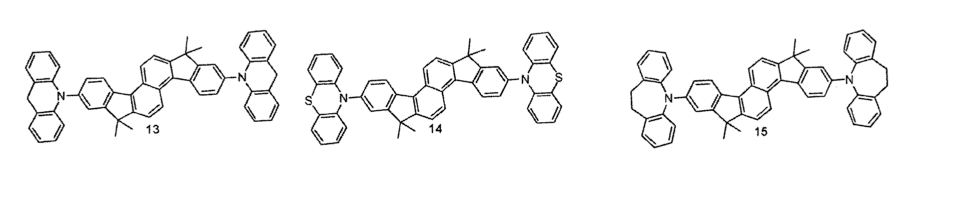

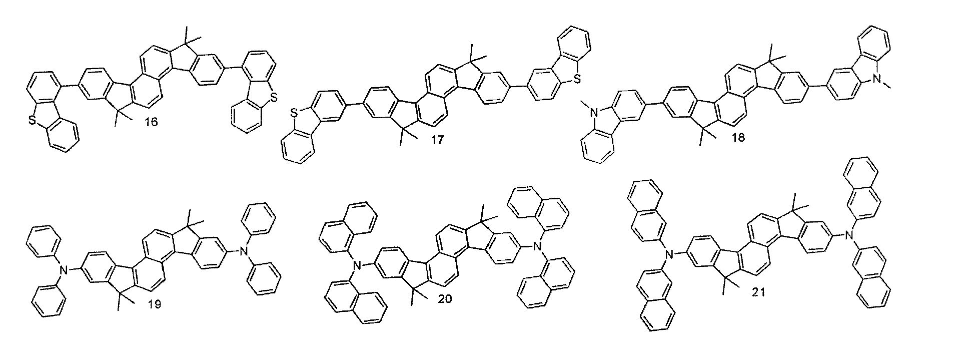

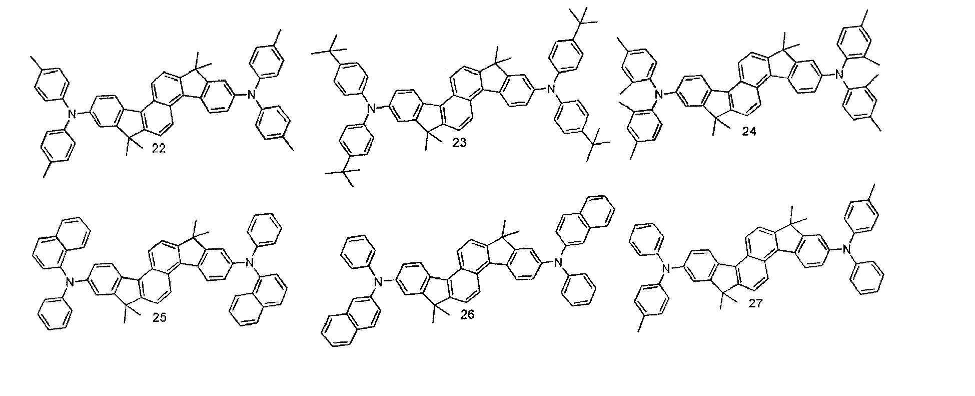

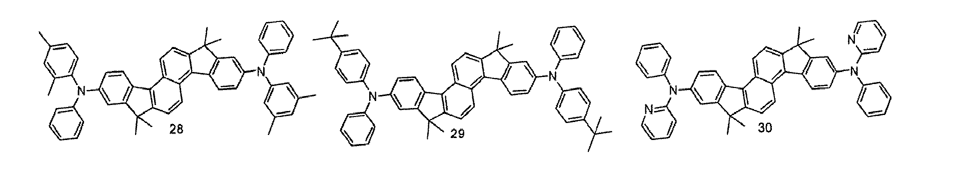

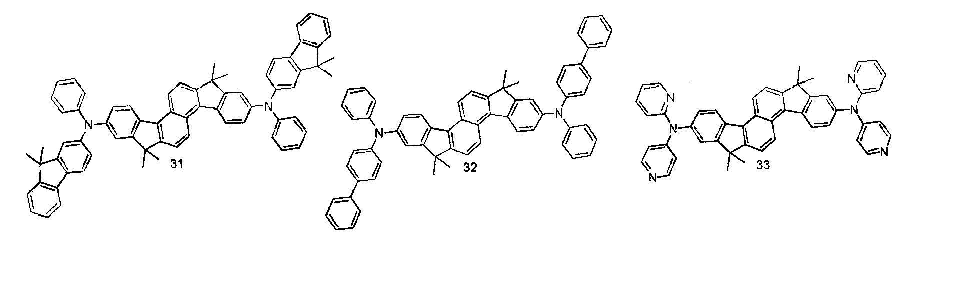

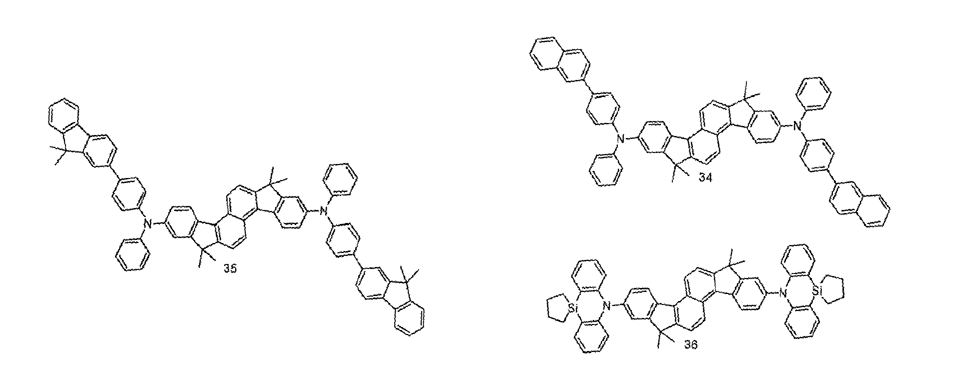

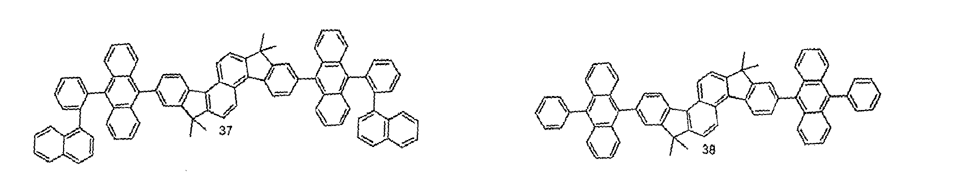

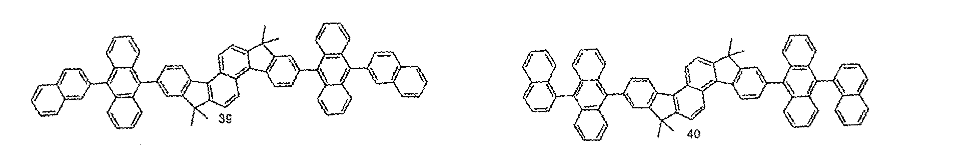

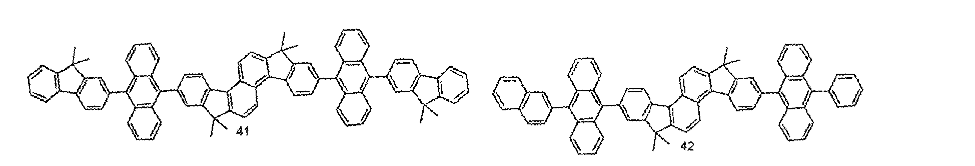

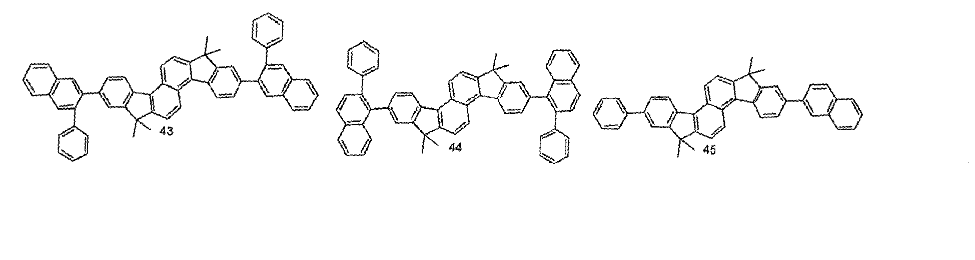

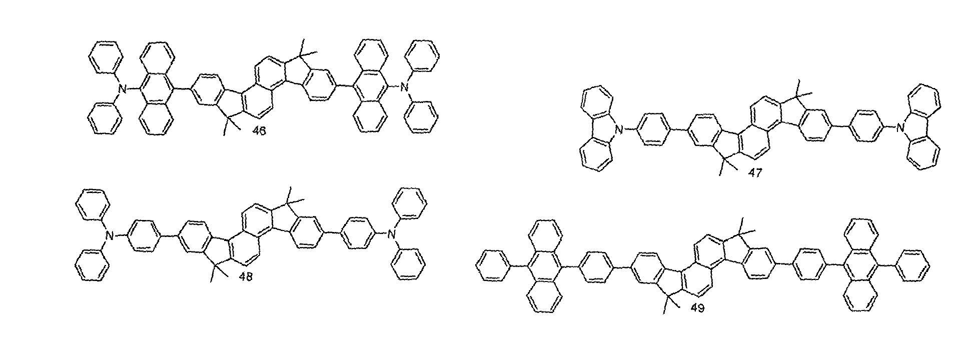

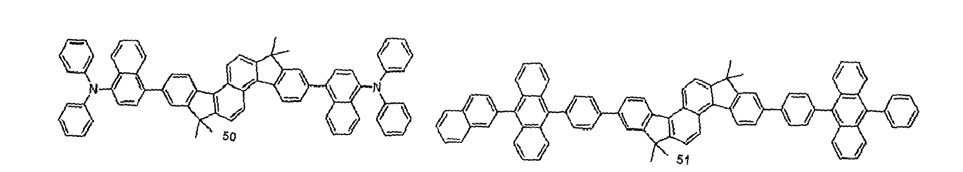

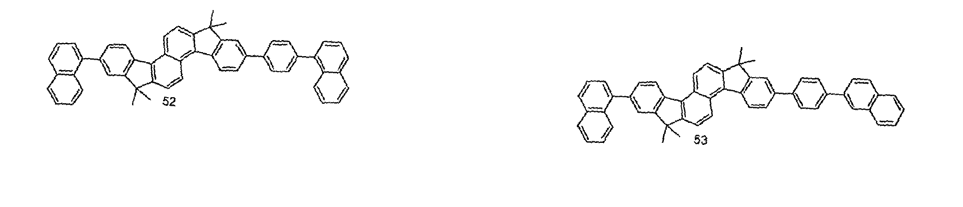

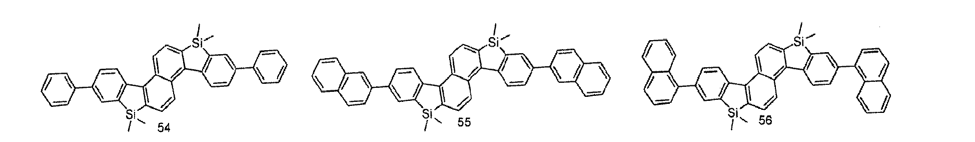

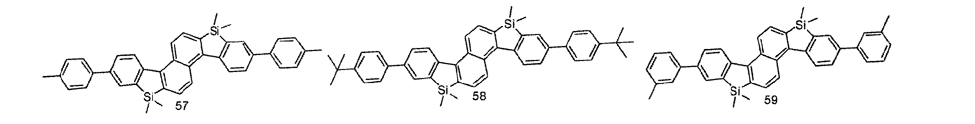

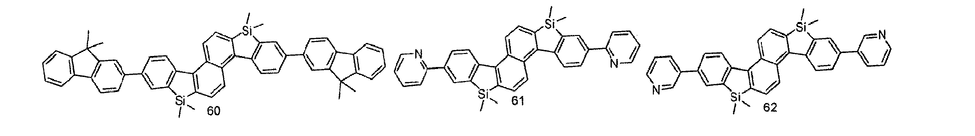

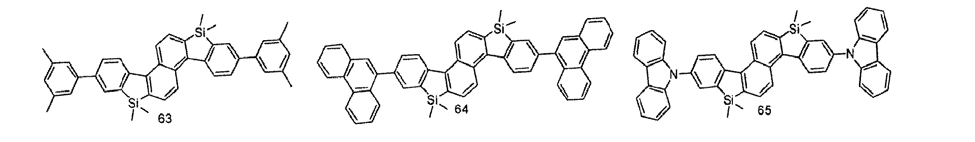

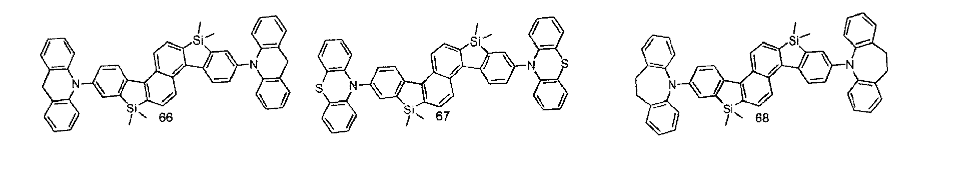

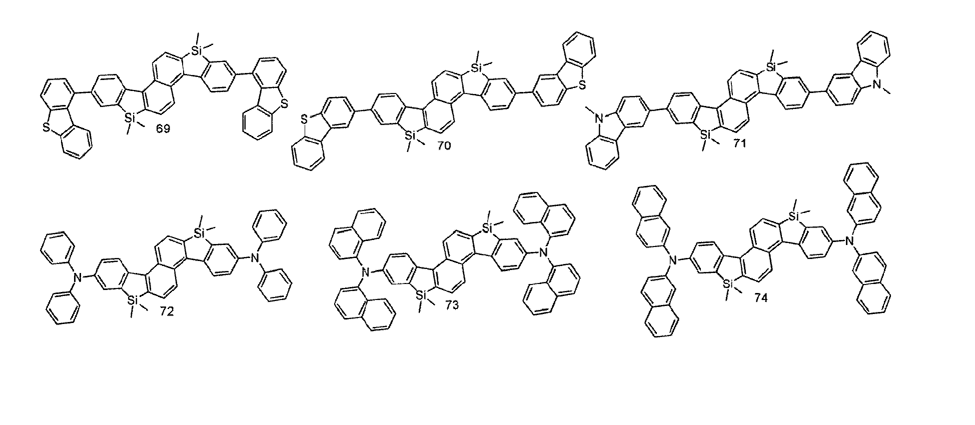

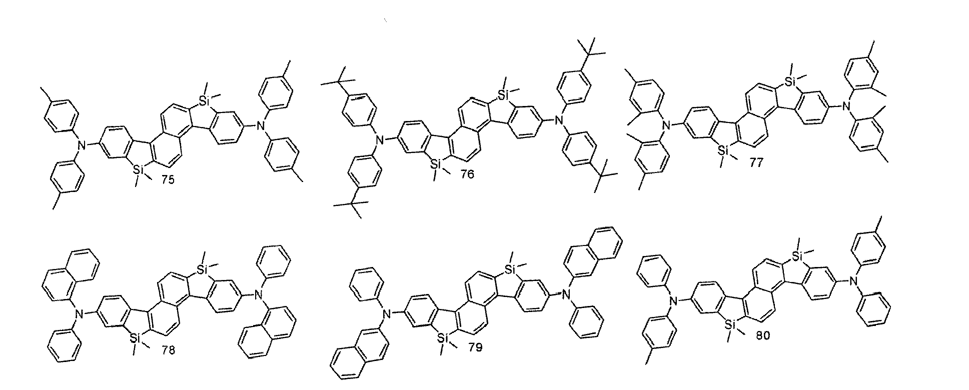

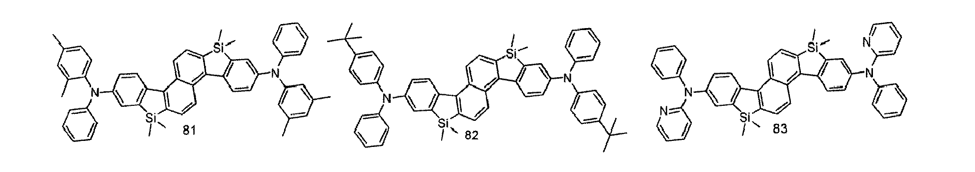

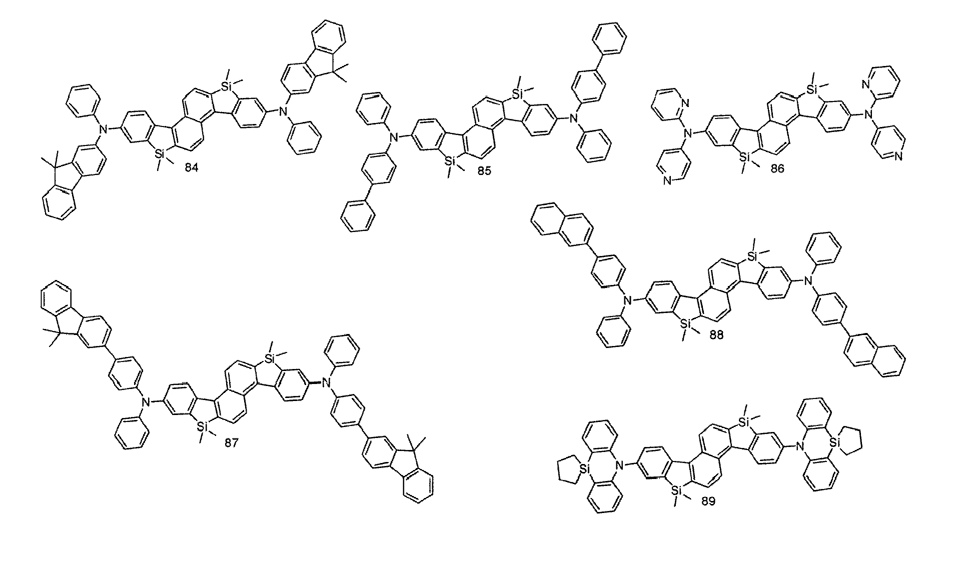

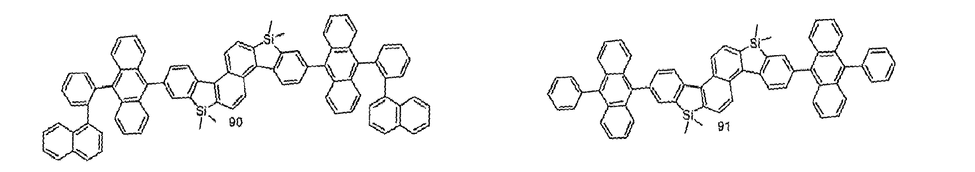

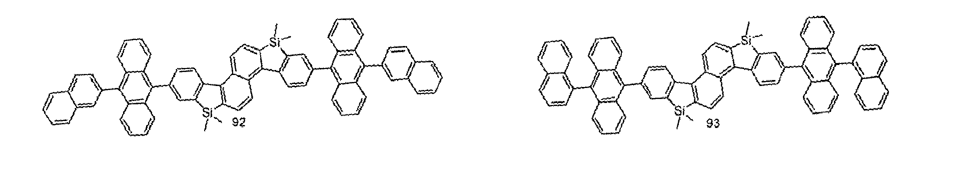

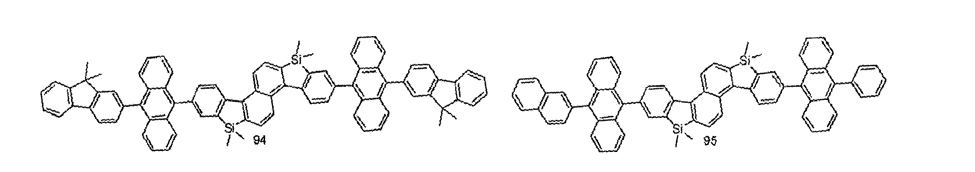

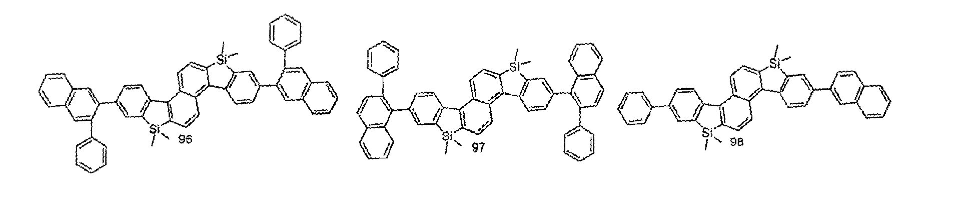

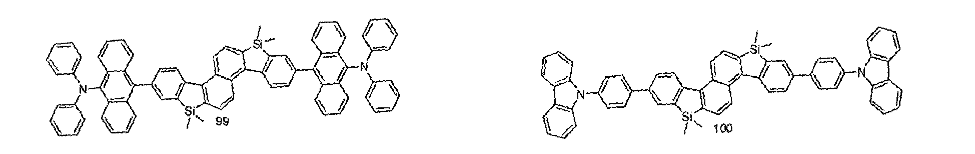

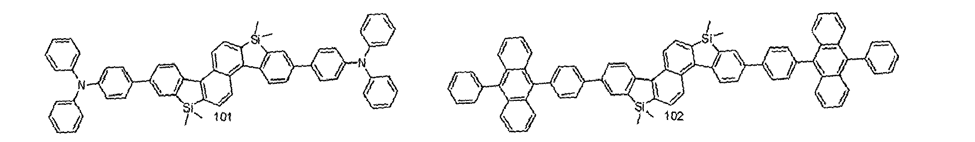

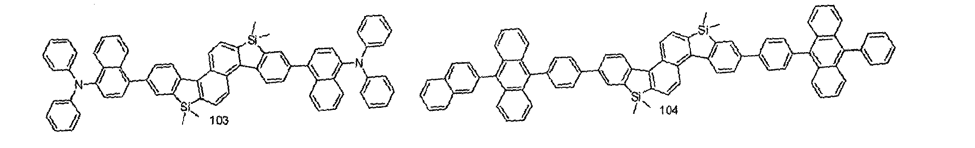

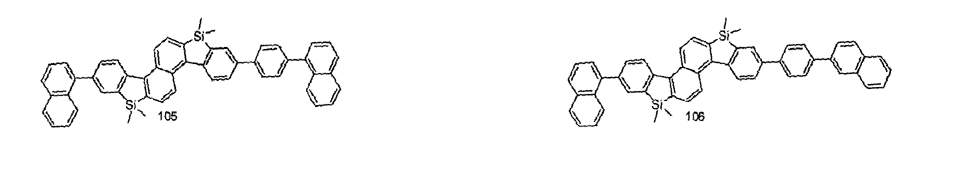

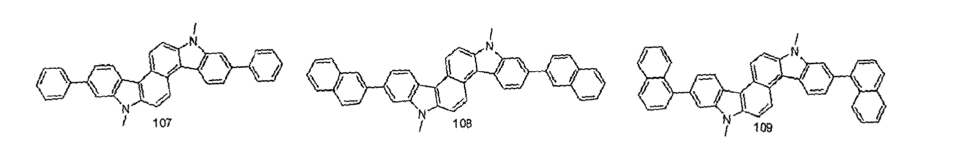

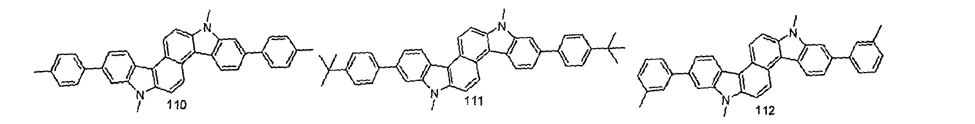

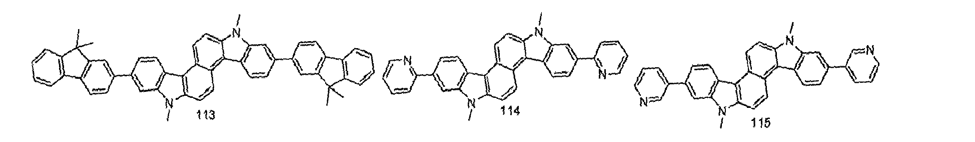

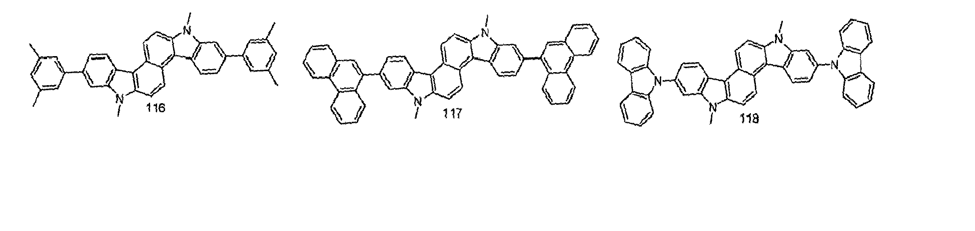

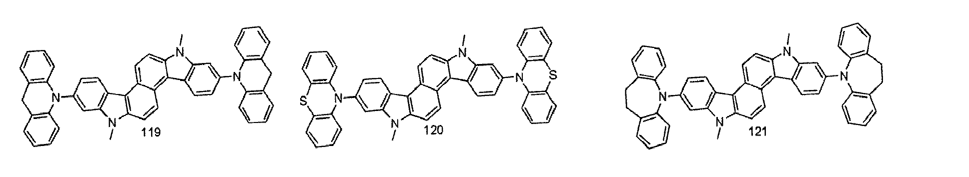

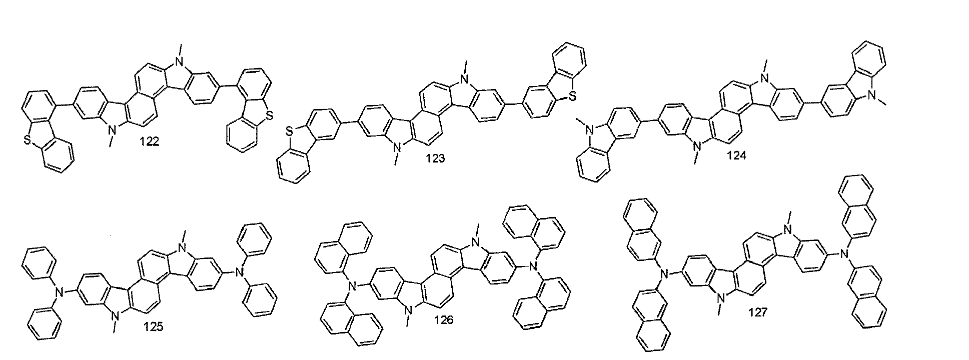

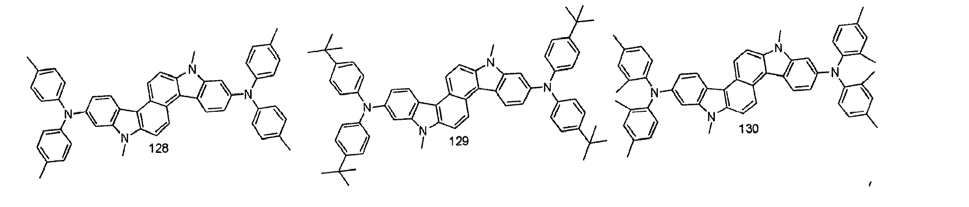

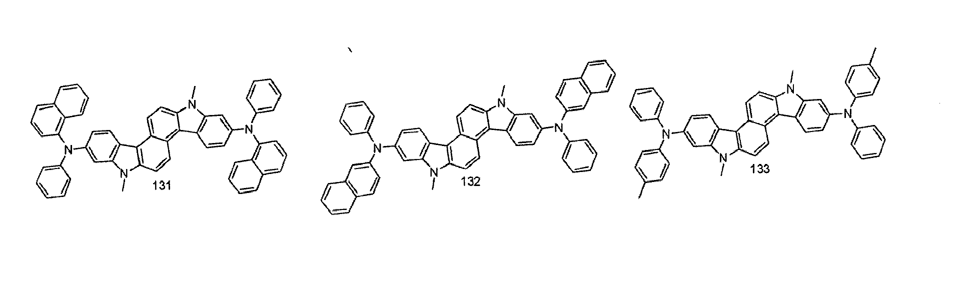

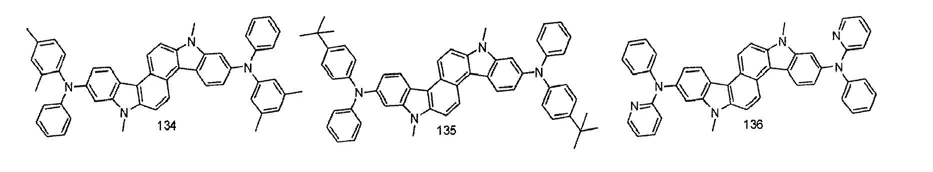

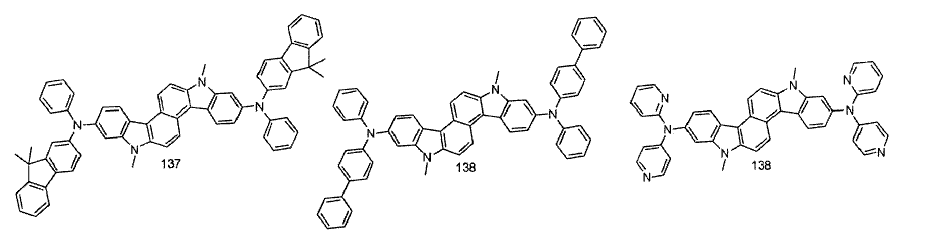

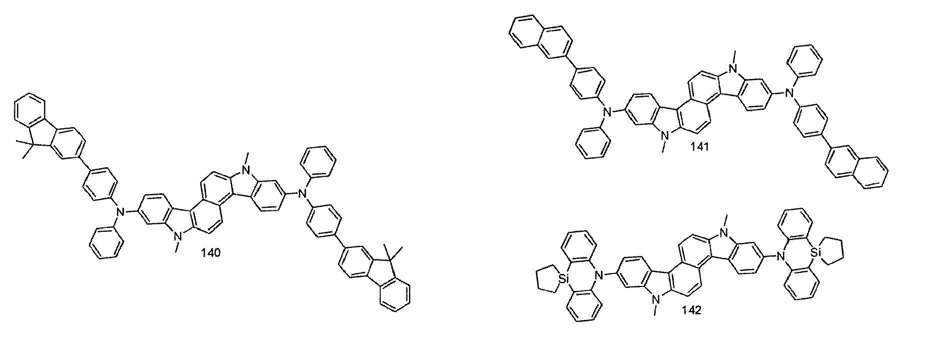

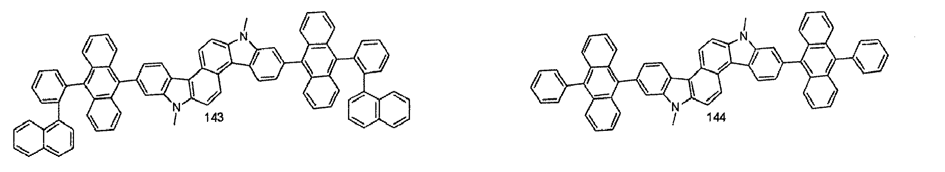

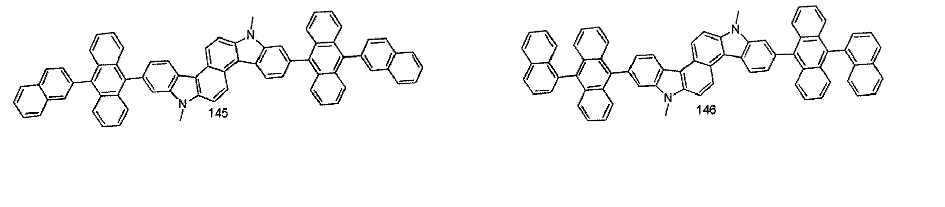

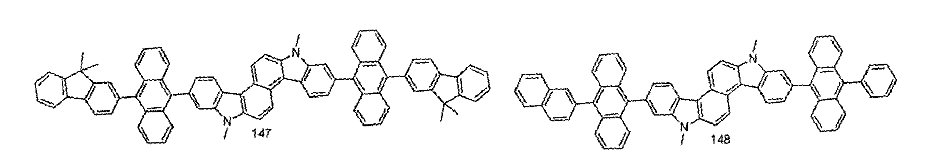

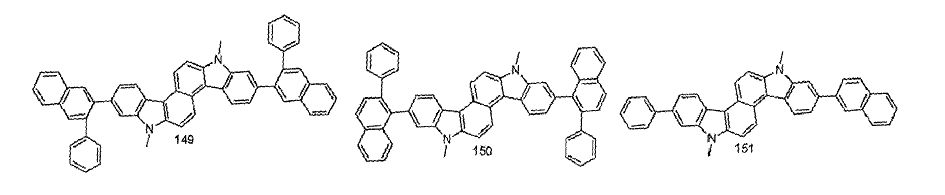

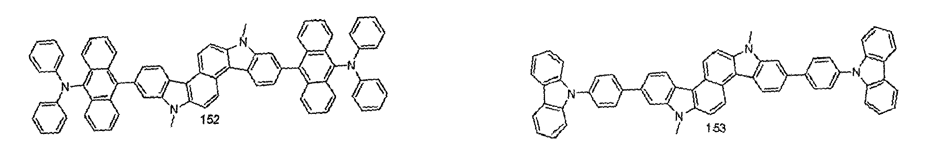

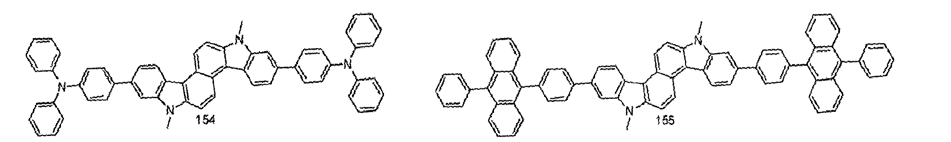

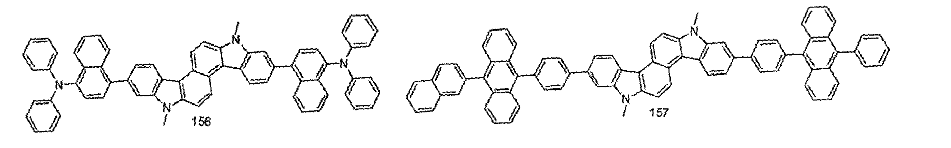

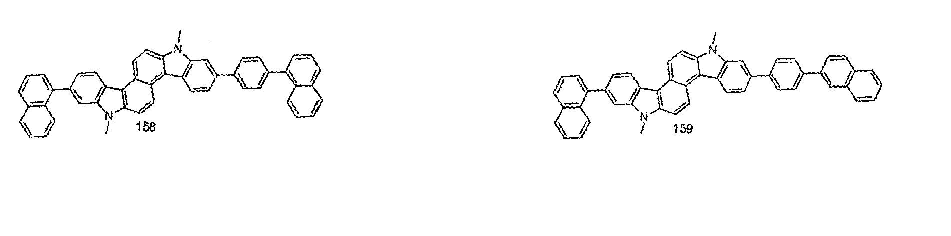

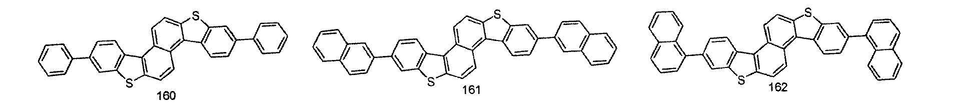

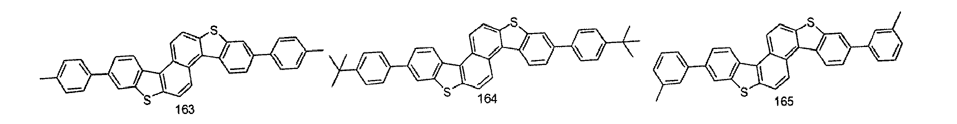

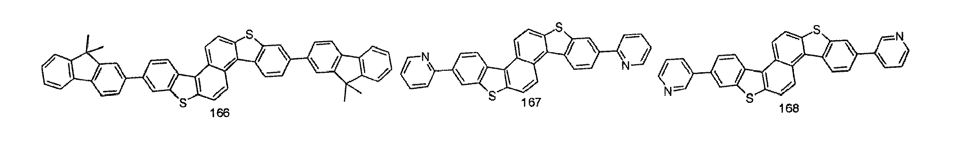

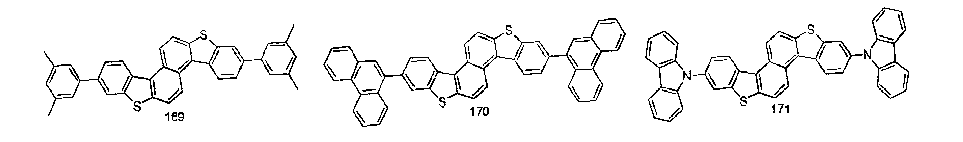

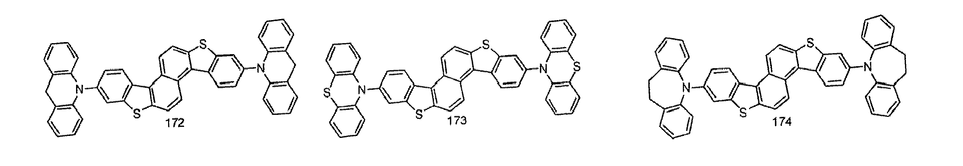

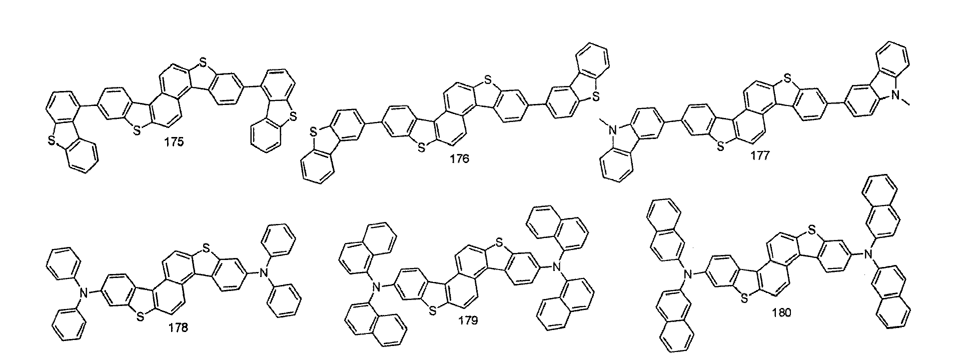

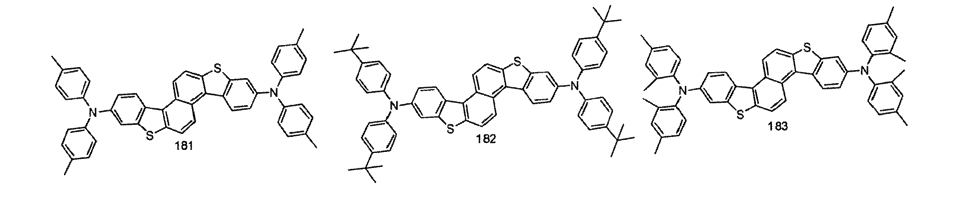

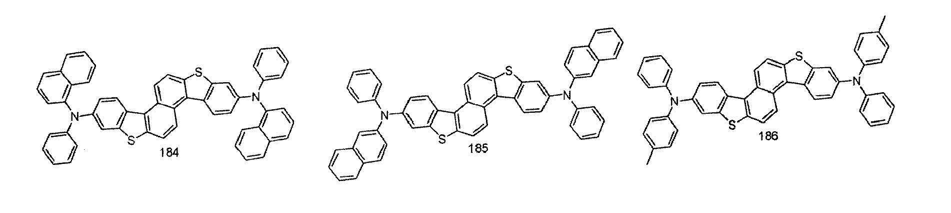

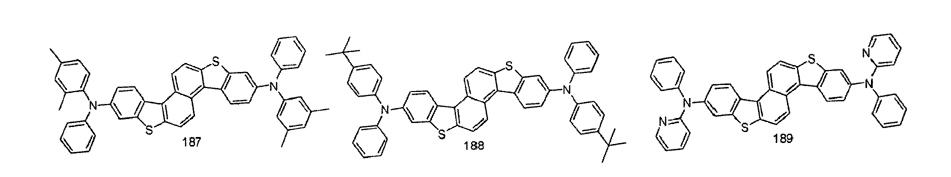

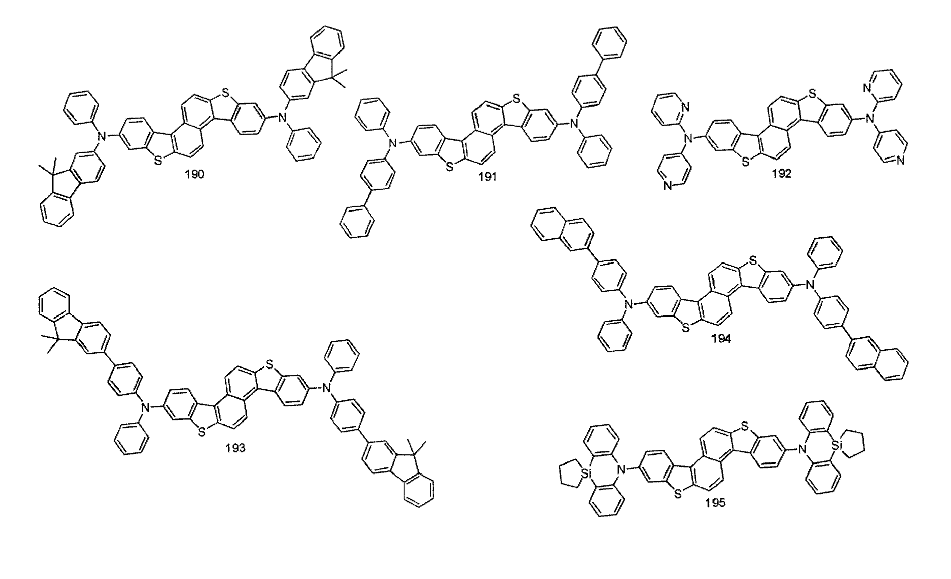

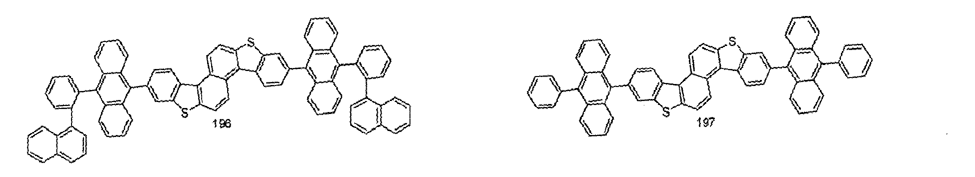

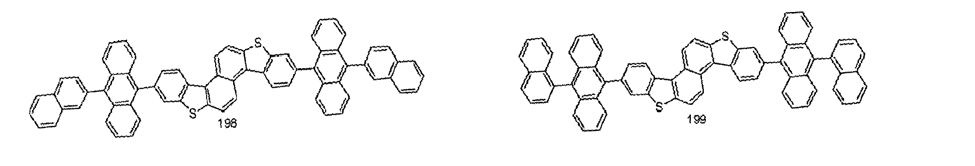

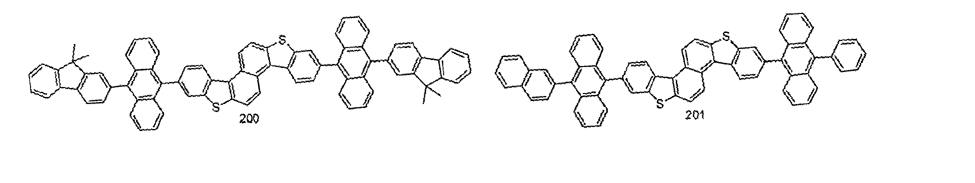

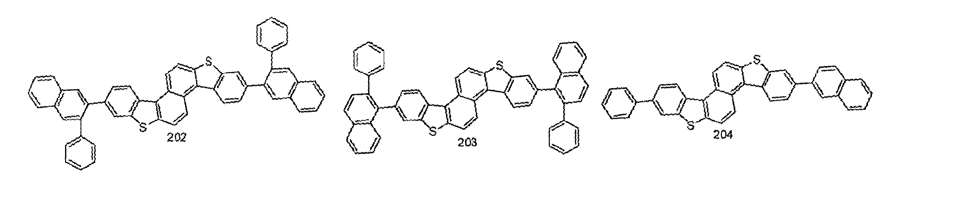

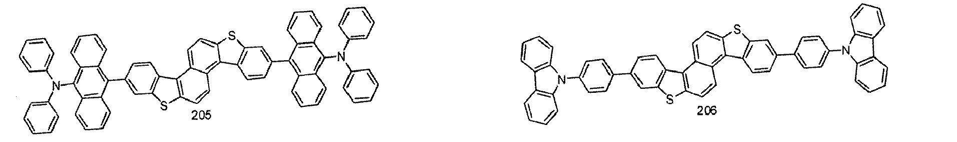

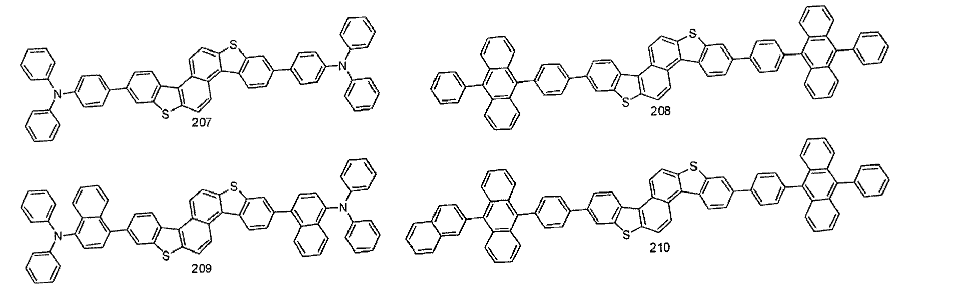

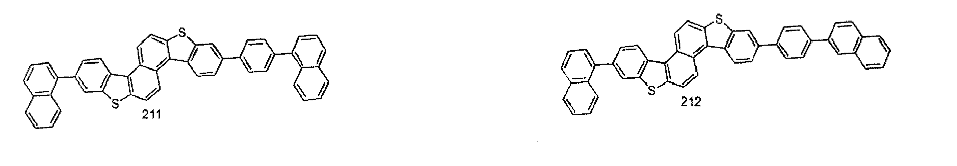

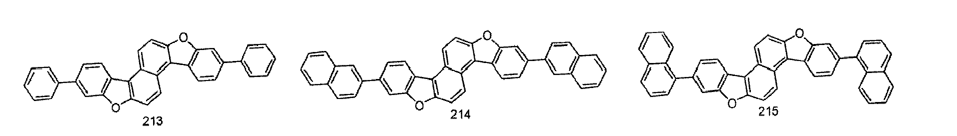

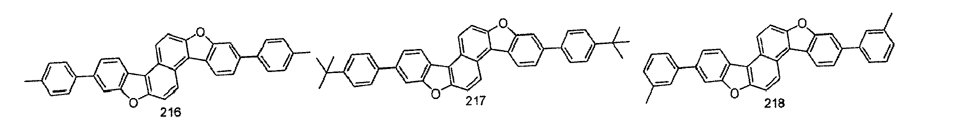

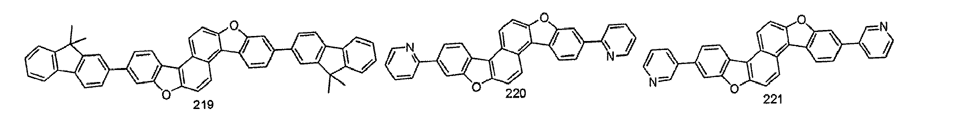

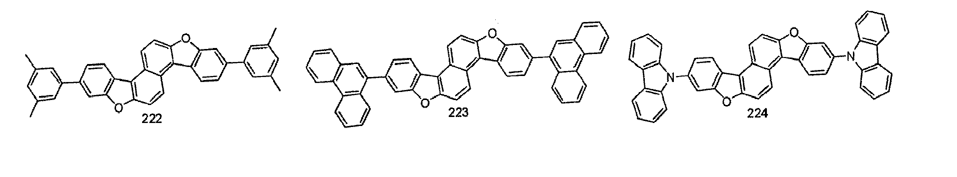

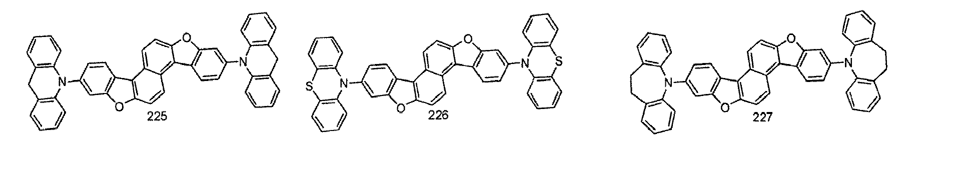

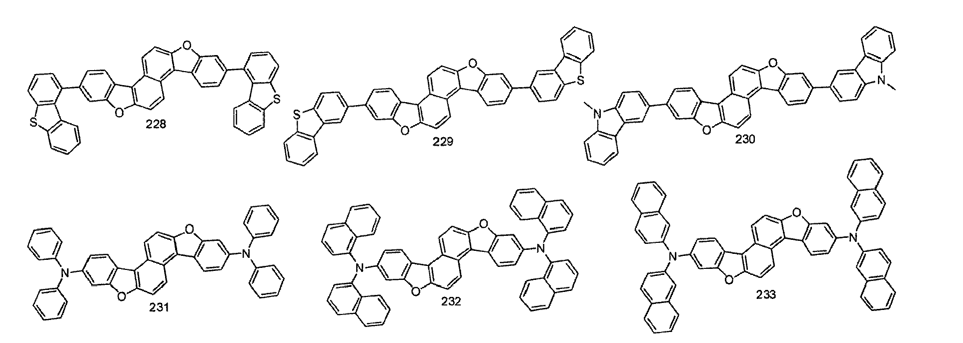

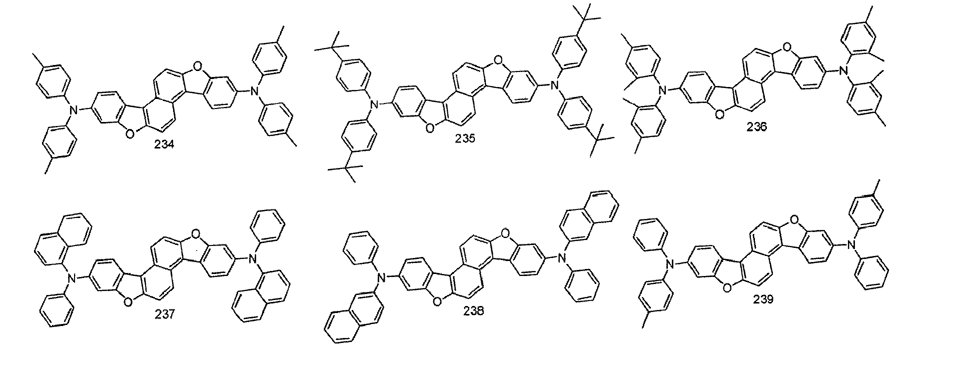

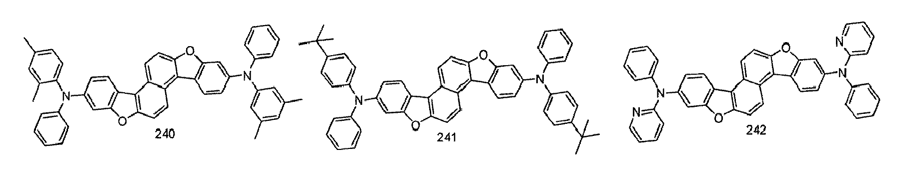

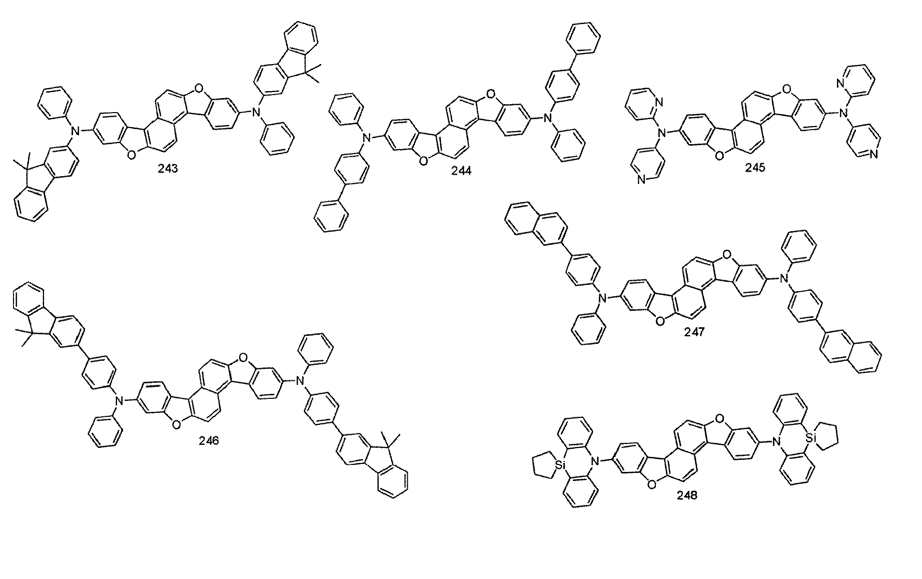

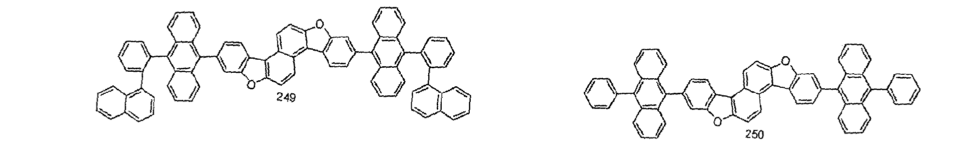

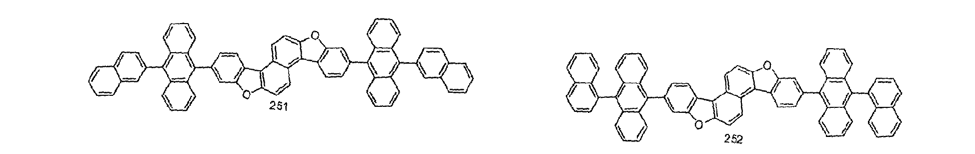

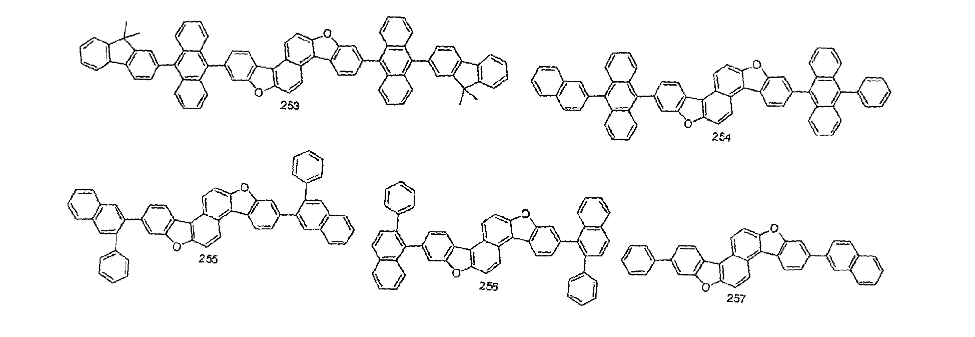

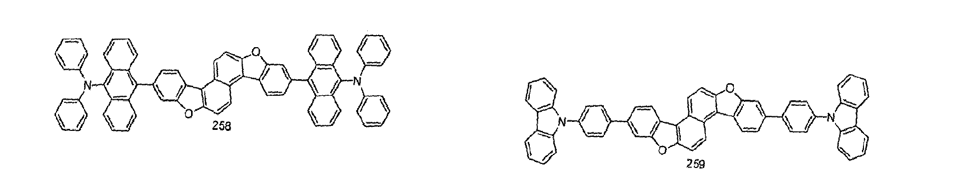

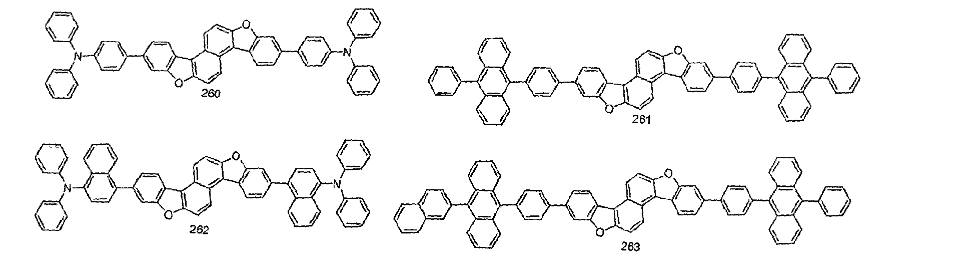

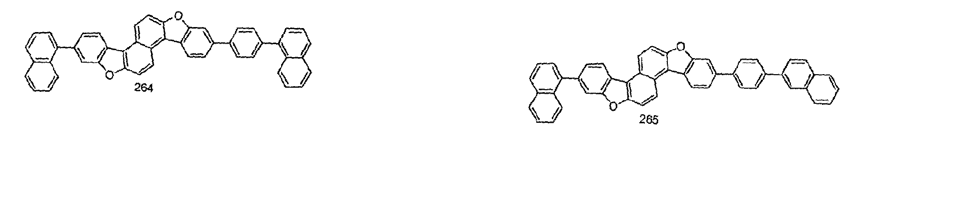

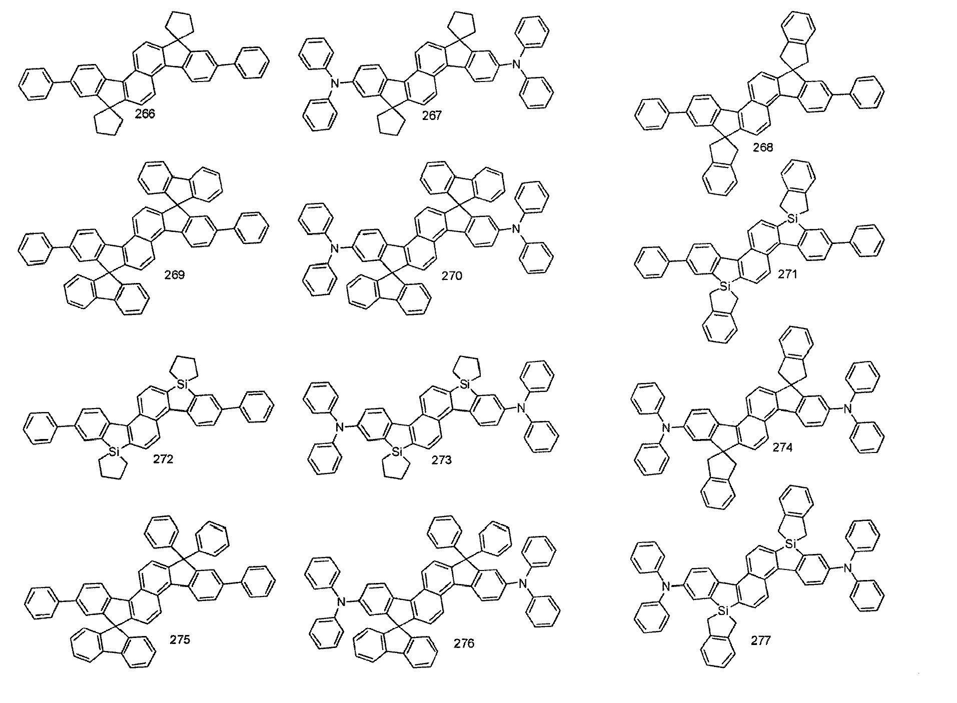

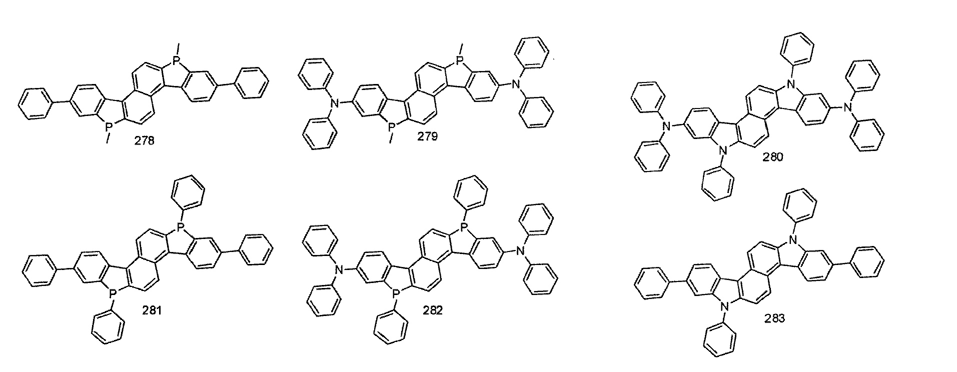

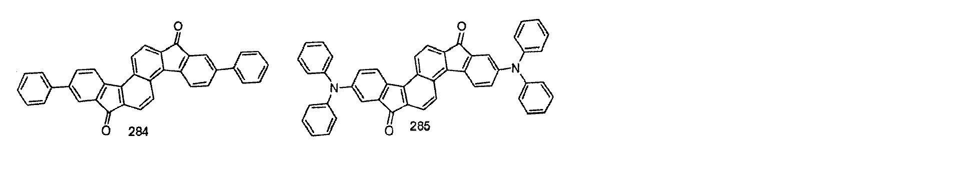

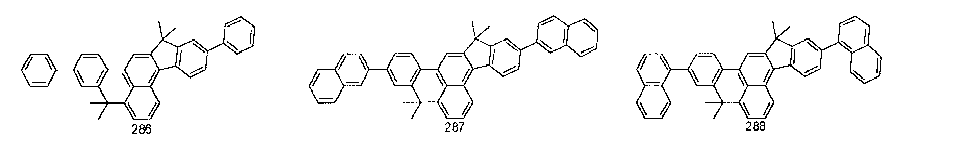

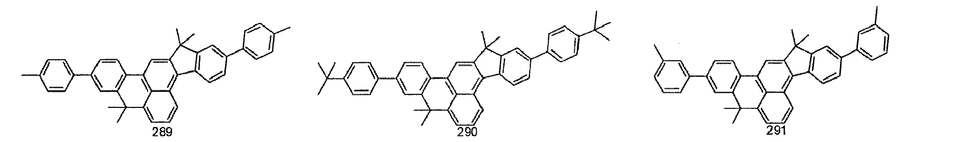

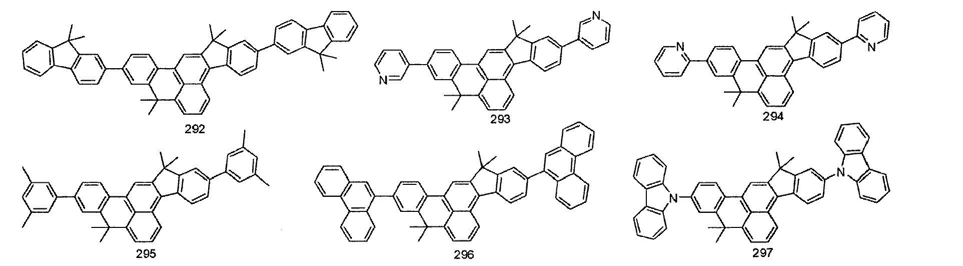

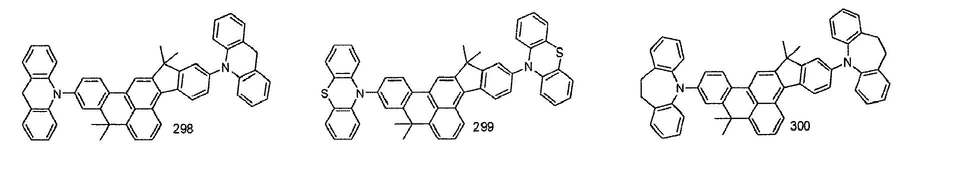

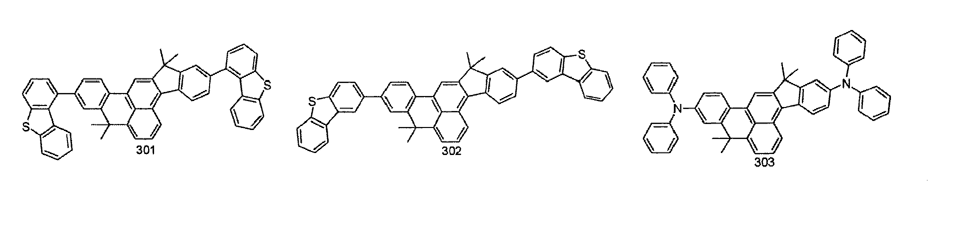

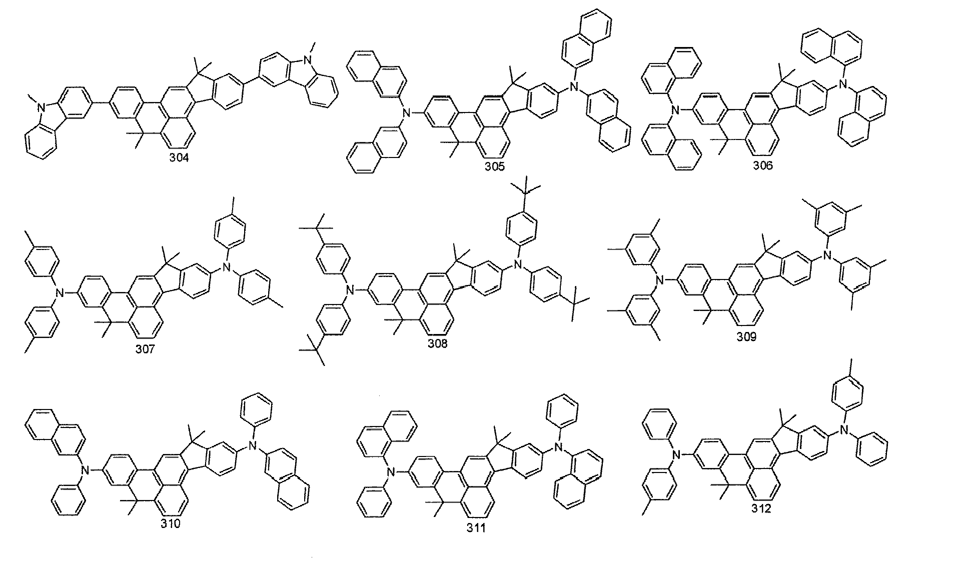

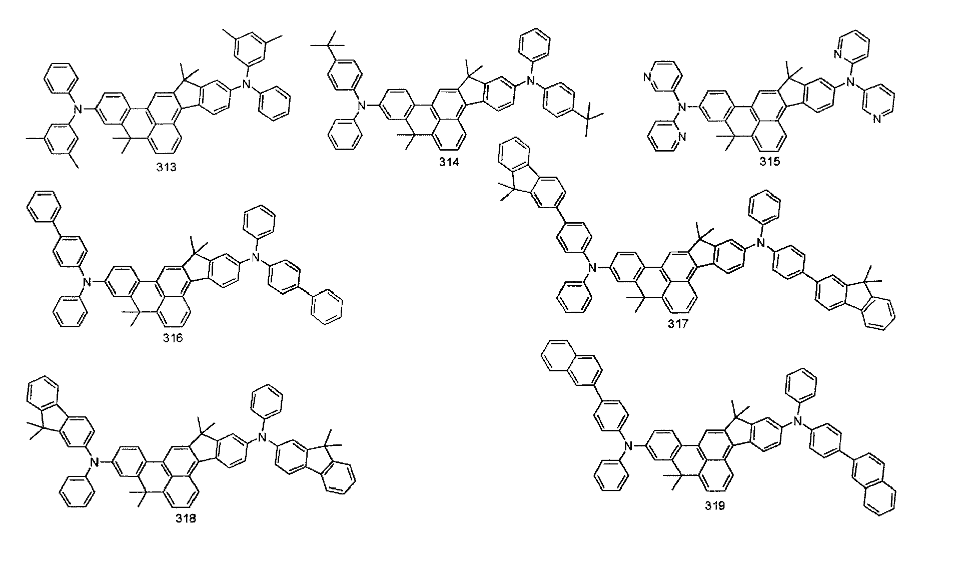

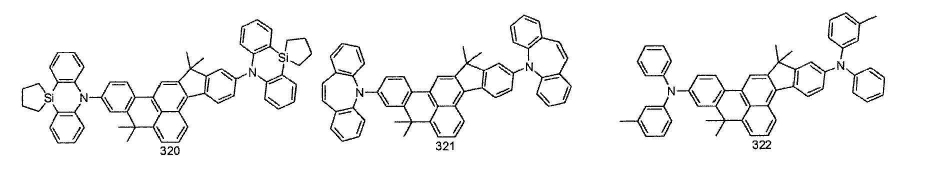

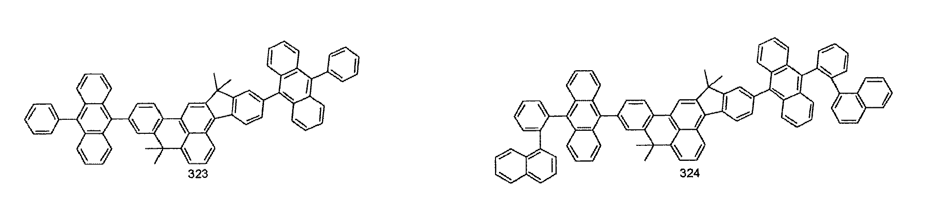

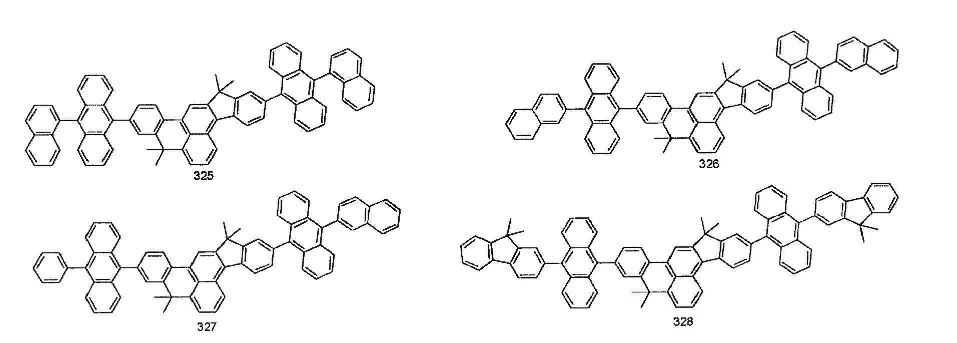

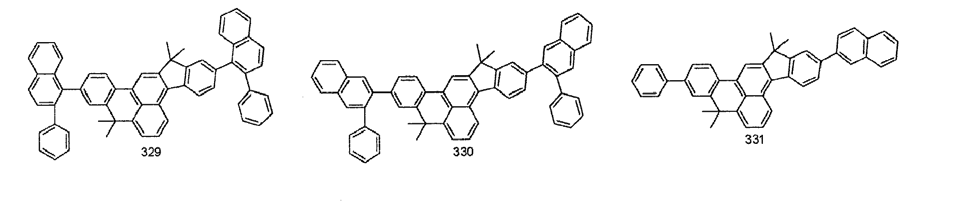

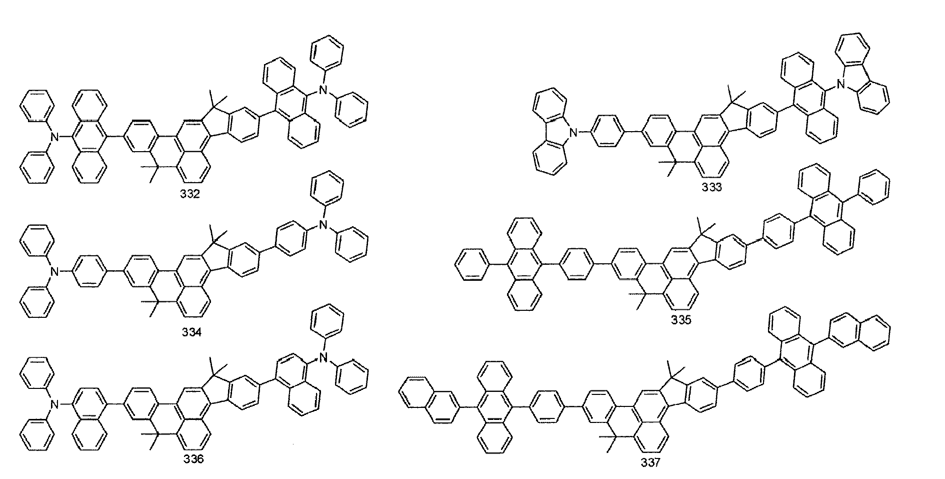

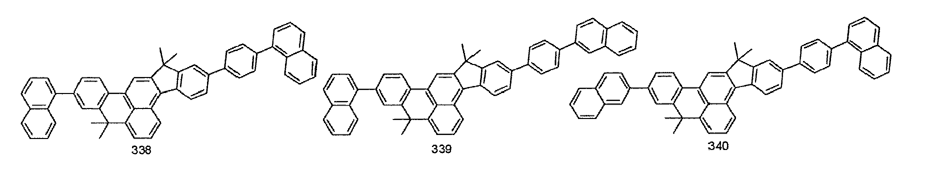

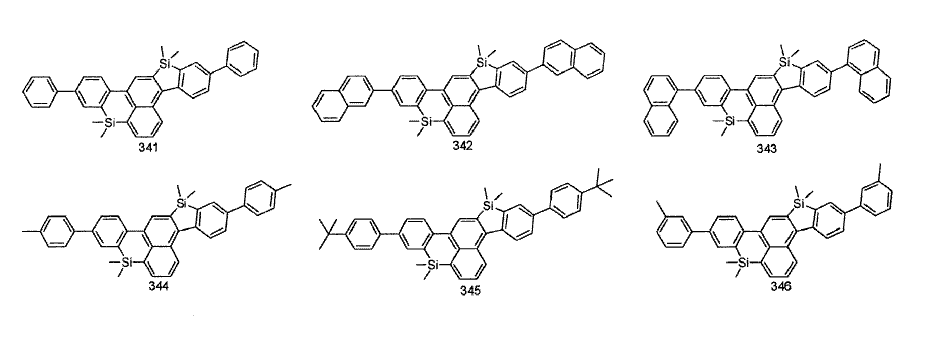

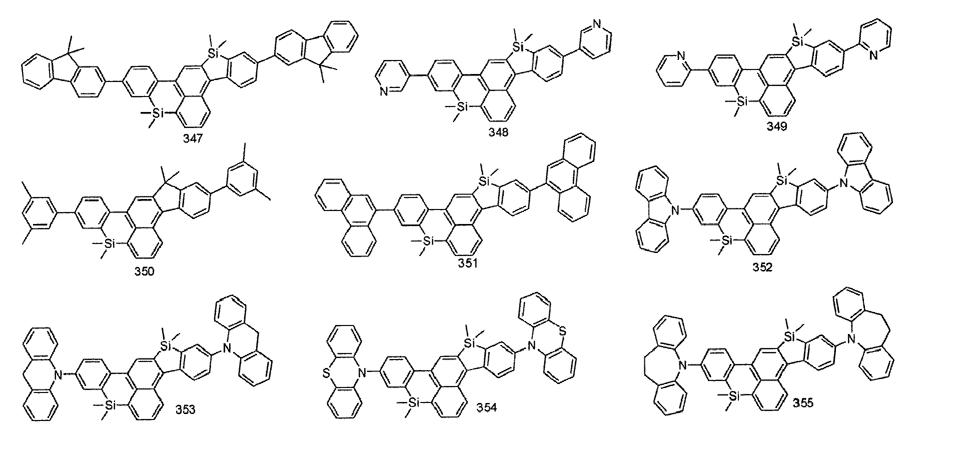

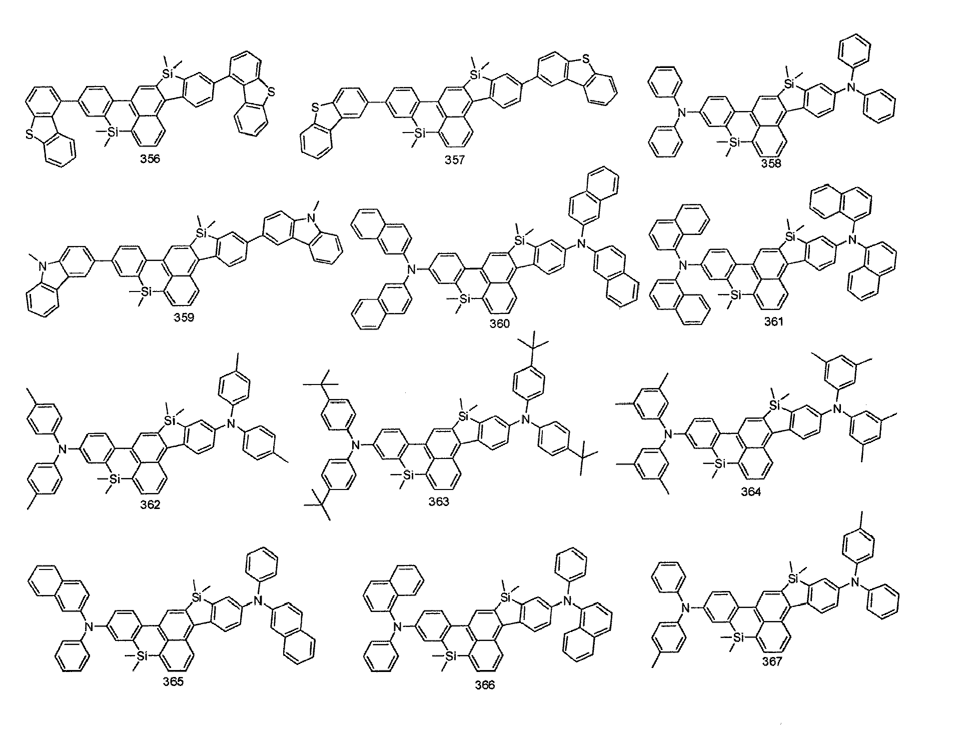

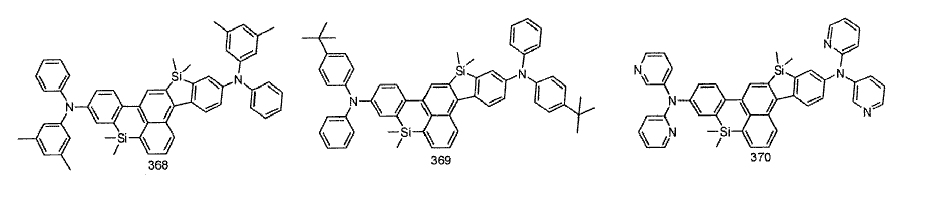

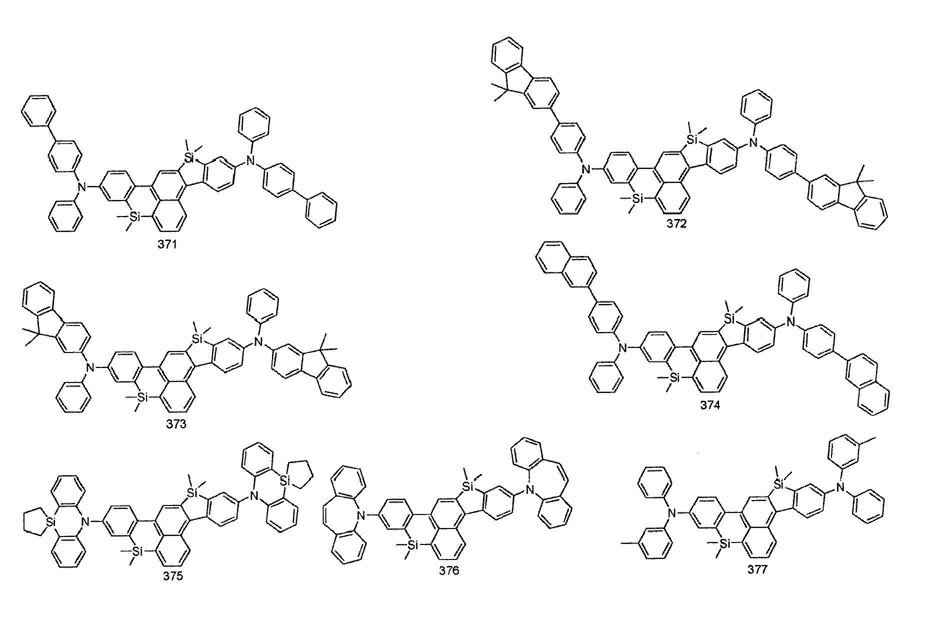

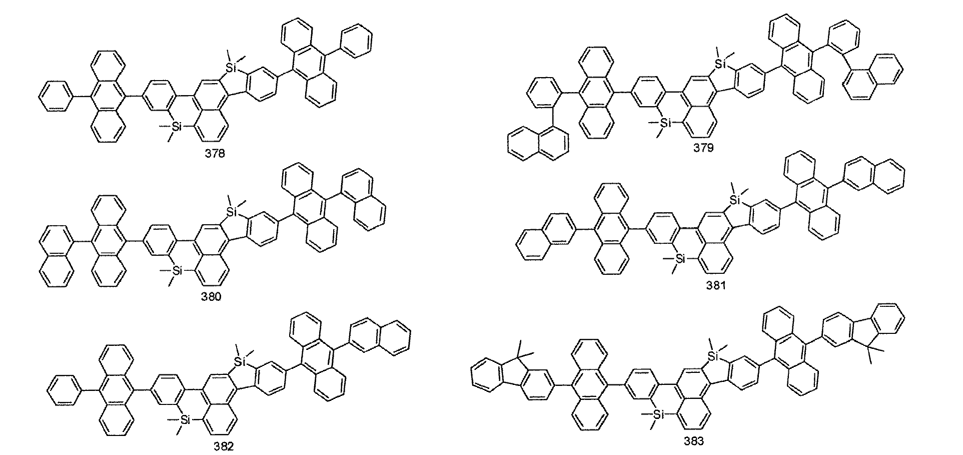

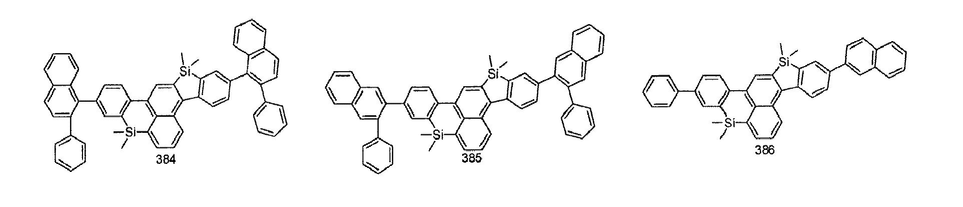

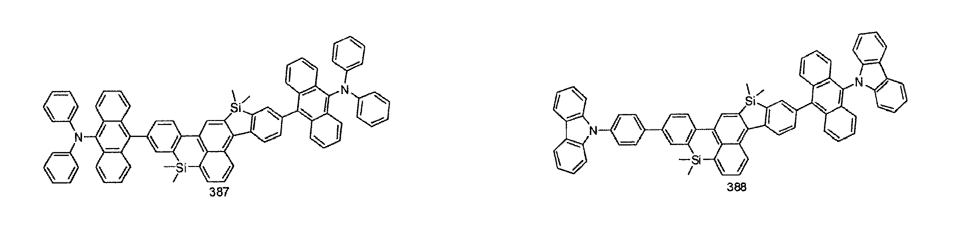

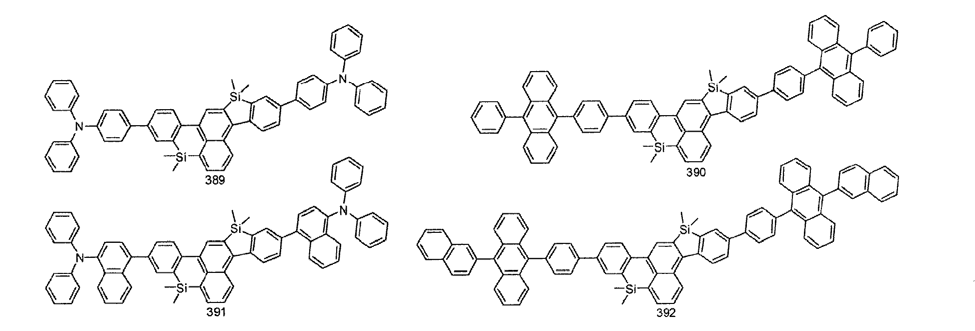

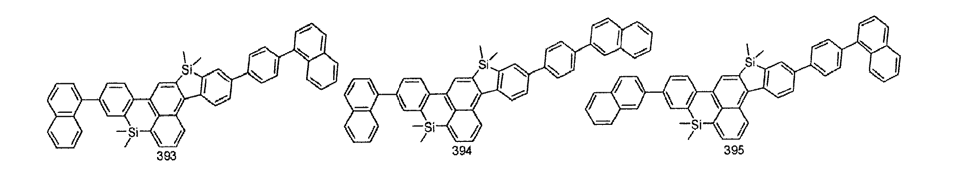

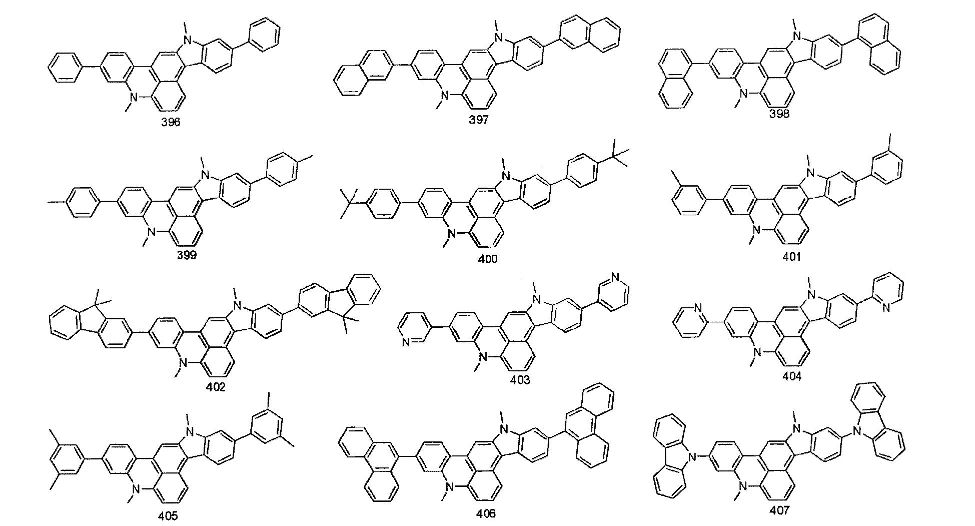

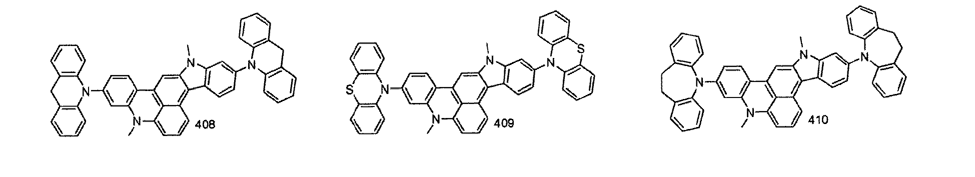

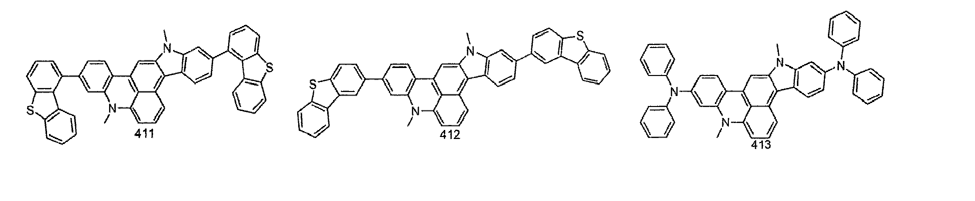

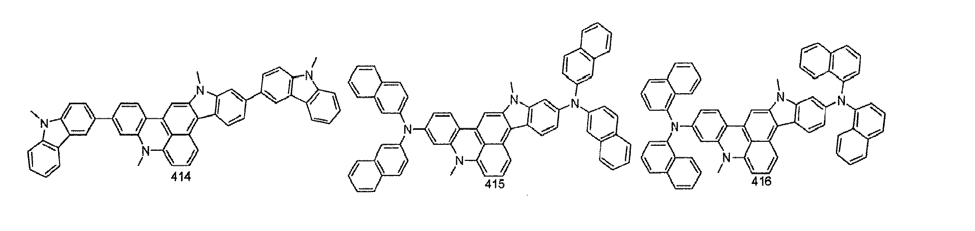

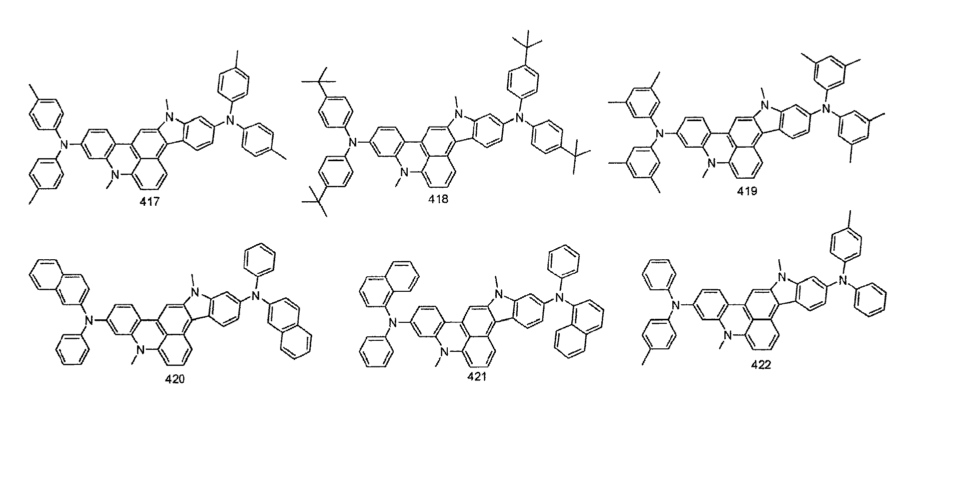

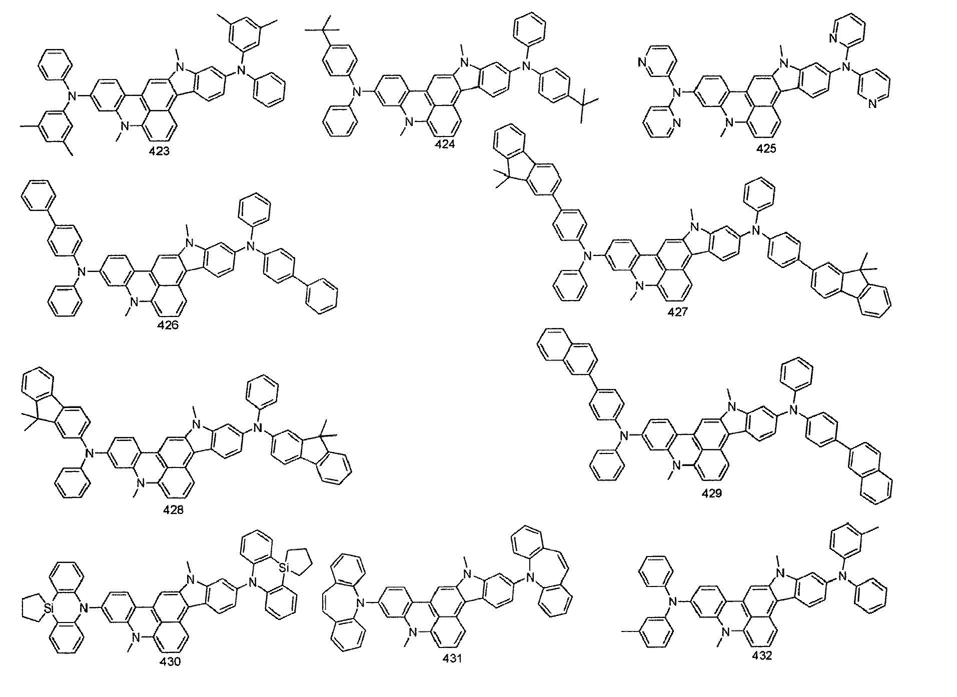

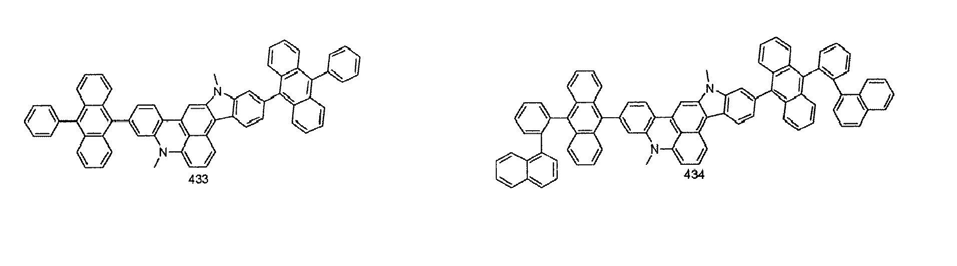

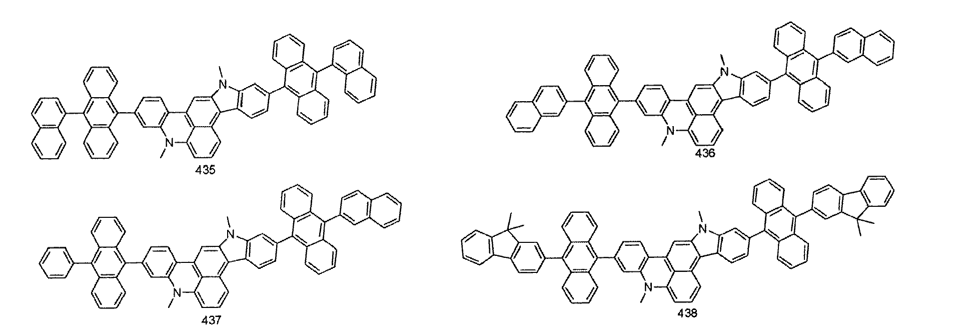

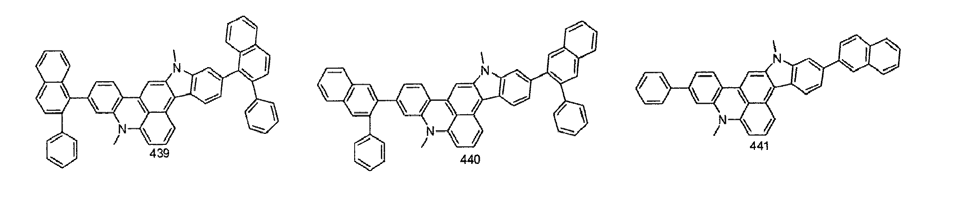

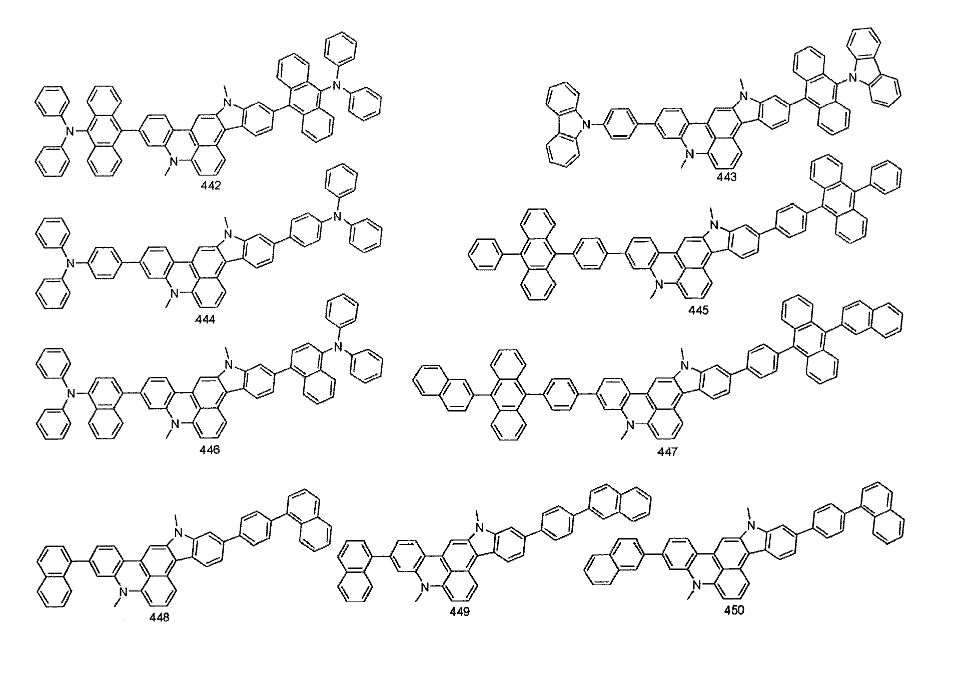

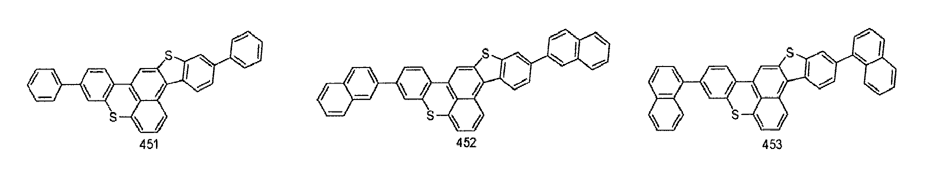

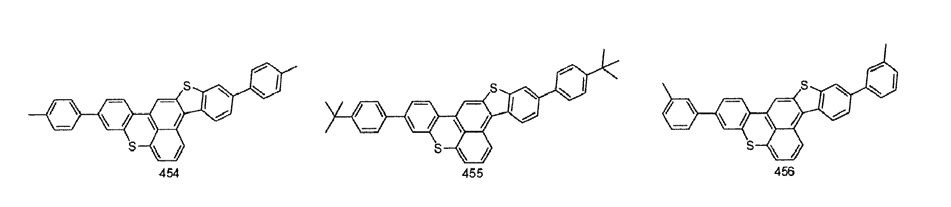

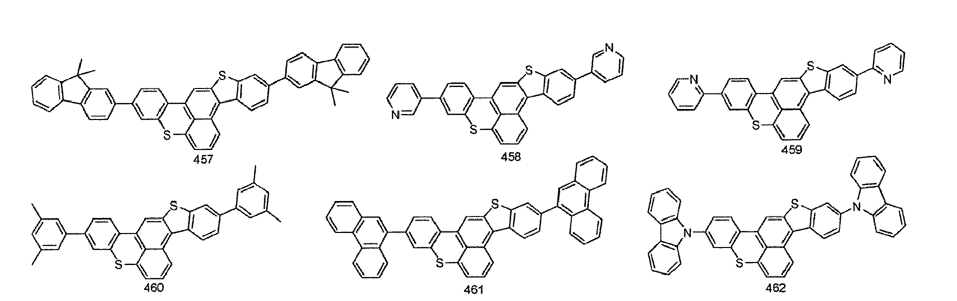

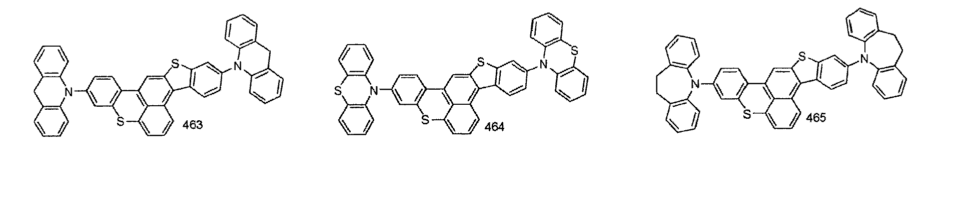

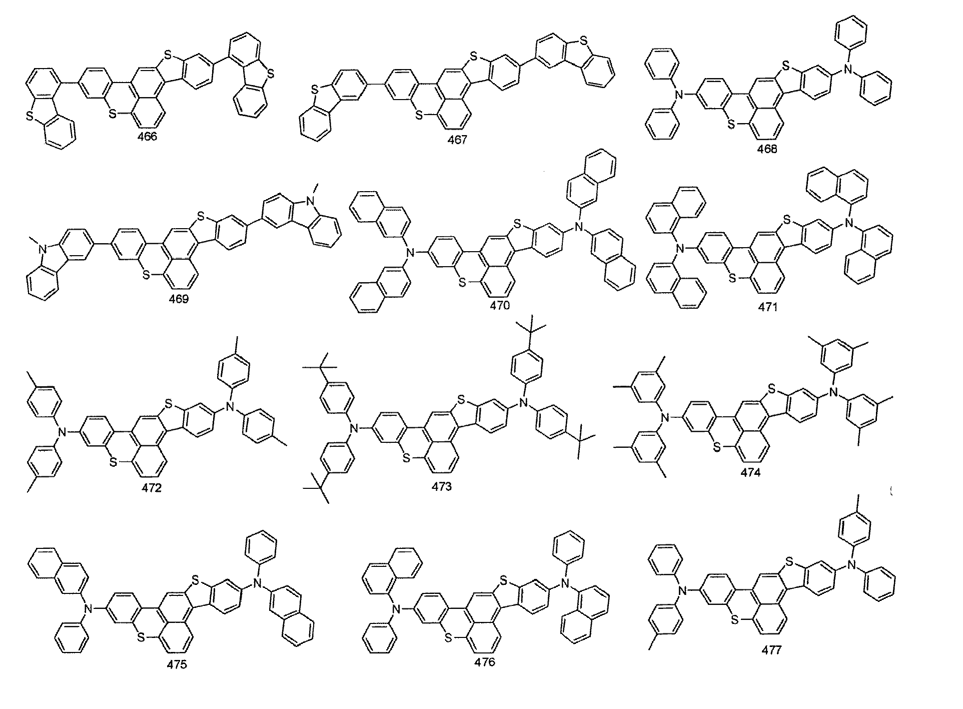

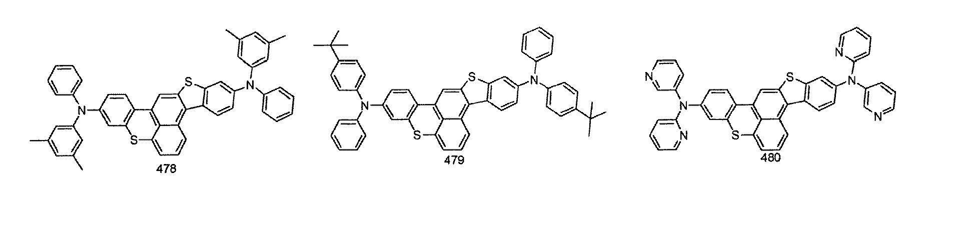

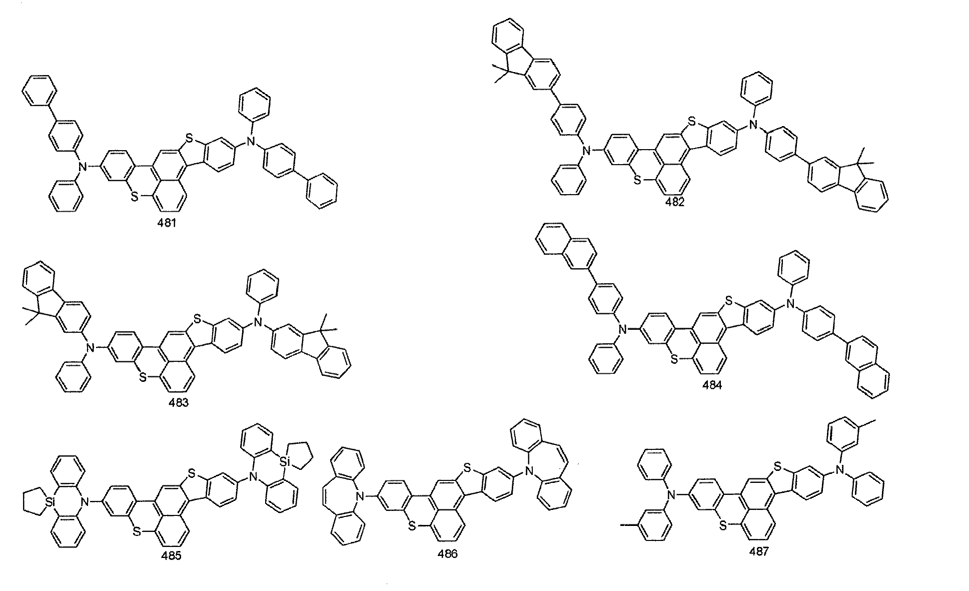

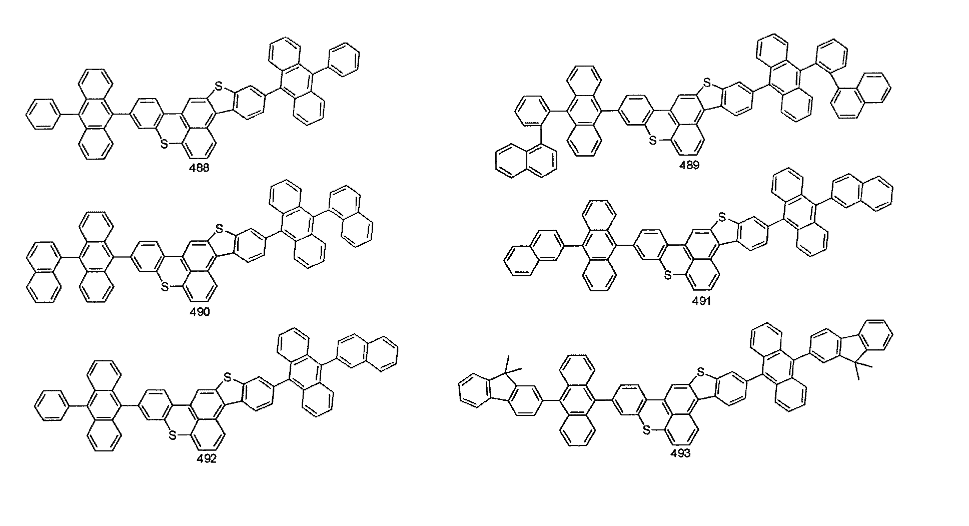

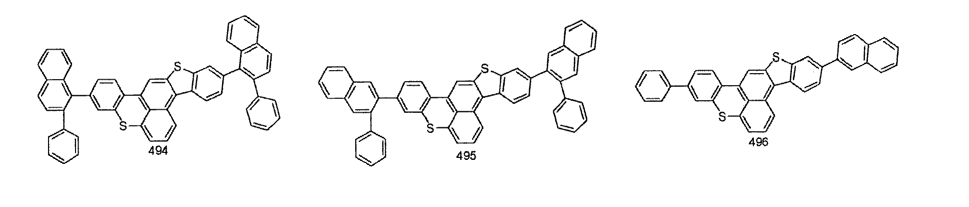

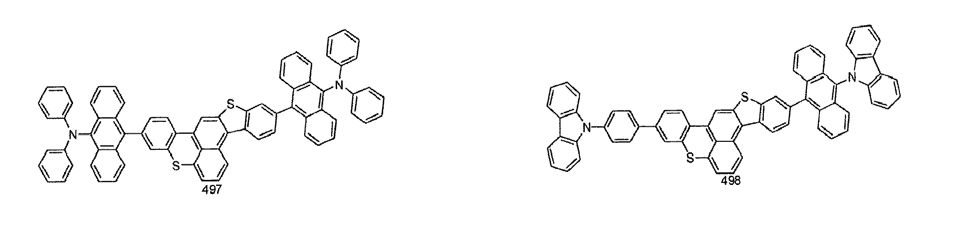

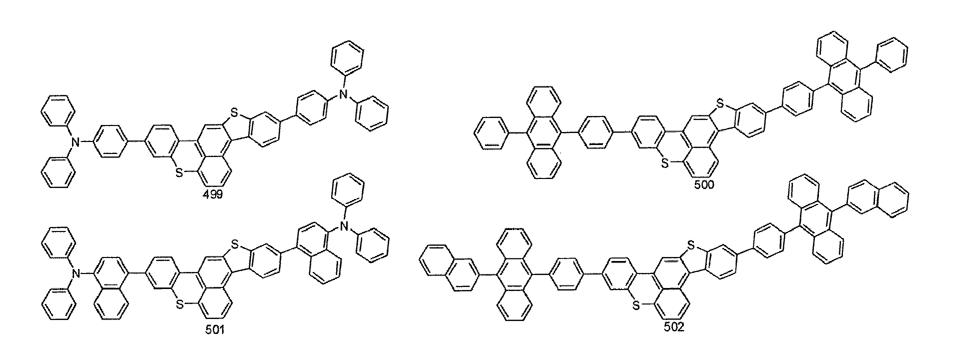

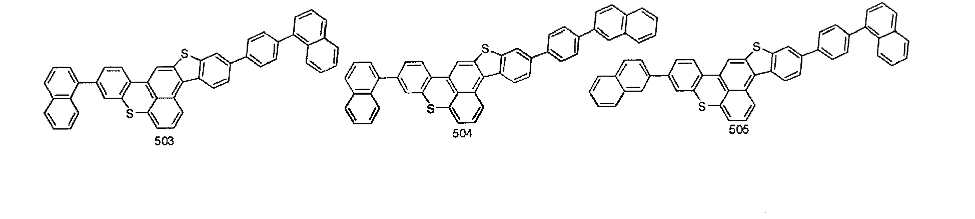

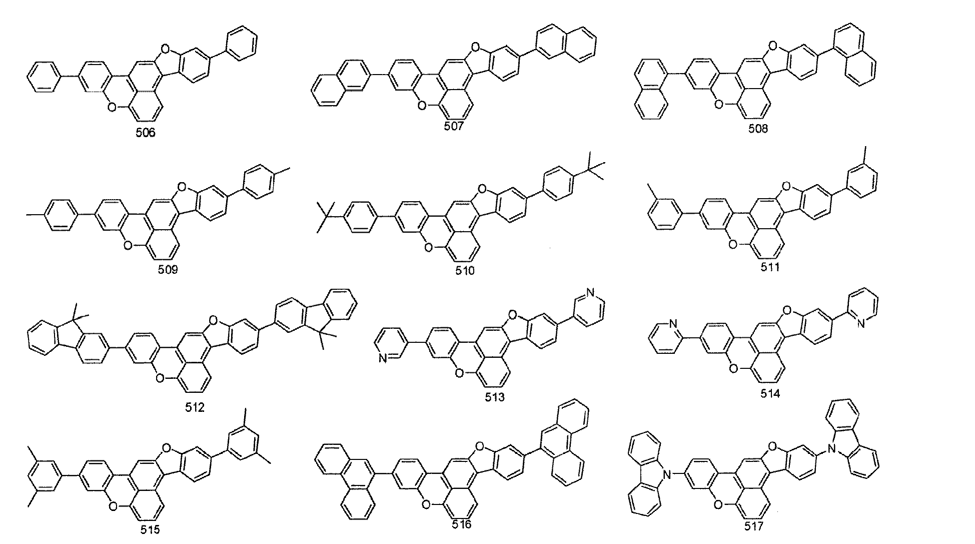

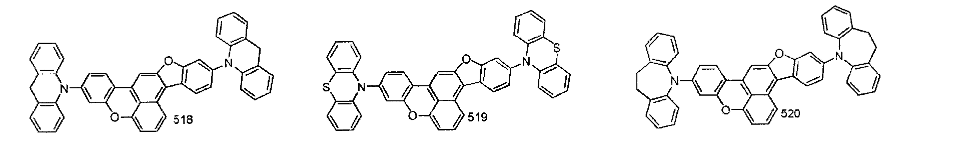

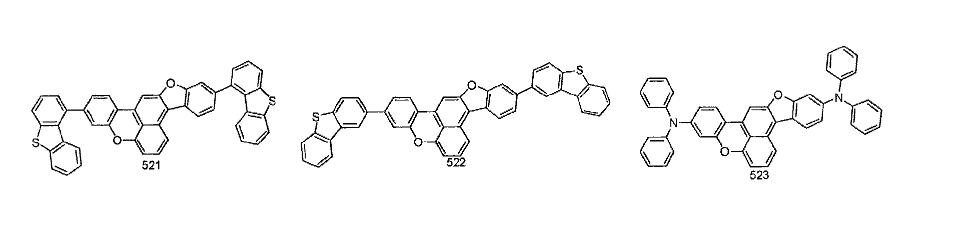

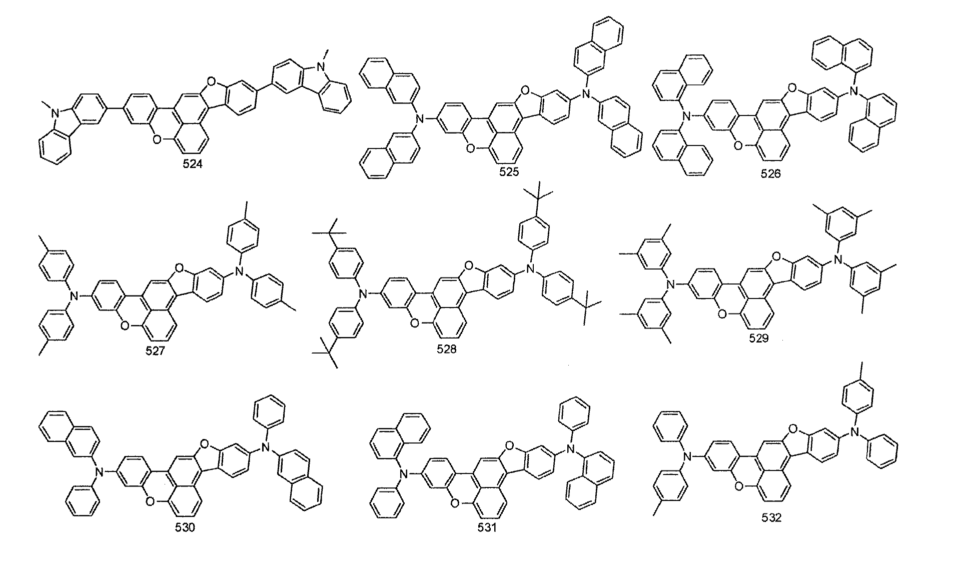

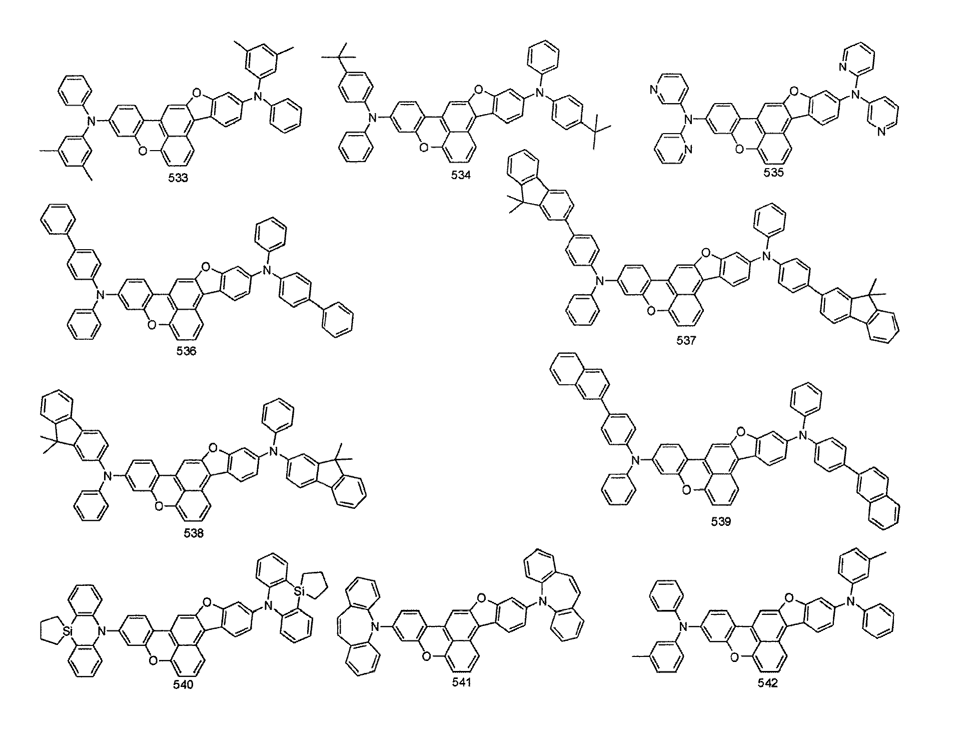

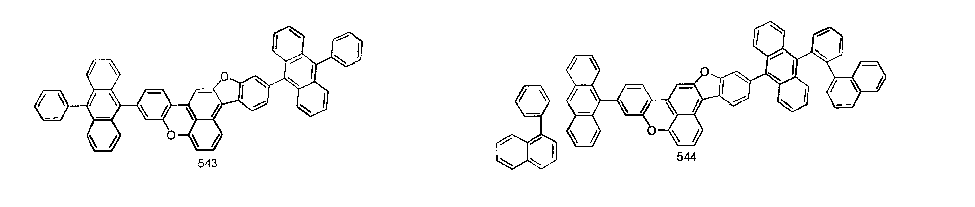

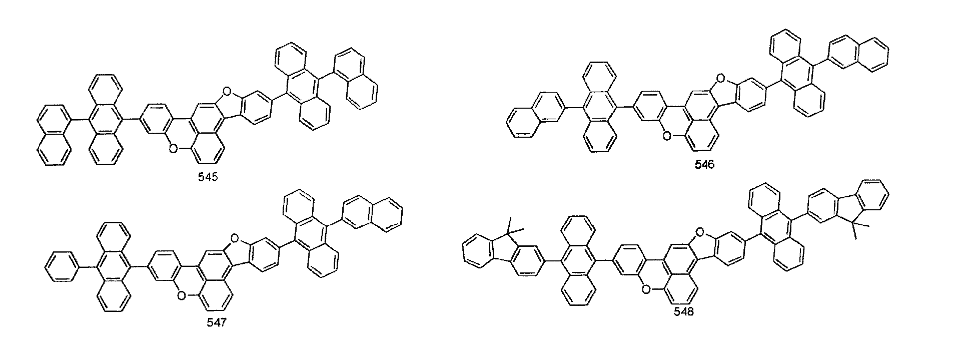

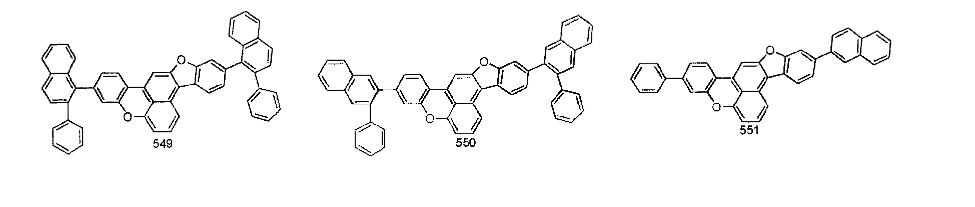

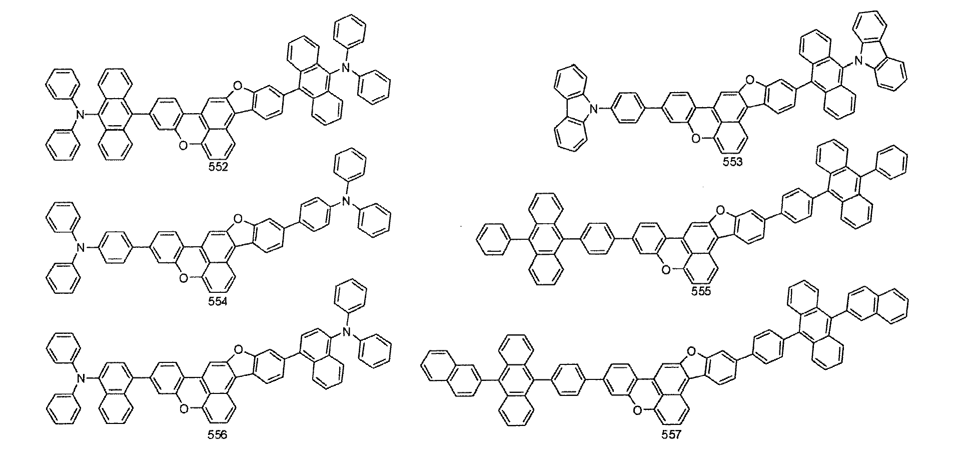

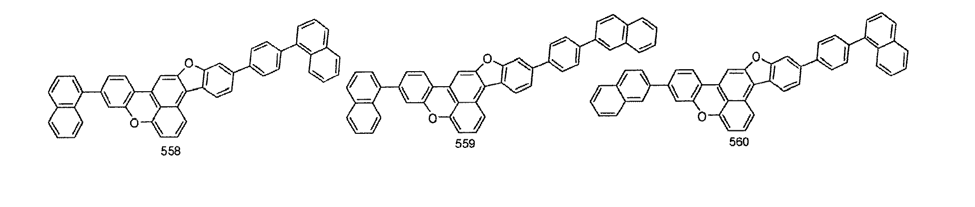

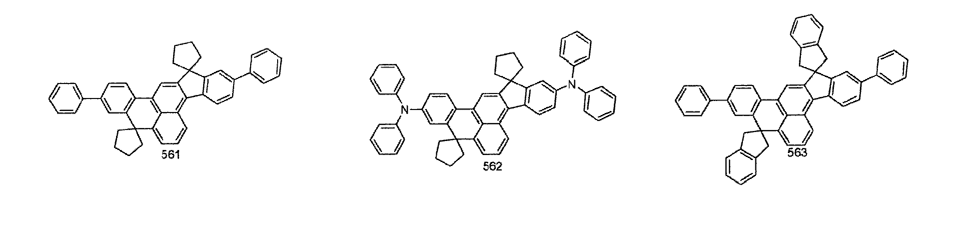

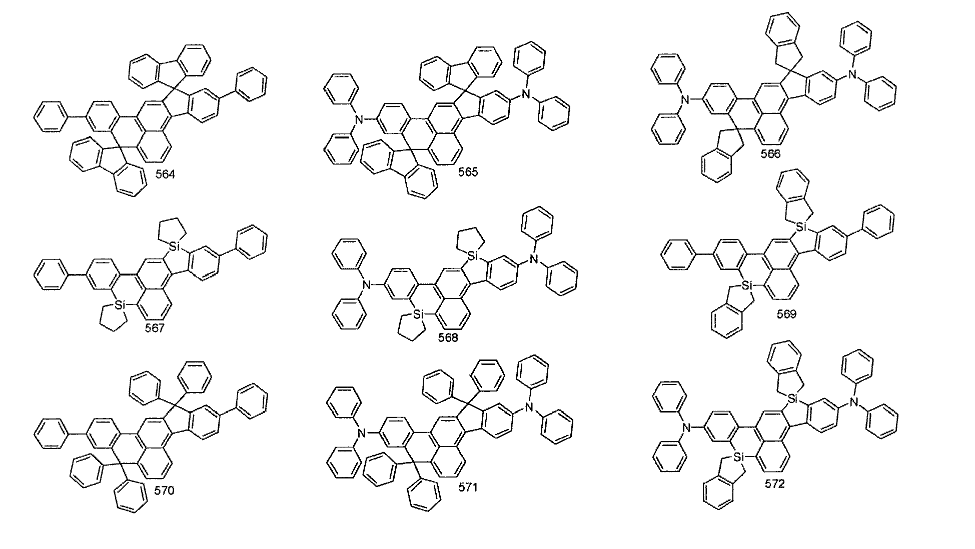

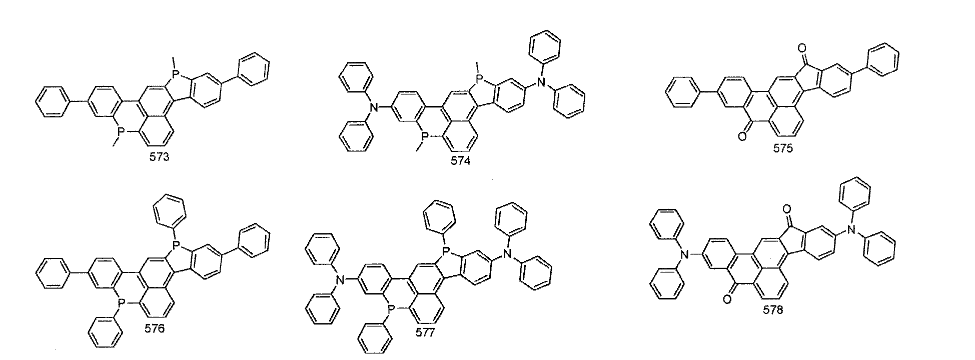

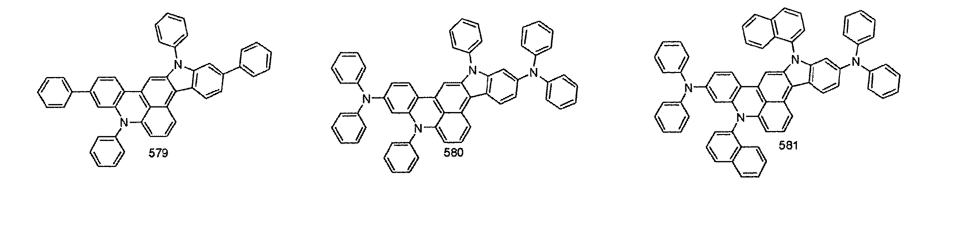

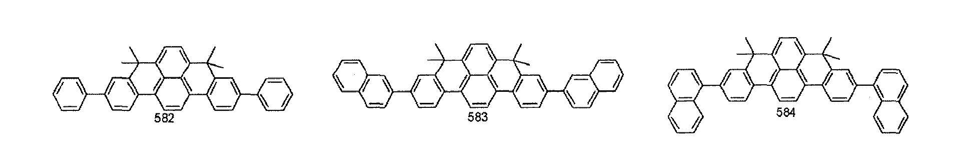

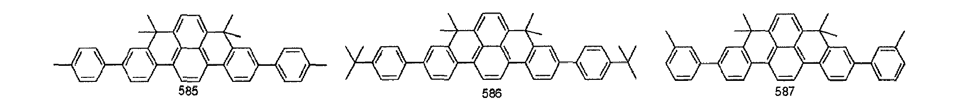

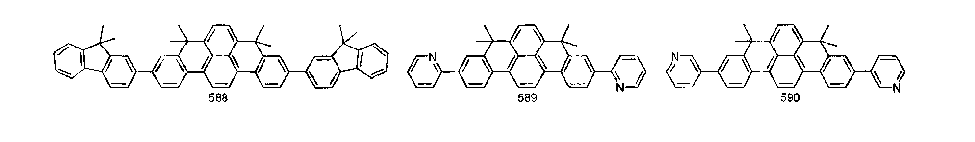

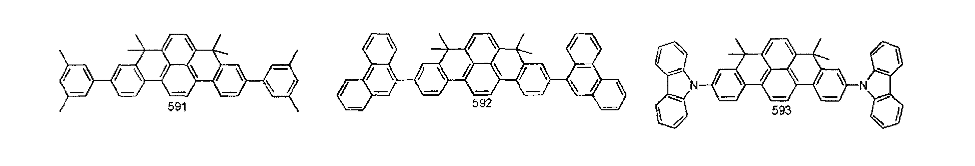

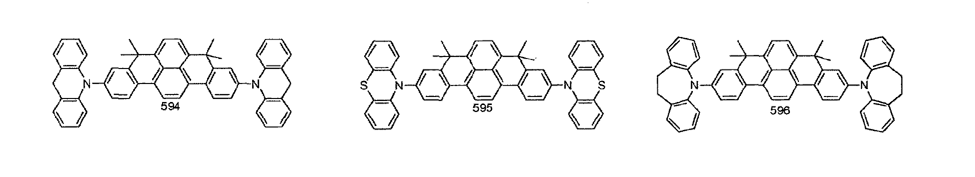

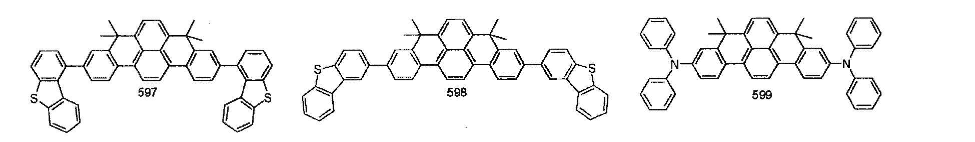

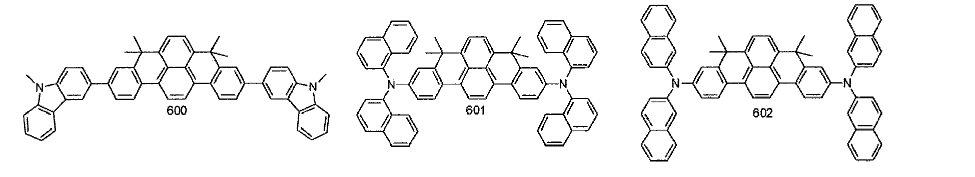

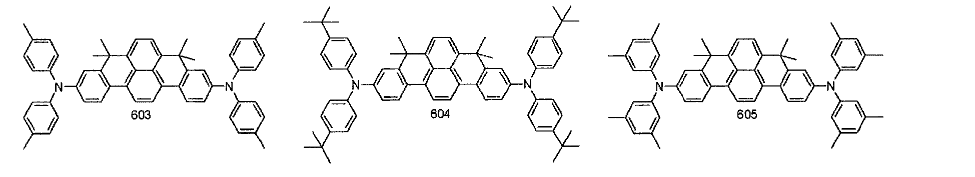

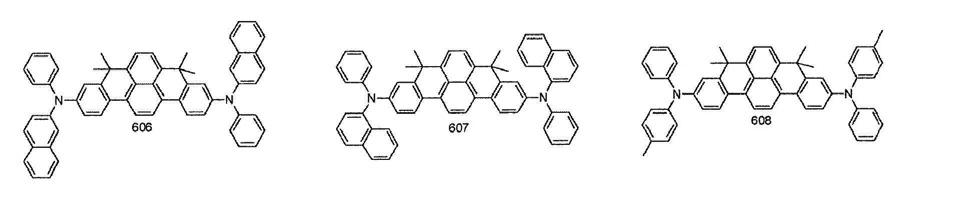

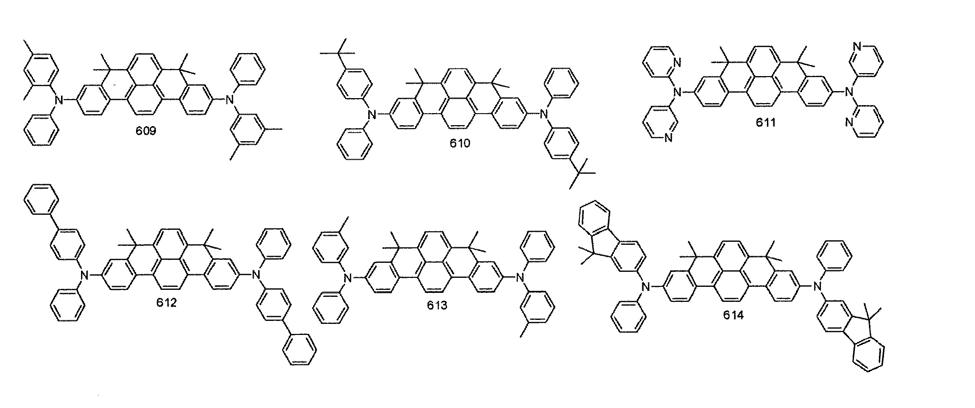

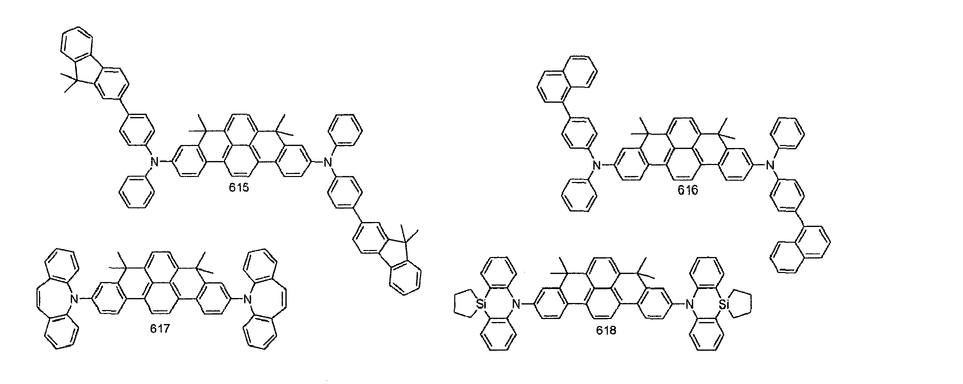

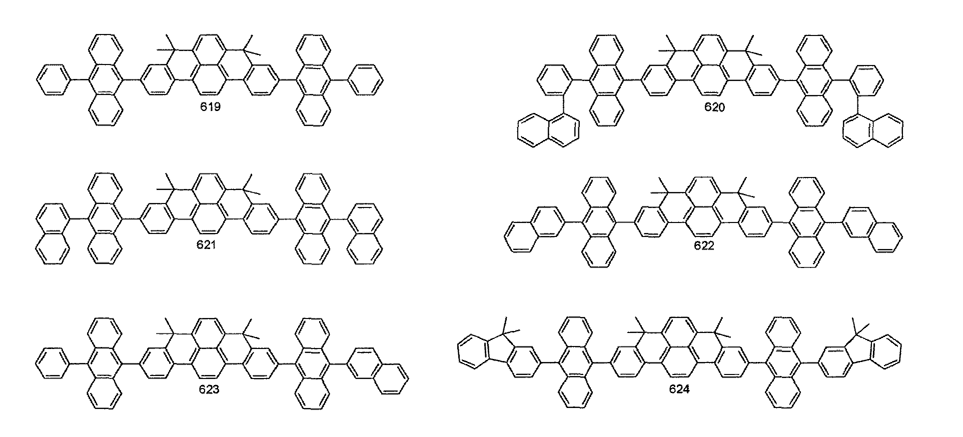

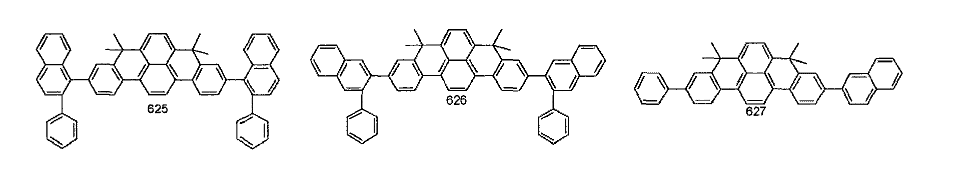

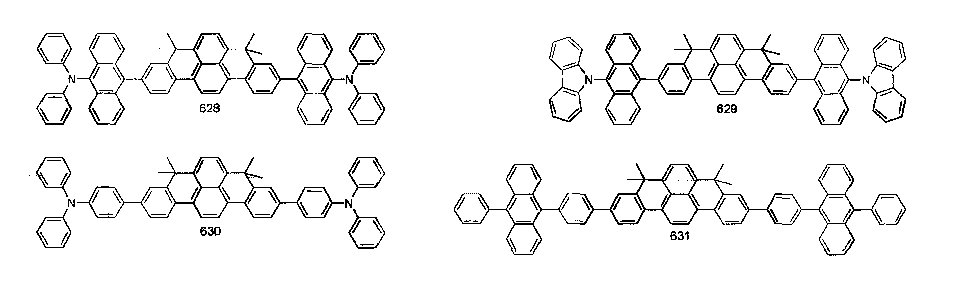

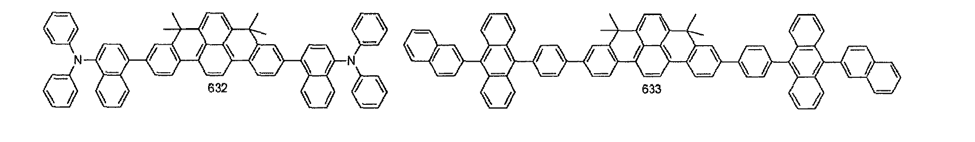

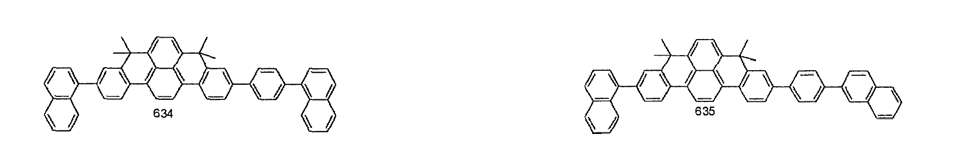

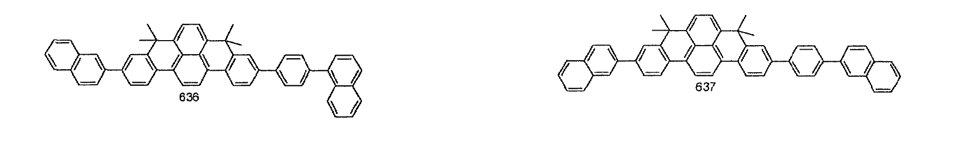

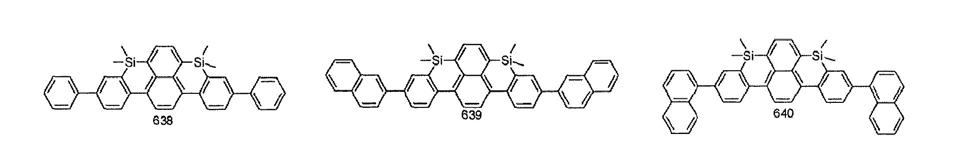

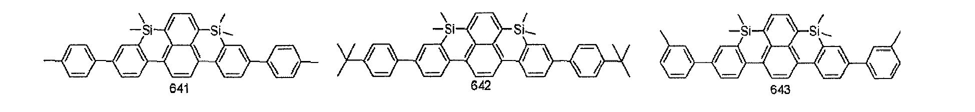

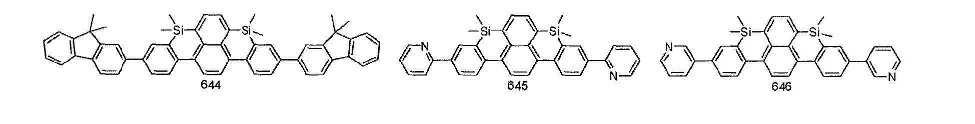

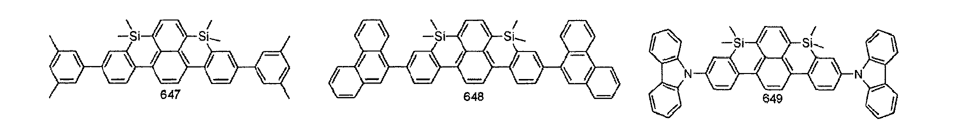

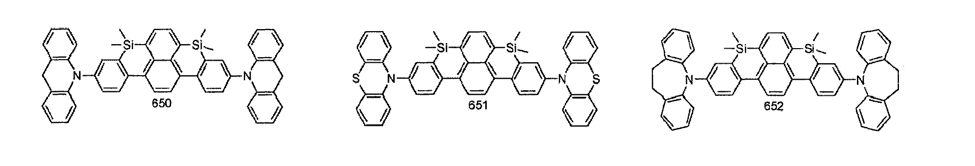

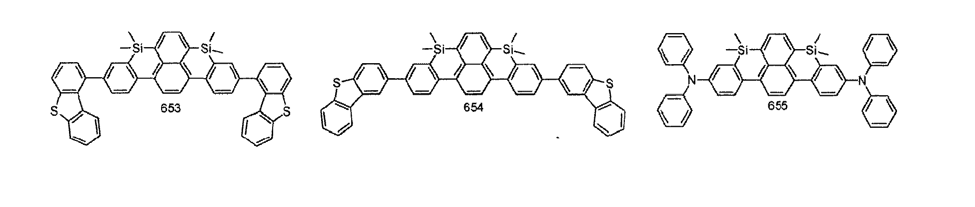

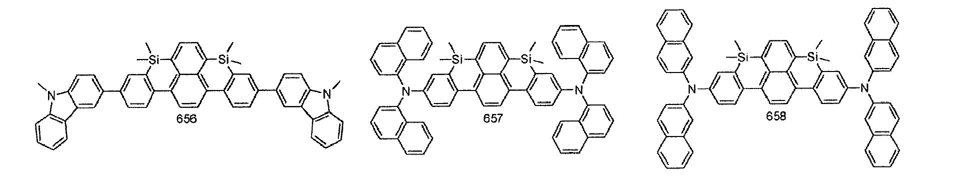

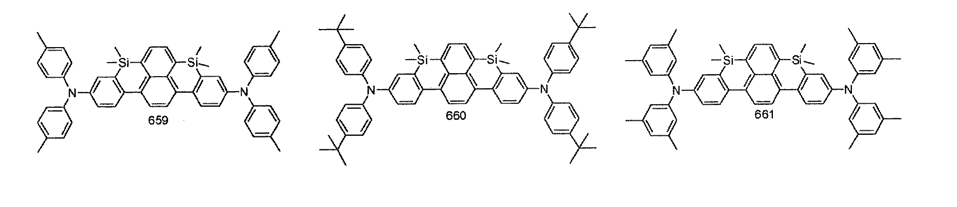

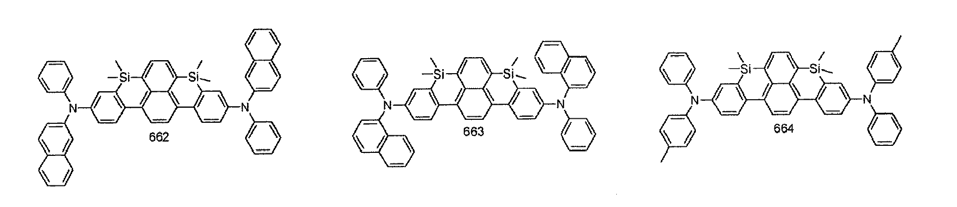

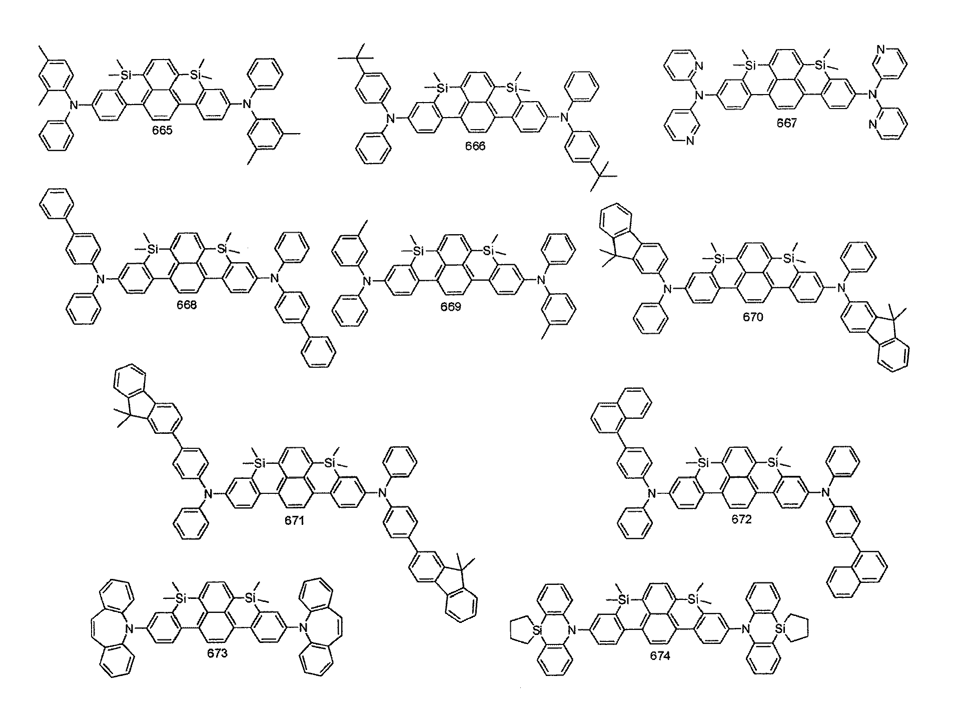

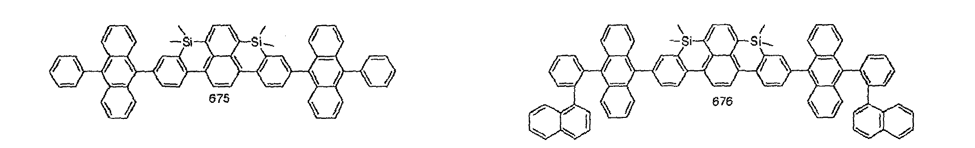

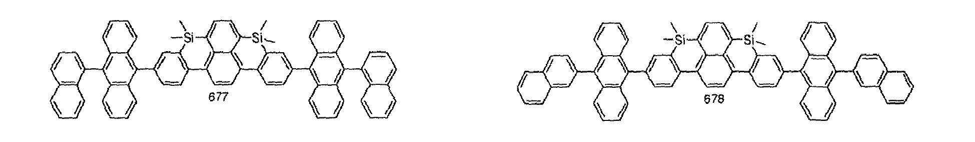

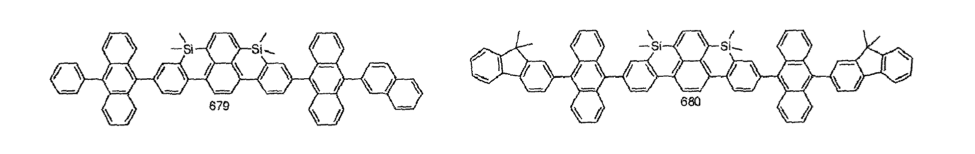

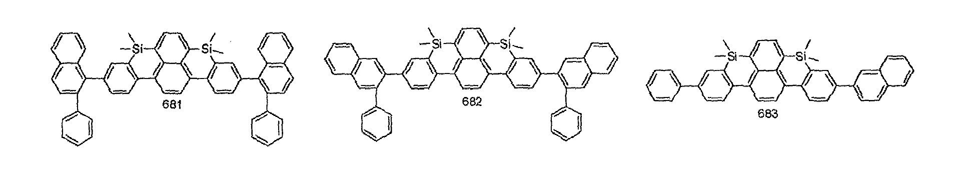

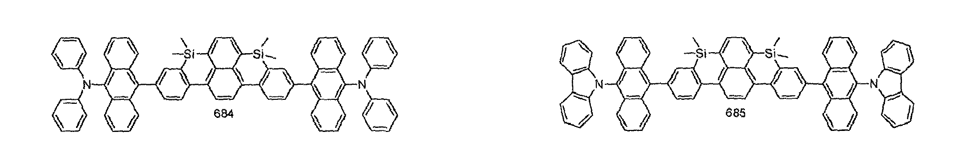

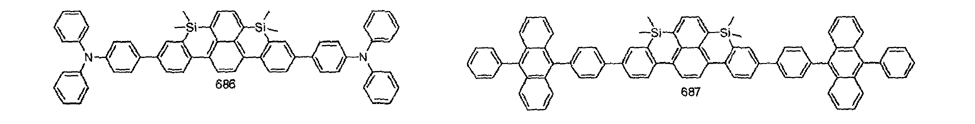

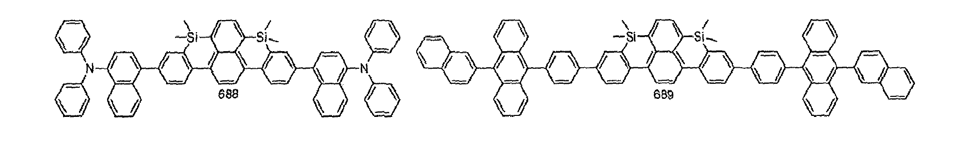

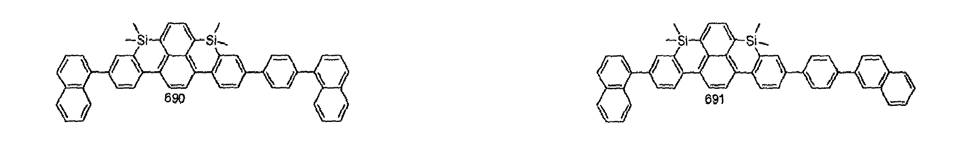

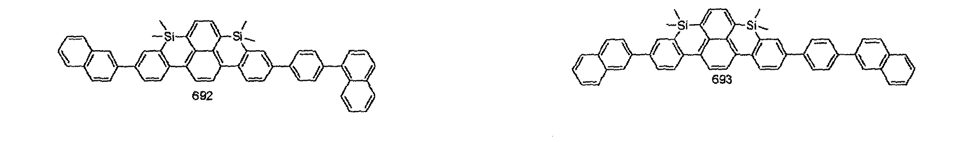

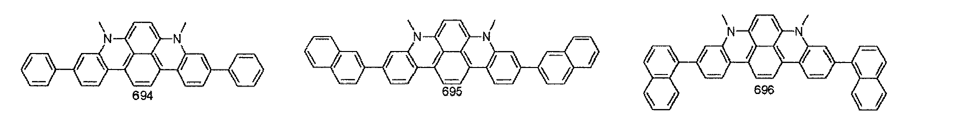

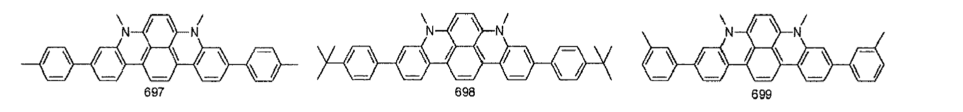

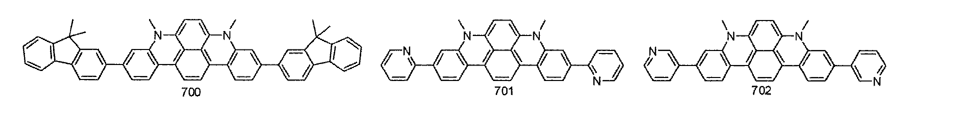

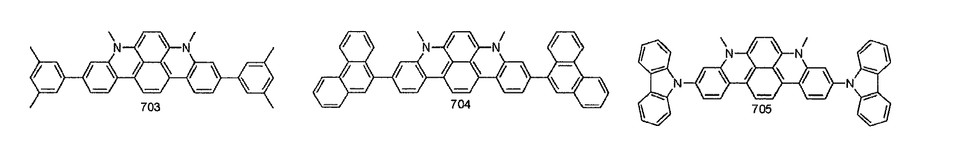

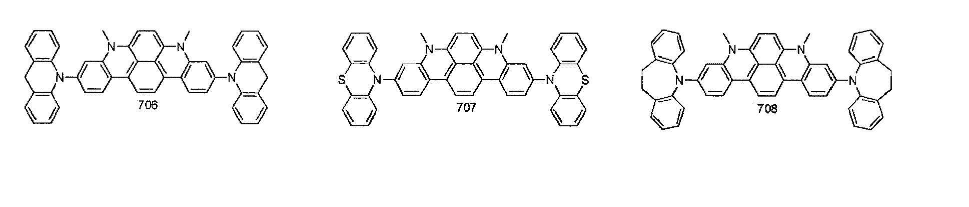

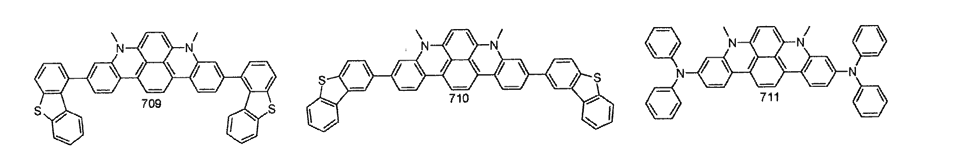

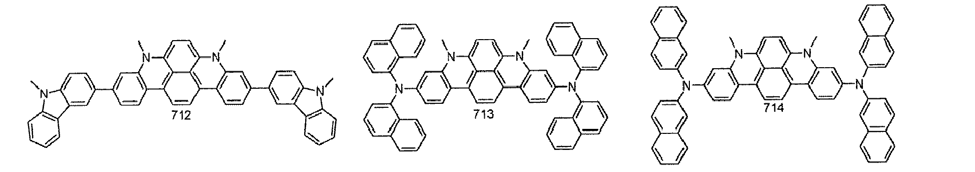

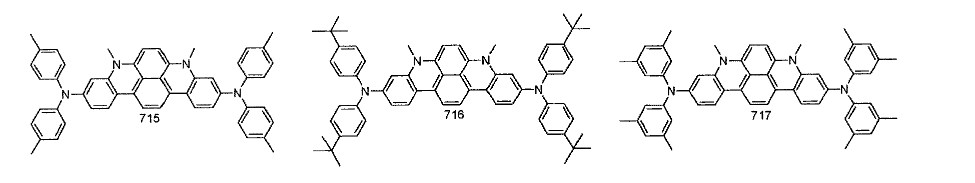

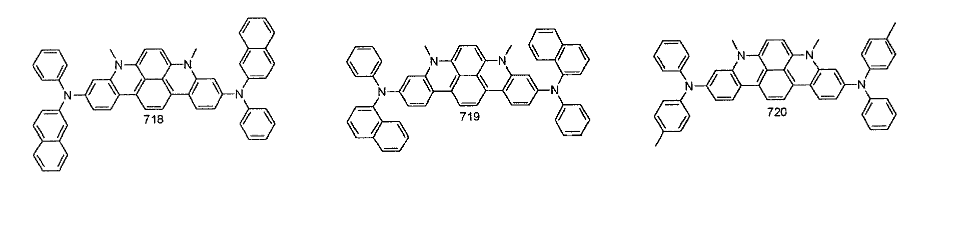

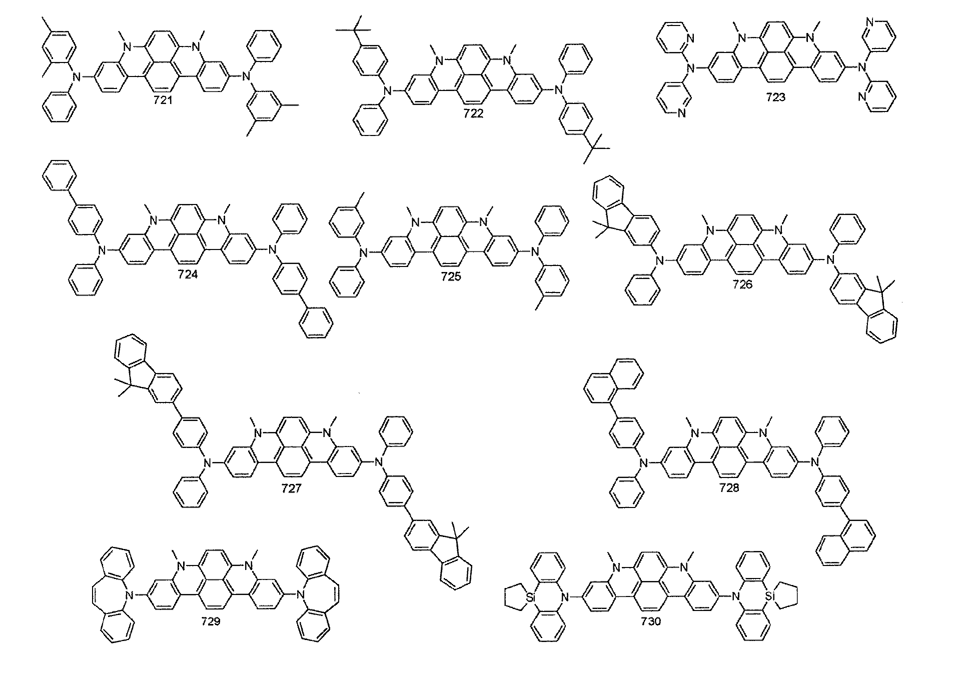

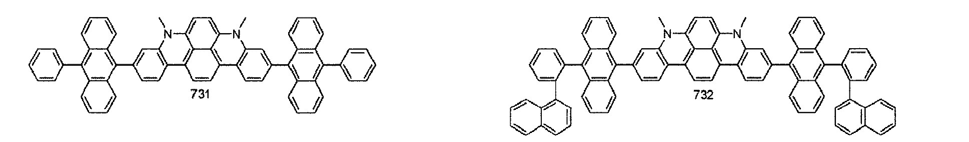

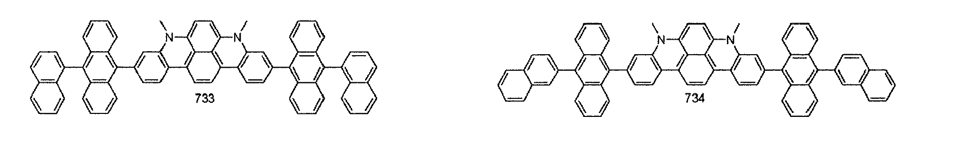

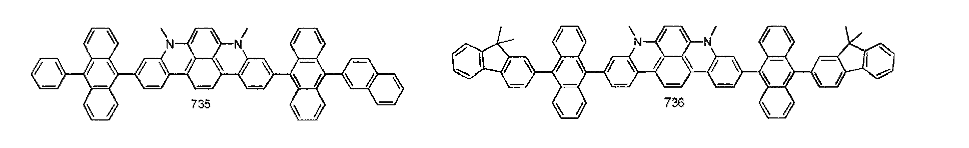

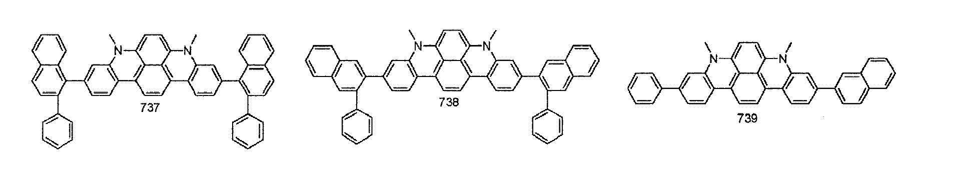

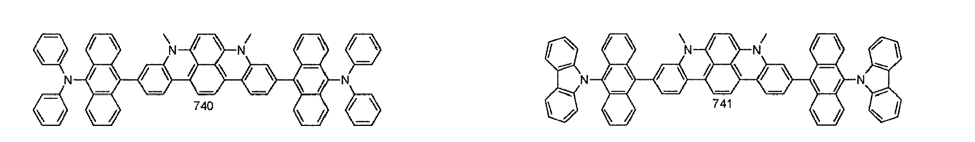

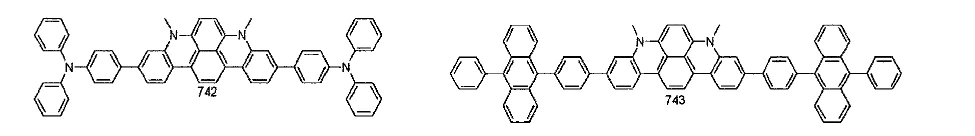

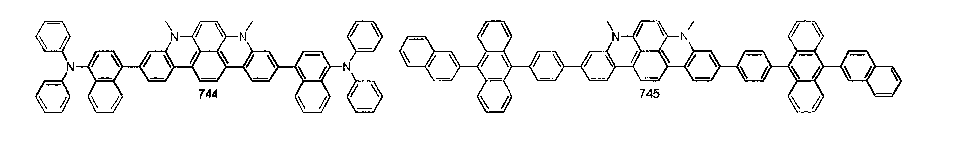

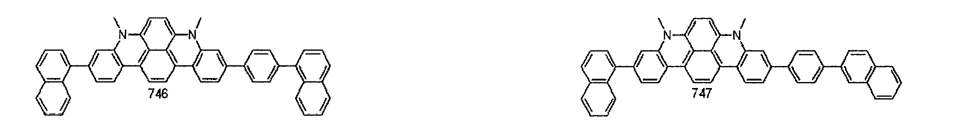

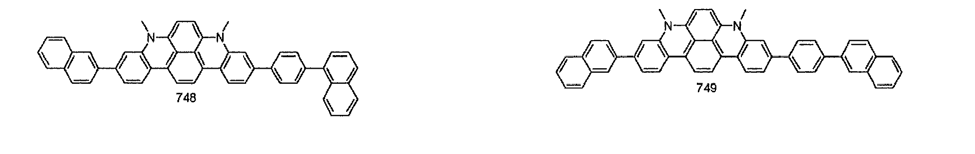

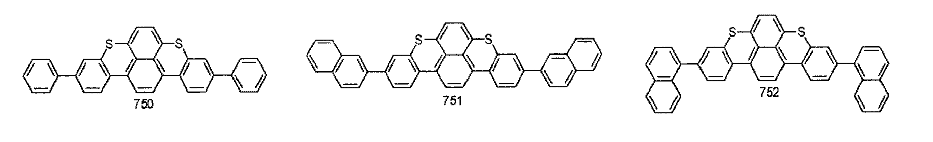

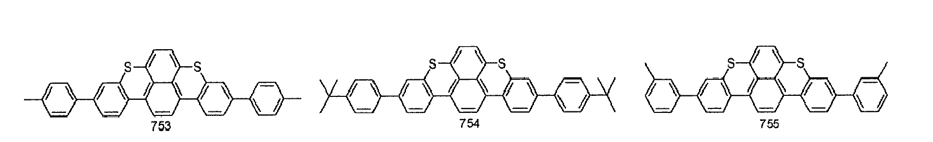

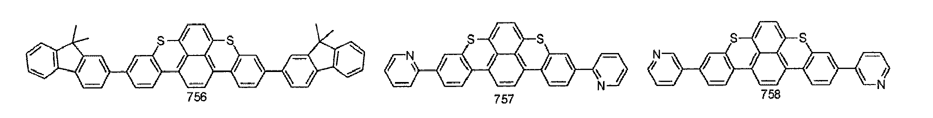

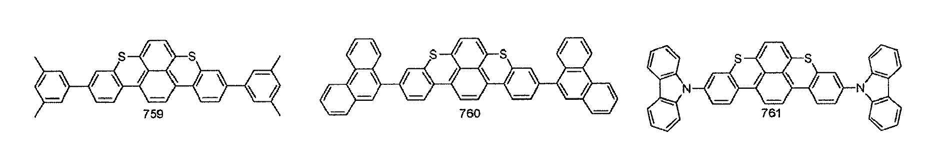

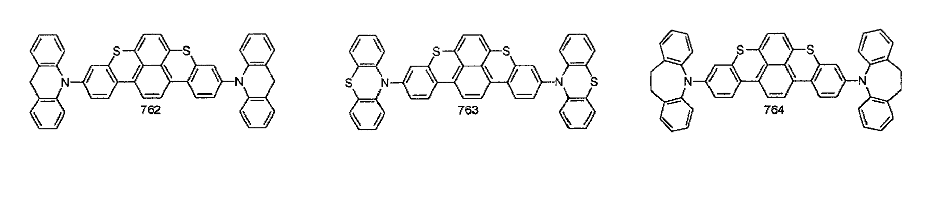

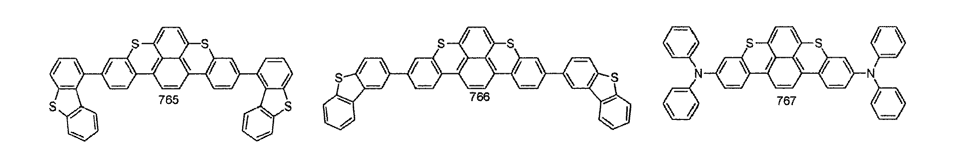

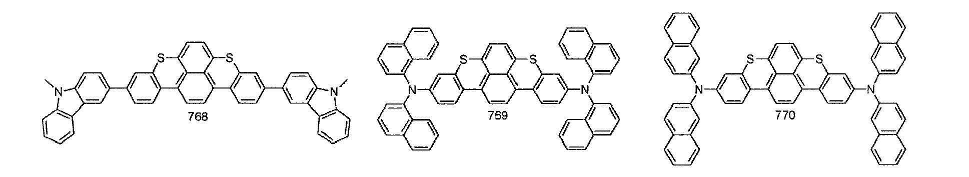

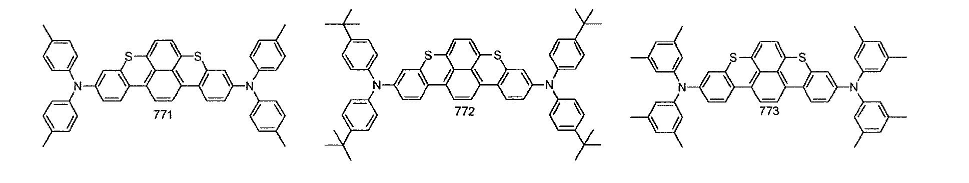

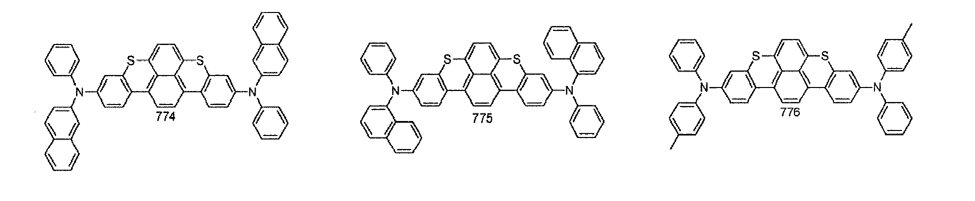

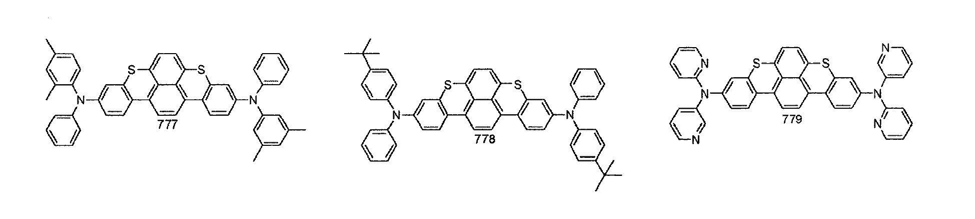

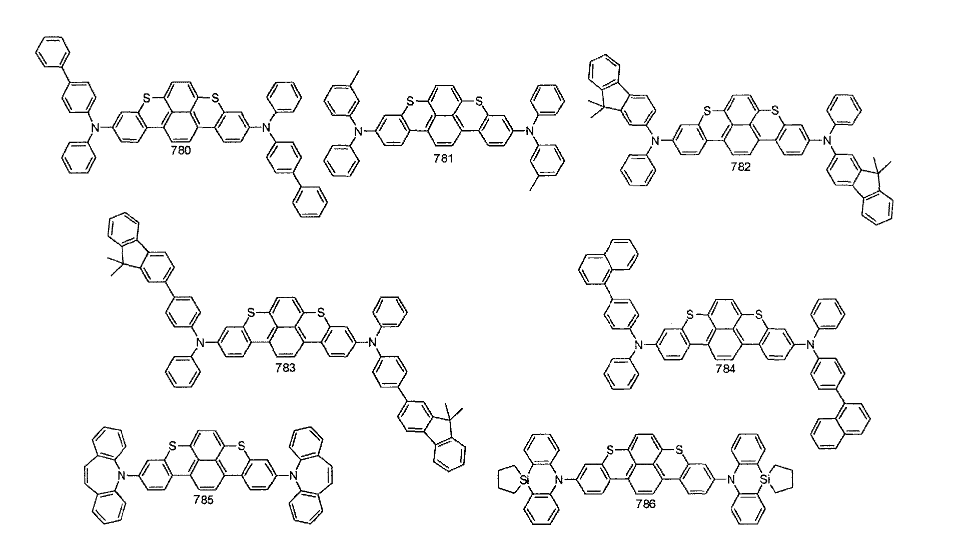

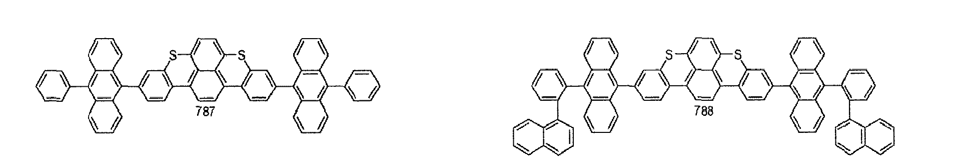

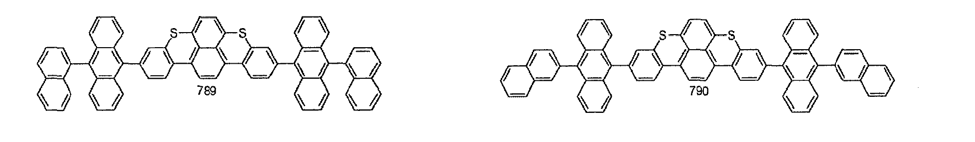

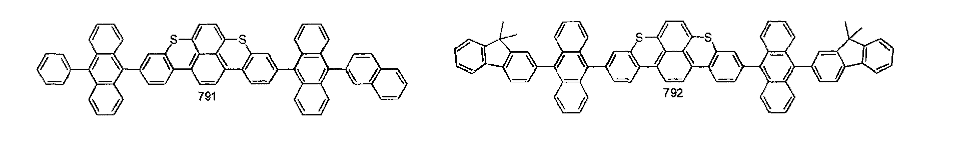

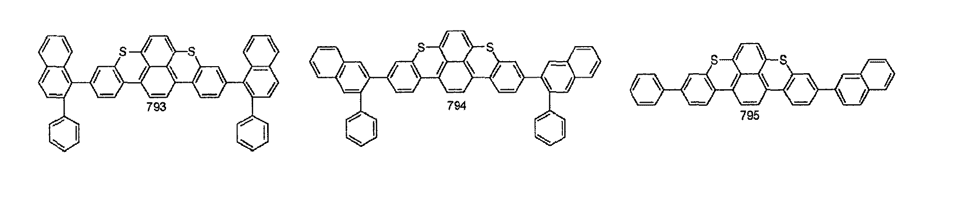

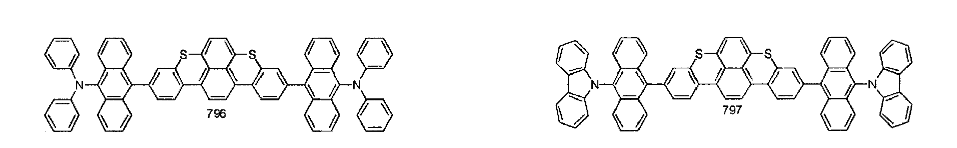

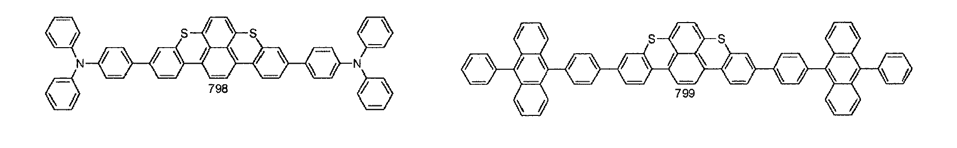

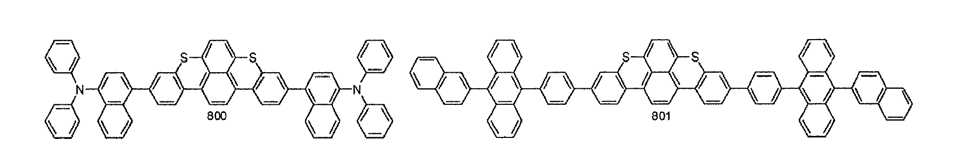

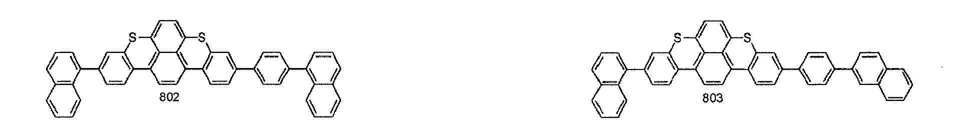

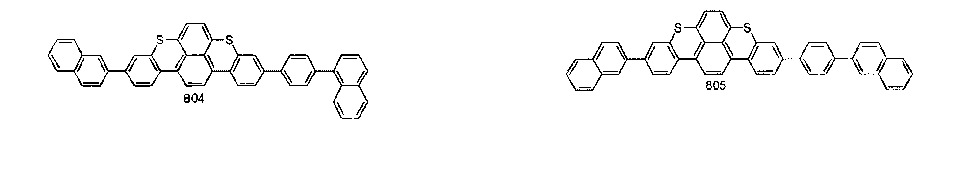

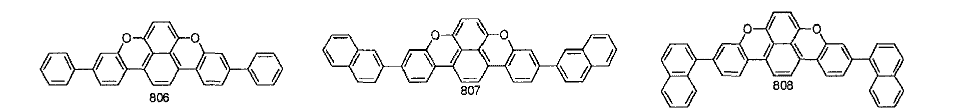

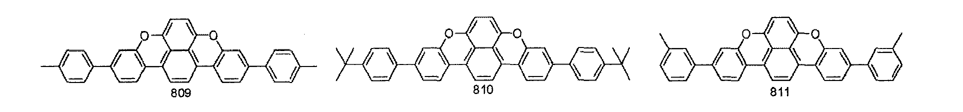

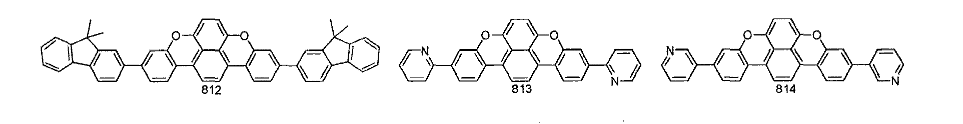

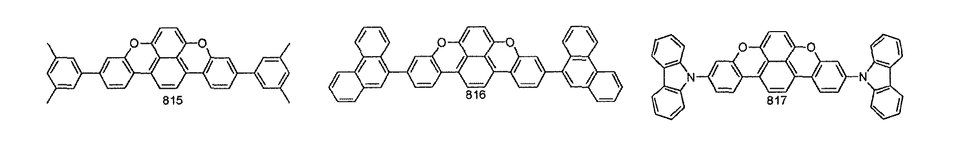

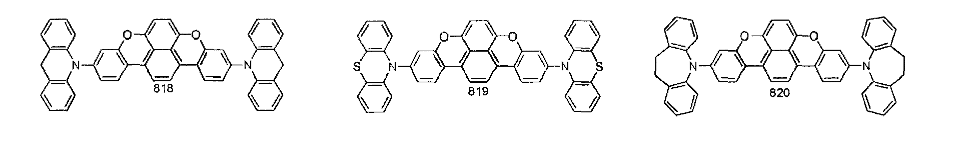

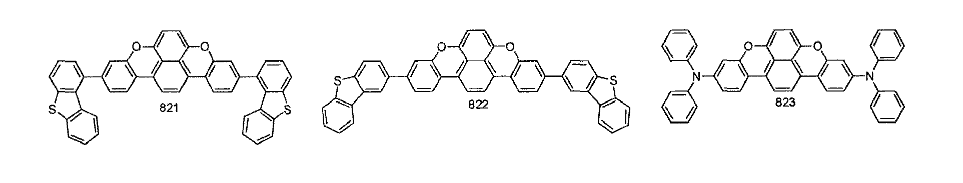

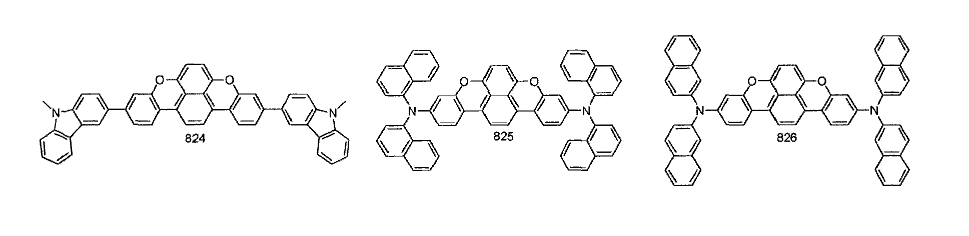

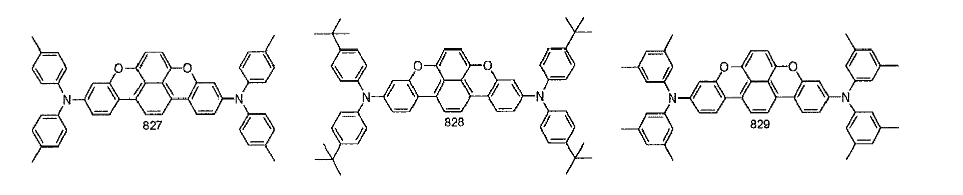

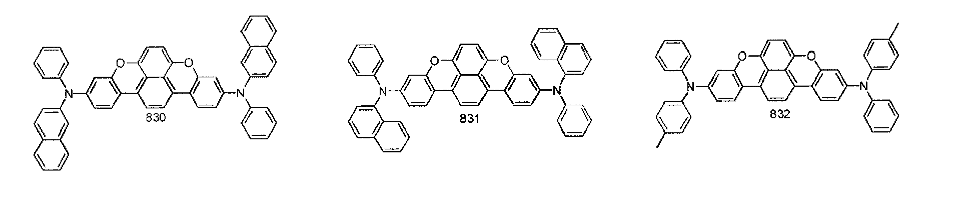

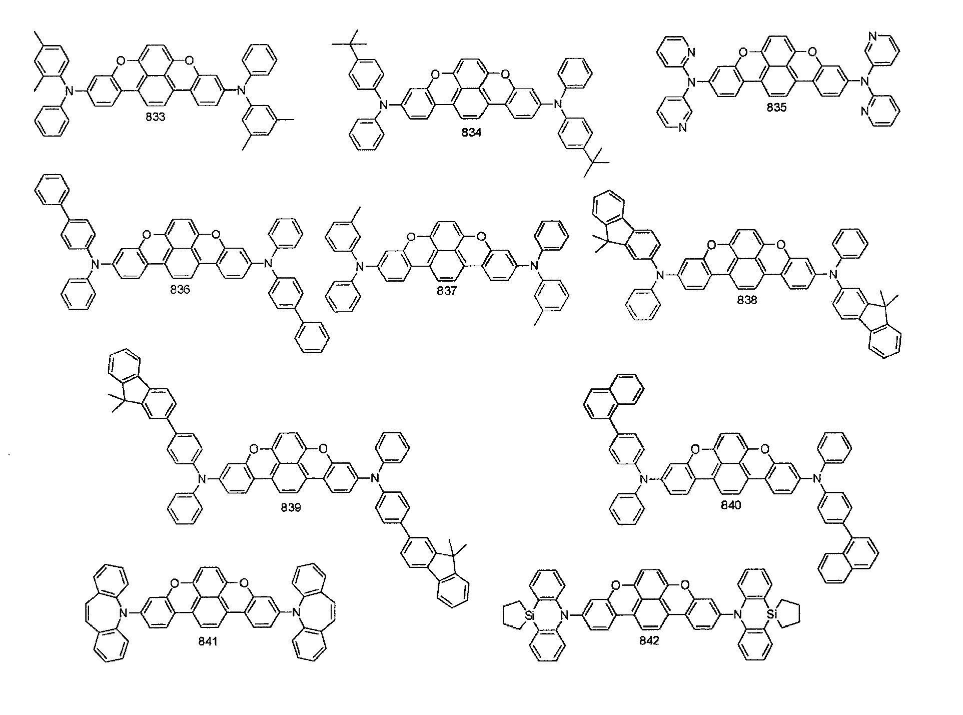

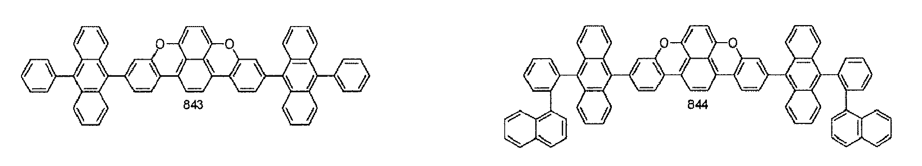

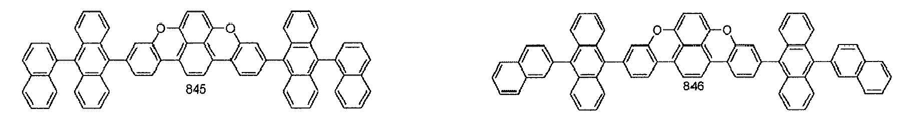

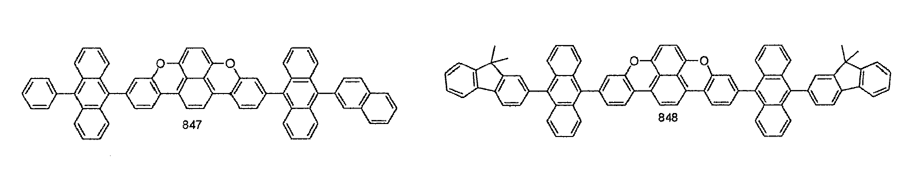

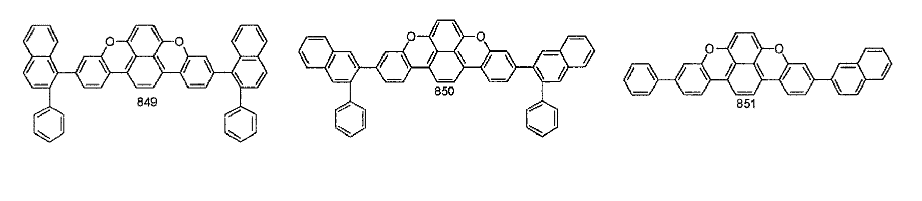

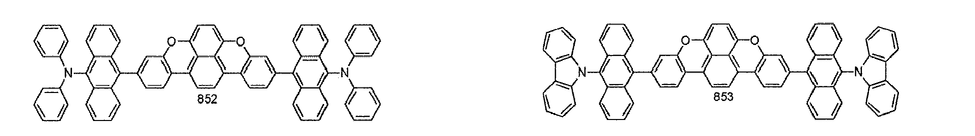

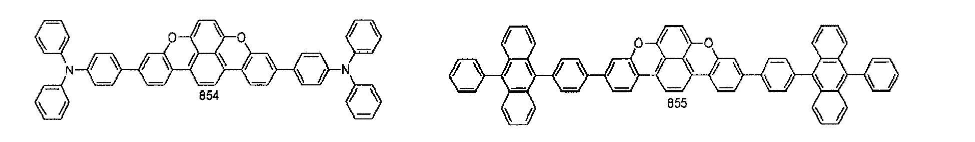

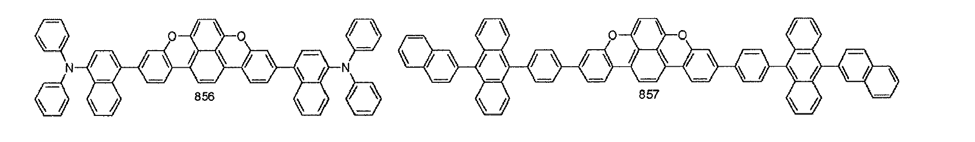

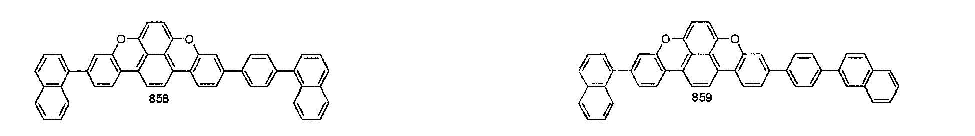

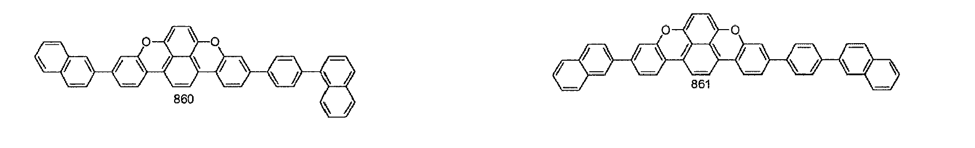

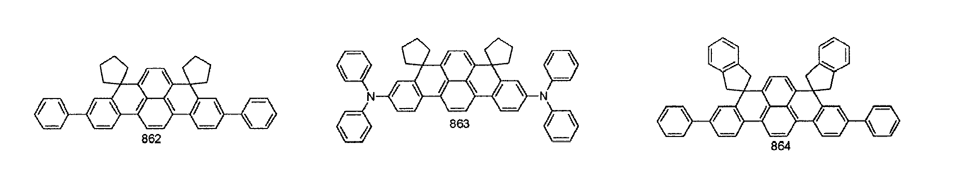

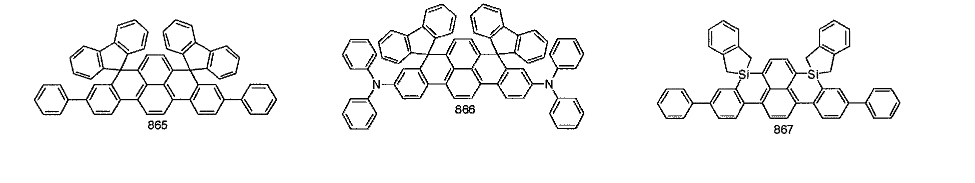

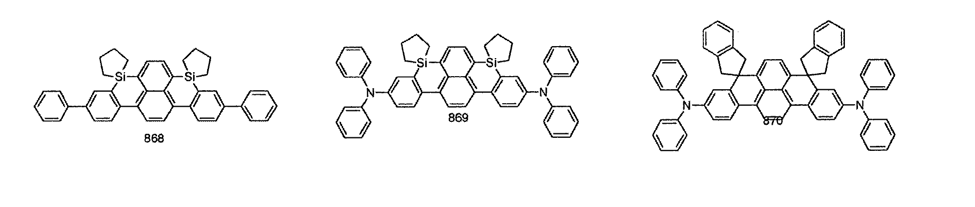

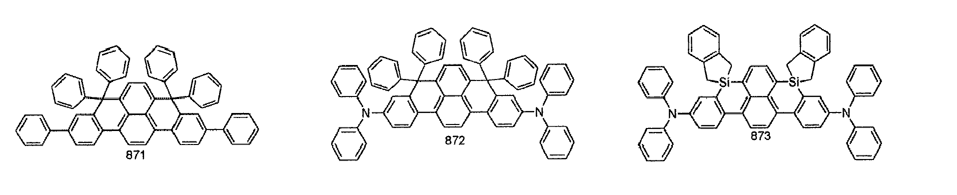

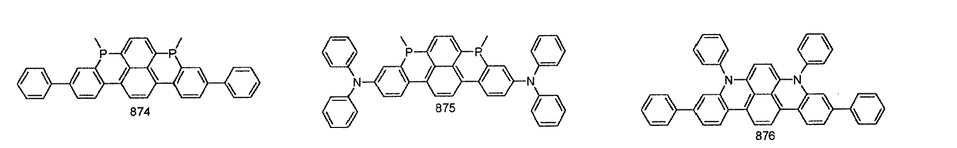

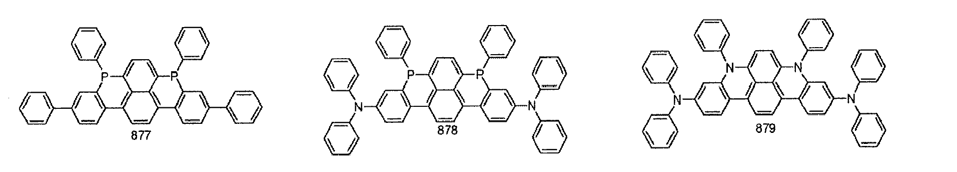

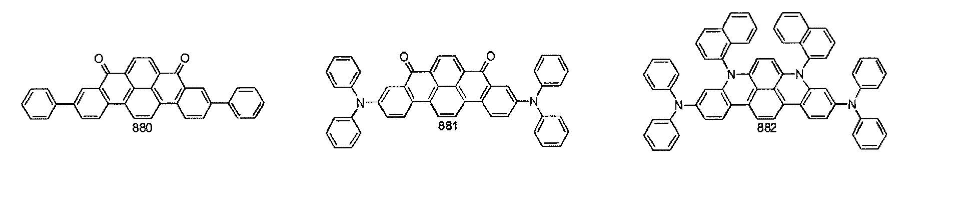

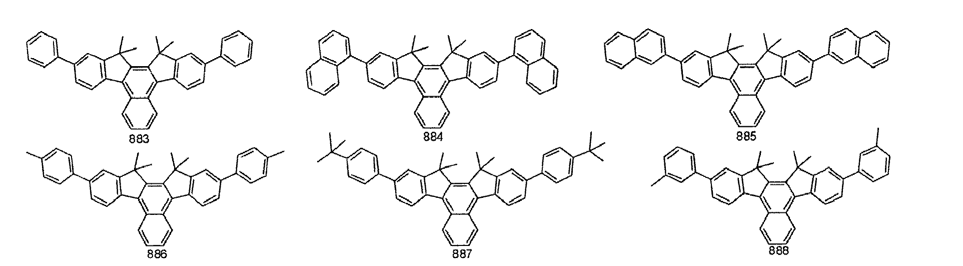

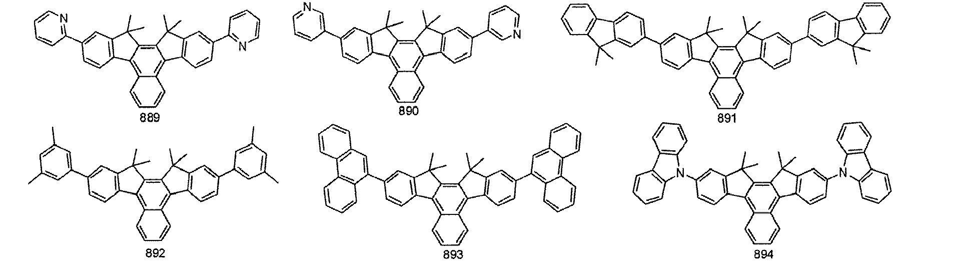

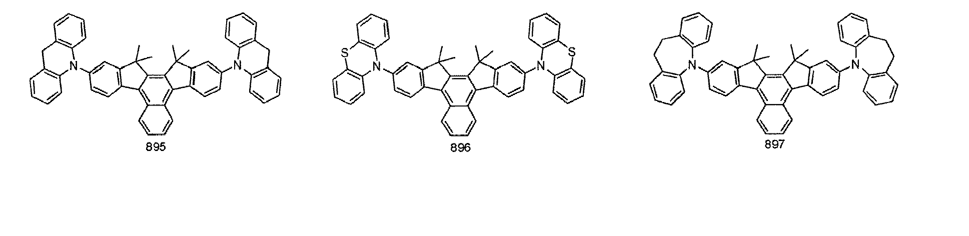

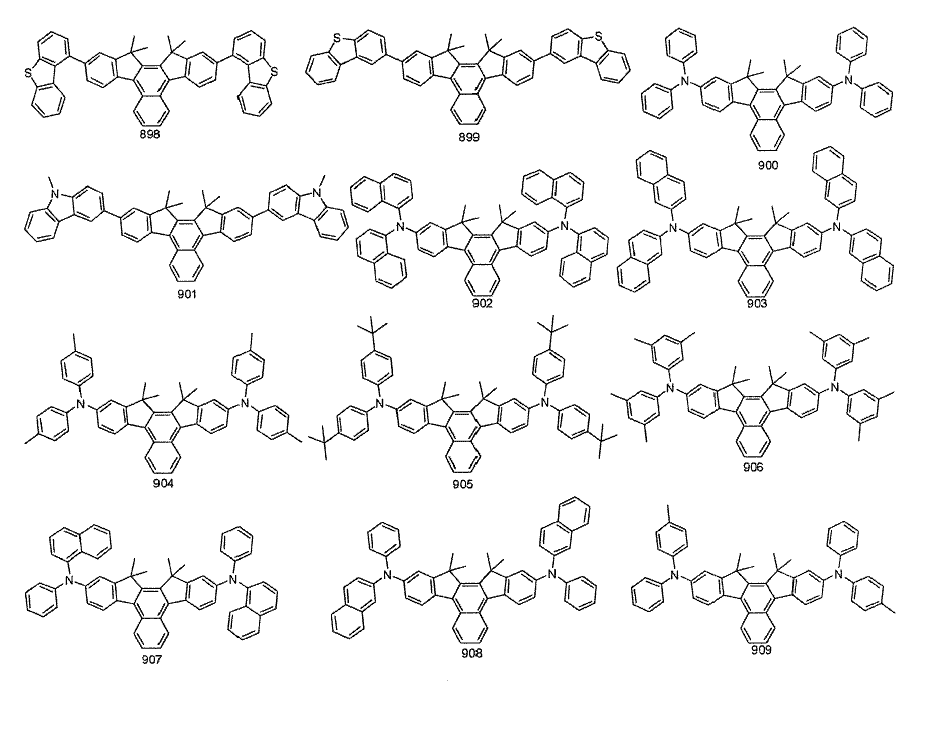

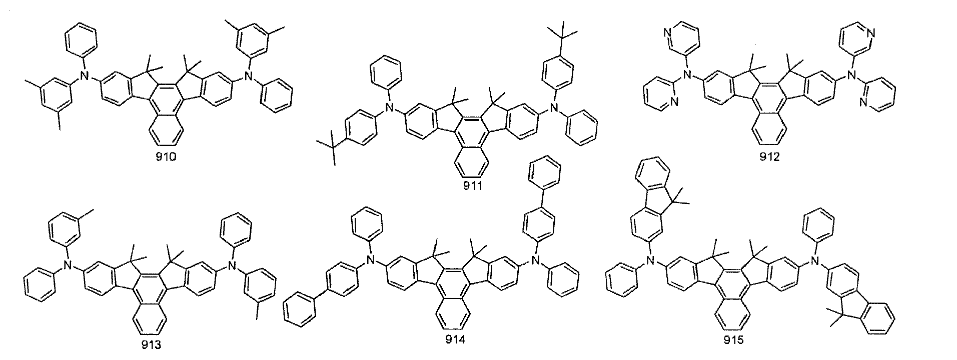

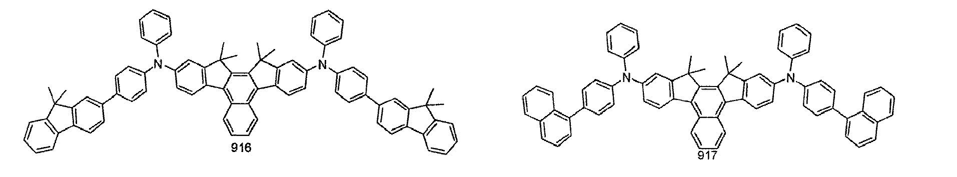

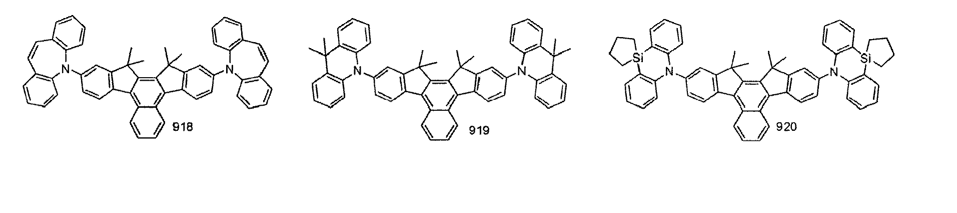

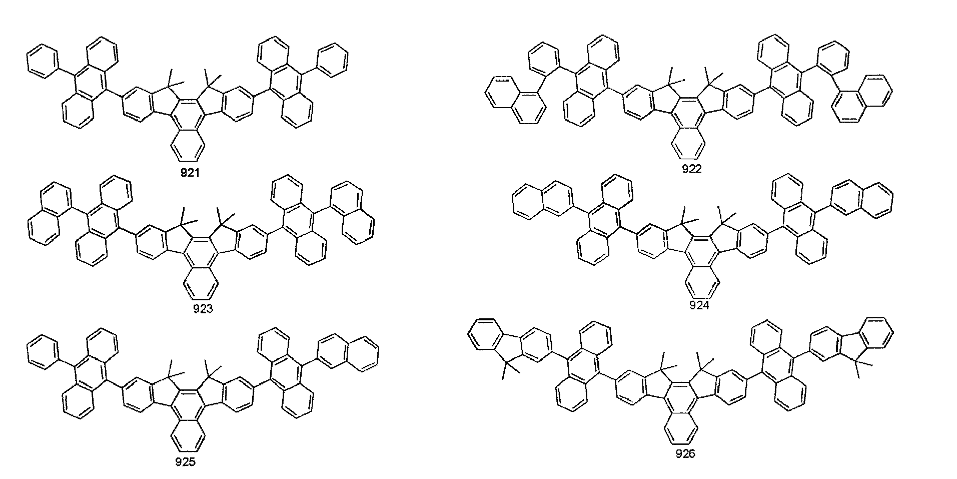

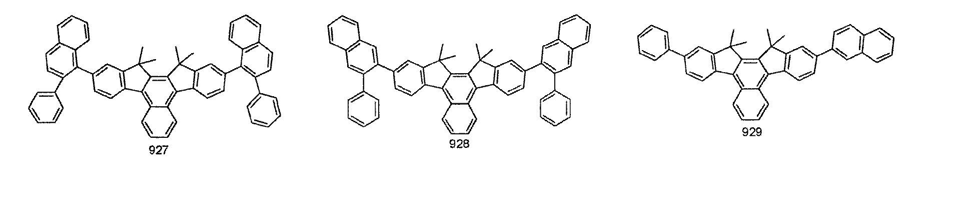

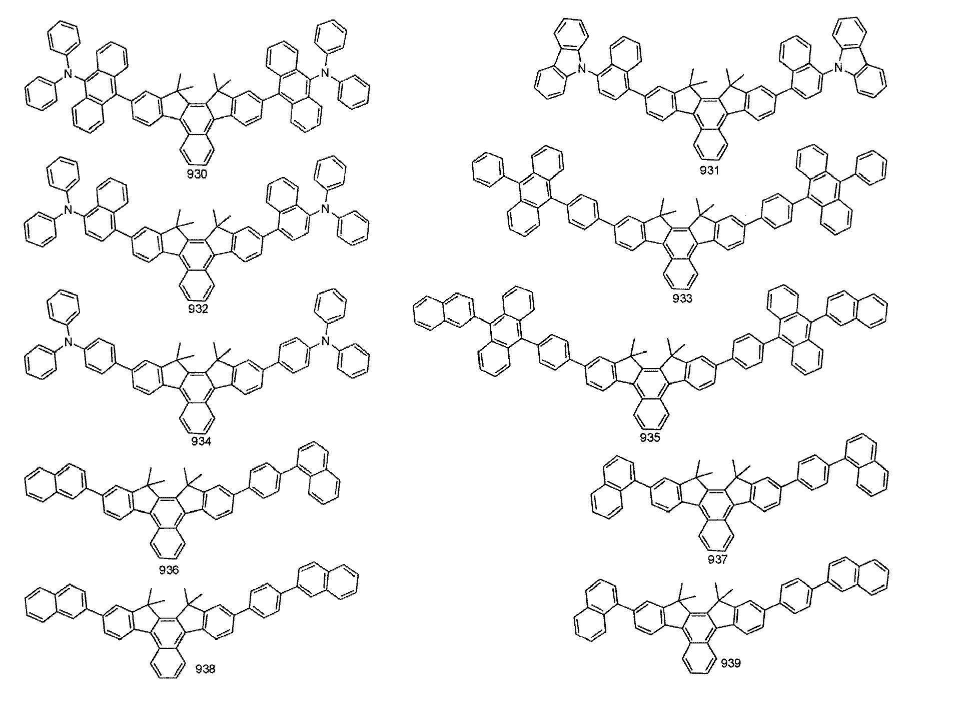

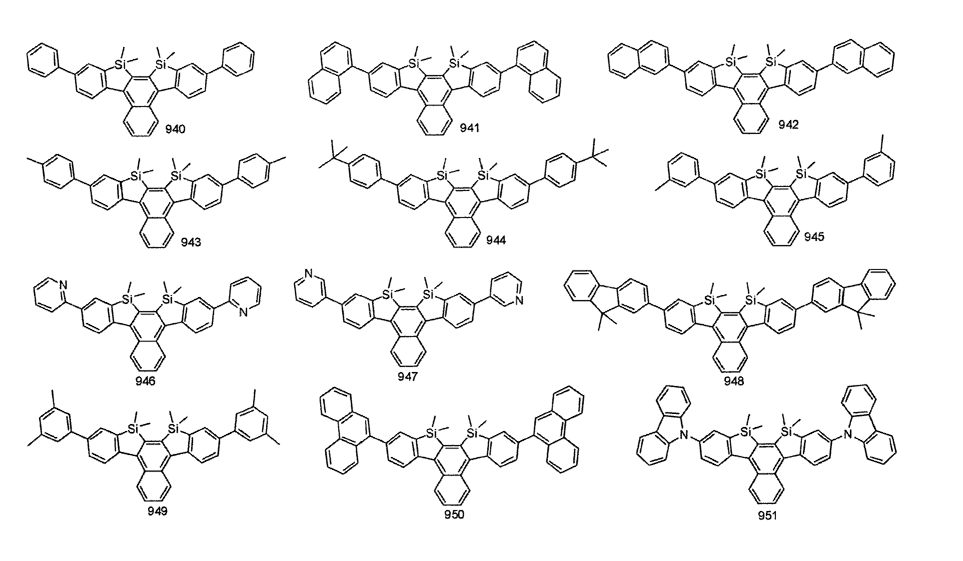

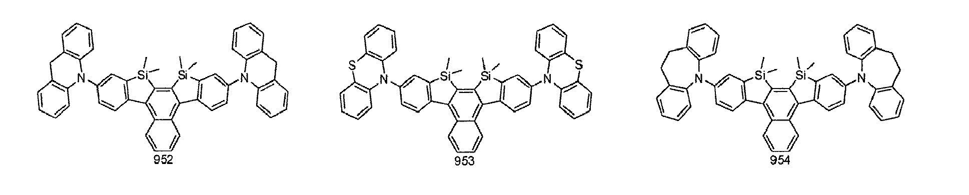

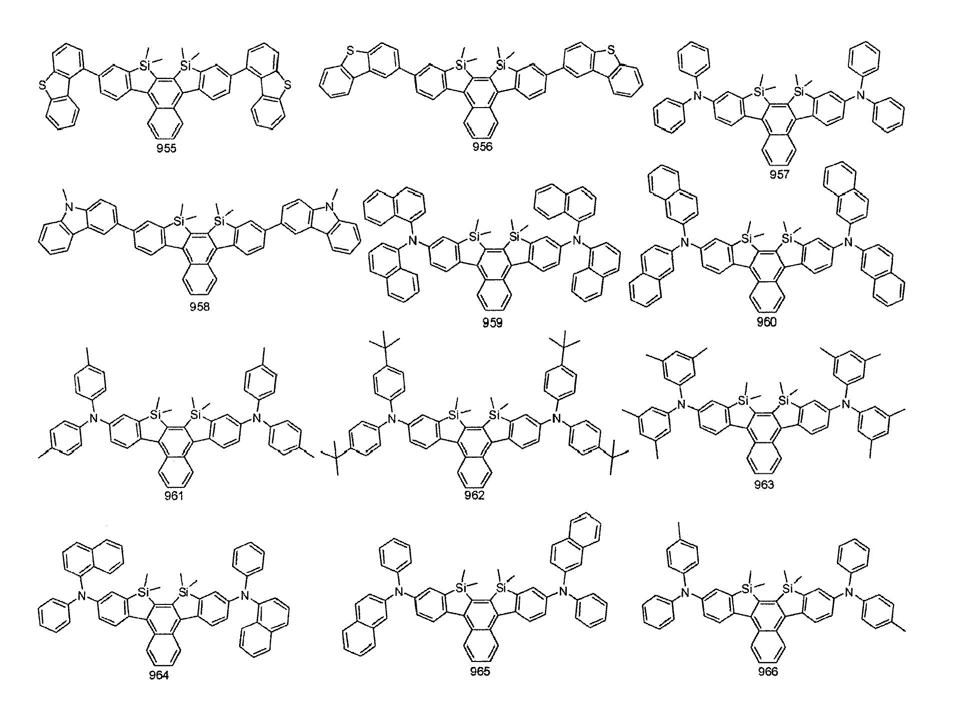

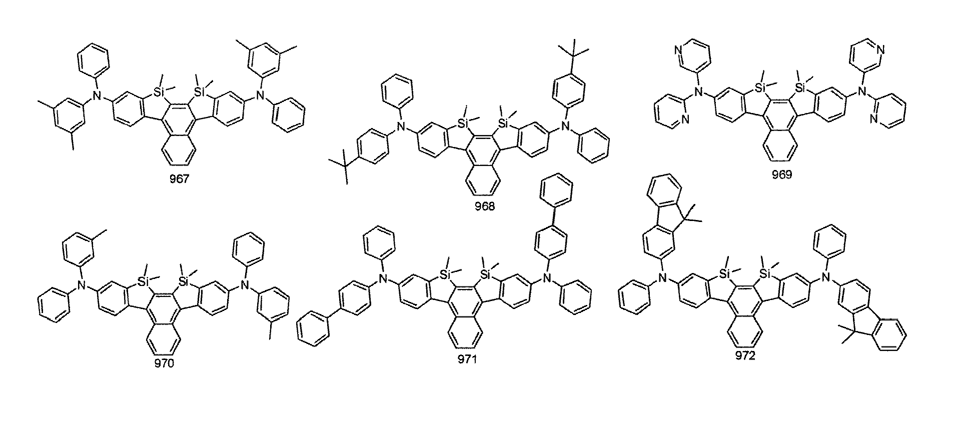

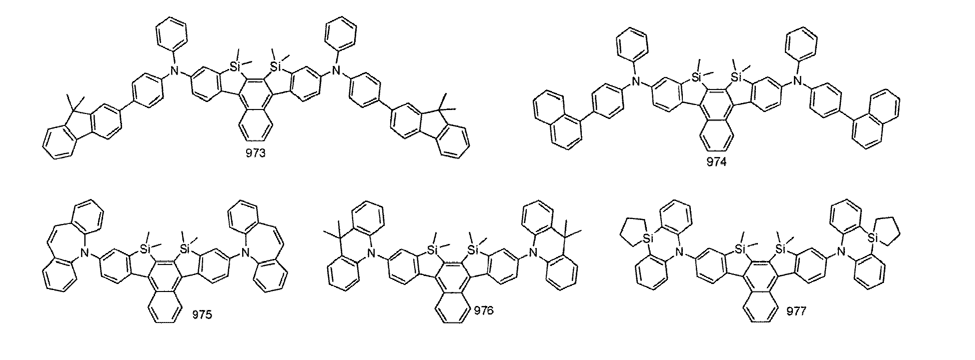

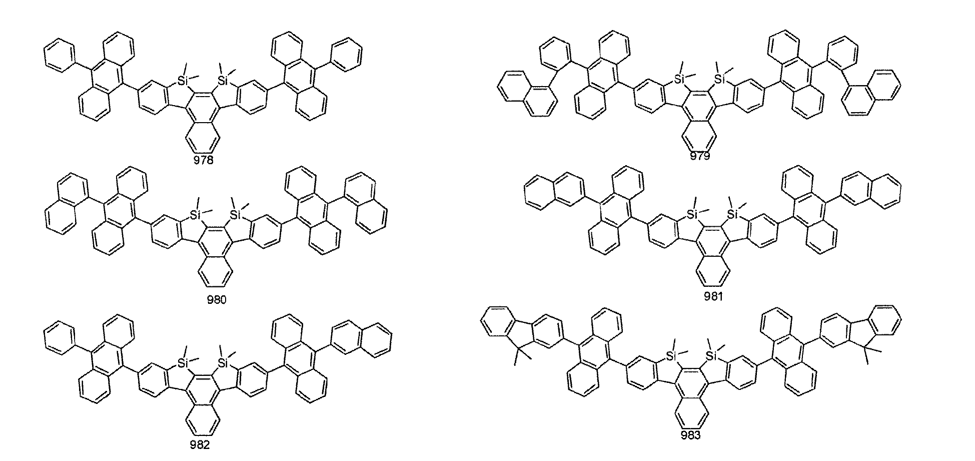

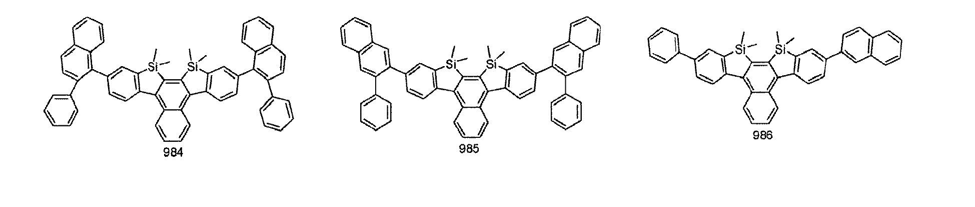

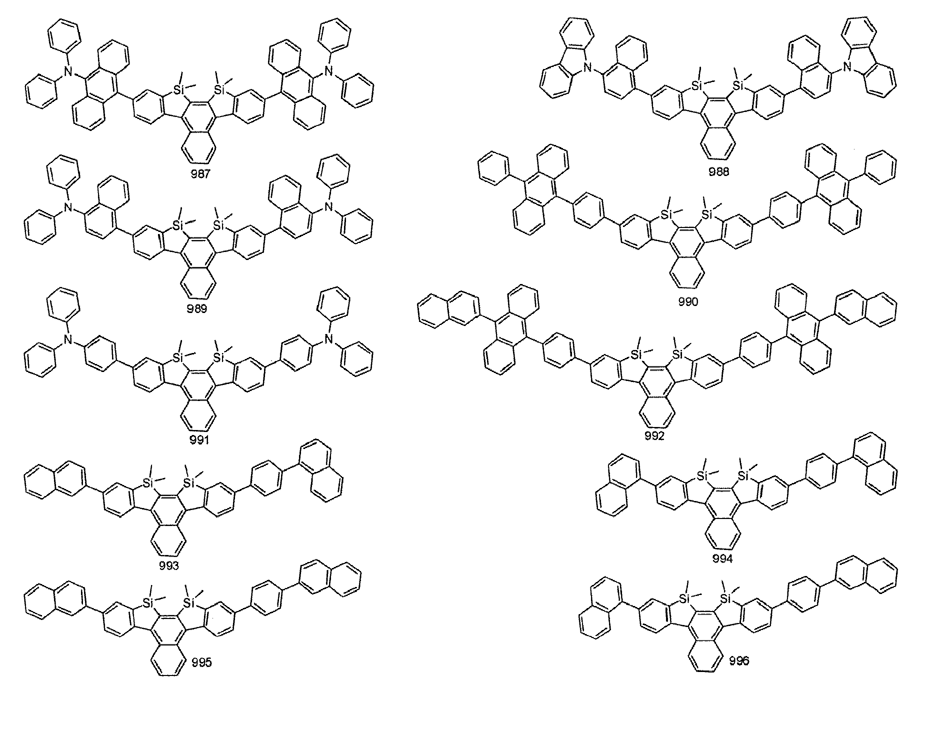

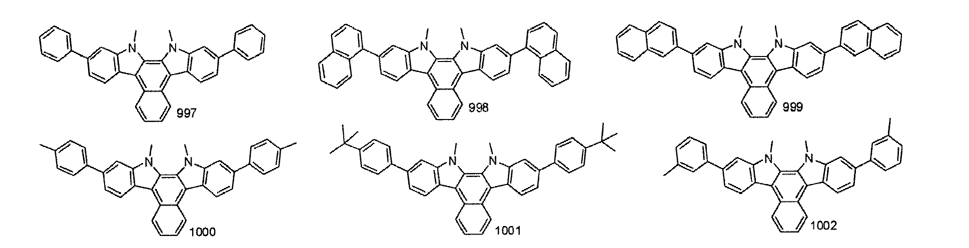

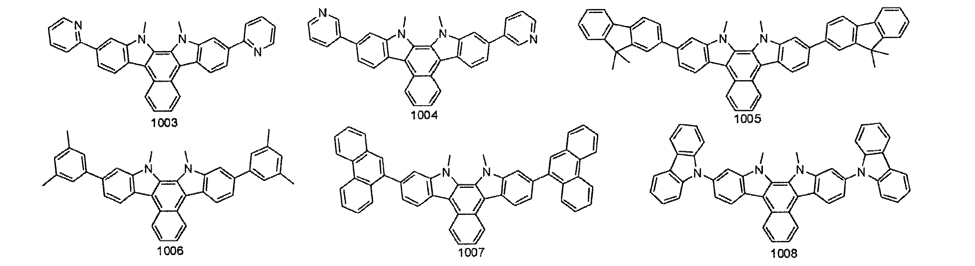

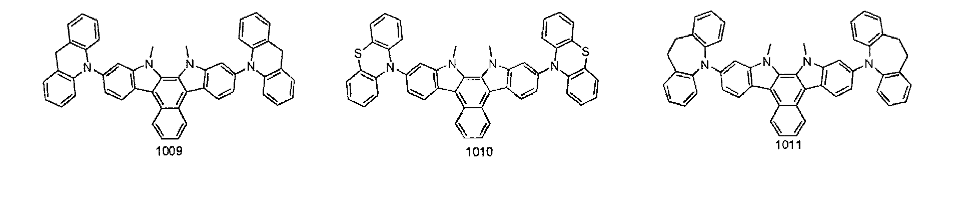

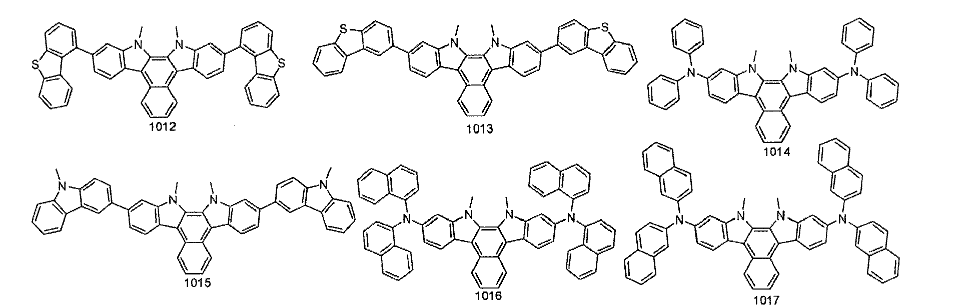

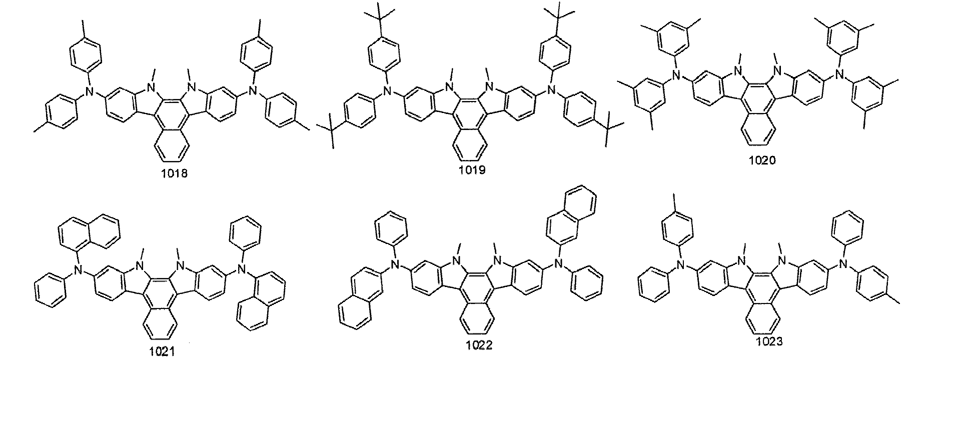

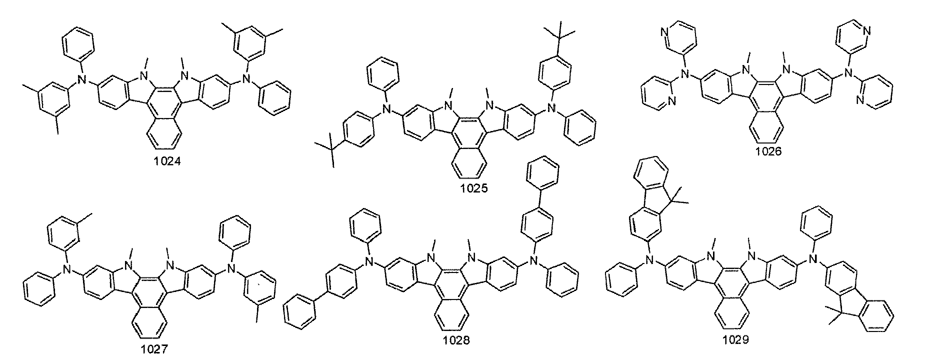

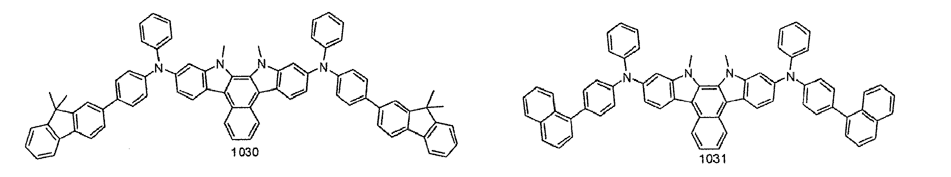

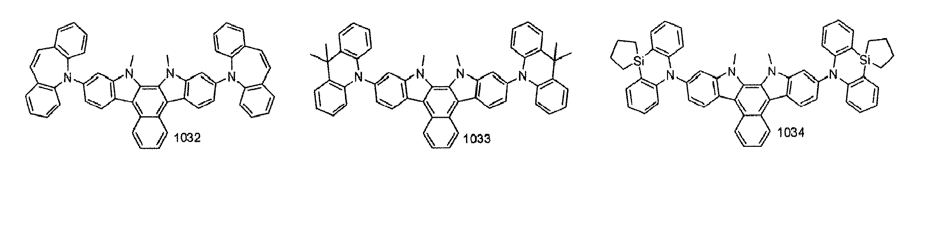

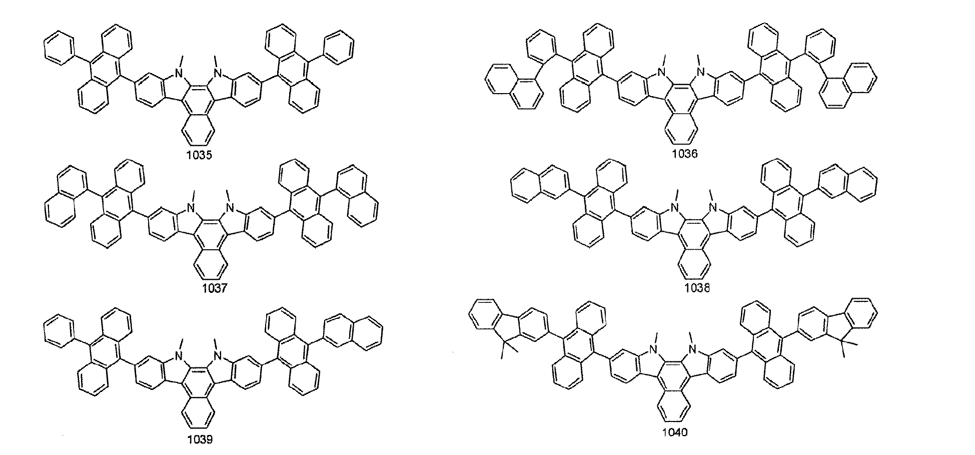

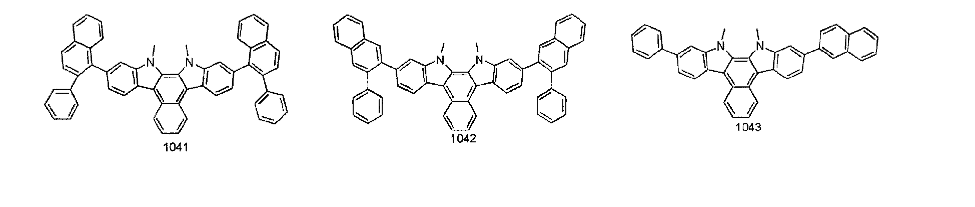

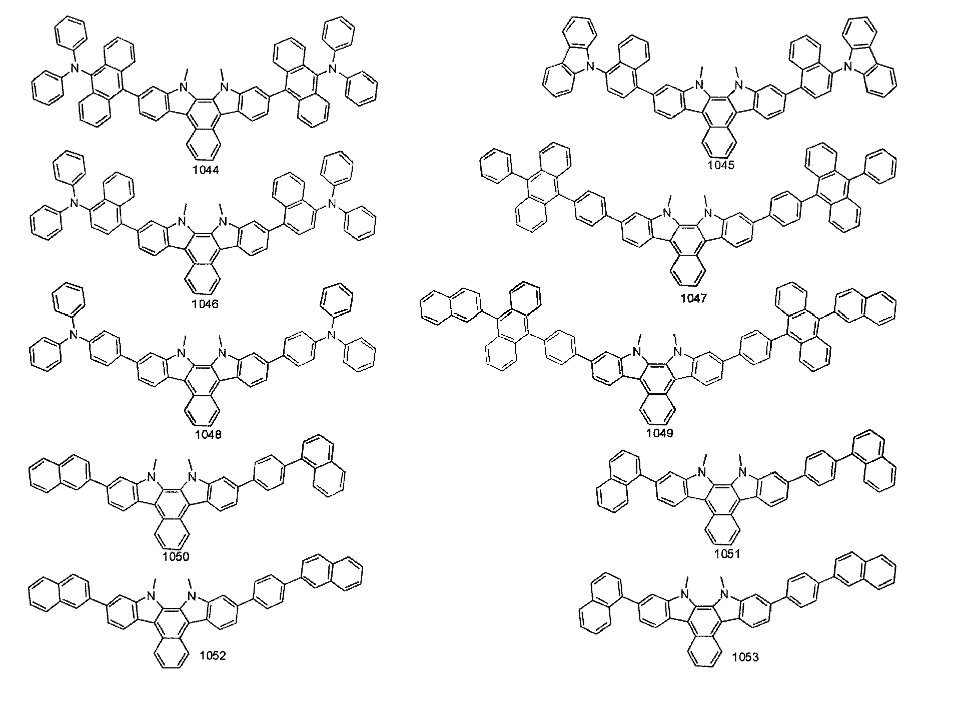

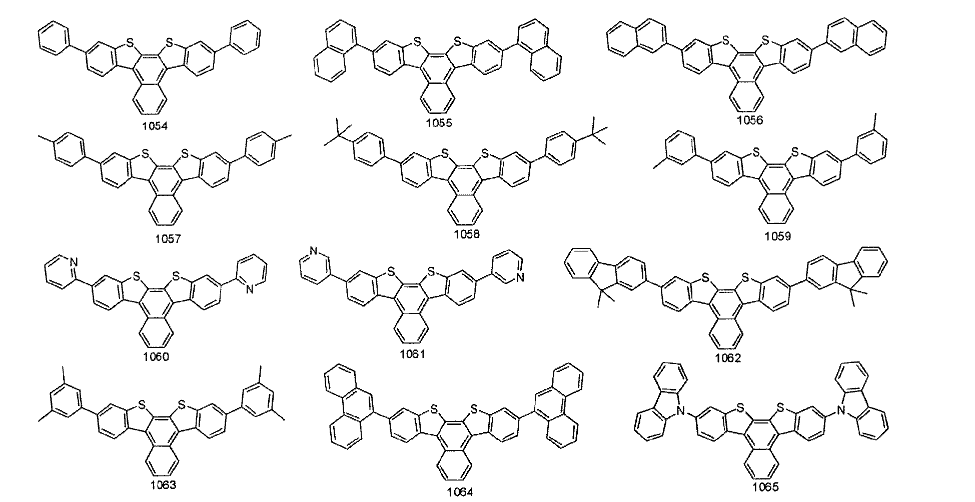

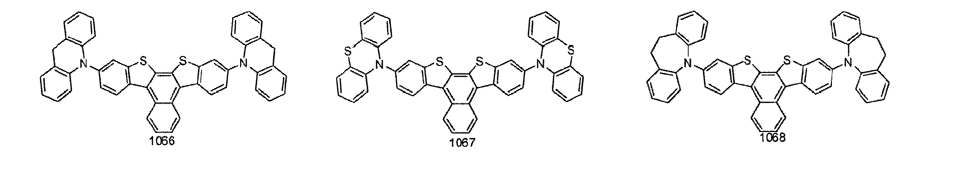

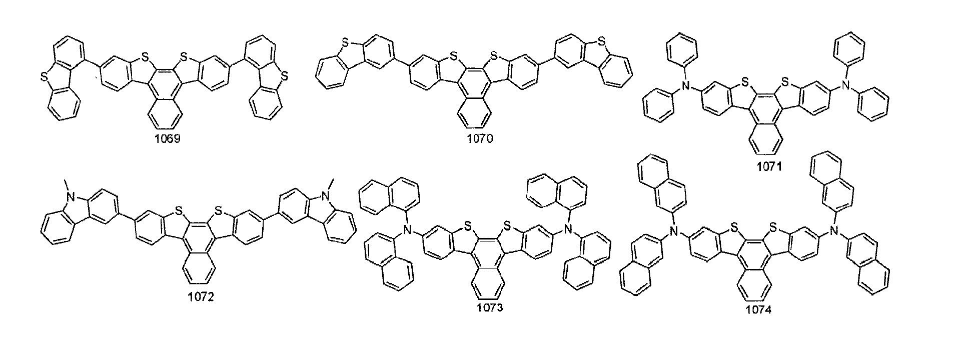

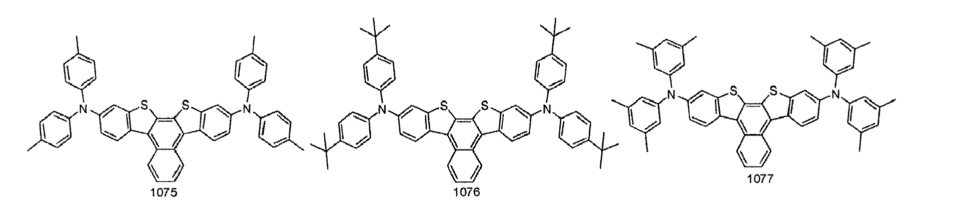

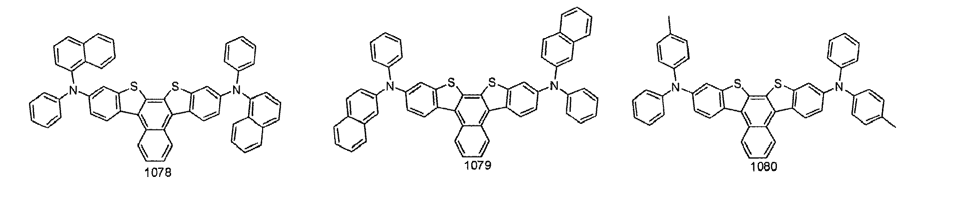

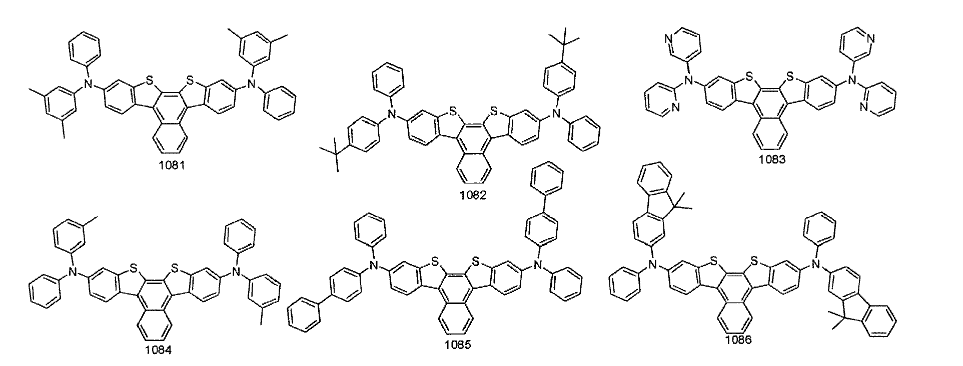

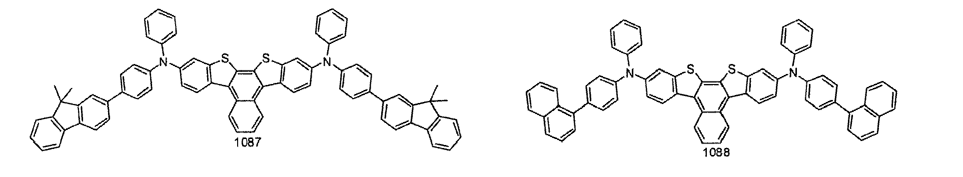

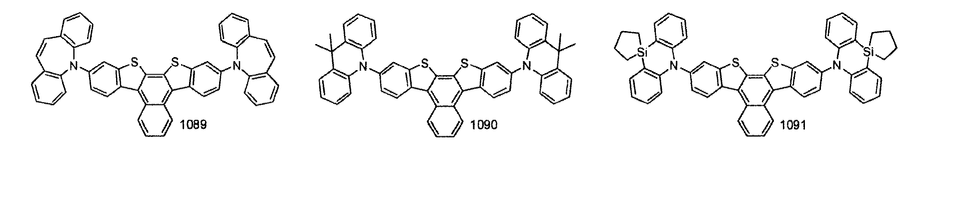

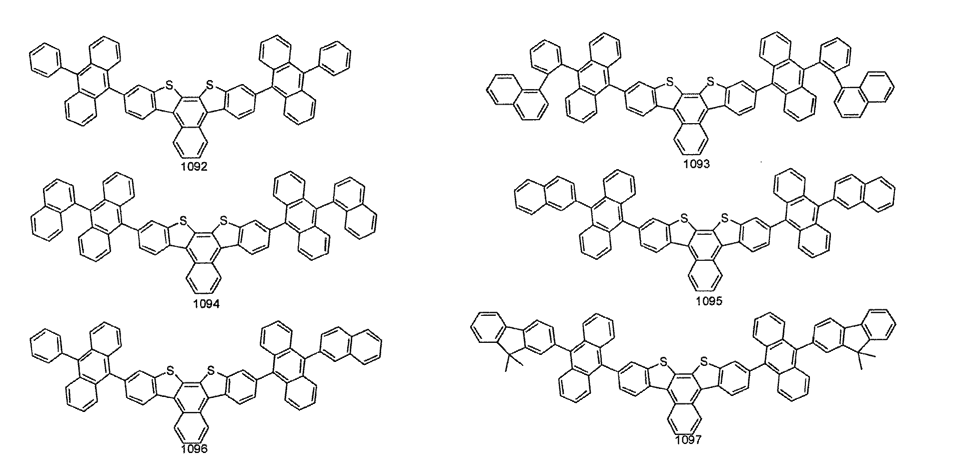

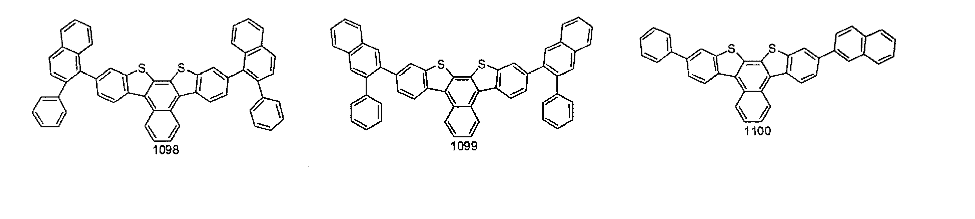

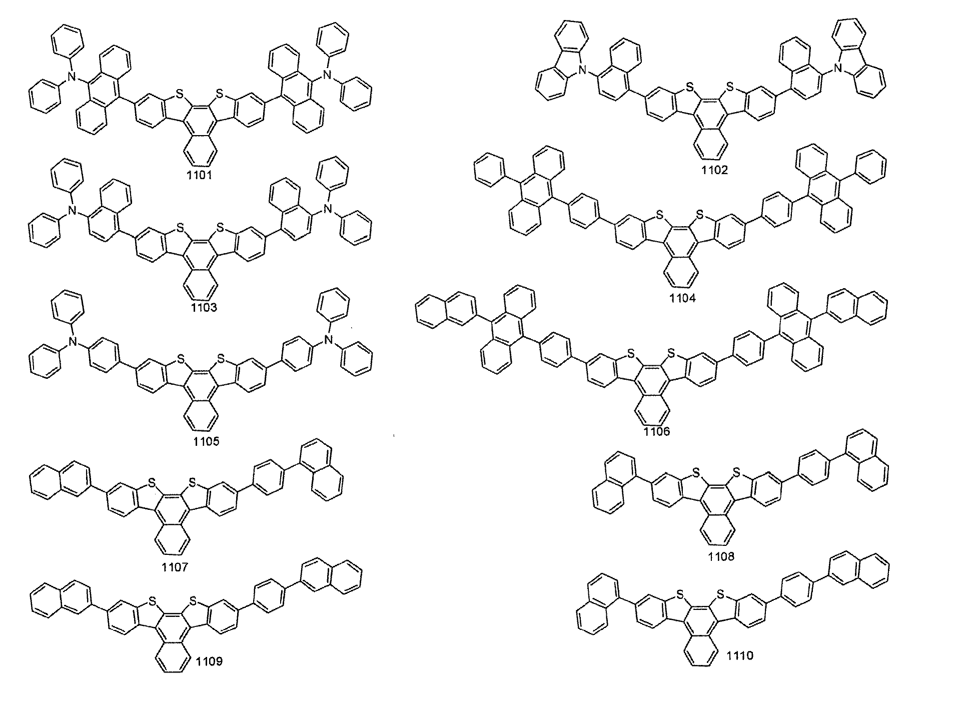

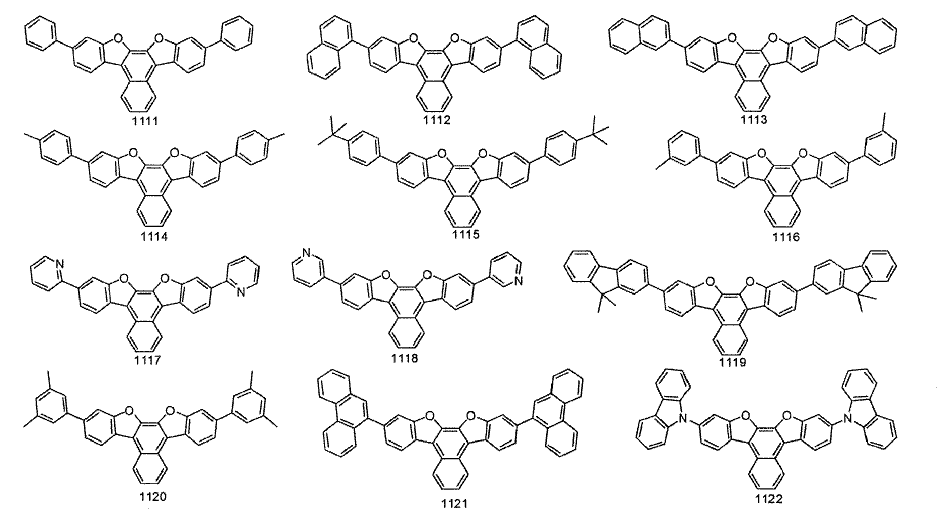

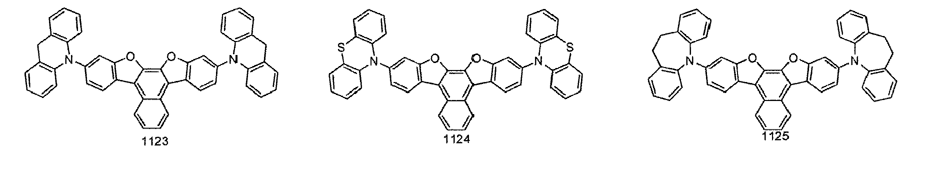

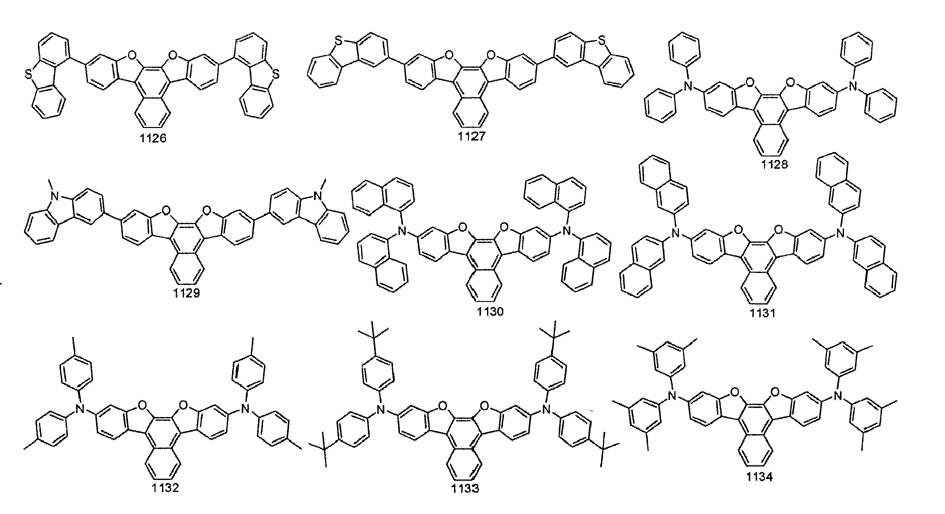

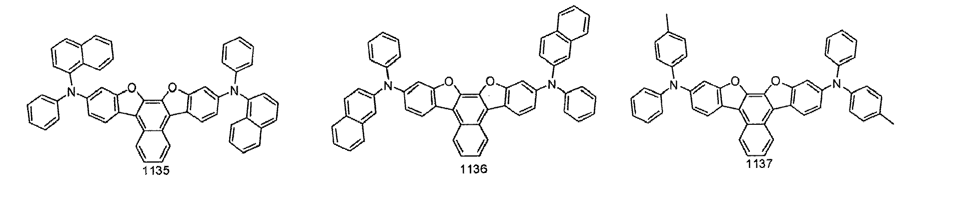

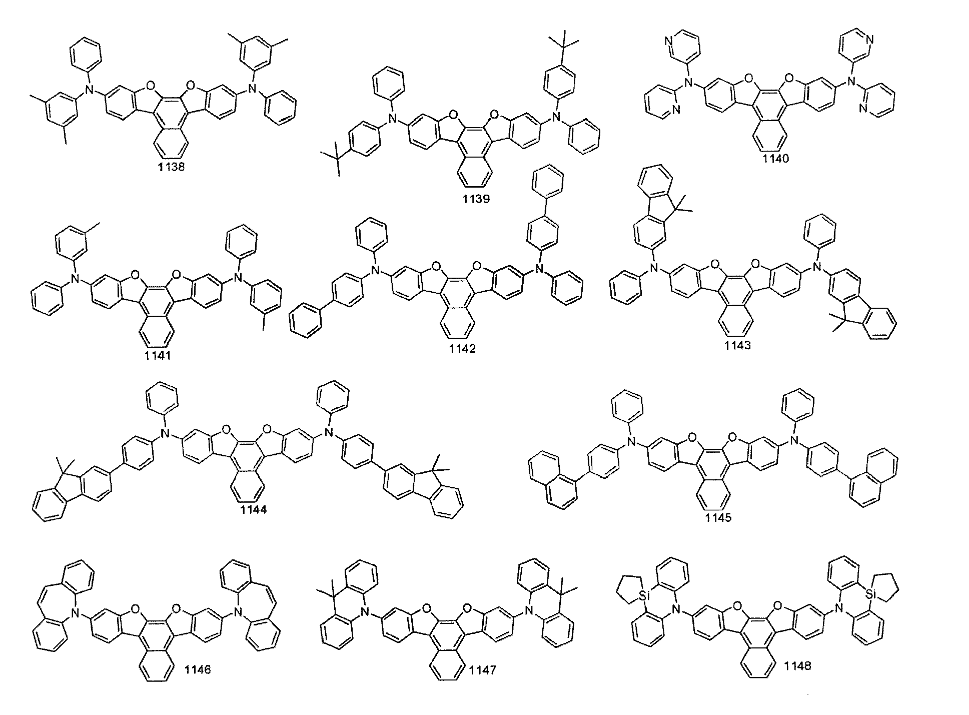

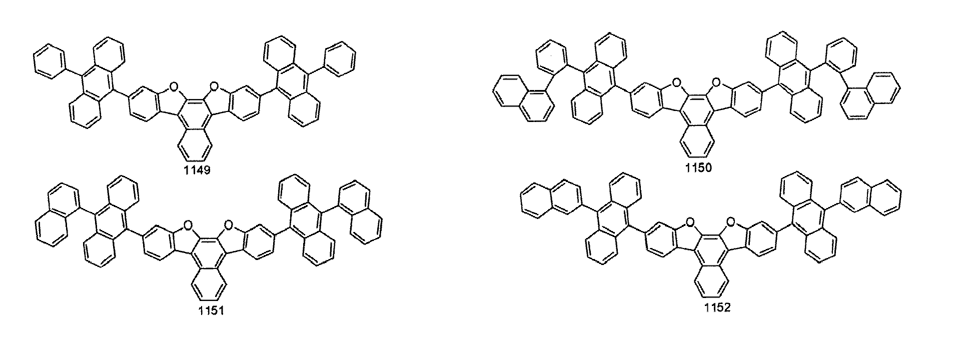

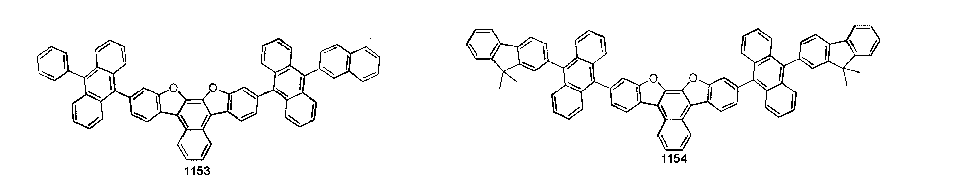

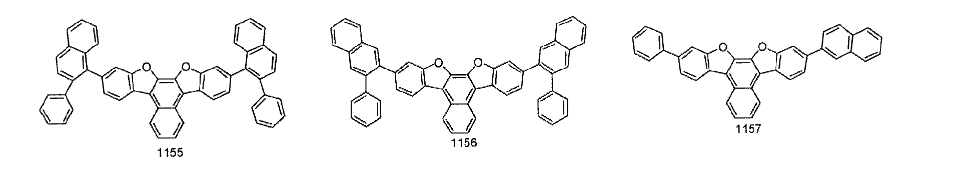

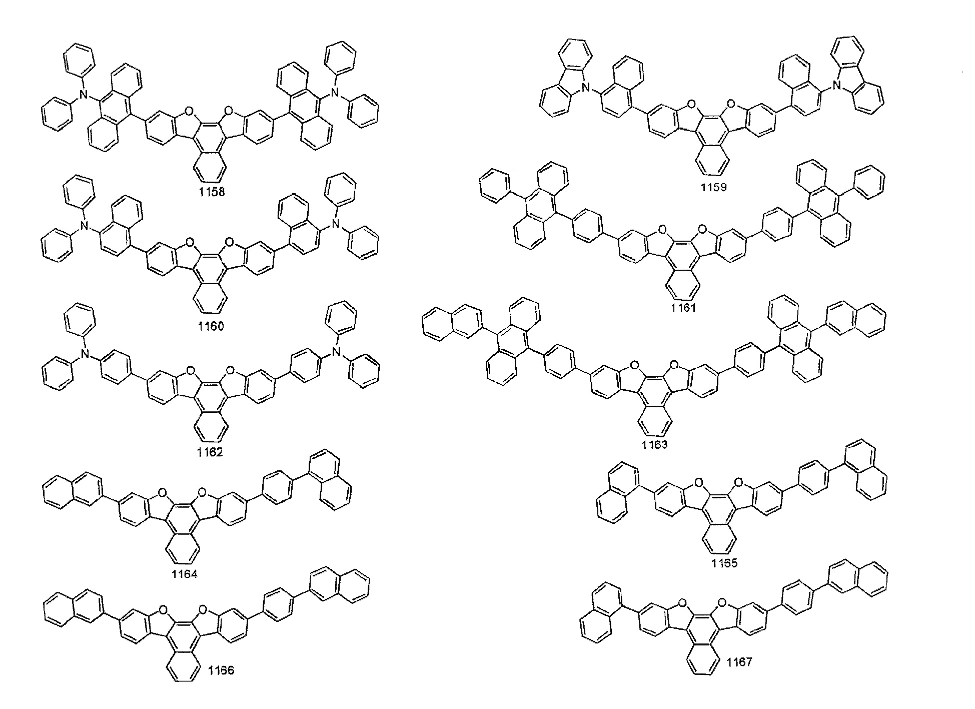

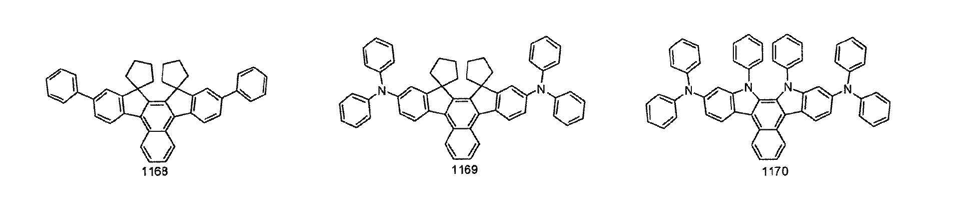

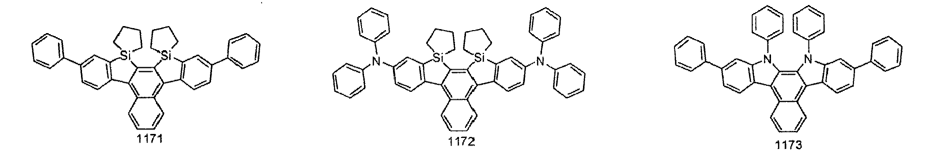

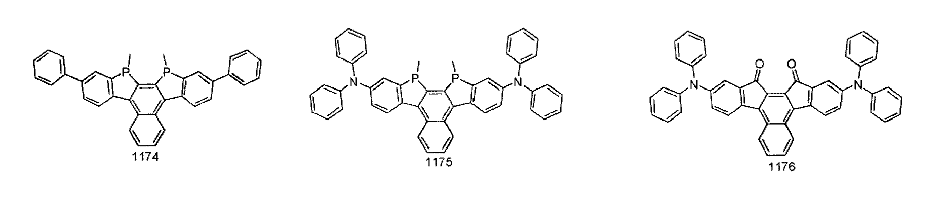

The organic electroluminescent compounds according to the present invention can be prepared according to the process illustrated by Reaction Scheme (1): 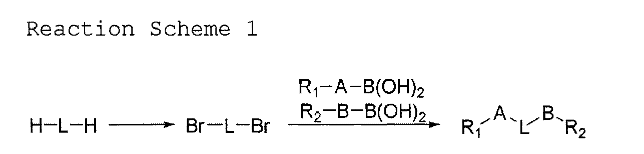 wherein, L, A, B, R₁ and R₂ are defined as in Chemical Formula (1).

Further, the present invention provides organic solar cells, which comprise one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more organic electroluminescent compound(s) represented by Chemical Formula (1). The organic electroluminescent compounds may be employed as material for a hole transport layer, or dopant or host material for an electroluminescent layer.

The organic electroluminescent device according to the present invention is **characterized in that** the organic layer comprises an electroluminescent layer, which contains one or more dopant(s) or host(s) in addition to one or more organic electroluminescent compound(s) represented by Chemical Formula (1). The dopant or host to be applied to an organic electroluminescent device according to the present invention is not particularly restrictive. The dopant to be applied to the organic electroluminescent device according to the invention is preferably selected from the compounds represented by one of Chemical Formulas (6) to (8): 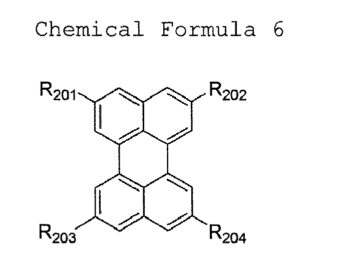 wherein, R₂₀₁ through R₂₀₄ independently represent hydrogen, halogen, (C1-C60)alky, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alky (C6-C60)aryloxy, (C1-C60)alkylthio, (C6-C60)arylthio, (C1-C60)alkoxycarbonyl, (C1-C60)alkylcarbonyl, (C6-C60)arylcarbonyl, carboxyl, nitro or hydroxyl, or R₂₀₁ through R₂₀₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkoxy, aryloxy, arylthio, alkylamino or arylamino of R₂₀₁ through R₂₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C1-C60)alkylthio, (C6-C60)arylthio, (C1-C60)alkoxycarbonyl, (C1-C60)alkylcarbonyl, (C6-C60)arylcarbonyl, carboxyl, nitro and hydroxyl; 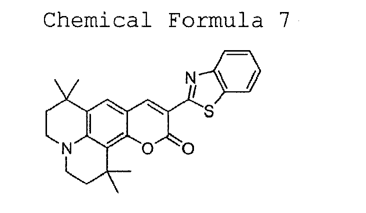 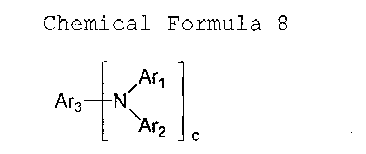
wherein, Ar₁ and Ar₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₁ and Ar₂ may be linked via (C3-C60)alkylene or (C3-C60)alkenylene with or without a fused ring to form an alicyclic ring, or a monocyclic or polycyclic aromatic ring;
the aryl, heteroaryl, arylamino or heterocycloalkyl of Ar₁ and Ar₂ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl;
Ar₃ represents (C6-C60)arylene, (C4-C60)heteroarylene or arylene represented by one of the following structures: 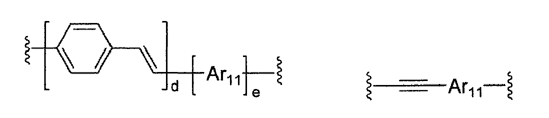
Ar₁₁ represents (C6-C60)arylene or (C4-C60)heteroarylene;
the arylene or heteroarylene of Ar₃ and Ar₁₁ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl;
c is an integer from 1 to 4,
d is an integer from 1 to 4, and
e is an integer of 0 or 1.

The dopant compounds represented by one of Chemical Formulas (6) to (8) can be exemplified by the following compounds, without restriction:

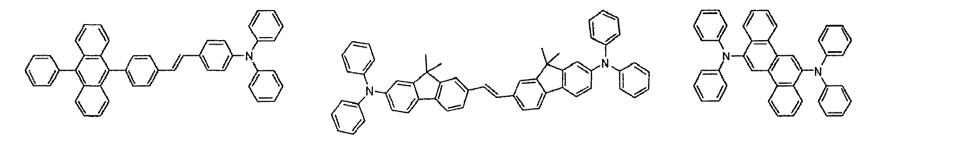

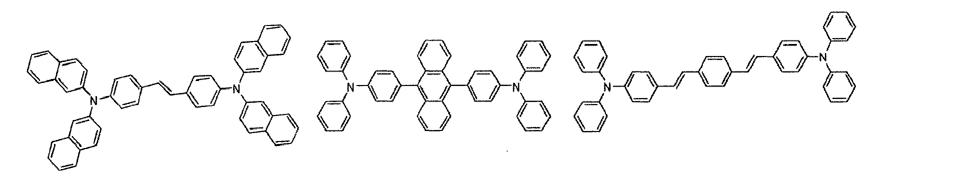

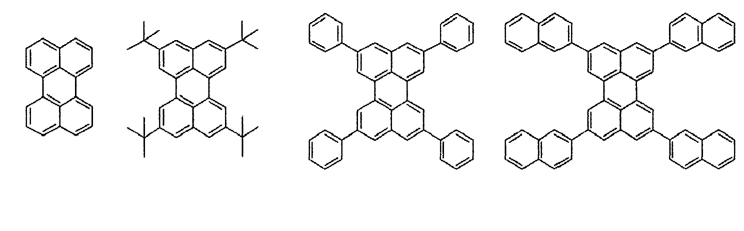

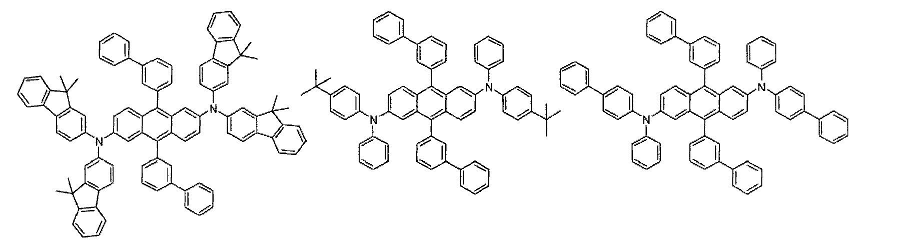

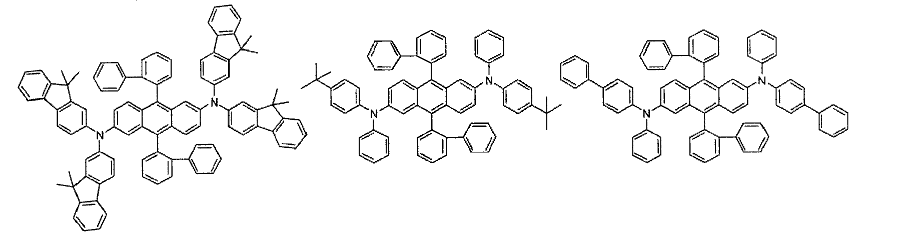

The host to be applied to organic electroluminescent devices according to the present invention is preferably selected from the compounds represented by Chemical Formula (9) or (10):

Chemical Formula 9 (Ar₂₁)_{f}-L₂₁-(Ar₂₂)_{g}

Chemical Formula 10 (Ar₂₃)ₕ-L₂₂-(Ar₂₄)ⱼ

wherein, L₂₁ represents (C6-C60)arylene or (C4-C60)heteroarylene;
L₂₂ represents anthracenylene;
Ar₂₁ through Ar₂₄ are independently selected from hydrogen, (C1-C60)alkyl, (C1-C60)alkoxy, halogen, (C4-C60)heteroaryl, (C5-C60)cycloalkyl and (C6-C60)aryl; the cycloalkyl, aryl or heteroaryl of Ar₂₁ through Ar₂₄ may be further substituted by one or more substituent(s) selected from a group consisting of (C6-C60)aryl or (C4-C60)heteroaryl with or without one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
f, g, h and i independently represent an integer from 0 to 4.

The host of Chemical Formula (9) or (10) can be exemplified by anthracene derivatives and benz[a]anthracene derivatives represented by one of Chemical Formulas (11) to (13): 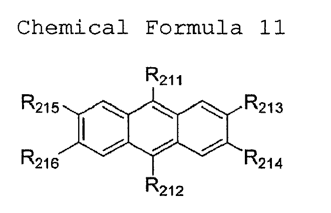 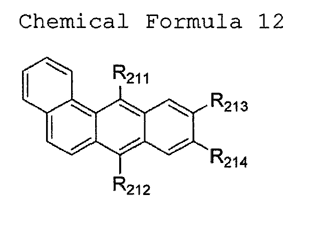 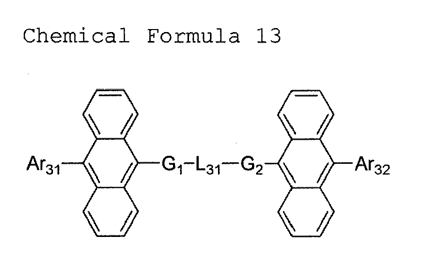
wherein, R₂₁₁ and R₂₁₂ independently represent (C6-C60)aryl, (C4-C60)heteroaryl, 5- or 6-membered heterocycloalkyl containing one or more heteroatom(s) selected from N, O and S, or (C3-C60)cycloalkyl; the aryl or heteroaryl of R₂₁₁ and R₂₁₂ may be further substituted by one or more substituent(s) selected from a group consisting of (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₂₁₃ through R₂₁₆ independently represent hydrogen, (C1-C60)alkyl, (C1-C60)alkoxy, halogen, (C4-C60)heteroaryl, (C5-C60)cycloalkyl or (C6-C60)aryl; the heteroaryl, cycloalkyl or aryl of R₂₀₃ through R₂₀₆ may be further substituted by one or more substituent(s) selected from a group consisting of (C1-C60)alkyl with or without halogen substituent(s), (C1-C60)alkoxy, (C3-C60) cycloalkyl, halogen, cyano, tri(C1-C60)alkylsilyl, di(C1-C60)alkyl(C6-C60)arylsilyl and tri(C6-C60)arylsilyl;
G₁ and G₂ independently represent a chemical bond, or (C6-C60)arylene with or without one or more substituent(s) selected from (C1-C60)alkyl, (C1-C60)alkoxy, (C6-C60) aryl, (C4-C60)heteroaryl and halogen;
Ar₃₁ and Ar₃₂ independently represent aryl or (C4-C60)heteroaryl selected from the following structures; 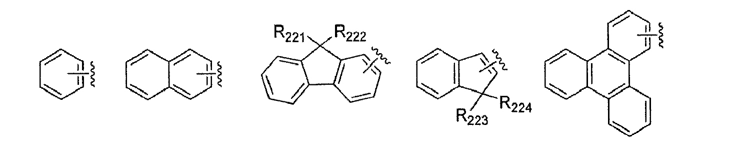 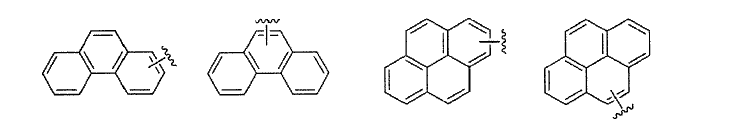 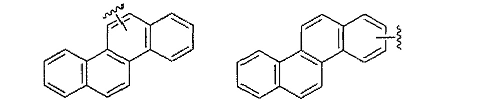
the aryl or heteroaryl of Ar₃₁ and Ar₃₂ may be substituted by one or more substituent(s) selected from (C1-C60)alkyl, (C1-C60)alkoxy, (C6-C60)aryl and (C4-C60)heteroaryl;
L₃₁ represents (C6-C60)arylene, (C4-C60)heteroarylene or a compound having the following structure; 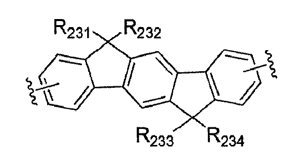
the arylene or heteroarylene of L₃₁ may be substituted by one or more substituent(s) selected from (C1-C60)alkyl, (C1-C60)alkoxy, (C6-C60)aryl, (C4-C60)heteroaryl and halogen;
R₂₂₁, R₂₂₂, R₂₂₃ and R₂₂₄ independently represent hydrogen, (C1-C60)alkyl or (C6-C60)aryl, or they may be linked to an adjacent substituent via (C3-C60)alkylene or (C3-C60)alkenylene with or without a fused ring to form an alicyclic ring, or a monocyclic or polycyclic aromatic ring; and
R₂₃₁, R₂₃₂, R₂₃₃ and R₂₃₄ independently represent hydrogen, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

The host compounds represented by one of Chemical Formulas (11) to (13) can be exemplified by the following compounds, but they are not restricted thereto.

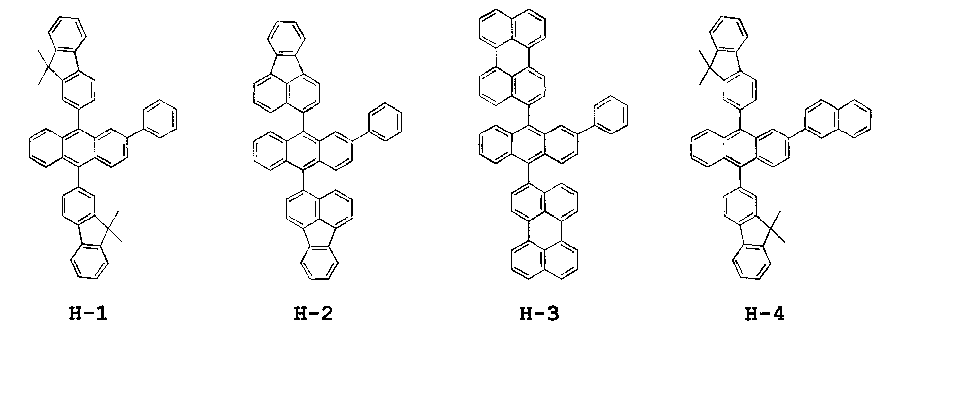

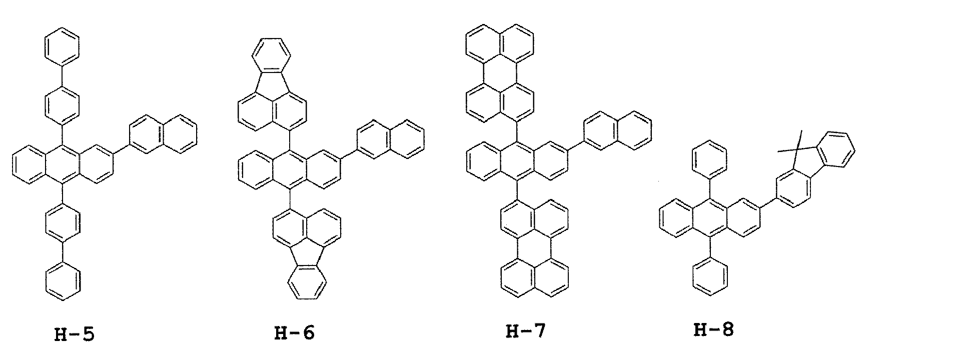

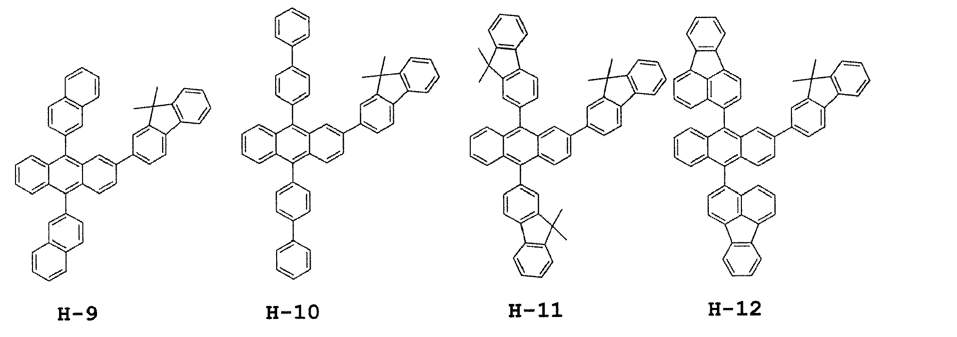

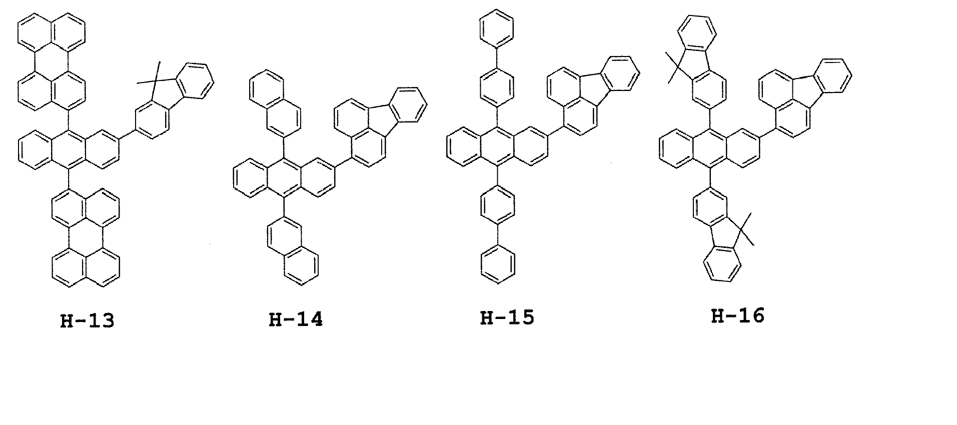

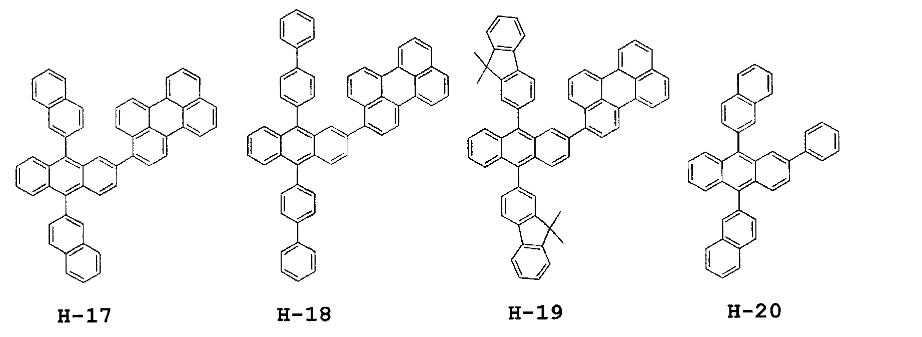

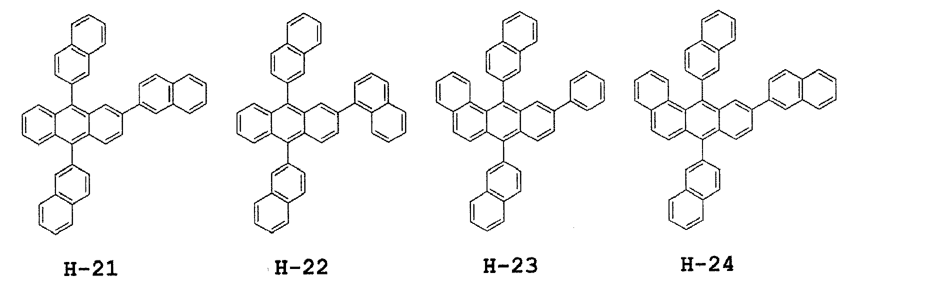

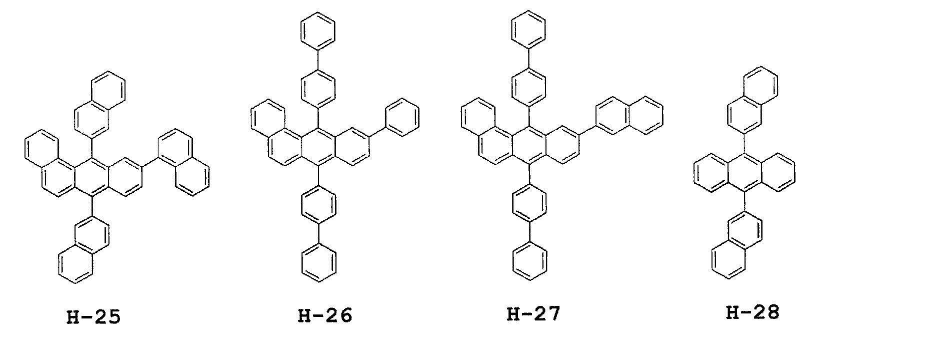

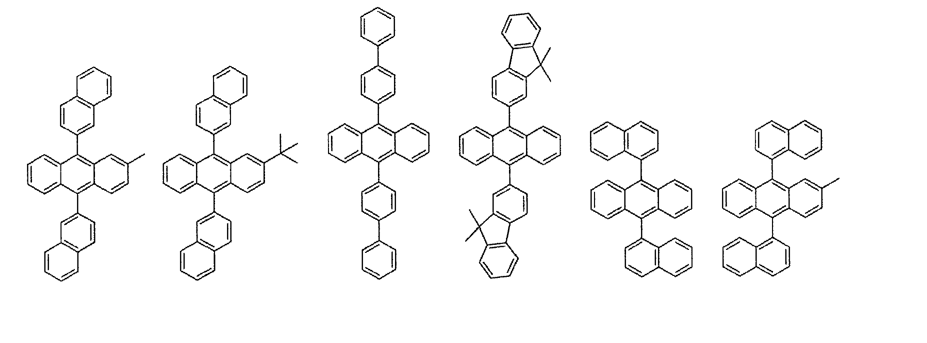

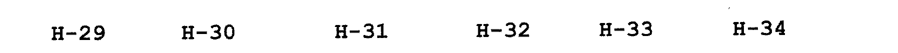

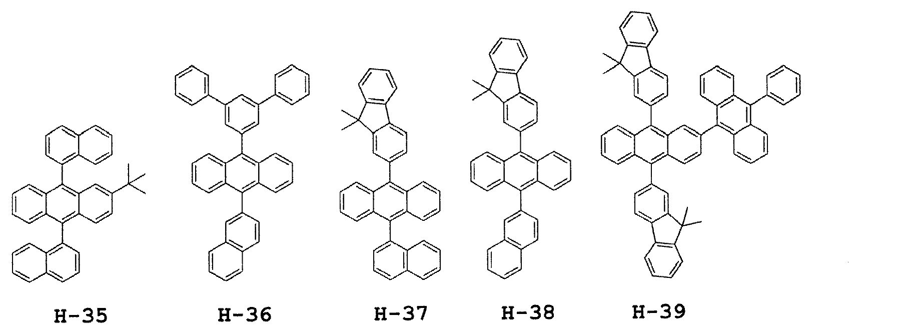

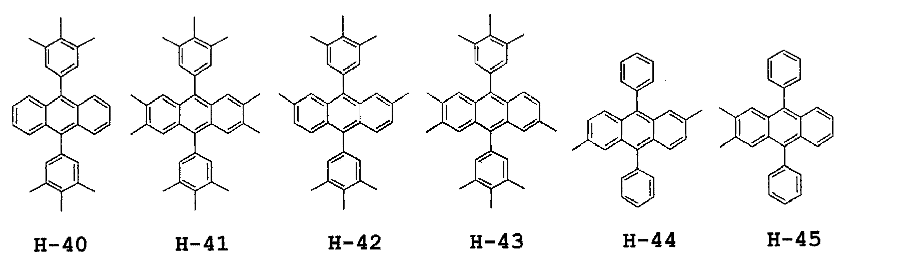

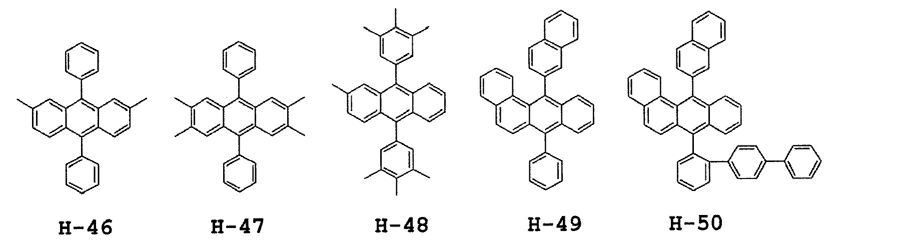

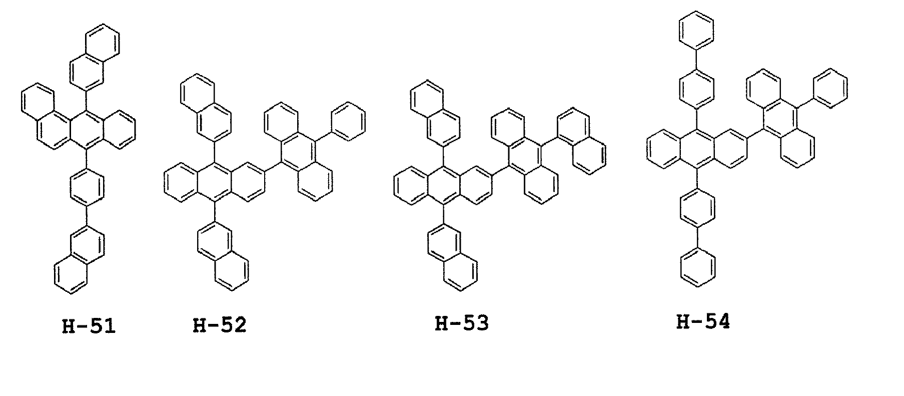

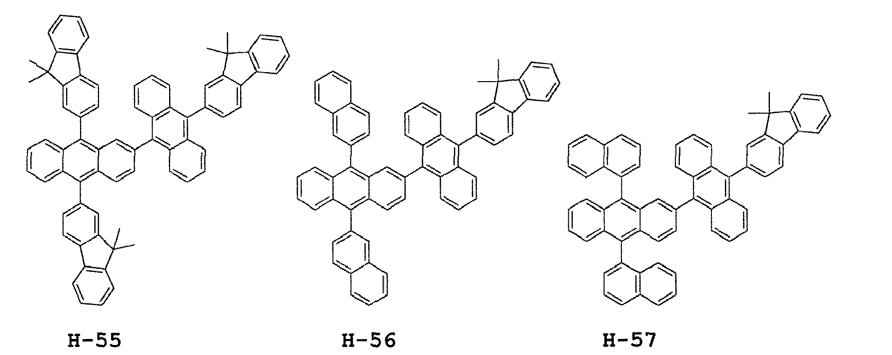

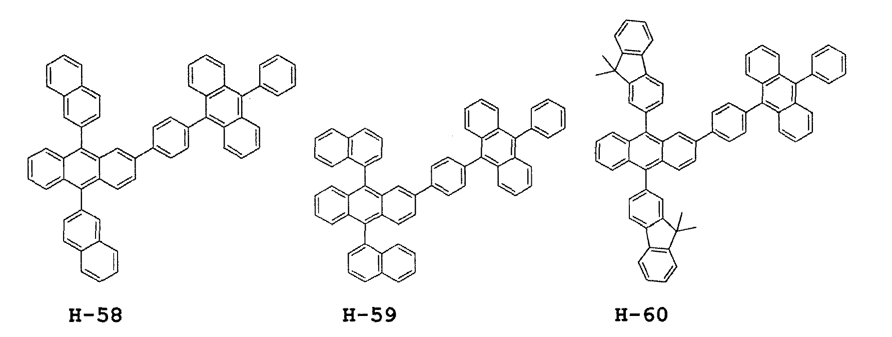

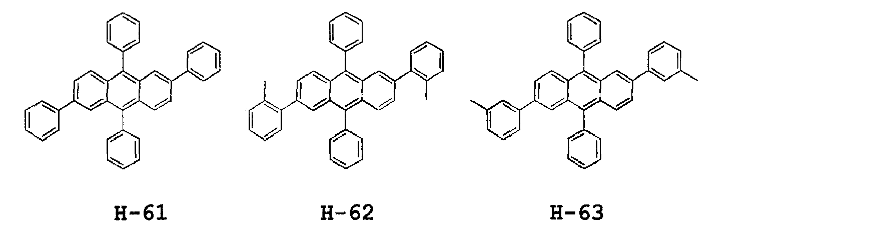

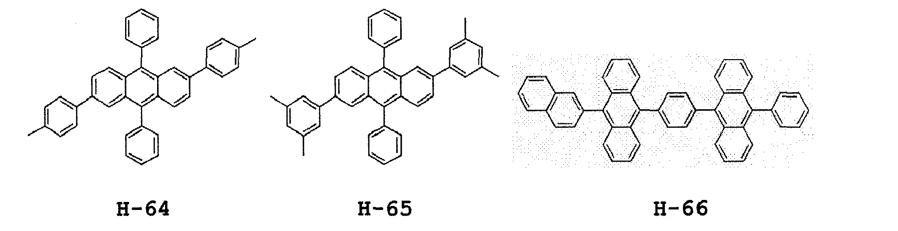

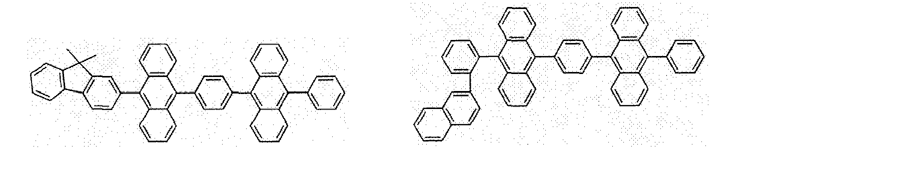

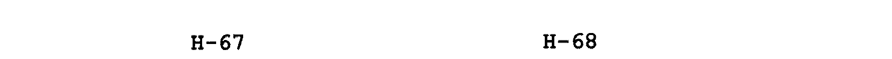

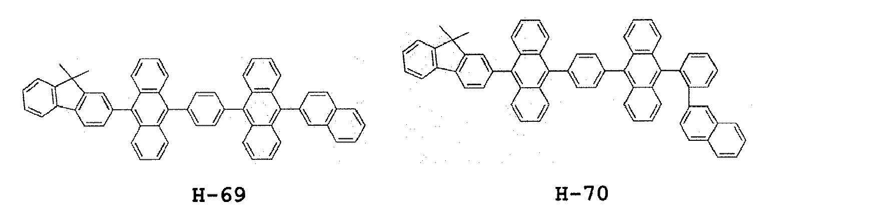

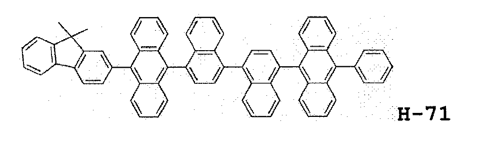

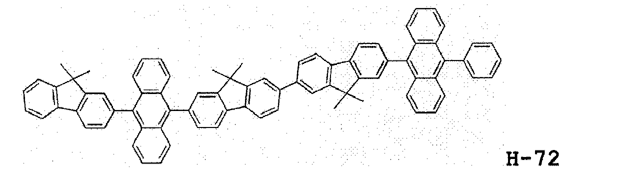

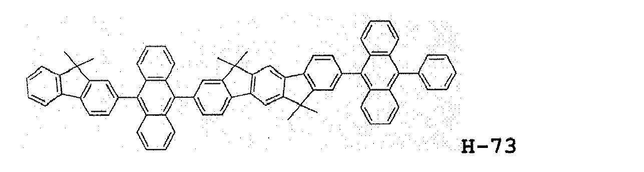

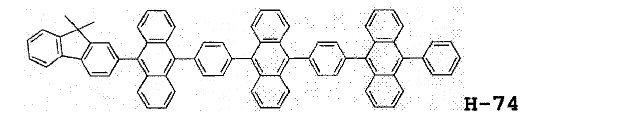

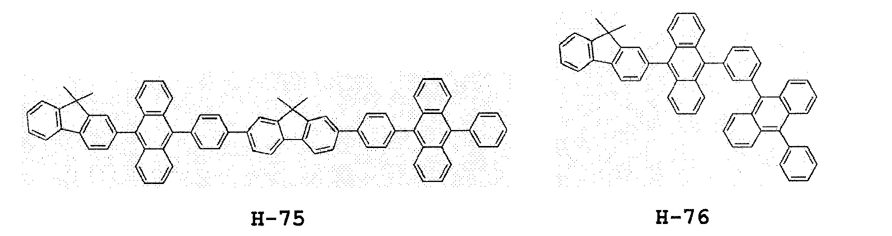

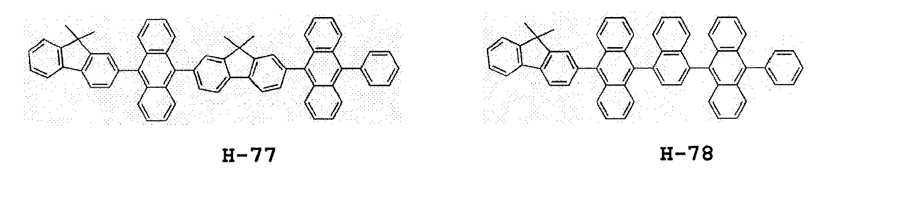

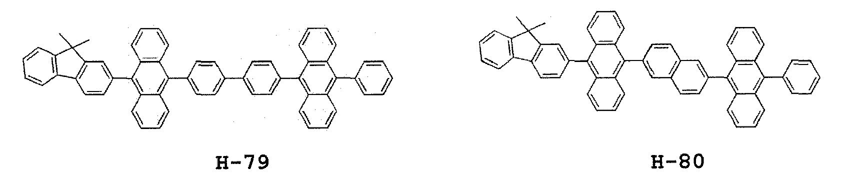

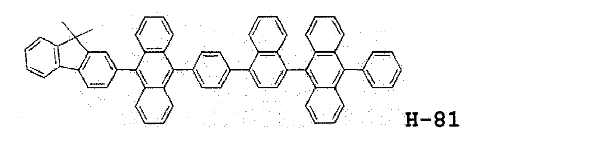

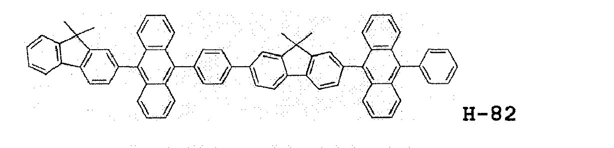

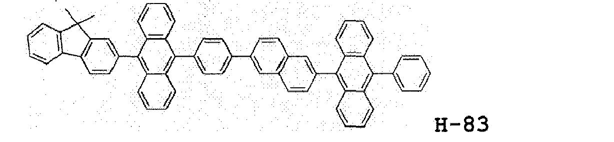

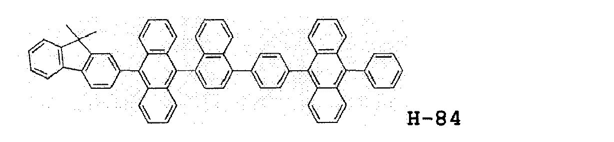

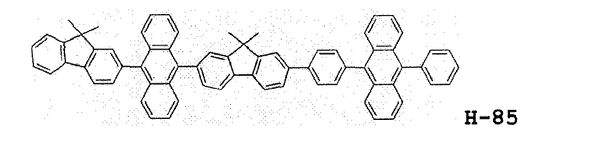

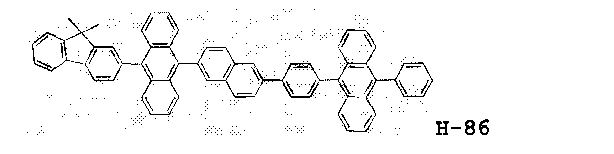

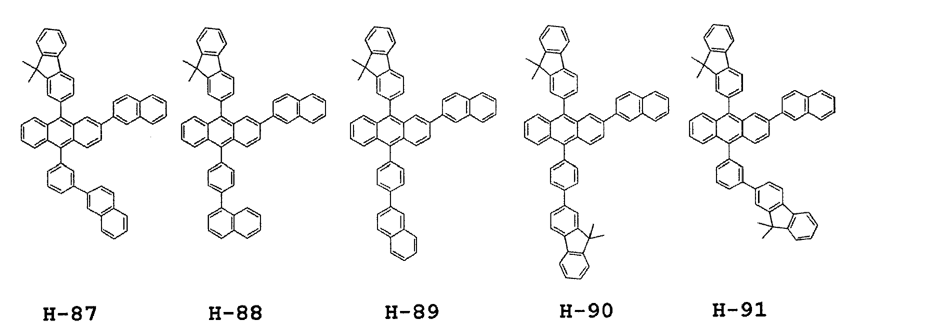

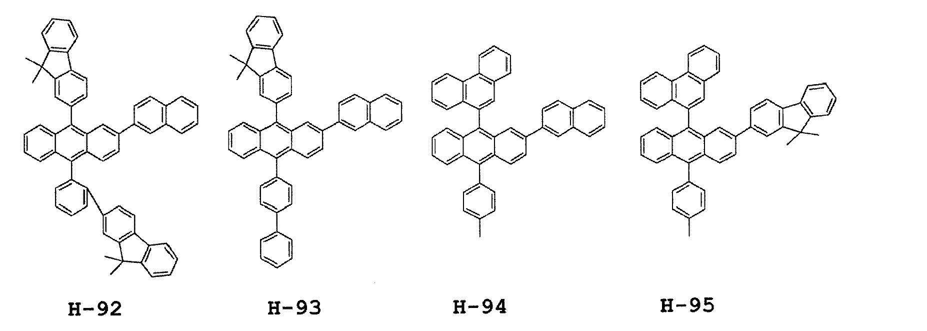

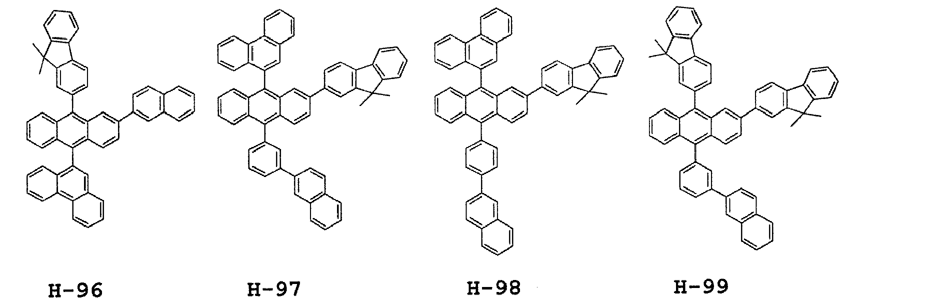

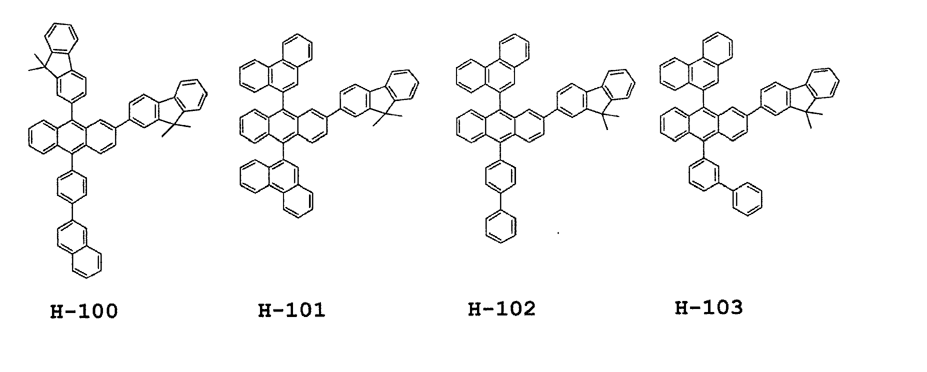

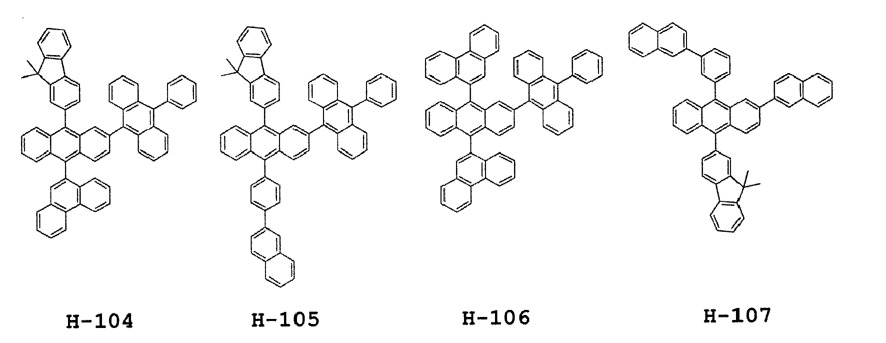

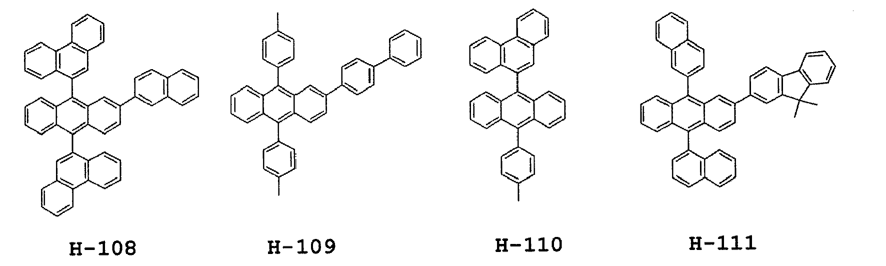

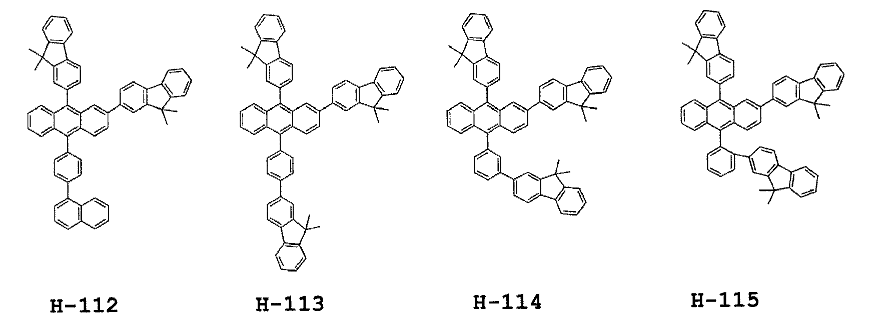

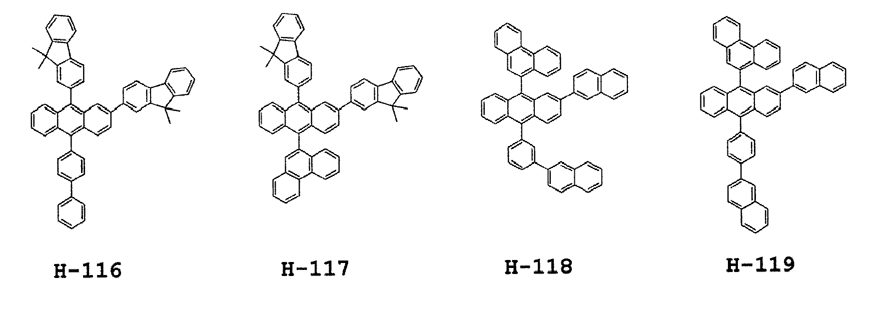

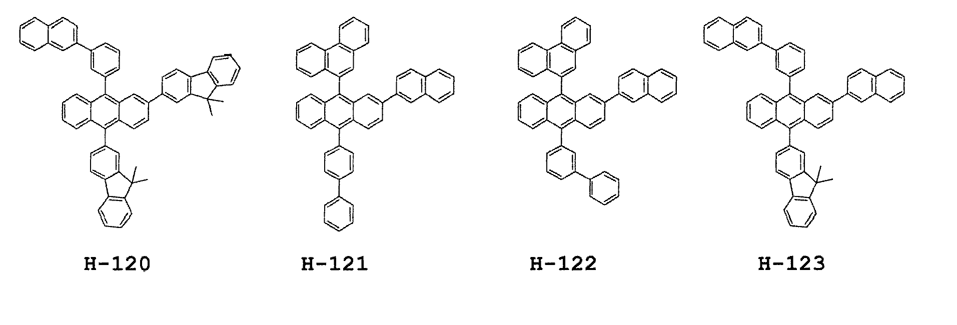

The organic electroluminescent device according to the invention may further comprise one or more compound(s) selected from a group consisting of arylamine compounds and styrylarylamine compounds, in addition to the organic electroluminescent compound represented by Chemical Formula (1). Examples of arylamine or styrylarylamine compounds include the compounds represented by Chemical Formula (14), but they are not restricted thereto: 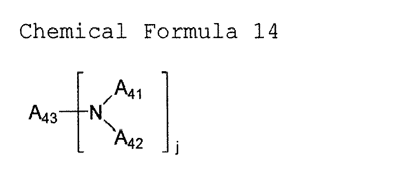
wherein, Ar₄₁ and Ar₄₂ independently represent (C1-C60)alkyl, (C6-C60)aryl, (C4-C60)heteroaryl, (C6-C60)arylamino, (C1-C60)alkylamino, morpholino, thiomorpholino, 5- or 6-membered heterocycloalkyl containing one or more heteroatom(s) selected from N, O and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring;
the aryl, heteroaryl, arylamino or heterocycloalkyl of Ar₄₁ and Ar₄₂ may be further substituted by one or more substituent(s) selected from a group consisting of halogen, (C1-C60)alkyl, (C2-C60)alkenyl, (C2-C60)alkynyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl;
Ar₄₃ represents (C6-C60)aryl, (C5-C60)heteroaryl or (C6-C60)arylamino; the aryl, heteroaryl or arylamino of Ar₄₃ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl; and
j is an integer from 1 to 4.

The arylamine compounds and styrylarylamine compounds may be more specifically exemplified by the following compounds, but are not restricted thereto.

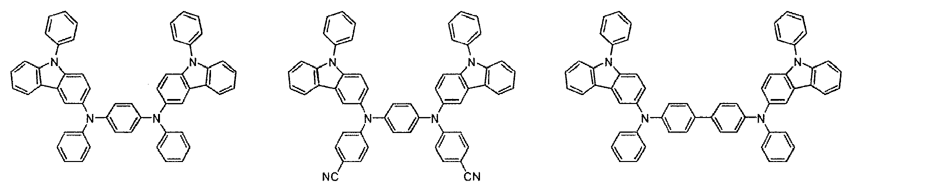

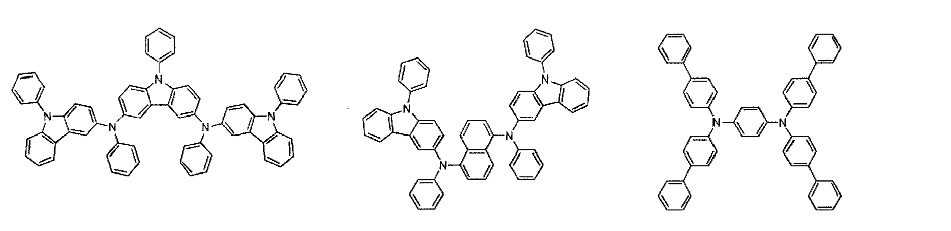

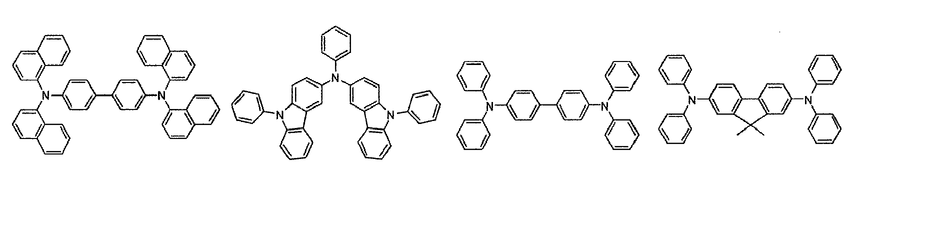

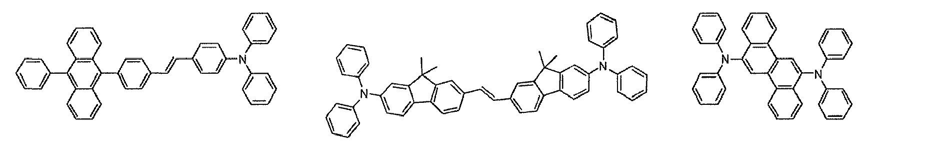

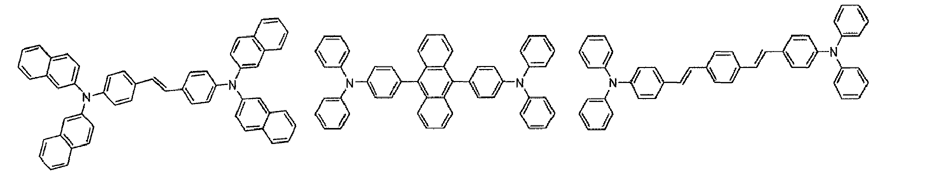

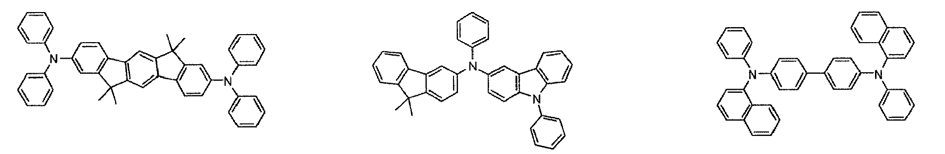

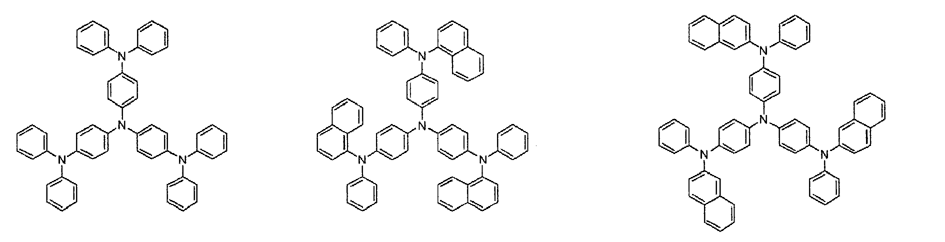

In an organic electroluminescent device according to the present invention, the organic layer may further comprise one or more metal(s) selected from a group consisting of organometals of Group 1, Group 2, 4^{th} period and 5^{th} period transition metals, lanthanide metals and d-transition elements in the Periodic Table of Elements, in addition to the organic electroluminescent compounds represented by Chemical Formula (1). The organic layer may comprise a charge generating layer as well as the electroluminescent layer.

The present invention can realize an organic electroluminescent device having a pixel structure of independent light-emitting mode, which comprises an organic electroluminescent device containing the compound of Chemical Formula (1) as a sub-pixel and one or more sub-pixel(s) comprising one or more metallic compound(s) selected from a group consisting of Ir, Pt, Pd, Rh, Re, Os, Tl, Pb, Bi, In, Sn, Sb, Te, Au and Ag, patterned in parallel at the same time.

Further, the organic electroluminescent device is an organic display wherein the organic layer further comprises one or more compound(s) selected from compounds having electroluminescent peak of wavelength of not less than 560 nm. The compounds having electroluminescent peak of wavelength of not less than 560 nm may be exemplified by the compounds represented by one of Chemical Formulas (15) to (19), but they are not restricted thereto.

Chemical Formula 15 **M¹L¹⁰¹L¹⁰²L¹⁰³**

In Chemical Formula (15), M¹ is selected from metals of Groups 7, 8, 9, 10, 11, 13, 14, 15 and 16 in the Periodic Table of Elements, and ligands L¹⁰¹, L¹⁰² and L¹⁰³ are independently selected from the following structures: 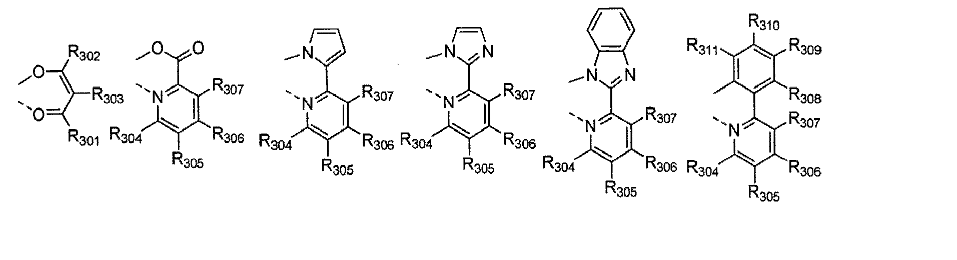 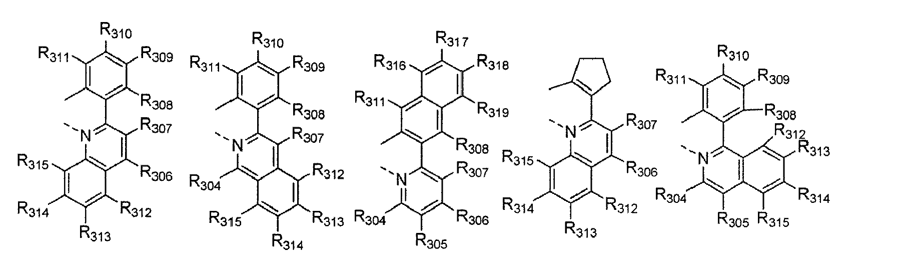 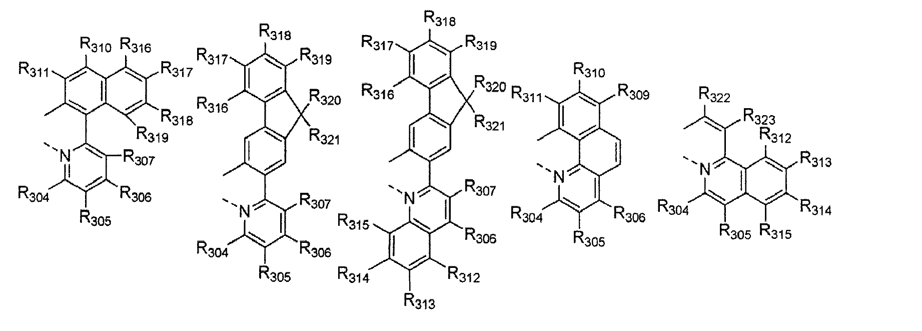 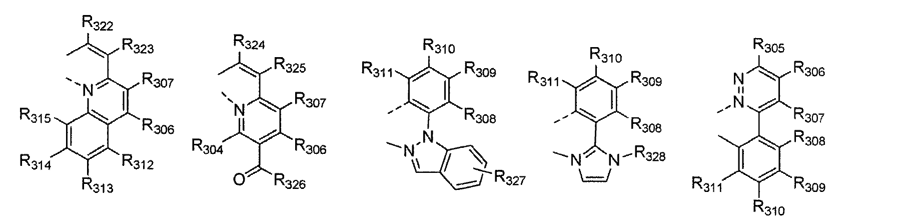 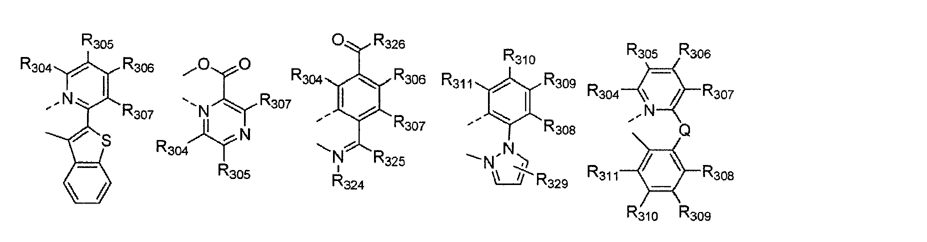
wherein, R₃₀₁ through R₃₀₃ independently represent hydrogen, (C1-C60)alkyl with or without halogen substituent(s), (C6-C60)aryl with or without (C1-C60)alkyl substituent(s), or halogen;
R₃₀₄ through R₃₁₉ independently represent hydrogen, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen; the alkyl, cycloalkyl, alkenyl or aryl of R₃₀₄ through R₃₁₉ may be further substituted by one or more substituent(s) selected from (C1-C60)alkyl, (C6-C60)aryl and halogen;
R₃₂₀ through R₃₂₃ independently represent hydrogen, (C1-C60)alkyl with or without halogen substituent(s), or (C6-C60)aryl with or without (C1-C60)alkyl substituent(s);
R₃₂₄ and R₃₂₅ independently represent hydrogen, (C1-C60) alkyl, (C6-C60) aryl or halogen, or R₃₂₄ and R₃₂₅ may be linked via (C3-C12)alkylene or (C3-C12)alkenylene with or without a fused ring to form an alicyclic ring, or a monocyclic or polycyclic aromatic ring; the alkyl or aryl of R₃₂₄ and R₃₂₅, or the alicyclic ring, or the monocyclic or polycyclic aromatic ring formed from R₃₂₄ and R₃₂₅ via (C3-C12)alkylene or (C3-C12)alkenylene with or without a fused ring may be further substituted by one or more substituent(s) selected from (C1-C60)alkyl with or without halogen substituent(s), (C1-C30)alkoxy, halogen, tri(C1-C30)alkylsilyl, tri(C6-C30)arylsilyl and (C6-C60)aryl;
R₃₂₆ represents (C1-C60)alkyl, (C6-C60)aryl, (C5-C60)heteroaryl or halogen;
R₃₂₇ through R₃₂₉ independently represent hydrogen, (C1-C60)alkyl, (C6-C60)aryl or halogen; the alkyl or aryl of R₃₂₆ through R₃₂₉ may be further substituted by halogen or (C1-C60)alkyl;
Q represents 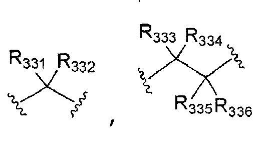 or 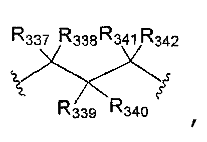 and R₃₃₁ through R₃₄₂ independently represent hydrogen, (C1-C60)alkyl with or without halogen substituent(s), (C1-C30)alkoxy, halogen, (C6-C60)aryl, cyano, (C5-C60)cycloalkyl, or each of R₃₃₁ through R₃₄₂ may be linked to an adjacent substituent via alkylene or alkenylene to form a (C5-C7) spiro-ring or a (C5-C9) fused ring, or each of them may be linked to R₃₀₇ or R₃₀₈ via alkylene or alkenylene to form a (C5-C7) fused ring.

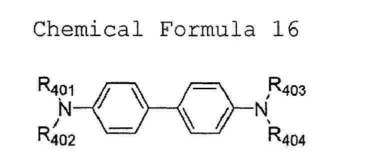

In Chemical Formula (16), R₄₀₁ through R₄₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₄₀₁ through R₄₀₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

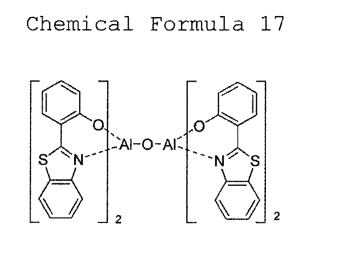

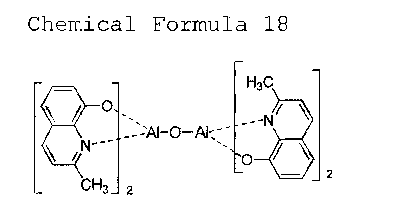

Chemical Formula 19 **L¹¹¹L¹¹²M²(T)ₖ**

In Chemical Formula (19), the ligands, L¹¹¹ and L¹¹² are independently selected from the following structures: 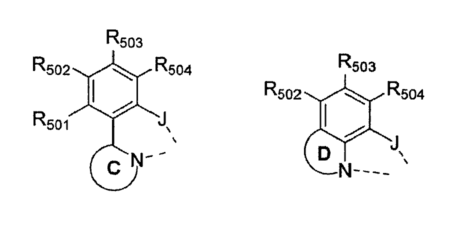
M² is a bivalent or trivalent metal;
k is 0 when M² is a bivalent metal, while k is 1 when M² is a trivalent metal;
T represents (C6-C60)aryloxy or tri(C6-C60)arylsilyl, and the aryloxy and triarylsilyl of T may be further substituted by (C1-C60)alkyl or (C6-C60)aryl;
J represents O, S or Se;
ring C represents oxazole, thiazole, imidazole, oxadiazole, thiadiazole, benzoxazole, benzothiazole, benzimidazole, pyridine or quinoline;
ring D represents pyridine or quinoline, and ring D may be further substituted by (C1-C60)alky, or phenyl or naphthyl with or without (C1-C60)alkyl substituent(s);
R₅₀₁ through R₅₀₄ independently represent hydrogen, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₅₀₁ to form a fused ring; and
ring C or the aryl of R₅₀₁ through R₅₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

The compounds for an electroluminescent layer, having electroluminescent peak of wavelength of not less than 560 nm, can be exemplified by the following compounds, but they are not restricted thereto.

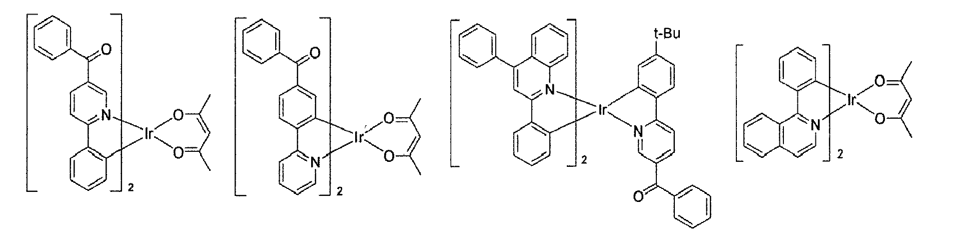

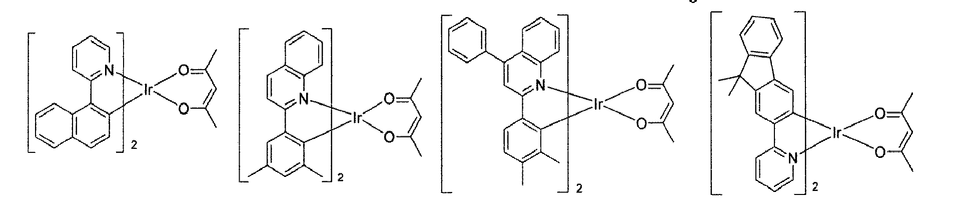

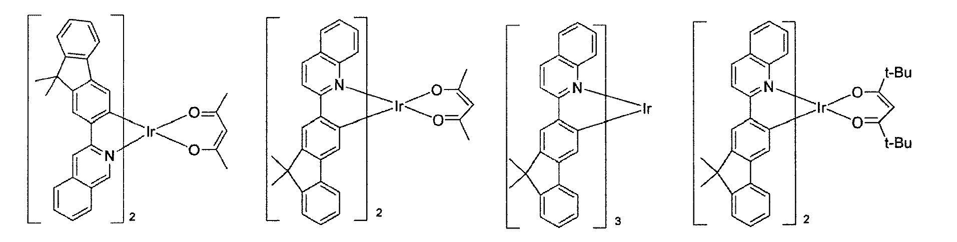

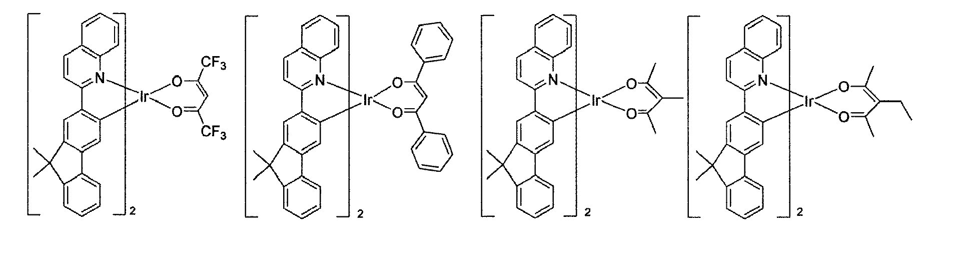

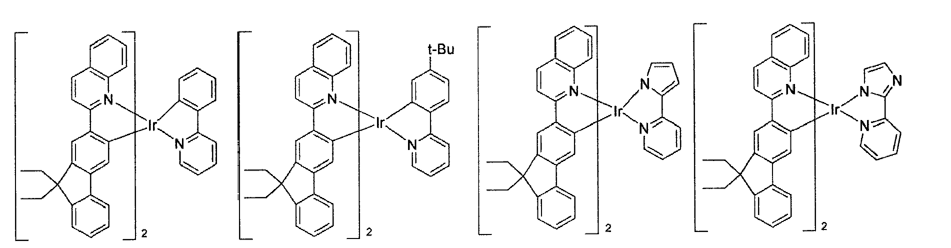

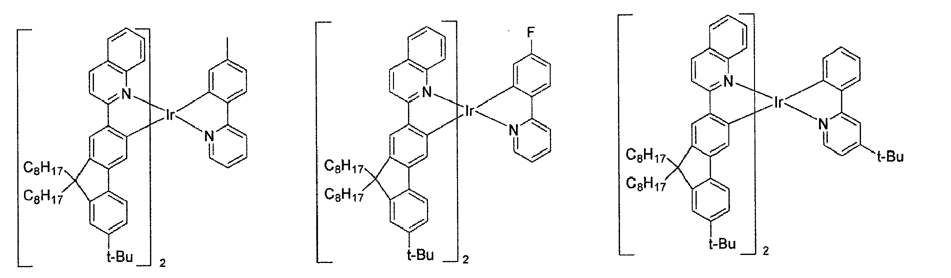

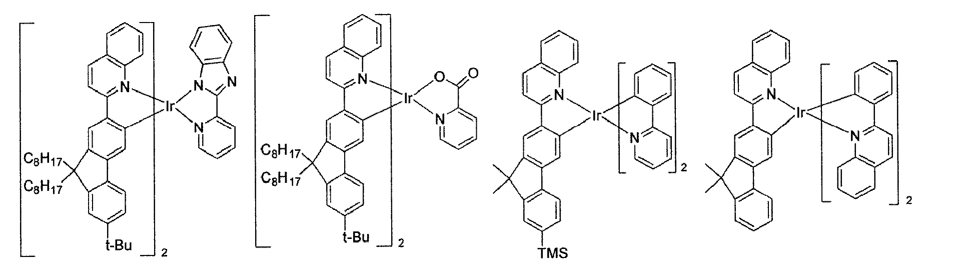

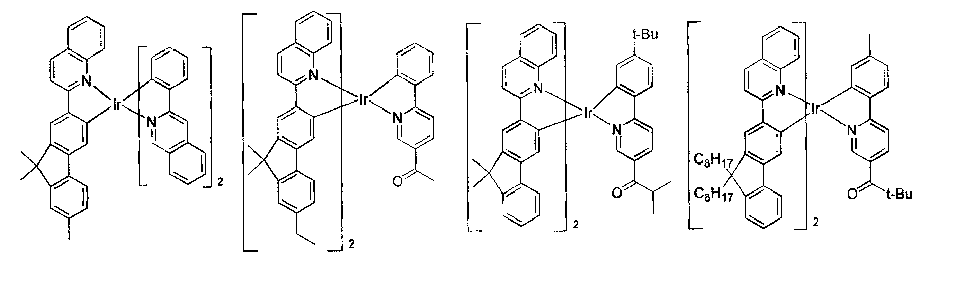

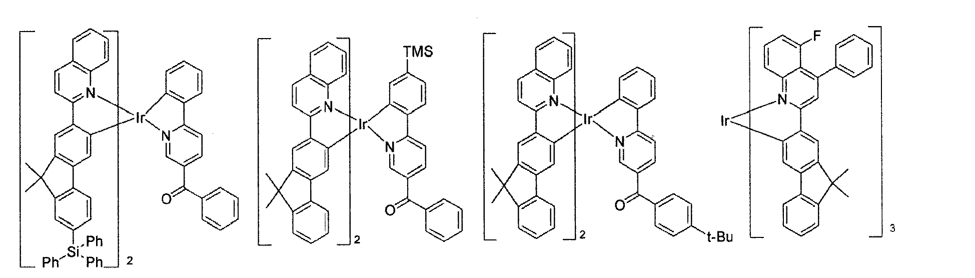

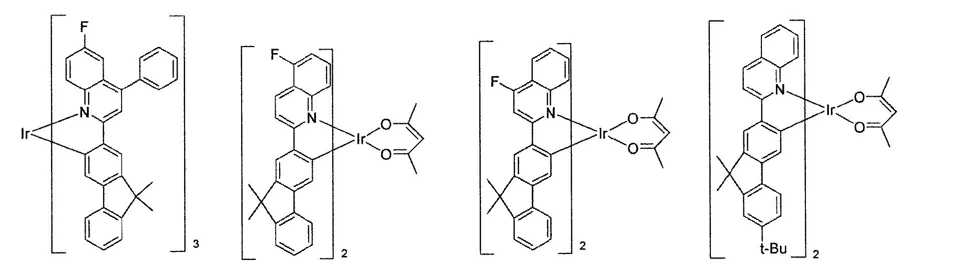

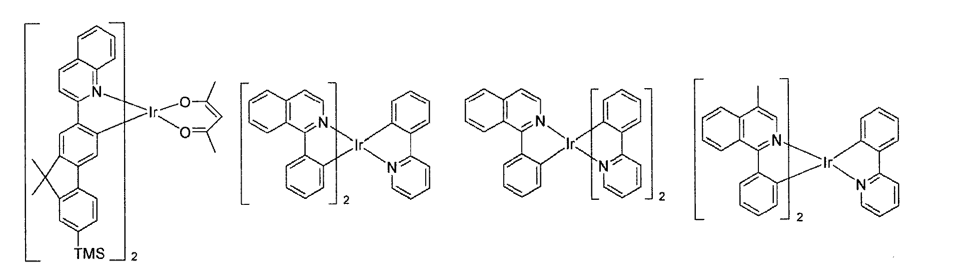

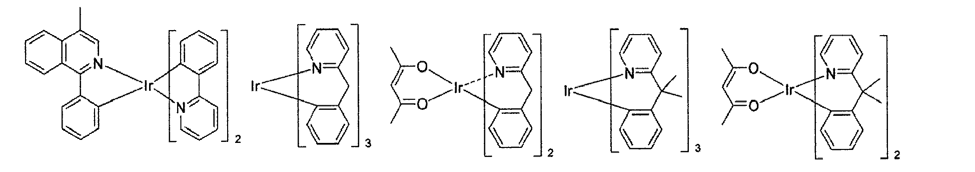

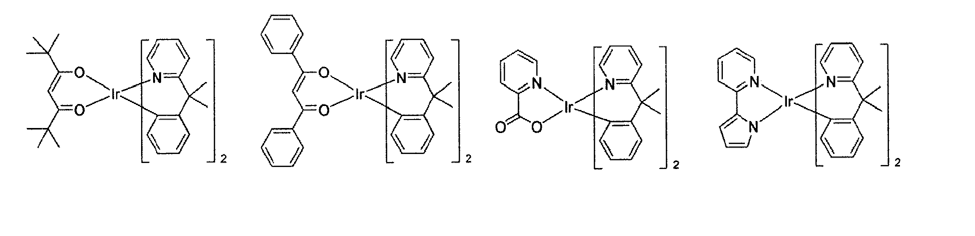

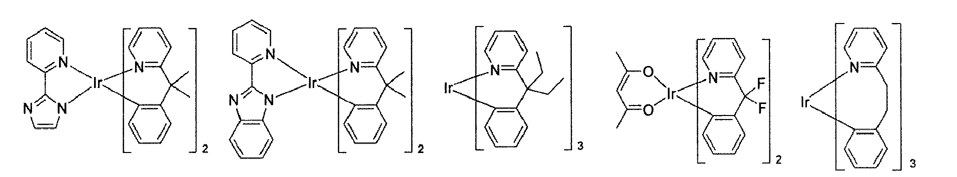

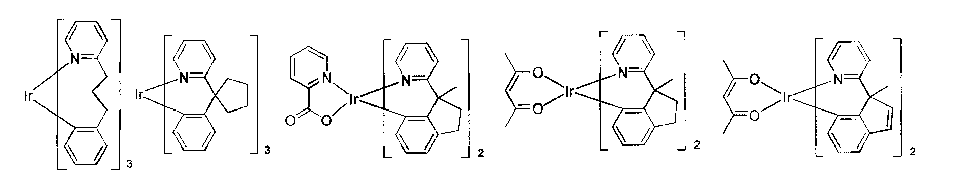

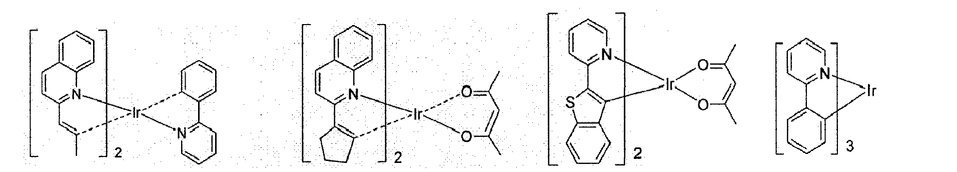

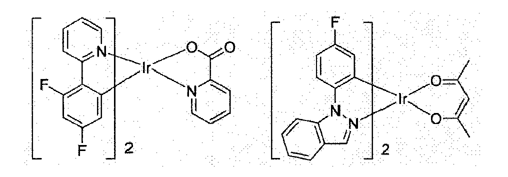

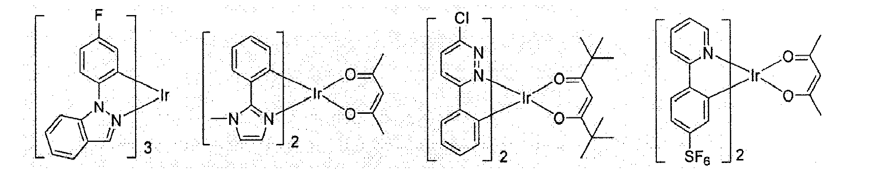

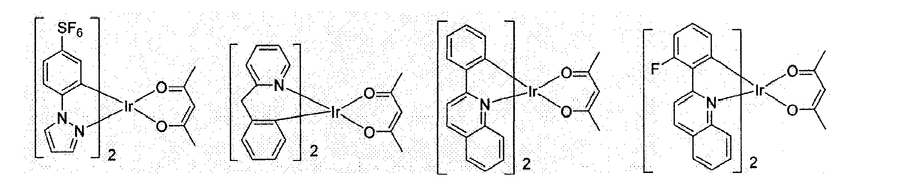

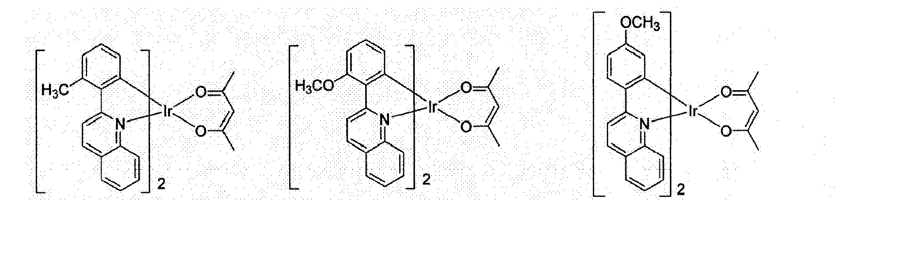

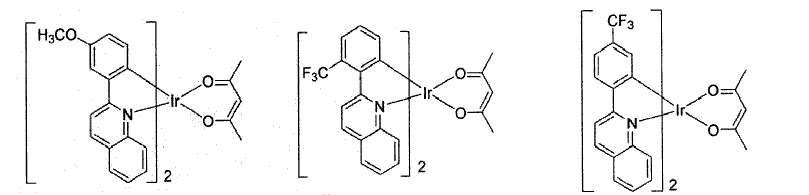

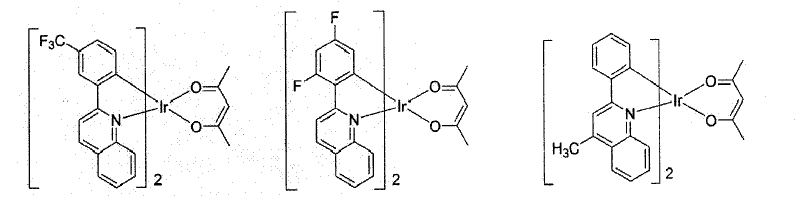

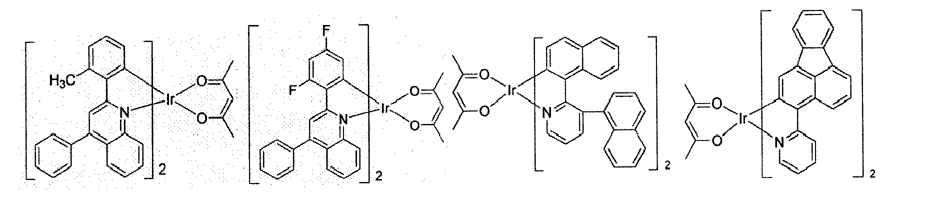

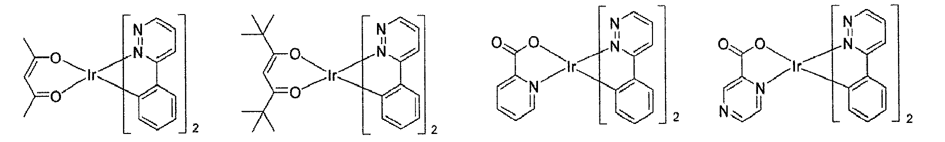

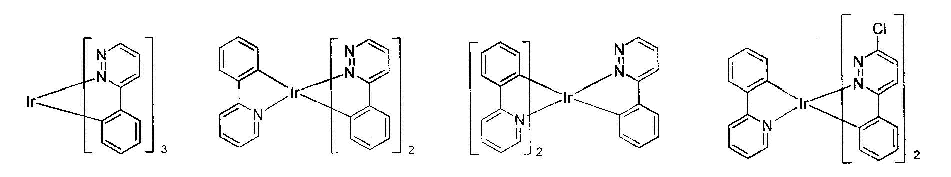

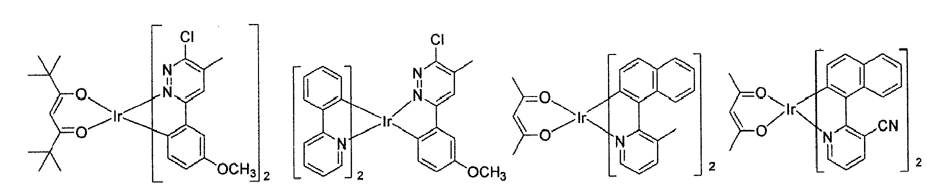

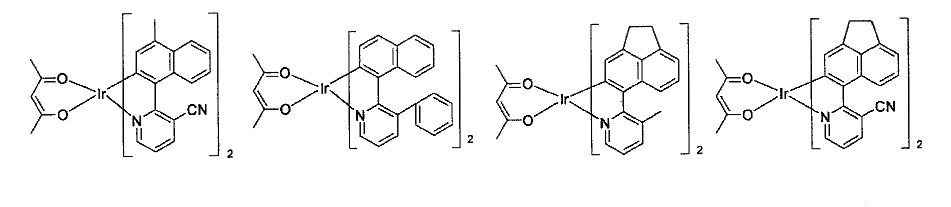

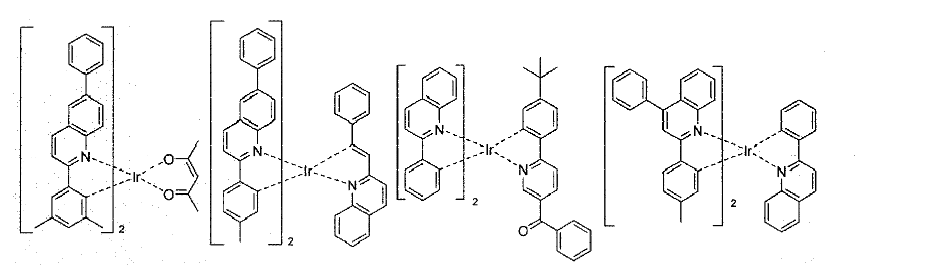

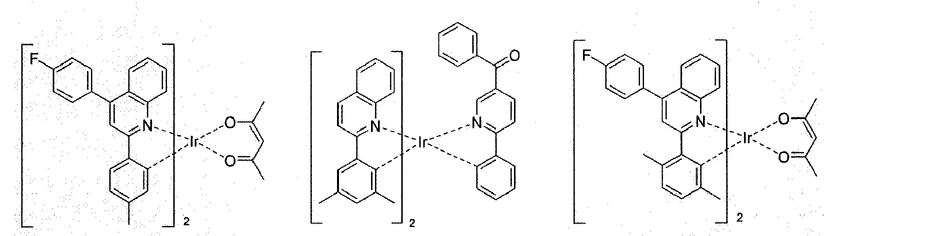

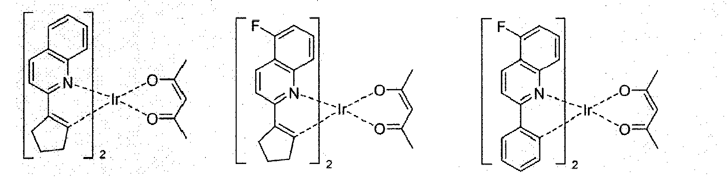

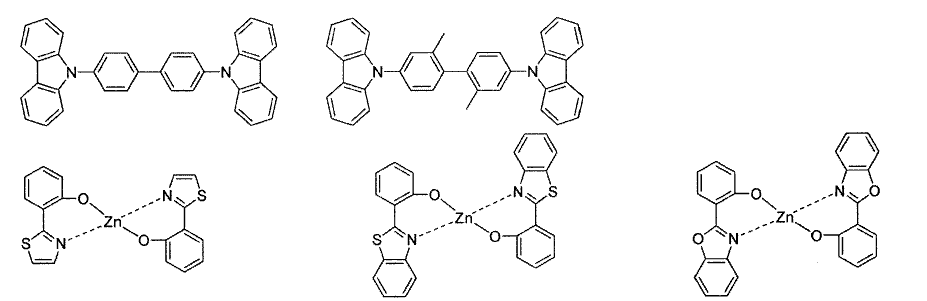

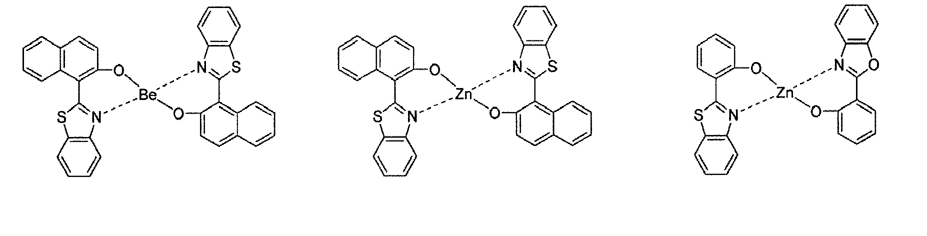

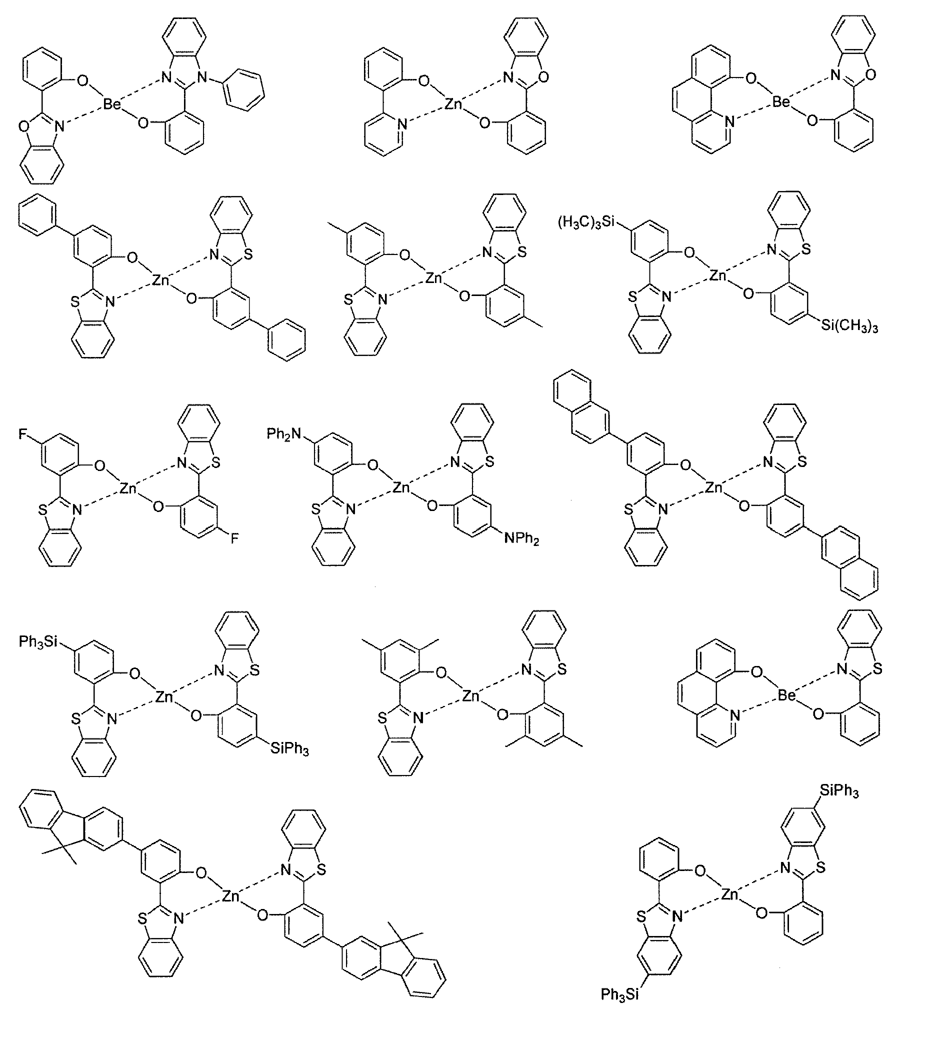

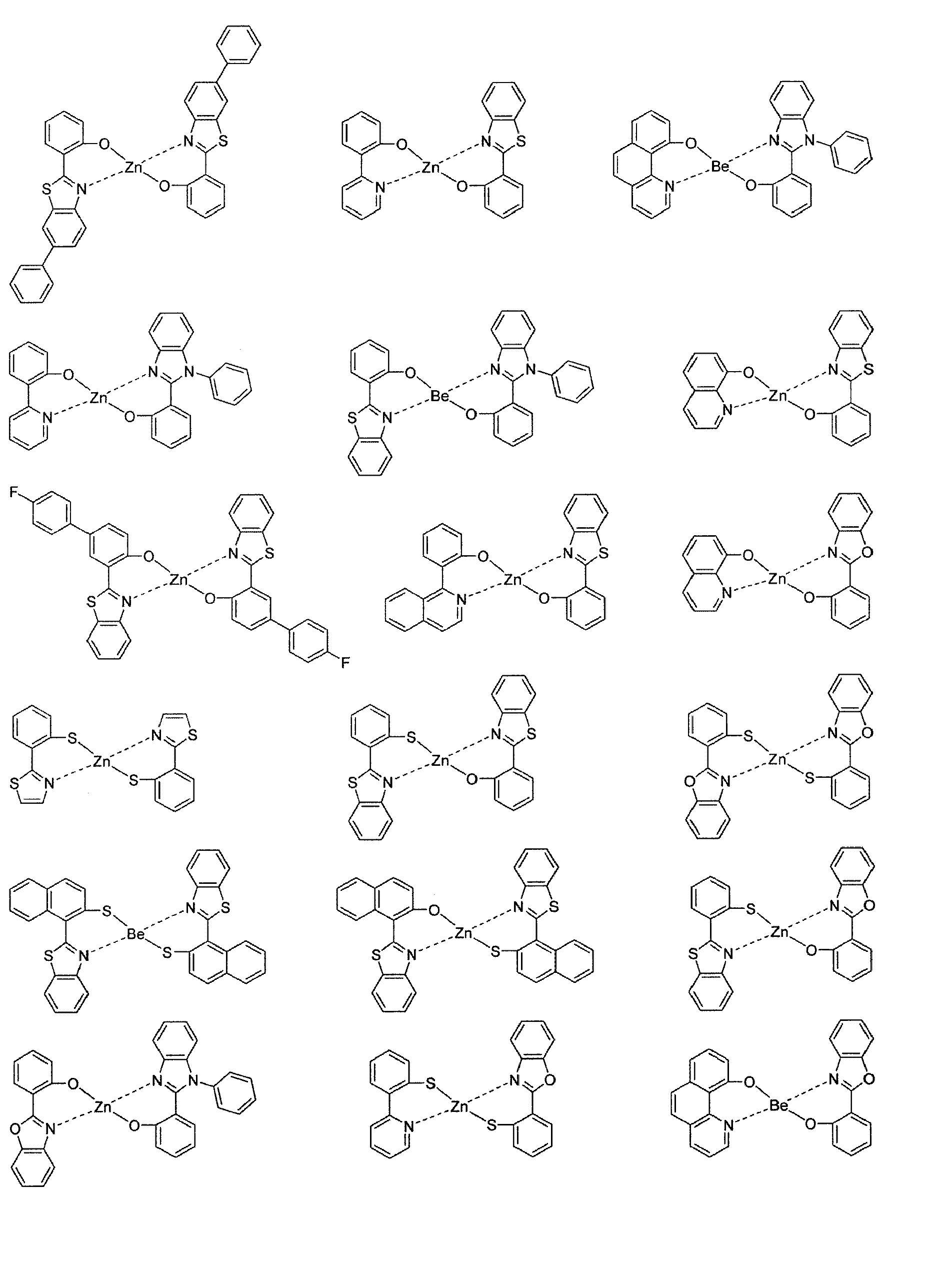

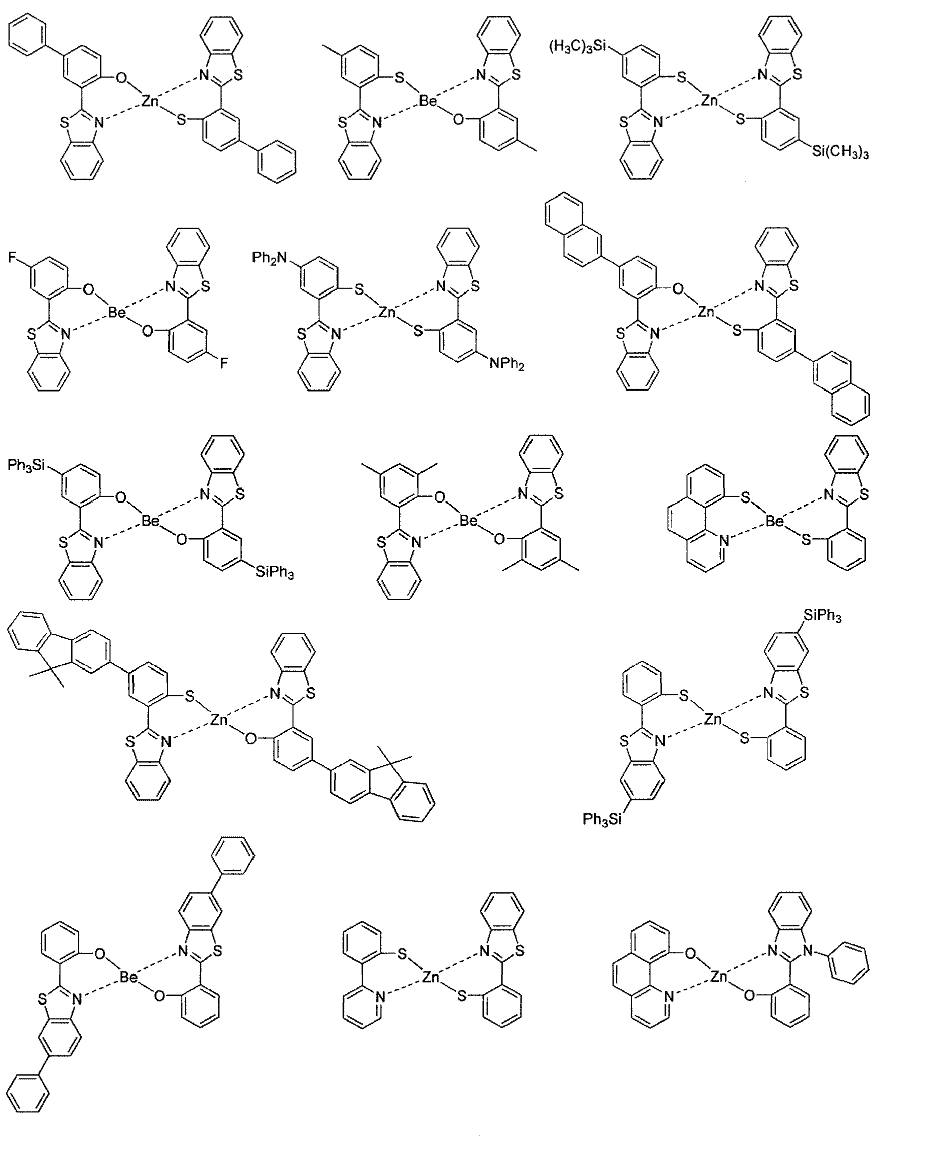

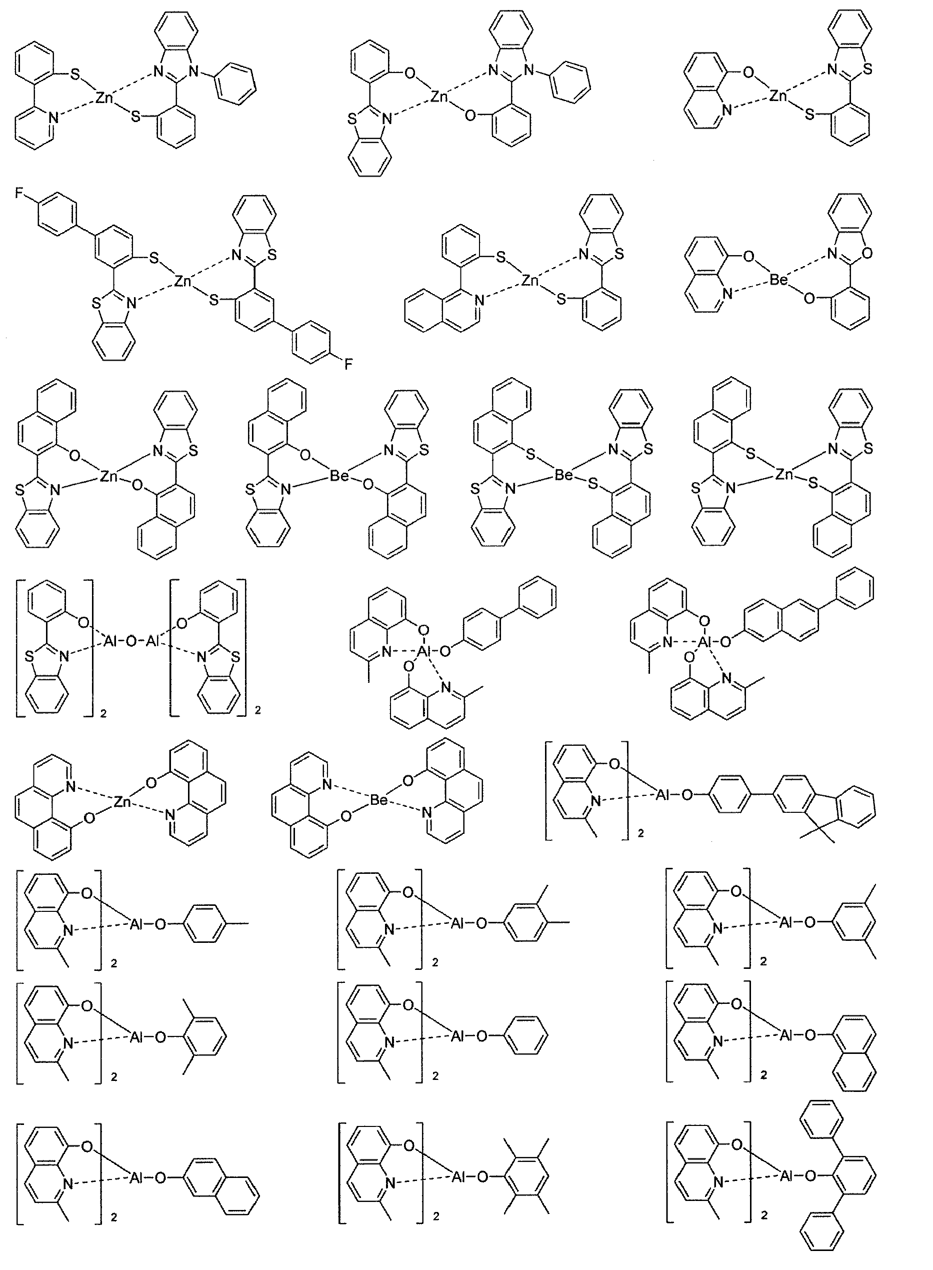

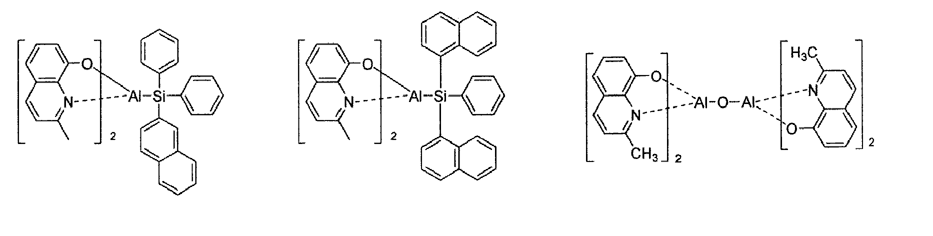

In an organic electroluminescent device according to the present invention, it is preferable to place one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal, or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the invention exhibit high luminous efficiency and excellent life property of material, so that OLED's having very good operation life can be manufactured therefrom.

### Best Mode

The present invention is further described by referring to representative compounds with regard to the organic electroluminescent compounds according to the invention, preparation thereof and luminescent properties of the devices manufactured therefrom, but those examples are provided for illustration of the embodiments only, not being intended to limit the scope of the invention by any means.

### Preparation Examples

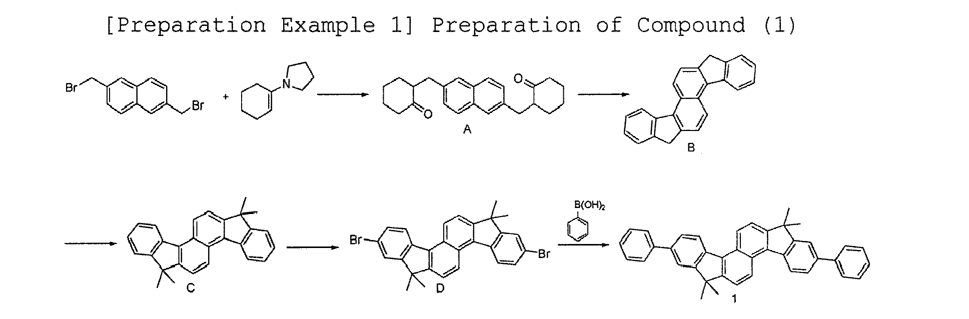

### Preparation of Compound (A)

Under nitrogen atmosphere, 2,6-bis(bromomethyl)naphthalene (5 g, 15.9 mmol) was dissolved in dioxane (150 mL), and 1-pyrrolidino-1-cyclohexene (5.3 g, 35 mmol) was added thereto, and the mixture was heated under reflux for 18 hours. After adding water (200 mL), the resultant mixture was heated for 2 hours. The solvent was evaporated off, and the residue was worked-up by using ether, 5% HCl and 5% NaHCO₃ solution. After evaporation, the residue was purified via column chromatography by using benzene and hexane (1:1) to obtain Compound (A) (4.3 g, 78%).

### Preparation of Compound (B)

Compound (A) (4.3 g, 12.3 mmol) was dissolved in a mixture (70 mL) of CHCl₃ and 10% methanesulfonic acid, and the solution was stirred at ambient temperature for 2 hours. After quenching the reaction by adding bicarbonate solution, the mixture was worked-up with NaHCO₃ and water. The solvent was evaporated, and the residue was purified via column chromatography. The organic product (1.3 g, 31%) was then dissolved in triglyme with 10% Pd/C, and the mixture was heated under reflux for 16 hours. After work-up, purification via column chromatography by using hexane gave Compound (B) (1.06 g, 91%).

### Preparation of Compound (C)

Compound B (5.2 g, 20.5 mmol) was dissolved in tetrahydrofuran (50 mL), and the solution was chilled to -78°C. To the solution, n-BuLi (1.6 M in hexane) (29.4 mL, 47.0 mmol) was slowly added dropwise. After 1 hour of reaction, iodomethane (7.6 mL, 53.2 mmol) was added thereto, and the mixture was slowly warmed to room temperature. After 1 hour of stirring, the temperature was lowered again to -78°C, and n-BuLi (1.6 M in hexane) (37.1 mL, 59.3 mmol) was slowly added dropwise thereto. After 1 hour of reaction, iodomethane (7.6 mL, 53.2 mmol) was added thereto. The temperature was slowly raised, and the mixture was stirred at room temperature for 15 hours. The reaction was quenched by adding aqueous NH₄Cl solution and distilled water, and the organic layer was evaporated under reduced pressure. Recrystallization from hexane gave Compound (C) (1.7 g, 4.6 mmol).

### Preparation of Compound (D)

Compound (C) (1.7 g, 4.6 mmol) and FeCl₃ (11.3 mg, 0.1 mmol) were dissolved in chloroform (30 mL), and the solution was chilled to 0°C by using an ice-water bath. Solution of bromine (0.7 mL, 13.9 mmol) dissolved in chloroform (5 mL) was slowly added dropwise thereto, and the resultant mixture was stirred for 24 hours. When the reaction was completed, aqueous saturated sodium thiosulfate solution (50 mL) was added to quench the reaction. The organic layer was separated and evaporated under reduced pressure. Recrystallization from boiling n-hexane (100 mL) gave Compound (D) (1.6 g, 3.0 mmol).

### Preparation of Compound (1)

Compound (D) (2.0 g, 4.13 mmol), phenylboronic acid (1.3 g, 10.33 mmol) and tetrakis palladium (0) triphenylphosphine (Pd(PPh₃)₄) (0.6 g, 0.41 mmol) were dissolved in toluene (100 mL) and ethanol (50 mL), and aqueous 2M sodium carbonate solution (50 mL) was added thereto. The mixture was stirred under reflux at 120°C for 4 hours. Then the temperature was lowered to 25°C, and distilled water (200 mL) was added to the reaction mixture to quench the reaction. The mixture was extracted with ethyl acetate (150 mL), and the extract was evaporated under reduced pressure to dryness. Purification via column chromatography gave the target compound (Compound 1) (1.6 g, 3.33 mmol).

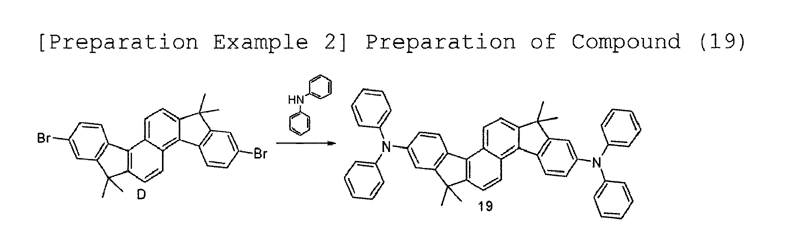

### Preparation of Compound (19)

In a reaction vessel, Compound (D) (7 g, 11.89 mmol), diphenylamine (6.1 g, 35.69 mmol), Pd(OAc)₂ (0.13 g, 0.59 mmol), tri(tert-butyl)phosphine (50% in toluene) (0.58 mL, 1.18 mmol), sodium tert-butoxide (4.57 g, 47.56 mmol) and DMF (100 mL) were stirred at 140°C for 12 hours. The mixture was then cooled to ambient temperature, and distilled water (100 mL) was added thereto. The solid produced was filtered under reduced pressure, and purified via column chromatography (ethyl acetate:hexane = 2:1) to obtain the target compound (Compound 19) (3.7 g, 40.47%).

The organic electroluminescent compounds (Compounds 1 to 1176) were prepared according to the same procedure as in Preparation Example 1 and 2, and the ¹H NMR and MS/FAB data of the compounds prepared are listed in Table 1.

**Table 1**

| Compound | ¹H NMR(CDCl₃, 200 MHz) | MS/FAB | |
|---|---|---|---|
| | | found | calculated |
| **1** | δ = 1.78(12H, s), 7.13(2H, m), 7.41(2H, m), 7.51∼7.52(8H, m), 7.69(2H, m), 7.83(2H, m), 7.95(2H, m), 8.15(2H, m) | 512.68 | 512.25 |
| **2** | δ = 1.78(12H, s), 7.13(2H, m), 7.58∼7.59(6H, m), 7.69∼7.73(4H, m), 7.83(2H, m), 7.92∼8(8H, m), 8.15(2H, m) | 612.80 | 612.28 |
| **5** | δ = 1.35(18H, s), 1.78(12H, s), 7.13(2H, m), 7.37∼7.38(8H, m), 7.69(2H, m), 7.83(2H, m), 7.95(2H, m), 8.15(2H, m) | 624.89 | 624.38 |
| **11** | δ = 1.78(12H, s), 7.13(2H, m), 7.69(2H, m), 7.82∼7.95(14H, m), 8.12∼8.15(6H, m), 8.93(4H, m) | 712.92 | 712.31 |
| **18** | δ = 1.78(12H, s), 3.82(6H, s), 7.13(2H, m), 7.29(2H, m), 7.39(2H, m), 7.5(2H, m), 7.69(2H, m), 7.77(4H, m), 7.83(2H, m), 7.95(2H, m), 8.12∼8.15(4H, m), 8.27(2H, m) | 718.92 | 718.33 |
| **19** | δ = 1.78(12H, s), 6.63∼6.64(10H, m), 6.81(6H, m), 7.13(2H, m), 7.2(8H, m), 7.84(2H, m), 7.95(2H, m) | 694.90 | 694.33 |
| **20** | δ = 1.78(12H, s), 6.64(2H, m), 6.81(2H, m), 6.98(4H, m), 7.13(2H, m), 7.38(4H, m), 7.53∼7.57(12H, m), 7.84(2H, m), 7.95(2H, m), 8.02∼8.07(8H, m) | 895.14 | 894.40 |
| **21** | δ = 1.78(12H, s), 6.64(2H, m), 6.81(2H, m), 7.13(2H, m), 7.36(4H, m), 7.49∼7.5(8H, m), 7.74∼7.77(8H, m), 7.84∼7.88(10H, m), 7.95(2H, m) | 895.14 | 894.40 |
| **22** | δ = 1.78(12H, s), 2.34(12H, s), 6.51(8H, m), 6.64(2H, m), 6.81(2H, m), 6.98(8H, m), 7.13(2H, m), 7.84(2H, m), 7.95(2H, m) | 751.01 | 750.40 |
| **25** | δ = 1.78(12H, s), 6.63∼6.64(6H, m), 6.81(4H, m), 6.98(2H, m), 7.13(2H, m), 7.2(4H, m), 7.38(2H, m), 7.53∼7.57(6H, m), 7.84(2H, m), 7.95(2H, m), 8.02∼8.07(4H, m) | 795.02 | 794.37 |
| **29** | δ = 1.35(18H, s), 1.78(12H, s), 6.55(4H, m), 6.63∼6.64(6H, m), 6.81(4H, m), 7.01(4H, m), 7.13(2H, m), 7.2(4H, m), 7.84(2H, m), 7.95(2H, m) | 807.12 | 806.46 |
| **31** | δ = 1.72(12H, s), 1.78(12H, s), 6.58∼6.64(8H, m), 6.75-6.81(6H, m), 7.13(2H, m), 7.2(4H, m), 7.28(2H, m), 7.38(2H, m), 7.55(2H, m), 7.62(2H, m), 7.84∼7.87(4H, m), 7.95(2H, m) | 927.22 | 926.46 |
| **34** | δ = 1.78(12H, s), 6.63∼6.69(10H, m), 6.81(4H, m), 7.13(2H, m), 7.2(4H, m), 7.54∼7.59(10H, m), 7.73(2H, m), 7.84(2H, m), 7.92∼8(8H, m) | 947.21 | 946.43 |
| **35** | δ = 1.72(12H, s), 1.78(12H, s), 6.63∼6.69(10H, m), 6.81(4H, m), 7.13(2H, m), 7.2(4H, m), 7.28(2H, m), 7.38(2H, m), 7.54∼7.55(6H, m), 7.63(2H, m), 7.77(2H, m), 7.84∼7.95(8H, m) | 1079.42 | 1078.52 |
| **38** | δ = 1.78(12H, s), 7.13(2H, m), 7.39∼7.41(10H, m), 7.51∼7.52(8H, m), 7.69(2H, m), 7.83(2H, m), 7.91∼7.95(10H, m), 8.15(2H, m) | 865.11 | 864.38 |
| **43** | δ = 1.78(12H, s), 7.13(2H, m), 7.41(2H, m), 7.51(4H, m), 7.59(4H, m), 7.69(2H, m), 7.79∼7.83(6H, m), 7.95∼8(6H, m), 8.15(2H, m), 8.4(4H, m) | 764.99 | 764.34 |
| **45** | δ = 1.78(12H, s), 7.13(2H, m), 7.41(1H, m), 7.51∼7.52(4H, m), 7.58∼7.59(3H, m), 7.69∼7.73(3H, m), 7.83(2H, m), 7.92∼8(5H, m), 8.15(2H, m) | 562.74 | 562.27 |
| **47** | δ = 1.78(12H, s), 7.13(2H, m), 7.25∼7.33(6H, m), 7.5(2H, m), 7.63∼7.69(8H, m), 7.79∼7.83(6H, m), 7.94∼7.95(4H, m), 8.12∼8.15(4H, m), 8.55(2H, m) | 843.06 | 842.37 |
| **48** | δ = 1.78(12H, s), 6.63(8H, m), 6.69(4H, m), 6.81(4H, m), 7.13(2H, m), 7.2(8H, m), 7.54(4H, m), 7.69(2H, m), 7.83(2H, m), 7.95(2H, m), 8.15(2H, m) | 847.10 | 846.40 |
| **52** | δ = 1.78(12H, s), 7.13(2H, m), 7.25(4H, m), 7.55(4H, m), 7.61(2H, m), 7.69(2H, m), 7.83(2H, m), 7.95(2H, m), 8.04∼8.08(4H, m), 8.15(2H, m), 8.42(2H, m), 8.55(2H, m) | 688.90 | 688.31 |
| **53** | δ = 1.78(12H, s), 7.13(2H, m), 7.25(4H, m), 7.55∼7.61(6H, m), 7.69∼7.73(3H, m), 7.83(2H, m), 7.92∼8.08(7H, m), 8.15(2H, m), 8.42(1H, m), 8.55(1H, m) | 688.90 | 688.31 |
| **63** | δ = 0.66(12H, s), 2.34(12H, s), 7.31∼7.35(4H, m), 7.6(4H, m), 7.66(2H, m), 7.82(2H, m), 7.95(2H, m), 8.14(2H, m) | 600.94 | 600.27 |
| **70** | δ = 0.66(12H, s), 7.35(2H, m), 7.5∼7.52(4H, m), 7.66(2H, m), 7.82∼7.86(4H, m), 7.95∼8(8H, m), 8.14(2H, m), 8.45(2H, m) | 757.12 | 756.18 |
| **72** | 8 = 0.66(12H, s), 6.63(8H, m), 6.79∼6.81(8H, m), 7.2(8H, m), 7.64∼7.66(4H, m), 8.14(2H, m) | 727.05 | 726.29 |
| **73** | δ = 0.66(12H, s), 6.79∼6.8(4H, m), 6.98(4H, m), 7.38(4H, m), 7.53∼7.57(12H, m), 7.64∼7.66(4H, m), 8.02∼8.07(8H, m), 8.14(2H, m) | 927.29 | 926.35 |
| **78** | δ = 0.66(12H, s), 6.63(4H, m), 6.79∼6.81(6H, m), 6.98(2H, m), 7.2(4H, m), 7.38(2H, m), 7.53∼7.57(6H, m), 7.64∼7.66(4H, m), 8.02∼8.07(4H, m), 8.14(2H, m) | 827.17 | 826.32 |
| **84** | δ = 0.66(12H, s), 1.72(12H, s), 6.58∼6.63(6H, m), 6.75∼6.81(8H, m), 7.2(4H, m), 7.28(2H, m), 7.38(2H, m), 7.55(2H, m), 7.62∼7.66(6H, m), 7.87(2H, m), 8.14(2H, m) | 959.37 | 958.41 |
| **91** | δ = 0.66(12H, s), 7.35∼7.41(12H, m), 7.51∼7.52(8H, m), 7.66(2H, m), 7.82(2H, m), 7.91∼7.95(10H, m), 8.14(2H, m) | 897.26 | 896.33 |
| **106** | δ = 0.66(12H, s), 7.25(4H, m), 7.35(2H, m), 7.55∼7.66(8H, m), 7.73(1H, m), 7.82(2H, m), 7.92∼8.08(7H, m), 8.14(2H, m), 8.42(1H, m), 8.55(1H, m) | 721.04 | 720.27 |
| **108** | δ = 3.82(6H, s), 7.5∼7.51(3H, m), 7.58∼7.59(6H, m), 7.69∼7.73(3H, m), 7.82∼7.92(4H, m), 7.99∼8(6H, m), 8.18(1H, m), 8.3 (1H, m) | 586.72 | 586.24 |
| **118** | δ = 3.82(6H, s), 7.25∼7.33(7H, m), 7.5∼7.51(3H, m), 7.62∼7.63(3H, m), 7.69∼7.71(3H, m), 7.82(1H, m), 7.93∼8(4H, m), 8.12(2H, m), 8.24(1H, m), 8.55(2H, m) | 664.79 | 664.26 |
| **125** | δ = 3.82(6H, s), 5.93(1H, m), 6.48(1H, m), 6.55(1H, m), 6.63(8H, m), 6.77∼6.81(5H, m), 7.2(8H, m), 7.51(1H, m), 7.69(1H, m), 7.82∼7.84(2H, m), 7.99∼8(2H, m) | 668.83 | 668.29 |
| **126** | δ = 3.82(6H, s), 5.93(1H, m), 6.48(1H, m), 6.55(1H, m), 6.77(1H, m), 6.98(4H, m), 7.38(4H, m), 7.51∼7.57(13H, m), 7.69(1H, m), 7.82∼7.84(2H, m), 7.99∼8.07(10H, m) | 869.06 | 868.36 |
| **127** | δ = 3.82(6H, s), 5.93(1H, m), 6.48(1H, m), 6.55(1H, m), 6.77(1H, m), 7.36(4H, m), 7.49∼7.51(9H, m), 7.69∼7.88(19H, m), 7.99∼8(2H, m) | 869.06 | 868.36 |
| **132** | δ = 3.82(6H, s), 5.93(1H, m), 6.48(1H, m), 6.55(1H, m), 6.63(4H, m), 6.77∼6.81(3H, m), 7.2(4H, m), 7.36(2H, m), 7.49∼7.51(5H, m), 7.69∼7.88(11H, m), 7.99∼8(2H, m) | 768.94 | 768.33 |
| **138** | δ = 3.82(6H, s), 5.93(1H, m), 6.48(1H, m), 6.55(1H, m), 6.63(4H, m), 6.69(4H, m), 6.77∼6.81(3H, m), 7.2(4H, m), 7.41(2H, m), 7.51∼7.54(13H, m), 7.69(1H, m), 7.82∼7.84(2H, m), 7.99∼8(2H, m) | 821.02 | 820.36 |
| **143** | δ = 3.82(6H, s), 7.39(8H, m), 7.47∼7.55(11H, m), 7.61(2H, m), 7.69(1H, m), 7.82∼7.91(14H, m), 7.99∼8.08(6H, m), 8.18(1H, m), 8.3(1H, m), 8.42(2H, m), 8.55(2H, m) | 1091.34 | 1090.43 |
| **153** | δ = 3.82(6H, s), 7.25∼7.33(6H, m), 7.5∼7.51(5H, m), 7.63∼7.69(7H, m), 7.79∼7.87(6H, m), 7.94∼8(4H, m), 8.12(2H, m), 8.18(1H, m), 8.3(1H, m), 8.55(2H, m) | 816.99 | 816.33 |
| **154** | δ = 3.82(6H, s), 6.63(8H, m), 6.69(4H, m), 6.81(4H, m), 7.2(8H, m), 7.5∼7.54(7H, m), 7.69(1H, m), 7.82∼7.87(2H, m), 7.99∼8(2H, m), 8.18(1H, m), 8.3(1H, m) | 821.02 | 820.36 |
| **180** | δ = 6.88(2H, m), 7.06(2H, m), 7.36(4H, m), 7.49∼7.5(8H, m), 7.58(2H, m), 7.74∼7.88(20H, m) | 875.11 | 874.25 |
| **182** | δ = 1.35(36H, s), 6.55(8H, m), 6.88(2H, m), 7.01∼7.06(10H, m), 7.58(2H, m), 7.8(4H, m) | 890.30 | 889.44 |
| **184** | δ = 6.63(4H, m), 6.81(2H, m), 6.88(2H, m), 6.98(2H, m), 7.06(2H, m), 7.2(4H, m), 7.38(2H, m), 7.53∼7.58(8H, m), 7.8(4H, m), 8.02∼8.07(4H, m) | 774.99 | 774.22 |
| **185** | δ = 6.63(4H, m), 6.81(2H, m), 6.88(2H, m), 7.06(2H, m), 7.2(4H, m), 7.36(2H, m), 7.49∼7.5(4H, m), 7.58(2H, m), 7.74∼7.88(12H, m) | 774.99 | 774.22 |
| **194** | δ = 6.63(4H, m), 6.69(4H, m), 6.81(2H, m), 6.88(2H, m), 7.06(2H, m), 7.2(4H, m), 7.54∼7.59(12H, m), 7.73(2H, m), 7.8(4H, m), 7.92(2H, m), 8(4H, m) | 927.18 | 926.28 |
| **241** | δ = 1.35(18H, s), 6.33(2H, m), 6.55(4H, m), 6.63(4H, m), 6.81(2H, m), 7.01(4H, m), 7.2(4H, m), 7.43(2H, m), 7.59∼7.66(6H, m) | 754.96 | 754.36 |
| **246** | δ = 1.72(12H, s), 6.33(2H, m), 6.63(4H, m), 6.69(4H, m), 6.81(2H, m), 7.2(4H, m), 7.28(2H, m), 7.38∼7.43(4H, m), 7.54∼7.66(14H, m), 7.77(2H, m), 7.87∼7.93(4H, m) | 1027.25 | 1026.42 |
| **260** | δ = 6.63(8H, m), 6.69(4H, m), 6.81(4H, m), 7.2(8H, m), 7.54∼7.66(10H, m), 7.75(2H, m), 7.95(2H, m) | 794.93 | 794.29 |
| **265** | δ = 7.25(4H, m), 7.55∼7.66(12H, m), 7.73∼7.75(3H, m), 7.92∼8.08(7H, m), 8.42(1H, m), 8.55(1H, m) | 636.73 | 636.21 |
| **267** | δ = 1.51(8H, m), 2.09(8H, m), 6.63∼6.64(10H, m), 6.81(6H, m), 7.13(2H, m), 7.2(8H, m), 7.84(2H, m), 7.95(2H, m) | 746.98 | 746.37 |
| **270** | δ = 6.63∼6.64(10H, m), 6.81(6H, m), 7.13∼7.2(18H, m), 7.35(4H, m), 7.75(4H, m), 7.84(2H, m), 7.95(2H, m) | 939.15 | 938.37 |
| **273** | δ = 1.3(8H, m), 1.45(8H, m), 6.63(8H, m), 6.79∼6.81(8H, m), 7.2(8H, m), 7.64∼7.66(4H, m), 8.14(2H, m) | 779.13 | 778.32 |
| **276** | δ = 6.63∼6.64(10H, m), 6.81(6H, m), 7.11∼7.26(20H, m), 7.33∼7.35(6H, m), 7.75(2H, m), 7.84(2H, m), 7.95(2H, m) | 941.16 | 940.38 |
| **280** | δ = 5.85(1H, m), 6.6∼6.69(11H, m), 6.81(4H, m), 7.2(8H, m), 7.45∼7.5(6H, m), 7.58∼7.65(6H, m), 7.94∼7.96(2H, m), 8.03(1H, m), 8.18(1H, m) | 792.96 | 792.33 |
| **282** | δ = 6.63(8H, m), 6.81(4H, m), 6.95(2H, m), 7.02(2H, m), 7.2(8H, m), 7.36(6H, m), 7.78(6H, m), 7.87(2H, m), 8.37(2H, m) | 826.90 | 826.27 |
| **285** | δ = 6.63(8H, m), 6.81(4H, m), 6.88∼6.9(4H, m), 7.2(8H, m), 7.48(2H, m), 8.14(2H, m), 8.24(2H, m) | 666.76 | 666.23 |
| **286** | δ = 1.78(6H, s), 1.85(6H, s), 6.96(1H, m), 7.07(1H, m), 7.41(3H, m), 7.51∼7.52(8H, m), 7.69(1H, m), 7.77(1H, m), 7.83(1H, m), 7.92(1H, m), 7.98(1H, s), 8.15(1H, m), 8.36(1H, m) | 512.68 | 512.25 |
| **287** | δ = 1.78(6H, s), 1.85(6H, s), 6.96(1H, m), 7.07(1H, m), 7.41(1H, m), 7.58∼7.59(6H, m), 7.69∼7.77(4H, m), 7.83(1H, m), 7.92(3H, m), 7.98(1H, s), 8(4H, m), 8.15(1H, m), 8.36(1H, m) | 612.80 | 612.28 |
| **289** | δ = 1.78(6H, s), 1.85(6H, s), 2.34(6H, s), 6.96(1H, m), 7.07(1H, m), 7.29∼7.33(8H, m), 7.41(1H, m), 7.69(1H, m), 7.77(1H, m), 7.83(1H, m), 7.92(1H, m), 7.98(1H, s), 8.15(1H, m), 8.36(1H, m) | 540.74 | 540.28 |
| **292** | δ = 1.72(12H, s), 1.78(6H, s), 1.85(6H, s), 6.96(1H, m), 7.07(1H, m), 7.28(2H, m), 7.38∼7.41(3H, m), 7.55(2H, m), 7.63(2H, m), 7.69(1H, m), 7.77(3H, m), 7.83∼7.93(6H, m), 7.98(1H, s), 8.15(1H, m), 8.36(1H, m) | 745.00 | 744.38 |
| **296** | δ = 1.78(6H, s), 1.85(6H, s), 6.96(1H, m), 7.07(1H, m), 7.41(1H, m), 7.69(1H, m), 7.77∼7.93(13H, m), 7.98(1H, s), 8.12∼8.15(5H, m), 8.36(1H, m), 8.93(4H, m) | 712.92 | 712.31 |
| **302** | δ = 1.78(6H, s), 1.85(6H, s), 6.96(1H, m), 7.07(1H, m), 7.41(1H, m), 7.5∼7.52(4H, m), 7.69(1H, m), 7.77(1H, m), 7.83∼7.92(4H, m), 7.98(3H, s), 7.98∼8(4H, m), 8.15(1H, m), 8.36(1H, m), 8.45(2H, m) | 724.97 | 724.23 |
| **303** | δ = 1.78(6H, s), 1.85(6H, s), 6.51(1H, m), 6.63∼6.68(10H, m), 6.81(5H, m), 6.96(1H, m), 7.2(8H, m), 7.41∼7.46(2H, m), 7.84(1H, m), 7.98(1H, s), 8.36(1H, m) | 694.90 | 694.33 |
| **305** | δ = 1.78(6H, s), 1.85(6H, s), 6.51(1H, m), 6.64∼6.68(2H, m), 6.81(1H, m), 6.96(1H, m), 7.36∼7.5(14H, m), 7.74∼7.77(8H, m), 7.84∼7.88(9H, m), 7.98(1H, s), 8.36(1H, m) | 895.14 | 894.40 |
| **306** | δ = 1.78(6H, s), 1.85(6H, s), 6.51(1H, m), 6.64∼6.68(2H, m), 6.81(1H, m), 6.96∼6.98(5H, m), 7.38∼7.46(6H, m), 7.53∼7.57(12H, m), 7.84(1H, m), 7.98(1H, s), 8.02∼8.07(8H, m), 8.36(1H, m) | 895.14 | 894.40 |
| **310** | δ = 1.78(6H, s), 1.85(6H, s), 6.51(1H, m), 6.63∼6.68(6H, m), 6.81(3H, m), 6.96(1H, m), 7.2(4H, m), 7.36∼7.5(8H, m), 7.74∼7.77(4H, m), 7.84∼7.88(5H, m), 7.98(1H, s), 8.36(1H, m) | 795.02 | 794.37 |
| **311** | δ = 1.78(6H, s), 1.85(6H, s), 6.51(1H, m), 6.63∼6.68(6H, m), 6.81(3H, m), 6.96∼6.98(3H, m), 7.2(4H, m), 7.38∼7.46(4H, m), 7.53∼7.57(6H, m), 7.84(1H, m), 7.98(1H, s), 8.02∼8.07(4H, m), 8.36(1H, m) | 795.02 | 794.37 |
| **313** | δ = 1.78(6H, s), 1.85(6H, s), 2.34(12H, s), 6.36(4H, m), 6.51(1H, m), 6.63∼6.71(8H, m), 6.81(3H, m), 6.96(1H, m), 7.2(4H, m), 7.41∼7.46(2H, m), 7.84(1H, m), 7.98(1H, s), 8.36(1H, m) | 751.01 | 750.40 |
| **319** | δ = 1.78(6H, s), 1.85(6H, s), 6.51(1H, m), 6.63∼6.69(10H, m), 6.81(3H, m), 6.96(1H, m), 7.2(4H, m), 7.41∼7.46(2H, m), 7.54∼7.59(10H, m), 7.73(2H, m), 7.84(1H, m), 7.92(2H, m), 7.98(1H, s), 8(4H, m), 8.36(1H, m) | 947.21 | 946.43 |
| **322** | δ = 1.78(6H, s), 1.85(6H, s), 2.34(6H, s), 6.44(2H, m), 6.51∼6.68(11H, m), 6.81(3H, m), 6.96(1H, m), 7.08(2H, m), 7.2(4H, m), 7.41∼7.46(2H, m), 7.84(1H, m), 7.98(1H, s), 8.36(1H, m) | 722.96 | 722.37 |
| **327** | δ = 1.78(6H, s), 1.85(6H, s), 6.96(1H, m), 7.07(1H, m), 7.39∼7.41(10H, m), 7.51∼7.52(4H, m), 7.58∼7.59(3H, m), 7.69∼7.77(3H, m), 7.83(1H, m), 7.91∼7.92(10H, m), 7.98(1H, s), 8(2H, m), 8.15(1H, m), 8.36(1H, m) | 915.17 | 914.39 |
| **334** | δ = 1.78(6H, s), 1.85(6H, s), 6.63(8H, m), 6.69(4H, m), 6.81(4H, m), 6.96(1H, m), 7.07(1H, m), 7.2(8H, m), 7.41(1H, m), 7.54(4H, m), 7.69(1H, m), 7.77(1H, m), 7.83(1H, m), 7.92(1H, m), 7.98(1H, s), 8.15(1H, m), 8.36(1H, m) | 847.10 | 846.40 |
| **340** | δ = 1.78(6H, s), 1.85(6H, s), 6.96(1H, m), 7.07(1H, m), 7.25(4H, m), 7.41(1H, m), 7.55∼7.61(6H, m), 7.69∼7.77(3H, m), 7.83(1H, m), 7.92(2H, m), 7.98(1H, s), 8∼8.08(4H, m), 8.15(1H, m), 8.36(1H, m), 8.42(1H, m), 8.55(1H, m) | 688.90 | 688.31 |
| **342** | δ = 0.66(12H, s), 7.35(2H, m), 7.58∼7.65(8H, m), 7.73(2H, m), 7.82(2H, m), 7.88(1H, s), 7.92∼8(8H, m), 8.65(1H, m) | 644.95 | 644.24 |
| **364** | δ = 0.66(12H, s), 2.34(24H, s), 6.36(8H, m), 6.71(4H, m), 6.79∼6.8(4H, m), 7.6∼7.65(4H, m), 7.88(1H, s), 8.65(1H, m) | 839.27 | 838.41 |
| **366** | δ = 0.66(12H, s), 6.63(4H, m), 6.79∼6.81(6H, m), 6.98(2H, m), 7.2(4H, m), 7.38(2H, m), 7.53∼7.65(10H, m), 7.88(1H, s), 8.02∼8.07(4H, m), 8.65(1H, m) | 827.17 | 826.32 |
| **370** | δ = 0.66(12H, s), 6.62(2H, m), 6.7(2H, m), 6.79∼6.8(4H, m), 7.27(2H, m), 7.36(2H, m), 7.55∼7.65(6H, m), 7.88(1H, s), 8.04∼8.09(6H, m), 8.65(1H, m) | 731.01 | 730.27 |
| **382** | δ = 0.66(12H, s), 7.35∼7.41(11H, m), 7.51∼7.52(4H, m), 7.58∼7.65(5H, m), 7.73(1H, m), 7.82(2H, m), 7.88(1H, s), 7.91∼8(13H, m), 8.65(1H, m) | 947.32 | 946.35 |
| **389** | δ = 0.66(12H, s), 6.63(8H, m), 6.69(4H, m), 6.81(4H, m), 7.2(8H, m), 7.35(2H, m), 7.54∼7.65(6H, m), 7.82(2H, m), 7.88(1H, s), 7.95(2H, m), 8.65(1H, m) | 879.24 | 878.35 |
| **405** | δ = 2.34(12H, s), 3.2(3H, s), 3.82(3H, s), 6.57(1H, m), 6.92(1H, m), 6.98(1H, m), 7.31(2H, m), 7.38(1H, m), 7.39(1H, s), 7.6∼7.63(6H, m), 7.79(1H, m), 8.04(1H, m), 8.18(1H, m) | 542.71 | 542.27 |
| **413** | δ = 3.2(3H, s), 3.82(3H, s), 5.8(1H, m), 6.01(1H, m), 6.63∼6.69(10H, m), 6.81(4H, m), 6.98(1H, m), 7.2(8H, m), 7.32∼7.38(2H, m), 7.39(1H, s), 8.03∼8.04(2H, m) | 668.83 | 668.29 |
| **417** | δ = 2.34(12H, s), 3.2(3H, s), 3.82(3H, s), 5.8(1H, m), 6.01(1H, m), 6.51(8H, m), 6.67∼6.69(2H, m), 6.98(9H, m), 7.32∼7.38(2H, m), 7.39(1H, s), 8.03∼8.04(2H, m) | 724.93 | 724.36 |
| **420** | δ = 3.2(3H, s), 3.82(3H, s), 5.8(1H, m), 6.01(1H, m), 6.63∼6.69(6H, m), 6.81(2H, m), 6.98(1H, m), 7.2(4H, m), 7.32∼7.38(4H, m), 7.39(1H, s), 7.49∼7.5(4H, m), 7.74∼7.77(4H, m), 7.84∼7.88(4H, m), 8.03∼8.04(2H, m) | 768.94 | 768.33 |
| **421** | δ = 3.2(3H, s), 3.82(3H, s), 5.8(1H, m), 6.01(1H, m), 6.63∼6.69(6H, m), 6.81(2H, m), 6.98(3H, m), 7.2(4H, m), 7.32∼7.38(4H, m), 7.39(1H, s), 7.53∼7.57(6H, m), 8.02∼8.07(6H, m) | 768.94 | 768.33 |
| **428** | δ = 1.72(12H, s), 3.2(3H, s), 3.82(3H, s), 5.8(1H, m), 6.01(1H, m), 6.58∼6.81(12H, m), 6.98(1H, m), 7.2(4H, m), 7.28∼7.38(6H, m), 7.39(1H, s), 7.55(2H, m), 7.62(2H, m), 7.87(2H, m), 8.03∼8.04(2H, m) | 901.15 | 900.42 |
| **432** | δ = 2.34(6H, s), 3.2(3H, s), 3.82(3H, s), 5.8(1H, m), 6.01(1H, m), 6.44(2H, m), 6.55∼6.69(10H, m), 6.81(2H, m), 6.98(1H, m), 7.08(2H, m), 7.2(4H, m), 7.32∼7.38(2H, m), 7.39(1H, s), 8.03∼8.04(2H, m) | 696.88 | 696.33 |
| **446** | δ = 3.2(3H, s), 3.82(3H, s), 6.57∼6.63(9H, m), 6.81(4H, m), 6.92(1H, m), 6.98∼7.04(3H, m), 7.2(8H, m), 7.38(1H, m), 7.39(1H, s), 7.53∼7.54(4H, m), 7.62∼7.63(2H, m), 7.78∼7.79(3H, m), 8.04∼8.07(3H, m), 8.18(1H, m), 8.49(2H, m) | 921.14 | 920.39 |
| **452** | δ = 6.99(1H, m), 7.26(1H, m), 7.35(1H, m), 7.48(1H, m), 7.58∼7.59(6H, m), 7.65(1H, m), 7.73(2H, m), 7.92(2H, m), 8∼8.05(5H, m), 8.08(2H, s), 8.08∼8.11(lH, m), 8.29(1H, m) | 592.77 | 592.13 |
| **468** | δ = 6.43(1H, m), 6.63(8H, m), 6.81(4H, m), 6.88(1H, m), 7.06(1H, m), 7.2∼7.26(9H, m), 7.33∼7.35(3H, m), 7.8(1H, m), 8.08(1H, s), 8.29(1H, m) | 674.87 | 674.19 |
| **470** | δ = 6.43(1H, m), 6.88(1H, m), 7.06(1H, m), 7.26(1H, m), 7.33∼7.36(7H, m), 7.49∼7.5(8H, m), 7.74∼7.88(17H, m), 8.08(1H, s), 8.29(1H, m) | 875.11 | 874.25 |
| **471** | δ = 6.43(1H, m), 6.88(1H, m), 6.98(4H, m). 7.06(1H, m), 7.26(1H, m), 7.33∼7.38(7H, m), 7.53∼7.57(12H, m), 7.8(1H, m), 8.02∼8.07(8H, m), 8.08(1H, s), 8.29(1H, m) | 875.11 | 874.25 |
| **472** | δ = 2.34(12H, s), 6.43(1H, m), 6.51(8H, m), 6.88(1H, m), 6.98(8H, m), 7.06(1H, m), 7.26(1H, m), 7.33∼7.35(3H, m), 7.8(1H, m), 8.08(1H, s), 8.29(1H, m) | 730.98 | 730.25 |
| **478** | δ = 2.34(12H, s), 6.36(4H, m), 6.43(1H, m), 6.63(4H, m), 6.71(2H, m), 6.81(2H, m), 6.88(1H, m), 7.06(1H, m), 7.2∼7.26(5H, m), 7.33∼7.35(3H, m), 7.8(1H, m), 8.08(1H, s), 8.29(1H, m) | 730.98 | 730.25 |
| **518** | δ = 3.81(4H, s), 6.33∼6.41(3H, m), 6.51(4H, m), 6.65∼6.69(5H, m), 6.98∼7.01(8H, m), 7.4∼7.43(2H, m), 7.5(1H, m), 7.64(2H, s), 8.19(1H, m) | 666.76 | 666.23 |
| **523** | δ = 6.33∼6.41(3H, m), 6.63∼6.65(9H, m), 6.81(4H, m), 7.2(8H, m), 7.4∼7.43(2H, m), 7.5(1H, m), 7.64(2H, s), 8.19(1H, m) | 642.74 | 642.23 |
| **525** | δ = 6.33∼6.41(3H, m), 6.65(1H, m), 7.36∼7.43(6H, m), 7.49∼7.5(9H, m), 7.64(2H, s), 7.74∼7.77(8H, m), 7.84∼7.88(8H, m), 8.19(1H, m) | 842.98 | 842.29 |
| **526** | δ = 6.33∼6.41(3H, m), 6.65(1H, m), 6.98(4H, m), 7.38∼7.43(6H, m), 7.5∼7.57(13H, m), 7.64(2H, s), 8.02∼8.07(8H, m), 8.19(1H, m) | 842.98 | 842.29 |
| **531** | δ = 6.33∼6.41(3H, m), 6.63∼6.65(5H, m), 6.81(2H, m), 6.98(2H, m), 7.2(4H, m), 7.38∼7.43(4H, m), 7.5∼7.57(7H, m), 7.64(2H, s), 8.02∼8.07(4H, m), 8.19(1H, m) | 742.86 | 742.26 |
| **562** | δ = 1.51(8H, m), 2.09∼2.11(8H, m), 6.51(1H, m), 6.63∼6.68(10H, m), 6.81(5H, m), 6.96(1H, m), 7.2(8H, m), 7.41∼7.46(2H, m), 7.84(1H, m), 7.98(1H, s), 8.36(1H, m) | 746.98 | 746.37 |
| **571** | δ = 6.5(1H, m), 6.61∼6.64(10H, m), 6.81(5H, m), 6.96(1H, m), 7.11(8H, m), 7.2∼7.26(12H, m), 7.33(8H, m), 7.41∼7.42(2H, m), 7.84(1H, m), 7.98(1H, s), 8.36(1H, m) | 943.18 | 942.40 |
| **577** | δ = 6.63(8H, m), 6.81(4H, m), 6.95(2H, m), 7.02(2H, m), 7.2(8H, m), 7.3(1H, m), 7.36(4H, m), 7.58(1H, m), 7.78(6H, m), 7.87∼7.88(3H, m), 8.88(1H, m) | 826.90 | 826.27 |
| **580** | δ = 5.83(1H, m), 6.05(1H, m), 6.63∼6.69(12H, m), 6.81(5H, m), 6.98(1H, m), 7.2(10H, m), 7.29(1H, m), 7.38(1H, m), 7.45∼7.5(3H, m), 7.58(2H, m), 7.62(1H, s), 8.03∼8.04(2H, m) | 792.96 | 792.33 |
| **583** | δ = 1.85(12H, s), 6.87(2H, m), 7.07(2H, m), 7.58∼7.59(6H, m), 7.73∼7.77(4H, m), 7.92∼8(10H, m) | 612.80 | 612.28 |
| **599** | δ = 1.85(12H, s), 6.51(2H, m), 6.63∼6.68(10H, m), 6.81(4H, m), 6.87(2H, m), 7.2(8H, m), 7.46(2H, m), 7.97(2H, m) | 694.90 | 694.33 |
| **601** | δ = 1.85(12H, s), 6.51(2H, m), 6.68(2H, m), 6.87(2H, m), 6.98(4H, m), 7.38(4H, m), 7.46(2H, m), 7.53∼7.57(12H, m), 7.97∼8.07(10H, m) | 895.14 | 894.40 |
| **602** | δ = 1.85(12H, s), 6.51(2H, m), 6.68(2H, m), 6.87(2H, m), 7.36(4H, m), 7.46∼7.5(10H, m), 7.74∼7.77(8H, m), 7.84∼7.88(8H, m), 7.97(2H, m) | 895.14 | 894.40 |
| **607** | δ = 1.85(12H, s), 6.51(2H, m), 6.63∼6.68(6H, m), 6.81(2H, m), 6.87(2H, m), 6.98(2H, m), 7.2(4H, m), 7.38(2H, m), 7.46(2H, m), 7.53∼7.57(6H, m), 7.97∼8.07(6H, m) | 795.02 | 794.37 |
| **655** | δ = 0.66(12H, s), 6.63(8H, m), 6.79∼6.81(8H, m), 7.2(8H, m), 7.64(2H, m), 7.7(2H, m), 8.01(2H, m) | 727.05 | 726.29 |
| **657** | δ = 0.66(12H, s), 6.79∼6.8(4H, m), 6.98(4H, m), 7.38(4H, m), 7.53∼7.57(12H, m), 7.64(2H, m), 7.7(2H, m), 8.01∼8.07(10H, m) | 927.29 | 926.35 |
| **662** | δ = 0.66(12H, s), 6.63(4H, m), 6.79∼6.81(6H, m), 7.2(4H, m), 7.36(2H, m), 7.49∼7.5(4H, m), 7.64(2H, m), 7.7∼7.77(6H, m), 7.84∼7.88(4H, m), 8.01(2H, m) | 827.17 | 826.32 |
| **663** | δ = 0.66(12H, s), 6.63(4H, m), 6.79∼6.81(6H, m), 6.98(2H, m), 7.2(4H, m), 7.38(2H, m), 7.53∼7.57(6H, m), 7.64(2H, m), 7.7(2H, m), 8.01∼8.07(6H, m) | 827.17 | 826.32 |
| **696** | δ = 3.2(6H, s), 6.57(2H, m), 6.81(2H, m), 6.92(2H, m), 7.55(4H, m), 7.61∼7.63(4H, m), 7.98∼8.08(6H, m), 8.42(2H, m), 8.55(2H, m) | 586.72 | 586.24 |
| **711** | δ = 3.2(6H, s), 5.8(2H, m), 6.01(2H, m), 6.63(8H, m), 6.81(6H, m), 7.2(8H, m), 7.32(2H, m), 7.98(2H, m) | 668.83 | 668.29 |
| **713** | δ = 3.2(6H, s), 5.8(2H, m), 6.01(2H, m), 6.81(2H, m), 6.98(4H, m), 7.32∼7.38(6H, m), 7.53∼7.57(12H, m), 7.98∼8.07(10H, m) | 869.06 | 869.36 |
| **719** | δ = 3.2(6H, s), 5.8(2H, m), 6.01(2H, m), 6.63(4H, m), 6.81(4H, m), 6.98(2H, m), 7.2(4H, m), 7.32∼7.38(4H, m), 7.53∼7.57(6H, m), 7.98∼8.07(6H, m) | 768.94 | 768.33 |
| **742** | δ = 3.2(6H, s), 6.57∼6.63(10H, m), 6.69(4H, m), 6.81(6H, m), 6.92(2H, m), 7.2(8H, m), 7.54(4H, m), 7.63(2H, m), 7.98(2H, m) | 821.02 | 820.36 |
| **760** | δ = 6.99(2H, m), 7.06(2H, m), 7.48(2H, m), 7.65(2H, m), 7.82∼7.93(10H, m), 8.01(2H, m), 8.12(4H, m), 8.93(4H, m) | 692.89 | 692.16 |
| **769** | δ = 6.43(2H, m), 6.98(4H, m), 7.06(2H, m), 7.33∼7.38(8H, m), 7.53∼7.57(12H, m), 8.01∼8.07(10H, m) | 875.11 | 874.25 |
| **773** | δ = 2.34(24H, s), 6.36(8H, m), 6.43(2H, m), 6.71(4H, m), 7.06(2H, m), 7.33∼7.34(4H, m), 8.01(2H, m) | 787.09 | 786.31 |
| **775** | δ = 6.43(2H, m), 6.63(4H, m), 6.81(2H, m), 6.98(2H. m), 7.06(2H, m), 7.2(4H, m), 7.33∼7.38(6H, m), 7.53∼7.57(6H, m), 8.01∼8.07(6H, m) | 774.99 | 774.22 |
| **795** | δ = 6.99(2H, m), 7.06(2H, m), 7.41(1H, m), 7.48∼7.52(6H, m), 7.58∼7.59(3H, m), 7.65(2H, m), 7.73(1H, m), 7.92(1H, m), 8∼8.01(4H, m) | 542.71 | 542.12 |
| **823** | δ = 6.39∼6.41(4H, m), 6.5(2H, m), 6.63(8H, m), 6.81(4H, m), 7.2(8H, m), 7.5(2H, m), 8.1(2H, m) | 642.74 | 642.23 |
| **831** | δ = 6.39∼6.41(4H, m), 6.5(2H, m), 6.63(4H, m), 6.81(2H, m), 6.98(2H, m), 7.2(4H, m), 7.38(2H, m), 7.5∼7.57(8H, m), 8.02∼8.1(6H, m) | 742.86 | 742.26 |
| **866** | δ = 6.5(2H, m), 6.61∼6.63(10H, m), 6.81(4H, m), 6.87(2H, m), 7.2(8H, m), 7.28(4H, m), 7.38∼7.42(6H, m), 7.55(4H, m), 7.87(4H, m), 7.97(2H, m) | 939.15 | 928.37 |
| **878** | δ = 6.63(8H, m), 6.81(4H, m), 6.95(2H, m), 7.02(2H, m), 7.2(8H, m), 7.36(4H, m), 7.63(2H, m), 7.78(6H, m), 7.87(2H, m), 8.01(2H, m) | 826.90 | 826.27 |
| **882** | δ = 5.83(2H, m), 6.05(2H, m), 6.63(8H, m), 6.81(6H, m), 6.98(2H, m), 7.2(8H, m), 7.29(2H, m), 7.38(2H, m), 7.53∼7.57(6H, m), 7.98∼8.07(6H, m) | 893.08 | 892.36 |
| **885** | δ = 1.78(12H, s), 7.5(2H, m), 7.58∼7.59(6H, m), 7.69∼7.73(4H, m), 7.83(2H, m), 7.92(2H, m), 8(4H, m), 8.15(2H, m), 8.53(2H, m) | 612.80 | 612.28 |
| **894** | δ = 1.78(12H, s), 7.23∼7.33(8H, m), 7.4(2H, m), 7.5(4H, m), 7.63(2H, m), 7.94(2H, m), 8.09∼8.12(4H, m), 8.53∼8.55(4H, m) | 690.87 | 690.30 |
| **900** | δ = 1.78(12H, s), 6.63∼6.64(10H, m), 6.81(6H, m), 7.2(8H, m), 7.5(2H, m), 7.84(2H, m), 8.53(2H, m) | 694.90 | 694.33 |
| **902** | δ = 1.78(12H, s), 6.64(2H, m), 6.81(2H, m), 6.98(4H, m), 7.38(4H, m), 7.5∼7.57(14H, m), 7.84(2H, m), 8.02∼8.07(8H, m), 8.53(2H, m) | 895.14 | 894.40 |
| **907** | δ = 1.78(12H, s), 6.63∼6.64(6H, m), 6.81(4H, m), 6.98(2H, m), 7.2(4H, m), 7.38(2H, m), 7.5∼7.57(8H, m), 7.84(2H, m), 8.02∼8.07(4H, m), 8.53(2H, m) | 795.02 | 794.37 |
| **908** | δ = 1.78(12H, s), 6.63∼6.64(6H, m), 6.81(4H, m), 7.2(4H, m), 7.36(2H, m), 7.49∼7.5(6H, m), 7.74∼7.77(4H, m), 7.84∼7.88(6H, m), 8.53(2H, m) | 795.02 | 794.37 |
| **911** | δ = 1.35(18H, s), 1.78(12H, s), 6.55(4H, m), 6.63∼6.64(6H, m), 6.81(4H, m), 7.01(4H, m), 7.2(4H, m), 7.5(2H, m), 7.84(2H, m), 8.53(2H, m) | 807.12 | 806.46 |
| **929** | δ = 1.78(12H, s), 7.41(1H, m), 7.5∼7.52(6H, m), 7.58∼7.59(3H, m), 7.69∼7.73(3H, m), 7.83(2H, m), 7.92(1H, m), 8(2H, m), 8.15(2H, m), 8.53(2H, m) | 562.74 | 562.27 |
| **939** | δ = 1.78(12H, s), 7.25(4H, m), 7.5∼7.61(8H, m), 7.69∼7.73(3H, m), 7.83(2H, m), 7.92(1H, m), 8∼8.08(4H, m), 8.15(2H, m), 8.42(1H, m), 8.53∼8.55(3H, m) | 688.90 | 688.31 |
| **957** | δ = 0.66(12H, s), 6.63(8H, m), 6.79∼6.81(8H, m), 7.2(8H, m), 7.55(2H, m), 7.64(2H, m), 8.55(2H, m) | 727.05 | 726.29 |
| **991** | δ = 0.66(12H, s), 6.63(8H, m), 6.69(4H, m), 6.81(4H, m), 7.2(8H, m), 7.35(2H, m), 7.54∼7.55(6H, m), 7.82(2H, m), 7.95(2H, m), 8.55(2H, m) | 879.24 | 878.35 |
| **1008** | δ = 3.82(6H, s), 7.25∼7.33(6H, m), 7.5(2H, m), 7.6∼7.71(8H, m), 7.94(2H, m), 8.05(2H, m), 8.12(2H, m), 8.54∼8.55(4H, m) | 664.79 | 664.26 |
| **1014** | δ = 3.82(6H, s), 6.02(2H, m), 6.37(2H, m), 6.63(8H, m), 6.81(4H, m), 7.2(8H, m), 7.67(2H, m), 7.8(2H, m), 8.54(2H, m) | 668.83 | 668.29 |
| **1016** | δ = 3.82(6H, s), 6.02(2H, m), 6.37(2H, m), 6.98(4H, m), 7.38(4H, m), 7.53∼7.57(12H, m), 7.67(2H, m), 7.8(2H, m), 8.02∼8.07(8H, m), 8.54(2H, m) | 869.06 | 868.36 |
| **1021** | δ = 3.82(6H, s), 6.02(2H, m), 6.37(2H, m), 6.63(4H, m), 6.81(2H, m), 6.98(2H, m), 7.2(4H, m), 7.38(2H, m), 7.53∼7.57(6H, m), 7.67(2H, m), 7.8(2H, m), 8.02∼8.07(4H, m), 8.54(2H, m) | 768.94 | 768.33 |
| **1024** | δ = 2.34(12H, s), 3.82(6H, s), 6.02(2H, m), 6.36∼6.37(6H, m), 6.63(4H, m), 6.71(2H, m), 6.81(2H, m), 7.2(4H, m), 7.67(2H, m), 7.8(2H, m), 8.54(2H, m) | 724.93 | 727.36 |
| **1029** | δ = 1.72(12H, s), 3.82(6H, s), 6.02(2H, m), 6.37(2H, m), 6.58∼6.63(6H, m), 6.75∼6.81(4H, m), 7.2(4H, m), 7.28(2H, m), 7.38(2H, m), 7.55(2H, m), 7.62∼7.67(4H, m), 7.8(2H, m), 7.87(2H, m), 8.54(2H, m) | 901.15 | 900.42 |
| **1065** | δ = 7.25∼7.33(6H, m), 7.49∼7.5(4H, m), 7.63∼7.67(4H, m), 7.94(2H, m), 8.02∼8.05(4H, m), 8.12(2H, m), 8.54∼8.55(4H, m) | 670.84 | 670.15 |
| **1075** | δ = 2.34(12H, s), 6.51(8H, m), 6.88(2H, m), 6.98(8H, m), 7.06(2H, m), 7.67(2H, m), 7.8(2H, m), 8.54(2H, m) | 730.98 | 730.25 |
| **1121** | δ = 7.64∼7.67(4H, m), 7.75(2H, m), 7.82∼7.95(12H, m), 8.12(4H, m), 8.54(2H, m), 8.93(4H, m) | 660.76 | 660.21 |
| **1128** | δ = 6.33(2H, m), 6.63(8H, m), 6.81(4H, m), 7.2(8H, m), 7.43(2H, m), 7.64∼7.67(4H, m), 8.54(2H, m) | 642.74 | 642.23 |
| **1160** | δ = 6.63(8H, m), 6.81(4H, m), 7.04(2H, m), 7.2(8H, m), 7.53∼7.54(4H, m), 7.64∼7.67(4H, m), 7.75∼7.78(4H, m), 7.95(2H, m), 8.07(2H, m), 8.49∼8.54(4H, m) | 895.05 | 894.32 |
| **1165** | δ = 7.25(4H, m), 7.55(4H, m), 7.61∼7.67(6H, m), 7.75(2H, m), 7.95(2H, m), 8.04∼8.08(4H, m), 8.42(2H, m), 8.54∼8.55(4H, m) | 636.73 | 636.21 |
| **1169** | δ = 1.51(12H, m), 2.09(4H, m), 6.63∼6.64(10H, m), 6.81(6H, m), 7.2(8H, m), 7.5(2H, m), 7.84(2H, m), 8.53(2H, m) | 746.98 | 746.37 |
| **1170** | δ = 5.85(2H, m), 6.6∼6.63(10H, m), 6.81(4H, m), 7.2(8H, m), 7.45∼7.5(6H, m), 7.58(4H, m), 7.67(2H, m), 8.18(2H, m), 8.54(2H, m) | 792.96 | 792.33 |
| **1175** | δ = 1.06(6H, m), 6.63(8H, m), 6.69(2H, m), 6.81(4H, m), 6.97(2H, m), 7.2(8H, m), 7.54∼7.55(4H, m), 8.55(2H, m) | 702.76 | 702.24 |
| **1176** | δ = 6.63(8H, m), 6.81(4H, m), 6.88∼6.9(4H, m), 7.2(8H, m), 7.48(2H, m), 7.55(2H, m), 8.55(2H, m) | 666.76 | 666.23 |

### [Example 1] Electroluminescent properties of OLED employing the organic electroluminescent compound of the invention (I)

An OLED device was manufactured by using an electroluminescent material according to the invention.

First, a transparent electrode ITO thin film (15 Ω/□) (2) prepared from glass for OLED (1) (manufactured by Samsung-Corning) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) (of which the structure is shown below) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injecting layer (3) having 60 nm thickness on the ITO substrate.

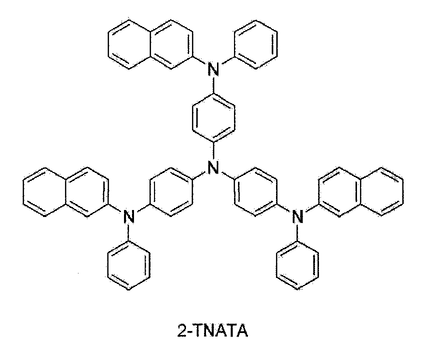

Then, to another cell of the vacuum vapor-deposit device, charged was N,N'-bis(α-naphthyl)-N,N'-diphenyl-4,4'-diamine (NPB) (of which the structure is shown below), and electric current was applied to the cell to evaporate NPB, thereby providing vapor-deposit of a hole transport layer (4) with 20 nm thickness on the hole injecting layer.

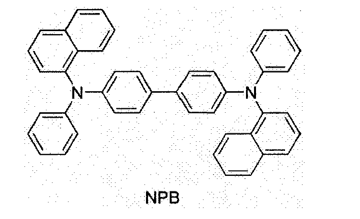

After forming the hole injecting layer and the hole transport layer, an electroluminescent layer was vapor-deposited as follows. To one cell of a vacuum vapor-deposit device, charged was a compound according to the invention (e.g., Compound 3) as electroluminescent material, and perylene (of which the structure is shown below) was charged to another cell. The two cells were simultaneously heated to carry out vapor-deposition at a vapor-deposition rate of perylene of 2 to 5% by weight, thereby providing vapor-deposit of an electroluminescent layer (5) with a thickness of 30 nm on the hole transport layer.

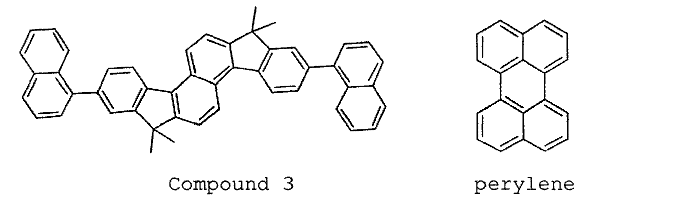

Then, tris(8-hydroxyquinoline)aluminum (III) (Alq) (of which the structure is shown below) was vapor-deposited as an electron transport layer (6) with a thickness of 20 nm, and lithium quinolate (Liq) (of which the structure shown below) was vapor-deposited as an electron injecting layer (7) with a thickness of 1 to 2 nm. Thereafter, an Al cathode (8) was vapor-deposited with a thickness of 150 nm by using another vacuum vapor-deposit device to manufacture an OLED.

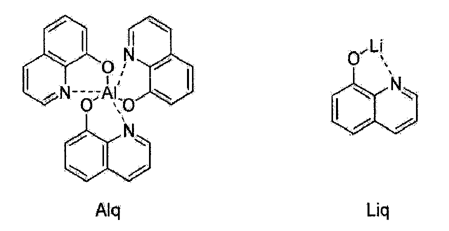

Each material employed for manufacturing an OLED was used as the electroluminescent material after purifying via vacuum sublimation at 10⁻⁶ torr.

### [Comparative Example 1] Electroluminescent properties of OLED employing conventional electroluminescent material

After forming a hole injecting layer (3) and a hole transport layer (4) according to the same procedure as described in Example 1, dinaphthylanthracene (DNA) was charged to one cell of said vacuum vapor-deposit device, while perylene was charged to another cell. An electroluminescent layer (5) was vapor-deposited by doping at 3% by weight on the basis of the host, with a thickness of 30 nm on the hole transport layer, with vapor-deposition rate of 100:1.

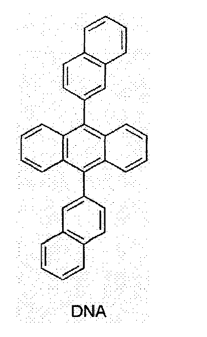

Then, an electron transport layer (6) and an electron injecting layer (7) were vapor-deposited according to the same procedures as in Example 1, and an Al cathode (8) was vapor-deposited with a thickness of 150 nm by using another vacuum vapor-deposit device to manufacture an OLED.

The luminous efficiencies of the OLED's comprising the organic electroluminescent compounds according to the present invention (Example 1) or conventional electroluminescent compound (Comparative Example 1) were measured at 5,000 cd/m², respectively, and the results are shown in Table 2.

**Table 2**

| No. | Host | Dopant | Doping concentration (wt%) | Efficiency (cd/A) | Color |
|---|---|---|---|---|---|
| | | | | @ 5,000 cd/m² | |
| 1 | Compound 3 | Perylene | 3 | 5.8 | Blue |
| 2 | Compound 11 | Perylene | 3 | 5.6 | Blue |
| 3 | Compound 39 | Perylene | 3 | 6.5 | Blue |
| 4 | Compound 91 | Perylene | 3 | 6.1 | Blue |
| 5 | Compound 736 | Perylene | 3 | 6.8 | Blue |
| 6 | Compound 846 | Perylene | 3 | 5.9 | Blue |
| 7 | Compound 980 | Perylene | 3 | 5.4 | Blue |
| 8 | Compound 1152 | Perylene | 3 | 5.8 | Blue |
| Comp.1 | DNA | Perylene | 3 | 4.5 | Blue |

As can be seen from Table 2, the device employing Compound 736 as host and perylene as dopant at a concentration of 3.0% by weight showed the highest luminous efficiency.

### [Example 2] Electroluminescent properties of OLED employing the organic electroluminescent compound of the invention (II)

After forming a hole injecting layer (3) and hole transport layer (4) according to the same procedure as described in Example 1, a compound according to the present invention (e.g., Compound 11) was charged to one cell of said vacuum vapor-deposit device as electroluminescent material, and Compound E (of which the structure is shown below) was charged to another cell. Then the two substances were evaporated at different rates to carry out doping at a concentration of 2 to 5% by weight on the basis of the host, thus providing an electroluminescent layer having 30 nm thickness vapor-deposited on the hole transport layer.

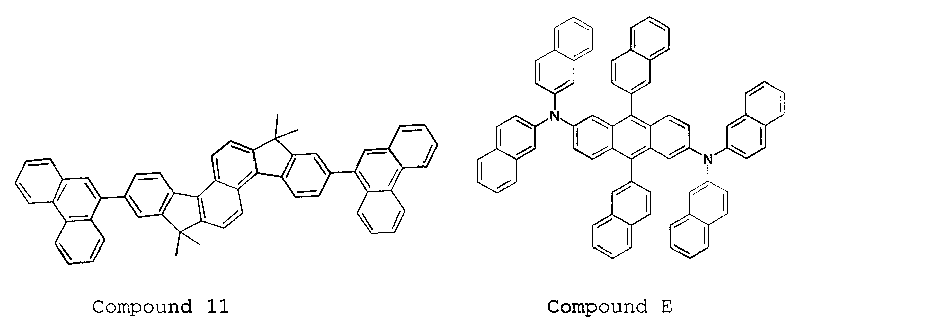

Then, an electron transport layer (6) and electron injecting layer (7) were vapor-deposited according to the same procedure as in Example 1, and an Al cathode (8) was vapor-deposited thereon with a thickness of 150 nm by using another vacuum vapor-deposit device to manufacture an OLED.

### [Comparative Example 2] Electroluminescent properties of OLED employing conventional electroluminescent material

After forming a hole injecting layer (3) and a hole transport layer (4) according to the same procedure as described in Example 1, tris(8-hydroxyquinoline)aluminum (III) (Alq) was charged to another cell of said vacuum vapor-deposit device, while Coumarin 545T (C545T) (of which the structure is shown below) was charged to still another cell. The two substances were evaporated at different rates to carry out doping, thereby providing an electroluminescent layer with a thickness of 30 nm on the hole transport layer. The doping concentration is preferably 1 to 3% by weight on the basis of Alq.

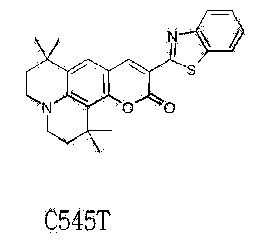

Then, an electron transport layer (6) and electron injecting layer (7) were vapor-deposited according to the same procedure as Example 1, and an Al cathode (8) was vapor-deposited thereon with a thickness of 150 nm by using another vacuum vapor-deposit device to manufacture an OLED.

The luminous efficiencies of the OLED's comprising the organic EL compound according to the present invention (Example 2) or conventional EL compound (Comparative Example 2) were measured at 5,000 cd/m², respectively, and the results are shown in Table 3.

**Table 3**

| No. | Host | Dopant | Doping concentration (wt%) | Efficiency (cd/A) @5000 cd/m² | Color |
|---|---|---|---|---|---|
| 1 | Compound 3 | Compound E | 3 | 18.5 | Green |
| 2 | Compound 11 | Compound E | 3 | 18.9 | Green |
| 3 | Compound 39 | Compound E | 3 | 20.3 | Green |
| 4 | Compound 91 | Compound E | 3 | 18.2 | Green |
| 5 | Compound 736 | Compound E | 3 | 19.5 | Green |
| 6 | Compound 846 | Compound E | 3 | 19.7 | Green |
| 7 | Compound 980 | Compound E | 3 | 18.5 | Green |
| Comp. 2 | Alq | C545T | 1 | 10.3 | Green |

As can be seen from Table 3, the device employing Compound (39) as host and Compound (E) as dopant at a doping concentration of 3.0% by weight showed the highest luminous efficiency.

### [Example 3] Electroluminescent properties of OLED employing the organic electroluminescent compound of the invention (III)

After forming a hole injecting layer (3) and hole transport layer (4) according to the same procedure described in Example 1, a compound according to the present invention (e.g., Compound H-5) was charged to one cell of said vacuum vapor-deposit device as host, and Compound (1125) according to the invention was charged to another cell as dopant. Then the two substances were evaporated at different rates to carry out doping at a concentration of 2 to 5% by weight on the basis of the host, thus providing an electroluminescent layer (5) having 30 nm thickness on the hole transport layer.

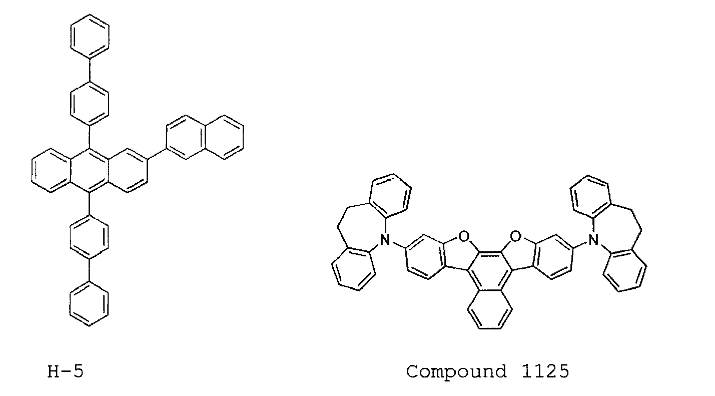

Then, an electron transport layer (6) and electron injecting layer (7) were vapor-deposited according to the same procedure as Example 1, and an Al cathode was vapor-deposited thereon with a thickness of 150 nm by using another vacuum vapor-deposit device to manufacture an OLED.

### [Comparative Example 3] Electroluminescent properties of OLED employing conventional electroluminescent material

After forming a hole injecting layer (3) and hole transport layer (4) according to the same procedure described in Example 1, Compound (H-5) was charged to another cell of said vacuum vapor-deposit device as electroluminescent host material, and Compound (G) was charged to still another cell. Then the two substances were evaporated at different rates to carry out doping at a concentration of 2 to 5% by weight on the basis of the host, and thus providing an electroluminescent layer having 30 nm thickness on the hole transport layer.

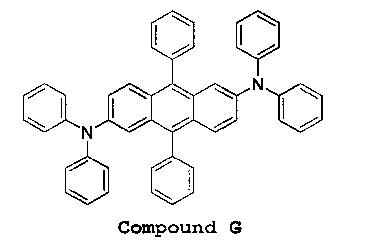

Then, an electron transport layer (6) and electron injecting layer (7) were vapor-deposited according to the same procedure as in Example 1, and an Al cathode (8) was vapor-deposited thereon with a thickness of 150 nm by using another vacuum vapor-deposit device to manufacture an OLED.

The luminous efficiencies of the OLED's comprising the organic electroluminescent compound according to the present invention (Example 3) or conventional electroluminescent compounds (Comparative Examples 2 and 3) were measured at 5,000 cd/m² and 20,000 cd/m², respectively, and the results are shown in Table 4.

**Table 4**

| No. | Host | Dopant | Doping concentration (wt%) | Efficiency (cd/A) | | Color |
|---|---|---|---|---|---|---|
| | | | | @5,000 cd/m² | @20,000 cd/m² | |
| 1 | H-5 | Compound **277** | 3 | 21.9 | 21.0 | Green |
| 2 | H-5 | Compound **300** | 3 | 18.1 | 17.3 | Green |
| 3 | H-5 | Compound **321** | 3 | 16.6 | 15.8 | Green |
| 4 | H-5 | Compound **355** | 3 | 19.7 | 19.1 | Green |
| 5 | H-5 | Compound **410** | 3 | 19.0 | 18.1 | Green |
| 6 | H-5 | Compound **673** | 3 | 19.0 | 18.5 | Green |
| 7 | H-5 | Compound **841** | 3 | 20.0 | 18.4 | Green |
| 8 | H-5 | Compound **1125** | 3 | 21.0 | 20.8 | Green |
| Comp.2 | Alq | C545T | 1 | 10.3 | 9.1 | Green |
| Comp.3 | H-5 | Compound G | 3.0 | 16.3 | 14.1 | Green |

As can be seen from Table 4, the device employing Compound (H-5) as host and Compound (277) as dopant at a doping concentration of 3.0% by weight showed the highest luminous efficiency.

### [Example 4] Electroluminescent properties of OLED employing the organic electroluminescent compound of the invention (IV)

After forming a hole injecting layer (3) and hole transport layer (4) according to the same procedure as described in Example 1, an anthracene-type host compound (Compound H-28) was charged to one cell of said vacuum vapor-deposit device as host, and Compound (19) according to the invention was charged to another cell as dopant. Then the two substances were evaporated at different rates to carry out doping at a concentration of 3% by weight on the basis of the host, thus providing an electroluminescent layer (5) having 30 nm thickness on the hole transport layer.

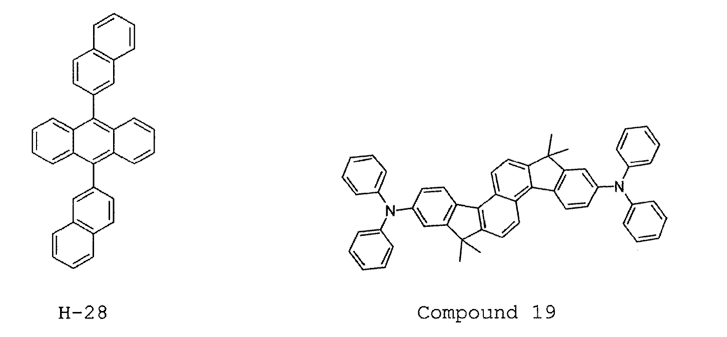

Then, an electron transport layer (6) and electron injecting layer (7) were vapor-deposited according to the same procedure as in Example 1, and an Al cathode was vapor-deposited thereon with a thickness of 150 nm by using another vacuum vapor-deposit device to manufacture an OLED.

The luminous efficiencies of the OLED's comprising the organic electroluminescent compound according to the present invention (Example 4) or conventional electroluminescent compound (Comparative Example 1) were measured at 5,000 cd/m², respectively, and the results are shown in Table 5.

**Table 5**

| No. | Host | Dopant | Doping concentration (wt%) | Efficiency (cd/A) | Color |
|---|---|---|---|---|---|
| | | | | @5,000 cd/m² | |
| 1 | H-28 | Compound **19** | 3 | 5.8 | Blue |
| 2 | H-36 | Compound **20** | 3 | 5.6 | Blue |
| 3 | H-38 | Compound **22** | 3 | 6.5 | Blue |
| 4 | H-50 | Compound **72** | 3 | 6.1 | Blue |
| 5 | H-66 | Compound **154** | 3 | 6.8 | Blue |
| 6 | H-77 | Compound **260** | 3 | 5.9 | Blue |
| 7 | H-79 | Compound **477** | 3 | 5.4 | Blue |
| 8 | H-82 | Compound **562** | 3 | 5.8 | Blue |
| Comp.1 | DNA | perylene | 3 | 4.5 | Blue |

As can be seen from Table 5, the device employing Compound (H-66) as host and Compound (154) as dopant at a doping concentration of 3.0% by weight showed the highest luminous efficiency.

### [Example 5] Electroluminescent properties of OLED employing the organic electroluminescent compound of the invention (V)

After forming a hole injecting layer (3) according to the same procedure as in Example 1, another cell of the vacuum vapor-deposit device was charged with Compound (19) (of which the structure is shown below), and electric current was applied to the cell to carry out evaporation, thereby providing vapor-deposit of a hole transport layer (4) with 20 nm thickness on the hole injecting layer.

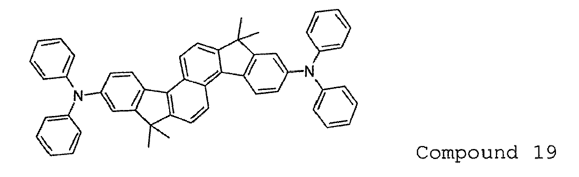

Then, an electroluminescent layer was vapor-deposited as follows. To one cell of a vacuum vapor-deposit device, charged was dinaphthylanthracene (DNA) as electroluminescent material, and perylene (of which the structure is shown below) was charged to another cell. The two cells were simultaneously heated to carry out vapor-deposition at a vapor-deposition rate of perylene of 2 to 5% by weight, thereby providing vapor-deposit of an electroluminescent layer (5) with a thickness of 30 nm on the hole transport layer.

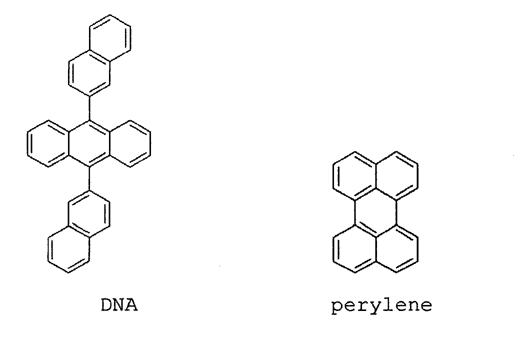

Then, an electron transport layer (6) and electron injecting layer (7) were vapor-deposited according to the same procedure as in Example 1, and an Al cathode was vapor-deposited thereon with a thickness of 150 nm by using another vacuum vapor-deposit device to manufacture an OLED.

### [Comparative Example 4] Electroluminescent properties of OLED employing conventional electroluminescent material

After forming a hole injecting layer (3) according to the same procedure as in Example 1, another cell of the vacuum vapor-deposit device was charged with N,N'-bis(α-naphthyl)-N,N'-diphenyl-4,4'-diamine (NPB) (of which the structure is shown below), and electric current was applied to the cell to evaporate NPB, thereby providing vapor-deposit of a hole transport layer (4) with 20 nm thickness on the hole injecting layer.

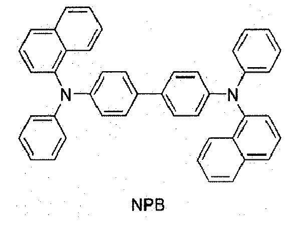

Then, an electroluminescent layer was vapor-deposited as follows. To one cell of a vacuum vapor-deposit device, charged was dinaphthylanthracene (DNA) as electroluminescent material, and perylene (of which the structure is shown below) was charged to another cell. The two cells were simultaneously heated to carry out vapor-deposition at a vapor-deposition rate of perylene of 2 to 5% by weight, thereby providing vapor-deposit of an electroluminescent layer (5) with a thickness of 30 nm on the hole transport layer.

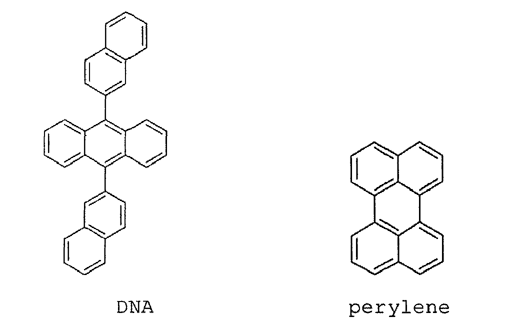

Then, an electron transport layer (6) and electron injecting layer (7) were vapor-deposited according to the same procedure as in Example 1, and an Al cathode was vapor-deposited thereon with a thickness of 150 nm by using another vacuum vapor-deposit device to manufacture an OLED.

The luminous efficiencies of the OLED's comprising the organic electroluminescent compound according to the present invention (Example 5) or conventional electroluminescent compound (Comparative Example 4) were measured at 5,000 cd/m², respectively, and the results are shown in Table 6.

**Table 6**

| No. | Material of hole transport layer | Operation voltage (V) @1,000 cd/m² | Luminous efficiency (cd/A) @1,000 cd/m² | Color |
|---|---|---|---|---|
| 1 | Compound **19** | 5 | 5.4 | Blue |
| 2 | Compound **48** | 4.8 | 5.6 | Blue |
| Comp.4 | NPB | 6 | 4.5 | Blue |

As is evident from the experimental results, the compounds developed by the present invention exhibited excellent performances as compared to those of conventional material.

## Claims

1. An organic electroluminescent compound represented by Chemical Formula (1): 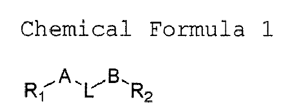 wherein, L is selected from the following structures: 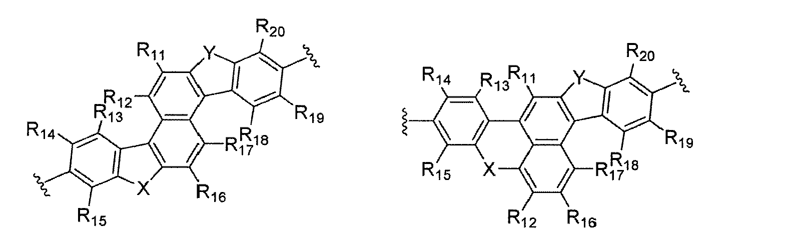 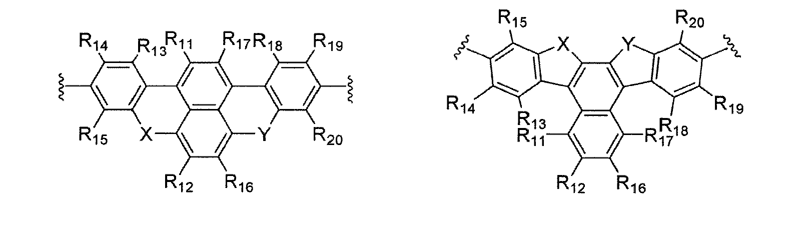
A and B independently represent a chemical bond, or (C6-C60)arylene, (C3-C60)heteroarylene, 5- or 6-membered heterocycloalkylene containing one or more heteroatom(s) selected from N, O and S, (C3-C60)cycloalkylene, (C2-C60)alkenylene, (C2-C60)alkynylene, (C1-C60)alkylenoxy, (C6-C60)arylenoxy or (C6-C60)arylenethio;
R₁ and R₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, NR₃R₄, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or a substituent selected from the following structures: 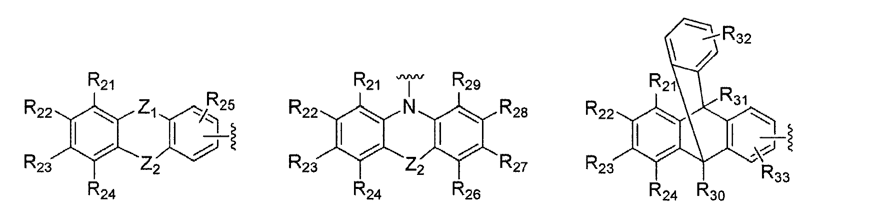
R₃ and R₄ independently represent (C1-C60)alkyl, (C6-C60)aryl, 5- or 6-membered heterocycloalkyl containing one or more heteroatom(s) selected from N, O and S, adamantyl, (C7-C60)bicycloalkyl, (C3-C60)cycloalkyl, (C3-C60)heteroaryl, tri(C1-C60)alkylsilyl, di(C1-C60)alkyl(C6-C60)arylsilyl or tri(C6-C60)arylsilyl, or R₃ and R₄ may be linked via (C3-C60)alkylene or (C3-C60)alkenylene with or without a fused ring to form an alicyclic ring, or a monocyclic or polycyclic aromatic ring;
X and Y independently represent a chemical bond, or - C(R₄₁)(R₄₂)-, -N(R₄₃)-, -S-, -O-, -Si(R₄₄)(R₄₅)-, -P(R₄₆)-,-C(=O)-, -B(R₄₇)-, -In(R₄₈)-, -Se-, -Ge(R₄₉)(R₅₀)-, -Sn(R₅₁)(R₅₂)-or -Ga(R₅₃)-;
R₄₁ through R₅₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₄₁ and R₄₂, R₄₄ and R₄₅, R₄₉ and R₅₀, or R₅₁ and R₅₂ may be linked via (C3-C60)alkylene or (C3-C60)alkenylene with or without a fused ring to form an alicyclic ring, or a monocyclic or polycyclic aromatic ring;
R₁₁ through R₂₀ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60) ar (C1-C60) alkyl, (C6-C60)aryloxy, (C6-C60)arylthio, (C6-C60) arylcarbonyl, (C1-C60) alkoxycarbonyl, (C1-C60)alkylcarbonyl, carboxyl, nitro or hydroxyl, or a substituent selected from the following structures: 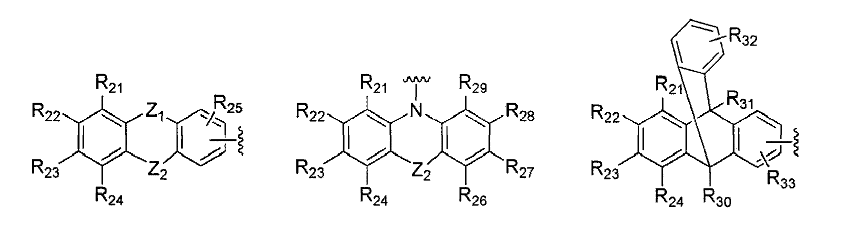
R₂₁ through R₃₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₂₁ through R₃₃ may be linked to an adjacent substituent via (C3-C60)alkylene or (C3-C60)alkenylene with or without a fused ring to form an alicyclic ring, or a monocyclic or polycyclic aromatic ring;
Z₁ and Z₂ independently represent a chemical bond, or - (CR₆₁R₆₂)ₘ-, -N(R₆₃)-, -S-, -O-, -Si(R₆₄)(R₆₅)-, -P(R₆₆)-, -C(=O)-, -B(R₆₇)-, -In(R₆₈)-, -Se-, -Ge(R₆₉)(R₇₀)-, -Sn(R₇₁)(R₇₂)-, - Ga(R₇₃)- or -(R₇₄)C=C(R₇₅)-;
wherein, R₆₁ through R₇₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60) bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₆₁ and R₆₂, R₆₄ and R₆₅, R₆₉ and R₇₀, R₇₁ and R₇₂, or R₇₄ and R₇₅ may be linked via (C3-C60)alkylene or (C3-C60)alkenylene with or without a fused ring to form an alicyclic ring, or a monocyclic or polycyclic aromatic ring;
the arylene, heteroarylene, heterocycloalkylene, cycloalkylene, alkenylene, alkynylene, alkylenoxy, arylenoxy or arylenethio of A and B; or the alkyl, aryl, heteroaryl, heterocycloalkyl, cycloalkyl, alkylsilyl, arylsilyl, adamantyl, bicycloalkyl, alkenyl, alkynyl, alkylamino or arylamino of R₁ through R₄, R₁₁ through R₃₃, R₄₁ through R₅₅ and R₆₁ through R₇₅ may be further substituted by one or more substituent(s) selected from halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl; and
m is an integer from 1 to 4.

2. An organic electroluminescent device which is comprised of a first electrode; a second electrode; and at least one organic layer(s) interposed between the first electrode and the second electrode; wherein the organic layer comprises an electroluminescent region containing one or more organic electroluminescent compound represented by Chemical Formula (1): 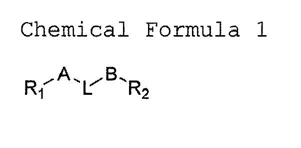
wherein, L is selected from the following structures: 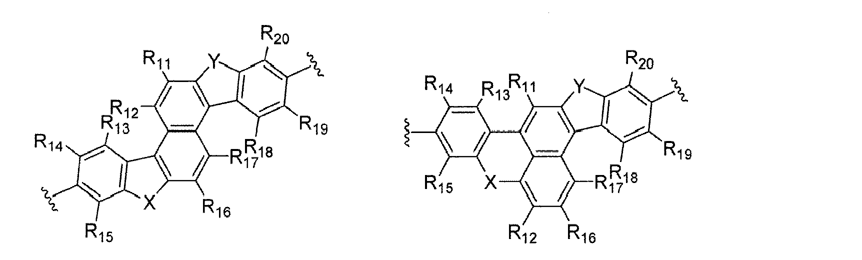 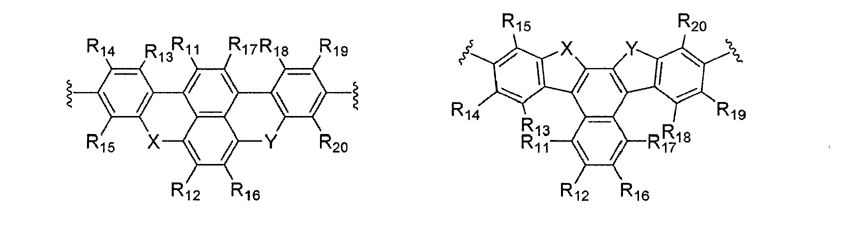
A and B independently represent a chemical bond, or (C6-C60)arylene, (C3-C60)heteroarylene, 5- or 6-membered heterocycloalkylene containing one or more heteroatom (s) selected from N, O and S, (C3-C60)cycloalkylene, (C2-C60)alkenylene, (C2-C60)alkynylene, (C1-C60)alkylenoxy, (C6-C60)arylenoxy or (C6-C60)arylenethio;
R₁ and R₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, NR₃R₄, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or a substituent selected from the following structures: 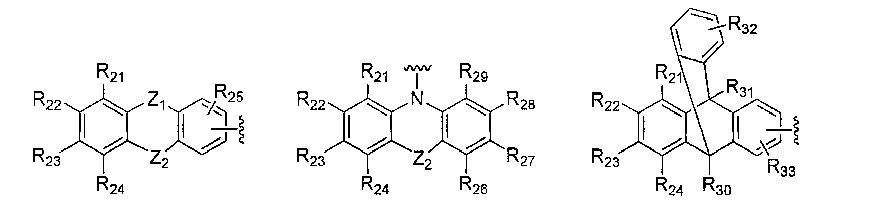
R₃ and R₄ independently represent (C1-C60)alkyl, (C6-C60)aryl, 5- or 6-membered heterocycloalkyl containing one or more heteroatom(s) selected from N, O and S, adamantyl, (C7-C60)bicycloalkyl, (C3-C60)cycloalkyl, (C3-C60)heteroaryl, tri(C1-C60)alkylsilyl, di(C1-C60)alkyl(C6-C60)arylsi or tri(C6-C60)arylsilyl, or R₃ and R₄ may be linked via (C3-C60)alkylene or (C3-C60)alkenylene with or without a fused ring to form an alicyclic ring, or a monocyclic or polycyclic aromatic ring;
X and Y independently represent a chemical bond, or - C(R₄₁)(R₄₂)-, -N(R₄₃)-, -S-, -O-, -Si(R₄₄)(R₄₅)-, -P(R₄₆)-, - C(=O)-, -B(R₄₇)-, -In(R₄₈)-, -Se-, -Ge(R₄₉)(R₅₀)-, -Sn(R₅₁)(R₅₂)- or -Ga(R₅₃)-;
R₄₁ through R₅₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₄₁ and R₄₂, R₄₄ and R₄₅, R₄₉ and R₅₀, or R₅₁ and R₅₂ may be linked via (C3-C60)alkylene or (C3-C60)alkenylene with or without a fused ring to form an alicyclic ring, or a monocyclic or polycyclic aromatic ring;
R₁₁ through R₂₀ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or a substituent selected from the following structures: 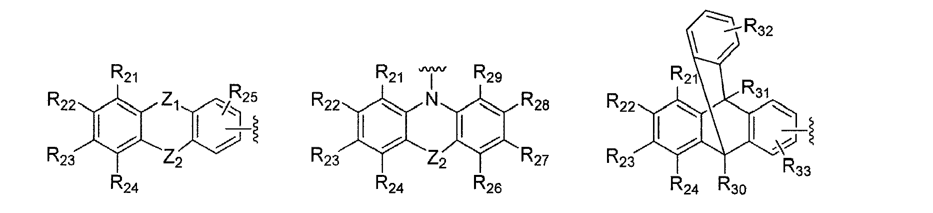
R₂₁ through R₃₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₂₁ through R₃₃ may be linked to an adjacent substituent via (C3-C60)alkylene or (C3-C60)alkenylene with or without a fused ring to form an alicyclic ring, or a monocyclic or polycyclic aromatic ring;
Z₁ and Z₂ independently represent a chemical bond, or - (CR₆₁R₆₂)ₘ-, -N(R₆₃)-, -S-, -O-, -Si(R₆₄)(R₆₅)-, -P(R₆₆)-, -C(=O)-, -B(R₆₇)-, -In(R₆₈) -Se-, -Ge(R₆₉)(R₇₀)-, -Sn(R₇₁)(R₇₂)-, Ga(R₇₃)- or -(R₇₄)C=C(R₇₅)-;
wherein, R₆₁ through R₇₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₆₁ and R₆₂, R₆₄ and R₆₅, R₆₉ and R₇₀, R₇₁ and R₇₂, or R₇₄ and R₇₅ may be linked via (C3-C60)alkylene or (C3-C60)alkenylene with or without a fused ring to form an alicyclic ring, or a monocyclic or polycyclic aromatic ring;
the arylene, heteroarylene, heterocycloalkylene, cycloalkylene, alkenylene, alkynylene, alkylenoxy, arylenoxy or arylenethio of A and B; or the alkyl, aryl, heteroaryl, heterocycloalkyl, cycloalkyl, alkylsilyl, arylsilyl, adamantyl, bicycloalkyl, alkenyl, alkynyl, alkylamino or arylamino of R₁ through R₄, R₁₁ through R₃₃, R₄₁ through R₅₅ and R₆₁ through R₇₅ may be further substituted by one or more substituent(s) selected from halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl; and
m is an integer from 1 to 4 and one or more dopant(s) selected from the compounds represented by one of Chemical Formulas (6) to (8): 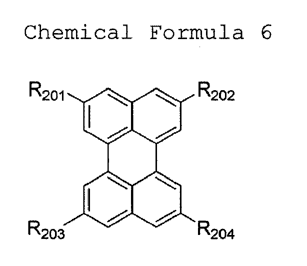
wherein, R₂₀₁ through R₂₀₄ independently represent hydrogen, halogen, (C1-C60)alkyl, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C1-C60)alkylthio, (C6-C60)arylthio, (C1-C60)alkoxycarbonyl, (C1-C60)alkylcarbonyl, (C6-C60)arylcarbonyl, carboxyl, nitro or hydroxyl, or R₂₀₁ through R₂₀₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkoxy, aryloxy, arylthio, alkylamino or arylamino of R₂₀₁ through R₂₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, (C1-C60)alkyl, (C6-C60) aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C1-C60)alkylthio, (C6-C60)arylthio, (C1-C60)alkoxycarbonyl, (C1-C60)alkylcarbonyl, (C6-C60)arylcarbonyl, carboxyl, nitro and hydroxyl; 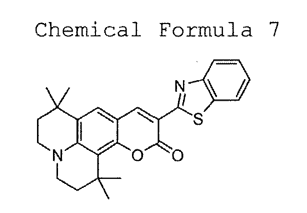 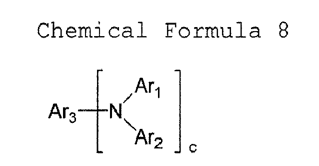
wherein, Ar₁ and Ar₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₁ and Ar₂ may be linked via (C3-C60)alkylene or (C3-C60)alkenylene with or without a fused ring to form an alicyclic ring, or a monocyclic or polycyclic aromatic ring;
the aryl, heteroaryl, arylamino or heterocycloalkyl of Ar₁ and Ar₂ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl;
Ar₃ represents (C6-C60)arylene, (C4-C60)heteroarylene or arylene represented by one of the following structures: 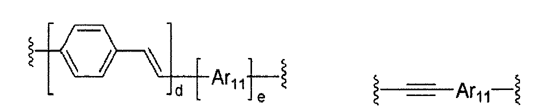
Ar₁₁ represents (C6-C60)arylene or (C4-C60)heteroarylene;
the arylene or heteroarylene of Ar₃ and Ar₁₁ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl;
c is an integer from 1 to 4,
d is an integer from 1 to 4, and e is an integer of 0 or 1.

3. The organic electroluminescent device according to claim 2, which is comprised of a first electrode; a second electrode; and at least one organic layer(s) interposed between the first electrode and the second electrode; wherein the organic layer comprises an electroluminescent region containing one or more organic electroluminescent compound(s) according to claim 1, and one or more host(s) selected from the compounds represented Chemical Formula (9) or (10):
Chemical Formula 9 (Ar₂₁)_{f}-L₂₁-(Ar₂₂)_{g}
Chemical Formula 10 (Ar₂₃)ₕ-L₂₂-(Ar₂₄)ⱼ
wherein, L₂₁ represents (C6-C60)arylene or (C4-C60)heteroarylene;
L₂₂ represents anthracenylene;
Ar₂₁ through Ar₂₄ are independently selected from hydrogen, (C1-C60)alkyl, (C1-C60)alkoxy, halogen, (C4-C60)heteroaryl, (C5-C60)cycloalkyl and (C6-C60)aryl; the cycloalkyl, aryl or heteroaryl of Ar₂₁ through Ar₂₄ may be further substituted by one or more substituent(s) selected from a group consisting of (C6-C60)aryl or (C4-C60)heteroaryl with or without one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
f, g, h and i independently represent an integer from 0 to 4.

4. The organic electroluminescent device according to claim 2, wherein the organic layer comprises one or more compound(s) selected from a group consisting of arylamine compounds and styrylarylamine compounds.

5. The organic electroluminescent device according to claim 2, wherein the organic layer comprises one or more metal(s) selected from a group consisting of organic metals of Group 1, Group 2, 4^{th} period and 5^{th} period transition metals, lanthanide metals and d-transition elements in the Periodic Table of Elements.

6. The organic electroluminescent device according to claim 2, which is an organic display further comprising a compound having electroluminescent peak of wavelength of not less than 560 nm in the electroluminescent layer.

7. The organic electroluminescent device according to claim 2, wherein the organic layer comprises an electroluminescent layer and a charge generating layer.

8. The organic electroluminescent device according to claim 2, wherein a mixed region of reductive dopant and organic substance, or a mixed region of oxidative dopant and organic substance is placed on the inner surface of one or both electrode(s) among the pair of electrodes.

9. An organic solar cell which comprises an organic electroluminescent compound represented by Chemical Formula (1) : 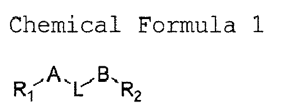
wherein, L is selected from the following structures: 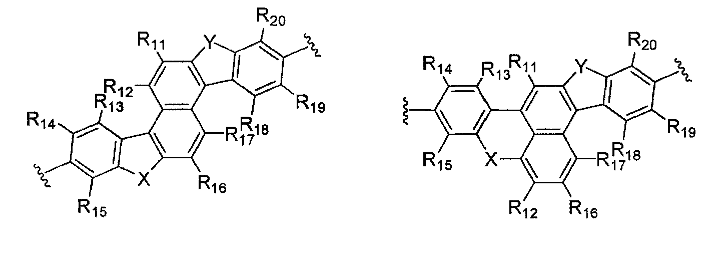 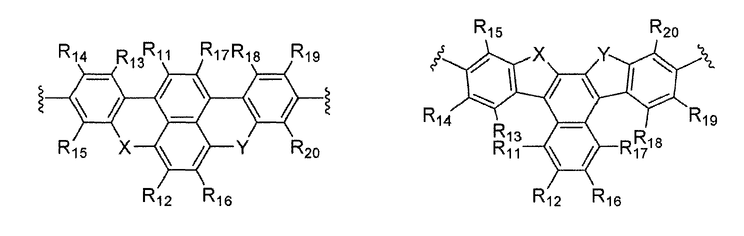
A and B independently represent a chemical bond, or (C6-C60)arylene, (C3-C60)heteroarylene, 5- or 6-membered heterocycloalkylene containing one or more heteroatom(s) selected from N, O and S, (C3-C60)cycloalkylene, (C2-C60)alkenylene, (C2-C60)alkynylene, (C1-C60)alkyleno (C6-C60)arylenoxy or (C6-C60)arylenethio;
R₁ and R₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, NR₃R₄, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or a substituent selected from the following structures: 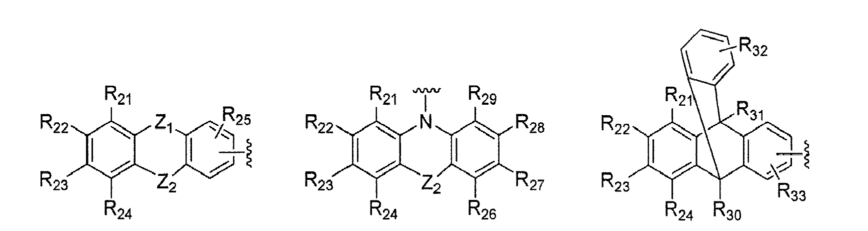
R₃ and R₄ independently represent (C1-C60)alkyl, (C6-C60)aryl, 5- or 6-membered heterocycloalkyl containing one or more heteroatom(s) selected from N, O and S, adamantyl, (C7-C60)bicycloalkyl, (C3-C60)cycloalkyl, (C3-C60)heteroaryl, tri(C1-C60)alkylsilyl, di(C1-C60)alkyl(C6-C60)arylsilyl or tri(C6-C60)arylsilyl, or R₃ and R₄ may be linked via (C3-C60)alkylene or (C3-C60)alkenylene with or without a fused ring to form an alicyclic ring, or a monocyclic or polycyclic aromatic ring;
X and Y independently represent a chemical bond, or - C(R₄₁)(R₄₂)-, -N(R₄₃)-, -S-, -O-, -Si(R₄₄)(R₄₅)-, -P(R₄₆)-, C(=O)-, -B(R₄₇)-, -In(R₄₈)-, -Se-, -Ge(R₄₉)(R₅₀)-, -Sn(R₅₁)(R₅₂)-or -Ga(R₅₃)-;
R₄₁ through R₅₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbanyl, (C1-C60)alkoxycarbonyl, (C1-C60)alkylcarbonyl, carboxyl, nitro or hydroxyl, or R₄₁ and R₄₂, R₄₄ and R₄₅, R₄₉ and R₅₀, or R₅₁ and R₅₂ may be linked via (C3-C60)alkylene or (C3-C60)alkenylene with or without a fused ring to form an alicyclic ring, or a monocyclic or polycyclic aromatic ring;
R₁₁ through R₂₀ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or a substituent selected from the following structures: 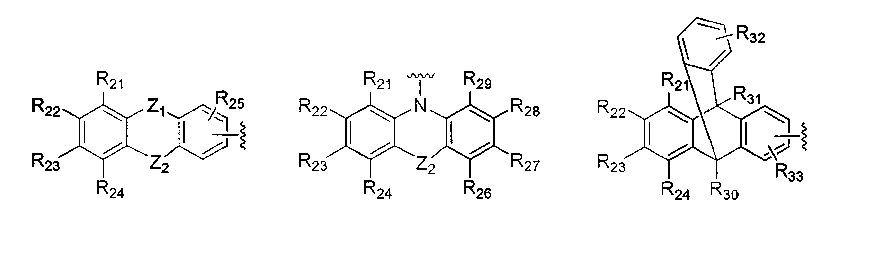
R₂₁ through R₃₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₂₁ through R₃₃ may be linked to an adjacent substituent via (C3-C60)alkylene or (C3-C60)alkenylene with or without a fused ring to form an alicyclic ring, or a monocyclic or polycyclic aromatic ring;
Z₁ and Z₂ independently represent a chemical bond, or - (CR₆₁R₆₂)ₘ-, -N(R₆₃)-, -S-, -O-, -Si(R₆₄)(R₆₅)-, -P(R₆₆)-, -C(=O)-, -B(R₆₇), -In(R₆₈)-, -Se-, -Ge(R₆₉)(R₇₀)-, -Sn(R₇₁)(R₇₂)-, Ga(R7₃₎- or-(R₇₄)C=C(R₇₅)-;
wherein, R₆₁ through R₇₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro or hydroxyl, or R₆₁ and R₆₂, R₆₄ and R₆₅, R₆₉ and R₇₀, R₇₁ and R₇₂, or R₇₄ and R₇₅ may be linked via (C3-C60)alkylene or (C3-C60)alkenylene with or without a fused ring to form an alicyclic ring, or a monocyclic or polycyclic aromatic ring;
the arylene, heteroarylene, heterocycloalkylene, cycloalkylene, alkenylene, alkynylene, alkylenoxy, arylenoxy or arylenethio of A and B; or the alkyl, aryl, heteroaryl, heterocycloalkyl, cycloalkyl, alkylsilyl, arylsilyl, adamantyl, bicycloalkyl, alkenyl, alkynyl, alkylamino or arylamino of R₁ through R₄, R₁₁ through R₃₃, R₄₁ through R₅₅ and R₆₁ through R₇₅ may be further substituted by one or more substituent(s) selected from halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C6-C60)arylcarbonyl, (C1-C60)alkoxycarbonyl, (C1-C60)alkylcarbonyl, carboxyl, nitro and hydroxyl; and
m is an integer from 1 to 4.
